(19) Europäisches Patentamt
European Patent Office
Office européen des brevets

(11) EP 2 690 095 A1

(12) **EUROPEAN PATENT APPLICATION**

(43) Date of publication:
**29.01.2014 Bulletin 2014/05**

(21) Application number: **13190133.2**

(22) Date of filing: **20.10.2009**

(51) Int Cl.:
*C07D 231/12* (2006.01) *C07D 333/16* (2006.01)
*C07D 233/64* (2006.01) *C07D 409/04* (2006.01)
*C07D 249/06* (2006.01) *C07D 261/08* (2006.01)
*C07D 263/32* (2006.01) *C07D 277/24* (2006.01)
*C07D 285/06* (2006.01) *C07D 307/42* (2006.01)
*A61K 31/4155* (2006.01) *A61K 31/42* (2006.01)
*A61K 31/4164* (2006.01)

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **21.10.2008 US 107314 P**

(62) Document number(s) of the earlier application(s) in accordance with Art. 76 EPC:
**09744008.5 / 2 350 016**

(71) Applicant: **Camabay Therapeutics, Inc.**
**Hayward, CA 94545 (US)**

(72) Inventors:
- **Ma, Jingyuan**
  **Hayward, CA 94545 (US)**
- **Novack, Aaron**
  **Hayward, CA 94545 (US)**
- **Nashashibi, Imad**
  **Hayward, CA 94545 (US)**
- **Pham, Phuongly**
  **Hayward, CA 94545 (US)**
- **Rabbat, Christopher J.**
  **Hayward, CA 94545 (US)**
- **Song, Jiangao**
  **Hayward, CA 94545 (US)**
- **Shi, Dong Fang**
  **Hayward, CA 94545 (US)**
- **Zhao, Zuchun**
  **Hayward, CA 94545 (US)**
- **Choi, Yun-Jung**
  **Hayward, CA 94545 (US)**
- **Chen, Xin**
  **Hayward, CA 94545 (US)**

(74) Representative: **Gibson, Mark**
**Sagittarius IP**
**Three Globeside**
**Fieldhouse Lane**
**Marlow, Buckinghamshire SL7 1HZ (GB)**

Remarks:
This application was filed on 24-10-2013 as a divisional application to the application mentioned under INID code 62.

(54) **Aryl gpr120 receptor agonists and uses thereof**

(57) Aryl GPR120 agonists are provided. These compounds are useful for the treatment of metabolic diseases, including Type II diabetes and diseases associated with poor glycemic control.

EP 2 690 095 A1

## Description

## BACKGROUND OF THE INVENTION

Diabetes mellitus can be divided into two clinical syndromes, Type I and Type II diabetes mellitus. Type I diabetes, or insulin-dependent diabetes mellitus, is a chronic autoimmune disease characterized by the extensive loss of beta cells in the pancreatic islets ofLangerhans (hereinafter referred to as "pancreatic islet cells" or "islet cells"), which produce insulin. As these cells are progressively destroyed, the amount of secreted insulin decreases, eventually leading to hyperglycemia (abnormally high level of glucose in the blood) when the amount secreted drops below the level required for euglycemia (normal blood glucose level). Although the exact trigger for this immune response is not known, patients with Type I diabetes have high levels of antibodies against pancreatic beta cells (hereinafter "beta cells"). However, not all patients with high levels of these antibodies develop Type I diabetes.

Type II diabetes, or non-insulin-dependent diabetes mellitus, develops when muscle, fat and liver cells fail to respond normally to insulin. This failure to respond (called insulin resistance) may be due to reduced numbers of insulin receptors on these cells, or a dysfunction of signaling pathways within the cells, or both. The beta cells initially compensate for this insulin resistance by increasing their insulin output. Over time, these cells become unable to produce enough insulin to maintain normal glucose levels, indicating progression to Type II diabetes (Kahn SE, Am J Med (2000) 108 Suppl 6a, 2S-8S).

The fasting hyperglycemia that characterizes Type II diabetes occurs as a consequence of the combined lesions of insulin resistance and beta cell dysfunction. The beta cell defect has two components: the first component, an elevation of basal insulin release (occurring in the presence of low, non-stimulatory glucose concentrations), is observed in obese, insulin-resistant pre-diabetic stages as well as in Type II diabetes. The second component is a failure to increase insulin release above the already elevated basal output in response to a hyperglycemic challenge. This lesion is absent in prediabetes and appears to define the transition from normo-glycemic insulin-resistant states to frank diabetes. There is currently no cure for diabetes. Conventional treatments for diabetes are very limited, and focus on attempting to control blood glucose levels in order to minimize or delay complications. Current treatments target either insulin resistance (metformin, thiazolidinediones ("TZDs")), or insulin release from the beta cell (sulphonylureas, exanatide). Sulphonylureas, and other compounds that act by depolarizing the beta cell, have the side effect of hypoglycemia since they cause insulin secretion independent of circulating glucose levels. One approve drug, Byetta (exanatide) stimulates insulin secretion only in the presence of high glucose, but is not orally available and must be injected. Januvia (sitagliptin) is another recently approved drug that increases blood levels of incretin hormones, which can increase insulin secretion, reduce glucagon secretion and have other less well characterized effects. However, Januvia and other dipeptidyl peptidases IV inhibitors may also influence the tissue levels of other hormones and peptides, and the long-term consequences of this broader effect have not been fully investigated. There is an unmet need for oral drugs that stimulate insulin secretion in a glucose dependent manner.

Progressive insulin resistance and loss of insulin secreting pancreatic beta cells are primary characteristics of Type II diabetes. Normally, a decline in the insulin sensitivity of muscle and fat is compensated for by increases in insulin secretion from the beta cell. However, loss of beta cell function and mass results in insulin insufficiency and diabetes (Kahn BB, Cell 92:593-596, 1998; Cavaghan MK, et al., J Clin Invest 106:329-333, 2000; Saltiel AR, Cell 104:517-529, 2001; Prentki M and Nolan CJ, J Clin Invest 116:1802-1812 (2006); and Kahn SE, J Clin Endicrinol Metab 86:4047-4058, 2001). Hyperglycemia further accelerates the decline in beta cell function (UKPDS Group, JAMA 281:2005-2012, 1999; Levy J, et al., Diabetes Med 15:290-296, 1998; and Zhou YP, et al., J Biol Chem 278:51316-23, 2003). Several of the genes in which allelic variation is associated with an increased risk of Type II diabetes are expressed selectively in the beta cell (Bell GI and Polonsky KS, Nature 414:788-791 (2001); Saxena R, et al., Science (2007) Apr 26; and Valgerdur Steinthorsdottir, et al., Nature Genetics (2007) Apr 26).

Insulin secretion from the beta cells of pancreatic islets is elicited by increased levels of blood glucose. Glucose is taken up into the beta cell primarily by the beta cell and liver selective transporter GLUT2 (Thorens B, Mol Membr Biol 2001 Oct-Dec;18(4):265-73). Once inside the cell, glucose is phosphorylated by glucokinase, which is the primary glucose sensor in the beta cell since it catalyzes the irreversible rate limiting step for glucose metabolism (Matschinsky FM, Curr Diab Rep 2005 Jun;5(3):171-6). The rate of glucose-6-phosphate production by glucokinase is dependent on the concentration of glucose around the beta cell, and therefore this enzyme allows for a direct relationship between level of glucose in the blood and the overall rate of glucose oxidation by the cell. Mutations in glucokinase produce abnormalities in glucose dependent insulin secretion in humans giving further evidence that this hexokinase family member plays a key role in the islet response to glucose (Gloyn AL, et al., J Biol Chem 2005 Apr 8;280(14):14105-13, Epub 2005 Jan 25). Small molecule activators of glucokinase enhance insulin secretion and may provide a route for therapeutic exploitation of the role of this enzyme (Guertin KR and Grimsby J, Curr Med Chem 2006;13(15):1839-43; and Matschinsky FM, et al., Diabetes 2006 Jan;55(1):1-12) in diabetes. Glucose metabolism via glycolysis and mitochondrial oxidative phosphorylation ultimately results in ATP production, and the amount of ATP produced in a beta cell

is directly related to the concentration of glucose to which the beta cell is exposed.

Glucose dependent insulin secretion from the beta cell is dependent on numerous neurotransmitters and blood-borne hormones, as well as local, intra-islet factors. CNS activation of the vagal innervation of the islet can lead to the release of small molecules such as acetylcholine and peptides such as vasoactive intestinal polypeptide (VIP), gastrin releasing peptide (GRP) and Pituitary Adenylate Cyclase Activating Peptide (PACAP). Acetylcholine activation of phospholipase C through the $G_{\alpha q}$-coupled GPCR M3 muscarinic receptor leads to release of $Ca^{2+}$ from intracellular stores (Gilon P and Henquin JC, Endocr Rev 2001 Oct;22(5):565-604). Cholinergic agonists also lead to a subtle $Na^{+}$-dependent plasma membrane depolarization that can work in concert with glucose-initiated depolarization to enhance insulin release (Gilon P and Henquin JC, Endocr Rev 2001 Oct;22(5):565-604). VIP and PACAP each bind to an overlapping set of $G_{\alpha}$-coupled GPCRs (PAC1, VIPER1, and VIPR2) on the beta cell that lead to stimulation of adenylate cyclase and an increase in intracellular cAMP (Filipsson K, et al., Diabetes 2001 Sep;50(9):1959-69; Yamada H, et al., Regul Pept 2004 Dec 15;123(1-3):147-53; and Qader SS, et al., Am J Physiol Endocrinol Metab 2007 May;292(5):E1447-55).

Incretin hormones such as Glucagon-Like Peptide 1 (GLP-1) and Glucose-dependent Insulinotropic Polypeptide (GIP, also known as Gastric Inhibitory Polypeptide) also bind to specific *Galpha*$_s$-coupled GPCRs receptors on the surface of islet cells, including beta cells, and raise intracellular cAMP (Drucker DJ, J Clin Invest 2007 Jan;117(1):24-32). Although the receptors for these hormones are present in other cells and tissues, the overall sum of effects of these peptides appear to be beneficial to control of glucose metabolism in the organism (Hansotia T, et al., J Clin Invest 2007 Jan;117(1):143-52, Epub 2006 Dec 21). GIP and GLP-1 are produced and secreted from intestinal K and L cells, respectively, and these peptide hormones are released in response to meals by both direct action of nutrients in the gut lumen and neural stimulation resulting from food ingestion. GIP and GLP-1 have short half-lives in human circulation due to the action of the protease dipeptidyl-peptidase IV (DPPIV), and inhibitors of this protease can lower blood glucose due to their ability to raise the levels of active forms of the incretin peptides. The glucose lowering that can be obtained with DPPIV inhibitors, however, is somewhat limited since these drugs are dependent on the endogenous release of the incretin hormones. Peptides (*e.g.,* exanatide (Byetta)) and peptide-conjugates that bind to the GIP or GLP-1 receptors but are resistant to serum protease cleavage can also lower blood glucose substantially (Gonzalez C, et al., Expert Opin Investig Drugs 2006 Aug;15(8):887-95), but these incretin mimetics must be injected and tend to induce a high rate of nausea and therefore are not ideal therapies for general use in the Type II diabetic population. The clinical success of DPPIV inhibitors and incretin mimetics, though far from ideal, do point to the potential utility of compounds that increase incretin activity in the blood. Some studies have indicated that beta cell responsiveness to GIP is diminished in Type II diabetes (Nauck MA, et al., J Clin Invest 91:301-307 (1993); and Elahi D, et al., Regul Pept 51:63-74 (1994)). Restoration of this responsiveness (Meneilly GS, et al., Diabetes Care 1993 Jan;16(1):110-4) may be a promising way to improve beta cell function *in vivo.*

Since increased incretin activity has a positive effect on glucose dependent insulin secretion and perhaps other mechanisms that lead to lower blood glucose, it is also of interest to explore therapeutic approaches to increasing incretin release from intestinal K and L cells. GLP-1 secretion appears to be attenuated in Type II diabetes (Vilsboll T, et al., Diabetes 50:609-613), so improving incretin release may ameliorate this component of metabolic dysregulation. Nutrients such as glucose and fat in the gut lumen prompt incretin secretion by interaction with apical receptors (Vilsboll T, et al., Diabetes 50:609-613). GLP-1 and GIP release can also result from neural stimulation; acetylcholine and GRP can enhance incretin release in a manner perhaps analogous to the effects of these neurotransmitters on the beta cell in regard to insulin secretion (Brubaker P, Ann N YAcad Sci 2006 Jul;1070:10-26; and Reimann F, et al., Diabetes 2006 Dec;55(Suppl 2):S78-S85). Somatostatin, leptin and free fatty acids also appear to modulate incretin secretion (Brubaker P, Ann N Y Acad Sci 2006 Jul;1070:10-26; and Reimann F, et al., Diabetes 2006 Dec;55(Suppl 2):S78-S85). To date, however, there does not appear to be a way to selectively impact these pathways to promote incretin secretion for therapeutic benefit. There is a need for oral drugs that stimulate incretin secretion in the treatment of diabetes.

Incretins can also increase the rate of beta cell proliferation and decrease the apoptotic rates of beta cells in animal models (Farilla L, et al., Endocrinology 2002 Nov;143(11):4397-408) and human islets *in vitro* (Farilla L, et al., Endocrinology 2003 Dec;144(12):5149-58). The net result of these changes is an increase in beta cell number and islet mass, and this should provide for increased insulin secretory capacity, which is another desired aim of anti-diabetic therapies. GLP-1 has also been shown to protect islets from the destructive effects of agents such as streptozotocin by blocking apoptosis (Li Y, et al., J Biol Chem 2003 Jan 3;278(1):471-8). Cyclin D1, a key regulator of progression through the cell cycle, is up-regulated by GLP-1, and other agents that increase cAMP and PKA activity also have a similar effect (Friedrichsen BN, et al., J Endocrinol 2006 Mar;188(3):481-92; and Kim MJ, et al., J Endocrinol 2006 Mar;188(3):623-33). Increased transcription of the cyclin D 1 gene occurs in response to PKA phosphorylation of CREB (cAMP-response element binding) transcription factors (Hussain MA, et al., Mol Cell Biol 2006 Oct;26(20):7747-59). There is a need for oral drugs that increase beta cell number and islet mass in the treatment of diabetes.

G protein-coupled receptors (GPCRs) are cell-surface receptors that play an important physiological role by transducing and amplifying extra-cellular signals such as hormones, growth factors, neurotransmitters and physiologically active substances. GPCRs are associated with changes in intracellular $Ca^{2+}$ concentration as well as increases in

intracellular inositol 1, 4, 5-triphosphate (IP3) concentration. These second messengers serve to focus the signal transduction events and stimulate other pathways. Hence, GPCRs are therapeutically important target classes in the pharmaceutical industry.

GPR120 is a GPCR for unsaturated long-chain free fatty acids (FFA) and is highly expressed in lung, intestine, adipocytes and taste cells as well as in the enteroendocrine cell lines such as STC-1 and GLUTag (Hirasawa et al., Nature Medicine 2005 Jan; 11: 90-94; and Iakoubov et al., Endocrinology 2007 Mar; 148(3): 1089-1098; and Katsuma et al., J. Biol. Chem. 2005 May; 280:19507-19515; Matsumura et al., Biomed. Res. 2007 Feb; 28(1) 49-55). The stimulation of GPR120 by FFAs increases the release of $Ca^{2+}$ from intracellular stores indicating that GPR120 is a Gαq-coupled receptor. GPR120 mediates the effect of unsaturated long-chain free fatty acids in stimulating GLP-1 and cholecystokinin (CCK) secretion, increases plasma insulin, activation of the extracellular signal-regulated kinase (ERK) cascade, proliferation of pancreatic β cells, inhibition of serum deprivation-induced apoptosis and adipogenesis (Katsuma et al., J. Biol. Chem. 2005 May; 280:19507-19515; and Rayasam et al., Expert Opin. Ther. Targets 2007 May; 11(5): 661-671; and Tanaka et al., Naunyn Schmiedeberg Arch Pharmacol 2008 Jun; 377(4-6):515-22; and Gotoh et al., Biochem. Biophys. Res. Commun. 2007 Mar; 354(2): 591-597).

Free fatty acids have been demonstrated as ligands for recently identified orphan GPCRs (Rayasam et al., Expert Opin Ther Targets 2007 May;11(5):661-671). GPR120 shares ligand specificity with other fatty acid receptors and there is a need for the development of small molecule agents that are specific modulators for GPR120 function. In particular, GPR120 is a promising target for the treatment of diabetes, obesity and the metabolic syndrome considering the significant role of GLP-1 and CCK in insulin secretion, gastric emptying and appetite feeding control.

## BRIEF SUMMARY OF THE INVENTION

Novel GPR120 compound agonists, methods for their preparation, and related synthetic intermediates and compositions are provided. The novel GPR120 agonists are useful in the treatment of diabetes and other related diseases including metabolic syndrome, dyslipidemia, insulin resistance, and complications of diabetes. The agonists include compounds of Formula (A)-(D) and (I)-(XIV) and pharmaceutically acceptable salts thereof.

Further provided are methods for treating diseases such as Type II diabetes and other diseases and conditions using one or more of these compounds or compositions, as described in further detail below. The invention also provides methods of raising intracellular levels of $Ca^{2+}$ by using one or more of the compounds described herein. Further, the compounds may be used to stimulate insulin production and stimulate secretion of insulin, glucagon-like peptide 1 (LP1), and glucose dependent insulinotropic polypeptide (GIP) in a mammal, in particular a human. Additionally, the compounds described herein are useful in lowering blood glucose when administered to a mammal in need of treatment to lower blood glucose.

## DETAILED DESCRIPTION OF THE INVENTION

The abbreviations used herein are conventional, unless otherwise defined: AcOH: acetic acid; nBuLi: *n*-butyllithium; $Cs_2CO_3$: cesium carbonate; $CH_2Cl_2$ or DCM: dichloromethane; $CH_3MgI$: methyl magnesium iodide; $CuCl_2$: copper chloride; DAST: (diethylamino)sulfur trifluoride; DEAD: diethyl azodicarboxylate; DIBAL: diisobutylaluminum hydride; DIPEA: diisopropylethylamine; DMF: dimethylformamide; DMSO: dimethyl sulfoxide; $Et_3N$: triethylamine; EtOAc: ethyl acetate; EtOH: ethanol; g: gram(s); h: hour; $H_2$: hydrogen; HBr: hydrogen bromide; HCl: hydrogen chloride; $H_2O$: water; $H_2O_2$: hydrogen peroxide; HPLC: high performance liquid chromatography; KCN: potassium cyanide; LHMDS: lithium hexamethyldisilazide; $LiAlH_4$: lithium aluminum hydride; LiOH: lithium hydroxide; M: molar; MeCN: acetonitrile; MeI: methyl iodide; MeOH: methanol; $MgSO_4$: magnesium sulfate; $MgCO_3$: magnesium carbonate; mg: millilgram; MsCl: mesyl chloride; mmol: millimoles mL: milliliter; sodium hydrogen sulfite; mCPBA: meta-chloroperoxybenzoic acid; N: normality; $N_2$: nitrogen; $Na_2CO_3$: sodium carbonate; $NaHCO_3$: sodium bicarbonate; $NaNO_2$: sodium nitrite; NaOH: sodium hydroxide; $Na_2S_2O_3$: sodium bisulfate; $Na_2SO_4$: sodium sulfate; NBS: N-bromosuccinimide; $NH_4Cl$: ammonium chloride; $NH_4OAc$: ammonium acetate; NMR: nuclear magnetic resonance; Pd/C: palladium on carbon; $PPh_3$: triphenyl phosphine; iPrOH: isopropyl alcohol; $SOCl_2$: thionyl chloride; THF: tetrahydrofuran; TLC: thin layer chromatography;
μL: microliter.

Unless otherwise stated, the following terms used in the specification and claims have the meanings given below.

"Alkyl" refers to monovalent saturated aliphatic hydrocarbyl groups having from 1 to 10 carbon atoms and, in some embodiments, from 1 to 6 carbon atoms. "$C_{u-v}$ alkyl" refers to alkyl groups having from u to v carbon atoms. This term includes, by way of example, linear and branched hydrocarbyl groups such as methyl ($CH_3$-), ethyl ($CH_3CH_2$-), *n*-propyl ($CH_3CH_2CH_2$-), isopropyl ($(CH_3)_2CH$-), *n*-butyl ($CH_3CH_2CH_2CH_2$-), isobutyl ($(CH_3)_2CHCH_2$-), *sec*-butyl ($(CH_3)(CH_3CH_2)CH$-), *t*-butyl ($(CH_3)_3C$-), *n*-pentyl ($CH_3CH_2CH_2CH_2CH_2$-), and neopentyl ($(CH_3)_3CCH_2$-).

"Substituted alkyl" and "substituted $C_{u-v}$ alkyl" refers to an alkyl group having from 1 to 5 and, in some embodiments, 1 to 3 or 1 to 2 substituents selected from the group consisting of alkenyl, substituted alkenyl, alkynyl, substituted

alkynyl, alkoxy, substituted alkoxy, acyl, acylamino, acyloxy, amino, substituted amino, aminocarbonyl, aminothiocarbonyl, aminocarbonylamino, aminothiocarbonylamino, aminocarbonyloxy, aminosulfonyl, aminosulfonyloxy, aminosulfonylamino, amidino, aryl, substituted aryl, aryloxy, substituted aryloxy, arylthio, substituted arylthio, azido, carboxyl, carboxyl ester, (carboxyl ester)amino, (carboxyl ester)oxy, cyano, cycloalkyl, substituted cycloalkyl, cycloalkyloxy, substituted cycloalkyloxy, cycloalkylthio, substituted cycloalkylthio, guanidino, substituted guanidino, halo, hydroxy, hydroxyamino, alkoxyamino, hydrazino, substituted hydrazino, heteroaryl, substituted heteroaryl, heteroaryloxy, substituted heteroaryloxy, heteroarylthio, substituted heteroarylthio, heterocyclic, substituted heterocyclic, heterocyclyloxy, substituted heterocyclyloxy, heterocyclylthio, substituted heterocyclylthio, nitro, spirocycloalkyl, $SO_3H$, substituted sulfonyl, sulfonyloxy, thioacyl, thiocyanate, thiol, alkylthio, and substituted alkylthio, wherein said substituents are as defined herein.

"Alkenyl" refers to a linear or branched hydrocarbyl group having from 2 to 10 carbon atoms and, in some embodiments, from 2 to 6 carbon atoms or 2 to 4 carbon atoms and having at least one site of vinyl unsaturation (>C = C<). "$C_u$-$_v$ alkenyl" refers to alkenyl groups having from u to v carbon atoms and is meant to include for example, ethenyl, propenyl, 1,3-butadienyl, and the like.

"Substituted alkenyl" and "substituted $C_u$-$_v$ alkenyl" refers to alkenyl groups having from 1 to 3 substituents and, in some embodiments, 1 to 2 substituents, selected from the group consisting of alkoxy, substituted alkoxy, acyl, acylamino, acyloxy, alkyl, substituted alkyl, alkynyl, substituted alkynyl, amino, substituted amino, aminocarbonyl, aminothiocarbonyl, aminocarbonylamino, aminothiocarbonylamino, aminocarbonyloxy, aminosulfonyl, aminosulfonyloxy, aminosulfonylamino, amidino, aryl, substituted aryl, aryloxy, substituted aryloxy, arylthio, substituted arylthio, carboxyl, carboxyl ester, (carboxyl ester)amino, (carboxyl ester)oxy, cyano, cycloalkyl, substituted cycloalkyl, cycloalkyloxy, substituted cycloalkyloxy, cycloalkylthio, substituted cycloalkylthio, guanidino, substituted guanidino, halo, hydroxy, heteroaryl, substituted heteroaryl, heteroaryloxy, substituted heteroaryloxy, heteroarylthio, substituted heteroarylthio, heterocyclic, substituted heterocyclic, heterocyclyloxy, substituted heterocyclyloxy, heterocyclylthio, substituted heterocyclylthio, nitro, $SO_3H$, substituted sulfonyl, sulfonyloxy, thioacyl, thiol, alkylthio, and substituted alkylthio, wherein said substituents are defined as herein and with the proviso that any hydroxy or thiol substitution is not attached to an acetylenic carbon atom.

"Alkynyl" refers to a linear monovalent hydrocarbon radical or a branched monovalent hydrocarbon radical containing at least one triple bond. The term "alkynyl" is also meant to include those hydrocarbyl groups having one triple bond and one double bond. "$C_{u-v}$ alkynyl" refers to alkynyl groups having from u to v carbon atoms and is meant to include ethynyl, propynyl, and the like.

"Substituted alkynyl" and "substituted $C_{u-v}$ alkynyl" refers to alkynyl groups having from 1 to 3 substituents and, in some embodiments, from 1 to 2 substituents, selected from the group consisting of alkoxy, substituted alkoxy, acyl, acylamino, acyloxy, amino, substituted amino, aminocarbonyl, aminothiocarbonyl, aminocarbonylamino, aminothiocarbonylamino, aminocarbonyloxy, aminosulfonyl, aminosulfonyloxy, aminosulfonylamino, amidino, aryl, substituted aryl, aryloxy, substituted aryloxy, arylthio, substituted arylthio, carboxyl, carboxyl ester, (carboxyl ester)amino, (carboxyl ester)oxy, cyano, cycloalkyl, substituted cycloalkyl, cycloalkyloxy, substituted cycloalkyloxy, cycloalkylthio, substituted cycloalkylthio, cycloalkenyl, substituted cycloalkenyl, cycloalkenyloxy, substituted cycloalkenyloxy, cycloalkenylthio, substituted cycloalkenylthio, guanidino, substituted guanidino, halo, hydroxy, heteroaryl, substituted heteroaryl, heteroaryloxy, substituted heteroaryloxy, heteroarylthio, substituted heteroarylthio, heterocyclic, substituted heterocyclic, heterocyclyloxy, substituted heterocyclyloxy, heterocyclylthio, substituted heterocyclylthio, nitro, $SO_3H$, substituted sulfonyl, sulfonyloxy, thioacyl, thiol, alkylthio, and substituted alkylthio, wherein said substituents are defined herein and with the proviso that any hydroxy or thiol substitution is not attached to an acetylenic carbon atom.

"Alkoxy" refers to the group -O-alkyl wherein alkyl is defined herein. Alkoxy includes, by way of example, methoxy, ethoxy, *n*-propoxy, isopropoxy, *n*-butoxy, *t*-butoxy, *sec*-butoxy, and *n*-pentoxy. "$C_u$-$_v$ alkoxy" refers to alkoxy groups having from u to v carbon atoms

"Substituted alkoxy" and "substituted $C_{u-v}$ alkoxy" refers to the group -O-(substituted alkyl) wherein substituted alkyl is as defined herein.

"Acyl" refers to the groups H-C(O)-, alkyl-C(O)-, substituted alkyl-C(O)-, alkenyl-C(O)-, substituted alkenyl-C (O)-, alkynyl-C(O)-, substituted alkynyl-C(O)-, cycloalkyl-C(O)-, substituted cycloalkyl-C(O)-, aryl-C(O)-, substituted aryl-C(O)-, substituted hydrazino-C(O)-, heteroaryl-C(O)-, substituted heteroaryl-C(O)-, heterocyclic-C(O)-, and substituted heterocyclic-C(O)-, wherein alkyl, substituted alkyl, alkenyl, substituted alkenyl, alkynyl, substituted alkynyl, cycloalkyl, substituted cycloalkyl, aryl, substituted aryl, substituted hydrazino, heteroaryl, substituted heteroaryl, heterocyclic and substituted heterocyclic are as defined herein. Acyl includes the "acetyl" group $CH_3C(O)$-.

"Acylamino" refers to the groups $-NR^{20}C(O)H$, $-NR^{20}C(O)$alkyl, $-NR^{20}C(O)$substituted alkyl, $-NR^{20}C(O)$cycloalkyl, $-NR^{20}C(O)$substituted cycloalkyl, $-NR^{20}C(O)$alkenyl, $-NR^{20}C(O)$substituted alkenyl, $-NR^{20}C(O)$alkynyl, $-NR^{20}C$ (O)substituted alkynyl, $-NR^{20}C(O)$aryl, $-NR^{20}C(O)$substituted aryl, $-NR^{20}C(O)$heteroaryl, $-NR^{20}C(O)$substituted heteroaryl, $-NR^{20}C(O)$heterocyclic, and $-NR^{20}C(O)$substituted heterocyclic wherein $R^{20}$ is hydrogen or alkyl and wherein alkyl, substituted alkyl, alkenyl, substituted alkenyl, alkynyl, substituted alkynyl, cycloalkyl, substituted cycloalkyl, aryl,

substituted aryl, heteroaryl, substituted heteroaryl, heterocyclic and substituted heterocyclic are as defined herein.

"Acyloxy" refers to the groups H-C(O)O-, alkyl-C(O)O-, substituted alkyl-C(O)O-, alkenyl-C(O)O-, substituted alkenyl-C(O)O-, alkynyl-C(O)O-, substituted alkynyl-C(O)O-, aryl-C(O)O-, substituted aryl-C(O)O-, cycloalkyl-C(O)O-, substituted cycloalkyl-C(O)O-, heteroaryl-C(O)O-, substituted heteroaryl-C(O)O-, heterocyclic-C(O)O-, and substituted heterocyclic-C(O)O- wherein alkyl, substituted alkyl, alkenyl, substituted alkenyl, alkynyl, substituted alkynyl, cycloalkyl, substituted cycloalkyl, aryl, substituted aryl, heteroaryl, substituted heteroaryl, heterocyclic, and substituted heterocyclic are as defined herein.

"Amino" refers to the group $-NH_2$.

"Substituted amino" refers to the group $-NR^{21}R^{22}$ where $R^{21}$ and $R^{22}$ are independently selected from the group consisting of hydrogen, alkyl, substituted alkyl, alkenyl, substituted alkenyl, alkynyl, substituted alkynyl, aryl, substituted aryl, cycloalkyl, substituted cycloalkyl, heteroaryl, substituted heteroaryl, heterocyclic, substituted heterocyclic, $-S(O)_2$-alkyl, $-S(O)_2$-substituted alkyl, $-S(O)_2$-alkenyl, $-S(O)_2$-substituted alkenyl, $-S(O)_2$-cycloalkyl, $-S(O_2$-substituted cycloalkyl, $-S(O)_2$-aryl, $-S(O)_2$-substituted aryl, $-S(O)_2$-heteroaryl, $-S(O)_2$-substituted heteroaryl, $-S(O)_2$-heterocyclyl, and $-S(O)_2$-substituted heterocyclyl and wherein $R^{21}$ and $R^{22}$ are optionally joined together with the nitrogen bound thereto to form a heterocyclyl or substituted heterocyclyl group, provided that $R^{21}$ and $R^{22}$ are both not hydrogen, and wherein alkyl, substituted alkyl, alkenyl, substituted alkenyl, alkynyl, substituted alkynyl, cycloalkyl, substituted cycloalkyl, aryl, substituted aryl, heteroaryl, substituted heteroaryl, heterocyclic, and substituted heterocyclic are as defined herein. When $R^{21}$ is hydrogen and $R^{22}$ is alkyl, the substituted amino group is sometimes referred to herein as alkylamino. When $R^{21}$ and $R^{22}$ are alkyl, the substituted amino group is sometimes referred to herein as dialkylamino. When referring to a monosubstituted amino, it is meant that either $R^{21}$ or $R^{22}$ is hydrogen but not both. When referring to a disubstituted amino, it is meant that neither $R^{21}$ nor $R^{22}$ are hydrogen.

"Hydroxyamino" refers to the group -NHOH.

"Alkoxyamino" refers to the group -NHO-alkyl wherein alkyl is defined herein.

"Aminocarbonyl" refers to the group $-C(O)NR^{23}R^{24}$ where $R^{23}$ and $R^{24}$ are independently selected from the group consisting of hydrogen, alkyl, substituted alkyl, alkenyl, substituted alkenyl, alkynyl, substituted alkynyl, aryl, substituted aryl, cycloalkyl, substituted cycloalkyl, heteroaryl, substituted heteroaryl, heterocyclic, substituted heterocyclic, hydroxy, alkoxy, substituted alkoxy, amino, substituted amino, and acylamino, and where $R^{23}$ and $R^{24}$ are optionally joined together with the nitrogen bound thereto to form a heterocyclic or substituted heterocyclic group, and wherein alkyl, substituted alkyl, alkenyl, substituted alkenyl, alkynyl, substituted alkynyl, cycloalkyl, substituted cycloalkyl, aryl, substituted aryl, heteroaryl, substituted heteroaryl, heterocyclic and substituted heterocyclic are as defined herein.

"Aminothiocarbonyl" refers to the group $-C(S)NR^{23}R^{24}$ where $R^{23}$ and $R^{24}$ are independently selected from the group consisting of hydrogen, alkyl, substituted alkyl, alkenyl, substituted alkenyl, alkynyl, substituted alkynyl, aryl, substituted aryl, cycloalkyl, substituted cycloalkyl, heteroaryl, substituted heteroaryl, heterocyclic, and substituted heterocyclic and where $R^{23}$ and $R^{24}$ are optionally joined together with the nitrogen bound thereto to form a heterocyclic or substituted heterocyclic group, and wherein alkyl, substituted alkyl, alkenyl, substituted alkenyl, alkynyl, substituted alkynyl, cycloalkyl, substituted cycloalkyl, aryl, substituted aryl, heteroaryl, substituted heteroaryl, heterocyclic and substituted heterocyclic are as defined herein.

"Aminocarbonylamino" refers to the group $-NR^{20}C(O)NR^{23}R^{24}$ where $R^{20}$ is hydrogen or alkyl and $R^{23}$ and $R^{24}$ are independently selected from the group consisting of hydrogen, alkyl, substituted alkyl, alkenyl, substituted alkenyl, alkynyl, substituted alkynyl, aryl, substituted aryl, cycloalkyl, substituted cycloalkyl, heteroaryl, substituted heteroaryl, heterocyclic, and substituted heterocyclic and where $R^{23}$ and $R^{24}$ are optionally joined together with the nitrogen bound thereto to form a heterocyclic or substituted heterocyclic group, and wherein alkyl, substituted alkyl, alkenyl, substituted alkenyl, alkynyl, substituted alkynyl, cycloalkyl, substituted cycloalkyl, aryl, substituted aryl, heteroaryl, substituted heteroaryl, heterocyclic and substituted heterocyclic are as defined herein.

"Aminothiocarbonylamino" refers to the group $-NR^{20}C(S)NR^{23}R^{24}$ where $R^{20}$ is hydrogen or alkyl and $R^{23}$ and $R^{24}$ are independently selected from the group consisting of hydrogen, alkyl, substituted alkyl, alkenyl, substituted alkenyl, alkynyl, substituted alkynyl, aryl, substituted aryl, cycloalkyl, substituted cycloalkyl, heteroaryl, substituted heteroaryl, heterocyclic, and substituted heterocyclic and where $R^{23}$ and $R^{24}$ are optionally joined together with the nitrogen bound thereto to form a heterocyclic or substituted heterocyclic group, and wherein alkyl, substituted alkyl, alkenyl, substituted alkenyl, alkynyl, substituted alkynyl, cycloalkyl, substituted cycloalkyl, aryl, substituted aryl, heteroaryl, substituted heteroaryl, heterocyclic and substituted heterocyclic are as defined herein.

"Aminocarbonyloxy" refers to the group $-O-C(O)NR^{23}R^{24}$ where $R^{23}$ and $R^{24}$ are independently selected from the group consisting of hydrogen, alkyl, substituted alkyl, alkenyl, substituted alkenyl, alkynyl, substituted alkynyl, aryl, substituted aryl, cycloalkyl, substituted cycloalkyl, heteroaryl, substituted heteroaryl, heterocyclic, and substituted heterocyclic and where $R^{23}$ and $R^{24}$ are optionally joined together with the nitrogen bound thereto to form a heterocyclic or substituted heterocyclic group, and wherein alkyl, substituted alkyl, alkenyl, substituted alkenyl, alkynyl, substituted alkynyl, cycloalkyl, substituted cycloalkyl, aryl, substituted aryl, heteroaryl, substituted heteroaryl, heterocyclic and substituted heterocyclic are as defined herein.

"Aminosulfonyl" refers to the group $-S(O)_2NR^{23}R^{24}$ where $R^{23}$ and $R^{24}$ are independently selected from the group consisting of hydrogen, alkyl, substituted alkyl, alkenyl, substituted alkenyl, alkynyl, substituted alkynyl, aryl, substituted aryl, cycloalkyl, substituted cycloalkyl, heteroaryl, substituted heteroaryl, heterocyclic, and substituted heterocyclic and where $R^{23}$ and $R^{24}$ are optionally joined together with the nitrogen bound thereto to form a heterocyclic or substituted heterocyclic group, and wherein alkyl, substituted alkyl, alkenyl, substituted alkenyl, alkynyl, substituted alkynyl, cycloalkyl, substituted cycloalkyl, aryl, substituted aryl, heteroaryl, substituted heteroaryl, heterocyclic and substituted heterocyclic are as defined herein.

"Aminosulfonyloxy" refers to the group $-O-S(O)_2NR^{23}R^{24}$ where $R^{23}$ and $R^{24}$ are independently selected from the group consisting of hydrogen, alkyl, substituted alkyl, alkenyl, substituted alkenyl, alkynyl, substituted alkynyl, aryl, substituted aryl, cycloalkyl, substituted cycloalkyl, heteroaryl, substituted heteroaryl, heterocyclic, and substituted heterocyclic and where $R^{23}$ and $R^{24}$ are optionally joined together with the nitrogen bound thereto to form a heterocyclic or substituted heterocyclic group, and wherein alkyl, substituted alkyl, alkenyl, substituted alkenyl, alkynyl, substituted alkynyl, cycloalkyl, substituted cycloalkyl, aryl, substituted aryl, heteroaryl, substituted heteroaryl, heterocyclic and substituted heterocyclic are as defined herein.

"Aminosulfonylamino" refers to the group $-NR^{20}-S(O)_2NR^{23}R^{24}$ where $R^{20}$ is hydrogen or alkyl and $R^{23}$ and $R^{24}$ are independently selected from the group consisting of hydrogen, alkyl, substituted alkyl, alkenyl, substituted alkenyl, alkynyl, substituted alkynyl, aryl, substituted aryl, cycloalkyl, substituted cycloalkyl, heteroaryl, substituted heteroaryl, heterocyclic, and substituted heterocyclic and where $R^{23}$ and $R^{24}$ are optionally joined together with the nitrogen bound thereto to form a heterocyclic or substituted heterocyclic group, and wherein alkyl, substituted alkyl, alkenyl, substituted alkenyl, alkynyl, substituted alkynyl, cycloalkyl, substituted cycloalkyl, aryl, substituted aryl, heteroaryl, substituted heteroaryl, heterocyclic and substituted heterocyclic are as defined herein.

"Amidino" refers to the group $-C(=NR^{25})NR^{23}R^{24}$ where $R^{25}$, $R^{23}$, and $R^{24}$ are independently selected from the group consisting of hydrogen, alkyl, substituted alkyl, alkenyl, substituted alkenyl, alkynyl, substituted alkynyl, aryl, substituted aryl, cycloalkyl, substituted cycloalkyl, heteroaryl, substituted heteroaryl, heterocyclic, and substituted heterocyclic and where $R^{23}$ and $R^{24}$ are optionally joined together with the nitrogen bound thereto to form a heterocyclic or substituted heterocyclic group, and wherein alkyl, substituted alkyl, alkenyl, substituted alkenyl, alkynyl, substituted alkynyl, cycloalkyl, substituted cycloalkyl, aryl, substituted aryl, heteroaryl, substituted heteroaryl, heterocyclic and substituted heterocyclic are as defined herein.

"Aryl" refers to an aromatic group of from 6 to 14 carbon atoms and no ring heteroatoms and having a single ring (*e.g.,* phenyl) or multiple condensed (fused) rings (*e.g.,* naphthyl or anthryl). For multiple ring systems, including fused, bridged, and spiro ring systems having aromatic and non-aromatic rings that have no ring heteroatoms, the term "Aryl" or "Ar" applies when the point of attachment is at an aromatic carbon atom (*e.g.,* 5,6,7,8-tetrahydronaphthalene-2-yl is an aryl group as its point of attachment is at the 2-position of the aromatic phenyl ring).

"Substituted aryl" refers to aryl groups which are substituted with 1 to 8 and, in some embodiments, 1 to 5, 1 to 3 or 1 to 2 substituents selected from the group consisting of alkyl, substituted alkyl, alkenyl, substituted alkenyl, alkynyl, substituted alkynyl, alkoxy, substituted alkoxy, acyl, acylamino, acyloxy, amino, substituted amino, aminocarbonyl, aminothiocarbonyl, aminocarbonylamino, aminothiocarbonylamino, aminocarbonyloxy, aminosulfonyl, aminosulfonyloxy, aminosulfonylamino, amidino, aryl, substituted aryl, aryloxy, substituted aryloxy, arylthio, substituted arylthio, azido, carboxyl, carboxyl ester, (carboxyl ester)amino, (carboxyl ester)oxy, cyano, cycloalkyl, substituted cycloalkyl, cycloalkyloxy, substituted cycloalkyloxy, cycloalkylthio, substituted cycloalkylthio, guanidino, substituted guanidino, halo, hydroxy, hydroxyamino, alkoxyamino, hydrazino, substituted hydrazino, heteroaryl, substituted heteroaryl, heteroaryloxy, substituted heteroaryloxy, heteroarylthio, substituted heteroarylthio, heterocyclic, substituted heterocyclic, heterocyclyloxy, substituted heterocyclyloxy, heterocyclylthio, substituted heterocyclylthio, nitro, $SO_3H$, substituted sulfonyl, sulfonyloxy, thioacyl, thiocyanate, thiol, alkylthio, and substituted alkylthio, wherein said substituents are defined herein.

"Arylalkyl" or "Aryl($C_1$-$C_z$)alkyl" refers to the radical $-R^uR^v$ where $R^u$ is an alkylene group (having 8 or fewer main chain carbon atoms) and $R^v$ is an aryl group as defined herein. Thus, "arylalkyl" refers to groups such as, for example, benzyl, and phenylethyl, and the like. Similarly, "Arylalkenyl" means a radical $-R^uR^v$ where $R^u$ is an alkenylene group (an alkylene group having 1 or 2 double bonds) and $R^v$ is an aryl group as defined herein, *e.g.,* styrenyl, 3-phenyl-2-propenyl, and the like.

"Aryloxy" refers to the group -O-aryl, where aryl is as defined herein, that includes, by way of example, phenoxy and naphthoxy.

"Substituted aryloxy" refers to the group -O-(substituted aryl) where substituted aryl is as defined herein.

"Arylthio" refers to the group -S-aryl, where aryl is as defined herein.

"Substituted arylthio" refers to the group -S-(substituted aryl), where substituted aryl is as defined herein.

"Azido" refers to the group $-N_3$.

"Hydrazino" refers to the group $-NHNH_2$.

"Substituted hydrazino" refers to the group $-NR^{26}NR^{27}R^{28}$ where $R^{26}$,$R^{27}$, and $R^{28}$ are independently selected from the group consisting of hydrogen, alkyl, substituted alkyl, alkenyl, substituted alkenyl, alkynyl, substituted alkynyl,

aryl, substituted aryl, carboxyl ester, cycloalkyl, substituted cycloalkyl, heteroaryl, substituted heteroaryl, heterocyclic, substituted heterocyclic, $-S(O)_2$-alkyl, $-S(O)_2$-substituted alkyl, $-S(O)_2$-alkenyl, $-S(O)_2$-substituted alkenyl, $-S(O)_2$-cycloalkyl, $-S(O)_2$-substituted cylcoalkyl, $-S(O)_2$-aryl, $-S(O)_2$-substituted aryl, $-S(O)_2$-heteroaryl, $-S(O)_2$-substituted heteroaryl, $-S(O)_2$-heterocyclic, and $-S(O)_2$-substituted heterocyclic and wherein $R^{27}$ and $R^{28}$ are optionally joined, together with the nitrogen bound thereto to form a heterocyclic or substituted heterocyclic group, provided that $R^{27}$ and $R^{28}$ are both not hydrogen, and wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic, and substituted heterocyclic are as defined herein.

"Cyano" or "carbonitrile" refers to the group -CN.

"Carbonyl" refers to the divalent group -C(O)- which is equivalent to -C(=O)-.

"Carboxyl" or "carboxy" refers to -COOH or salts thereof.

"Carboxyl ester" or "carboxy ester" refers to the groups -C(O)O-alkyl, -C(O)O-substituted alkyl, -C(O)O-alkenyl, -C(O)O-substituted alkenyl, -C(O)O-alkynyl, -C(O)O-substituted alkynyl, -C(O)O-aryl, -C(O)O-substituted aryl, -C(O)O-cycloalkyl, -C(O)O-substituted cycloalkyl, -C(O)O-heteroaryl, -C(O)O-substituted heteroaryl, -C(O)O-heterocyclic, and -C(O)O-substituted heterocyclic wherein alkyl, substituted alkyl, alkenyl, substituted alkenyl, alkynyl, substituted alkynyl, cycloalkyl, substituted cycloalkyl, aryl, substituted aryl, heteroaryl, substituted heteroaryl, heterocyclic, and substituted heterocyclic are as defined herein.

"(Carboxyl ester)amino" refers to the group $-NR^{20}$-C(O)O-alkyl, $-NR^{20}$-C(O)O-substituted alkyl, $-NR^{20}$-C(O)O-alkenyl, $-NR^{20}$-C(O)O-substituted alkenyl, $-NR^{20}$-C(O)O-alkynyl, $-NR^{20}$-C(O)O-substituted alkynyl, $-NR^{20}$-C(O)O-aryl, $-NR^{20}$-C(O)O-substituted aryl, $-NR^{20}$-C(O)O-cycloalkyl, $-NR^{20}$-C(O)O-substituted cycloalkyl, $-NR^{20}$-C(O)O-heteroaryl, $-NR^{20}$-C(O)O-substituted heteroaryl, $-NR^{20}$-C(O)O-heterocyclic, and $-NR^{20}$-C(O)O-substituted heterocyclic wherein $R^{20}$ is alkyl or hydrogen, and wherein alkyl, substituted alkyl, alkenyl, substituted alkenyl, alkynyl, substituted alkynyl, cycloalkyl, substituted cycloalkyl, aryl, substituted aryl, heteroaryl, substituted heteroaryl, heterocyclic, and substituted heterocyclic are as defined herein.

"(Carboxyl ester)oxy" refers to the group -O-C(O)O-alkyl, -O-C(O)O-substituted alkyl, -O-C(O)O-alkenyl, -O-C(O)O-substituted alkenyl, -O-C(O)O-alkynyl, -O-C(O)O-substituted alkynyl, -O-C(O)O-aryl, -O-C(O)O-substituted aryl, -O-C(O)O-cycloalkyl, -O-C(O)O-substituted cycloalkyl, -O-C(O)O-heteroaryl, -O-C(O)O-substituted heteroaryl, -O-C(O)O-heterocyclic, and -O-C(O)O-substituted heterocyclic wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic, and substituted heterocyclic are as defined herein.

"Cycloalkyl" refers to a saturated or partially saturated cyclic group of from 3 to 14 carbon atoms and no ring heteroatoms and having a single ring or multiple rings including fused, bridged, and spiro ring systems. For multiple ring systems having aromatic and non-aromatic rings that have no ring heteroatoms, the term "cycloalkyl" applies when the point of attachment is at a non-aromatic carbon atom (*e.g.,* 5,6,7,8,-tetrahydronaphthalene-5-yl). The term "cycloalkyl" includes cycloalkenyl groups. Examples of cycloalkyl groups include, for instance, adamantyl, cyclopropyl, cyclobutyl, cyclopentyl, cyclooctyl, and cyclohexenyl. "$C_{u-v}$ cycloalkyl" refers to cycloalkyl groups having u to v carbon atoms as ring members. "$C_{u-v}$ cycloalkenyl" refers to cycloalkenyl groups having u to v carbon atoms as ring members.

"Cycloalkenyl" refers to a partially saturated cycloalkyl ring having at least one site of >C = C< ring unsaturation.

"Substituted cycloalkyl" refers to a cycloalkyl group, as defined herein, having from 1 to 8, or 1 to 5, or, in some embodiments, 1 to 3 substituents selected from the group consisting of oxo, thione, alkyl, substituted alkyl, alkenyl, substituted alkenyl, alkynyl, substituted alkynyl, alkoxy, substituted alkoxy, acyl, acylamino, acyloxy, amino, substituted amino, aminocarbonyl, aminothiocarbonyl, aminocarbonylamino, aminothiocarbonylamino, aminocarbonyloxy, aminosulfonyl, aminosulfonyloxy, aminosulfonylamino, amidino, aryl, substituted aryl, aryloxy, substituted aryloxy, arylthio, substituted arylthio, azido, carboxyl, carboxyl ester, (carboxyl ester)amino, (carboxyl ester)oxy, cyano, cycloalkyl, substituted cycloalkyl, cycloalkyloxy, substituted cycloalkyloxy, cycloalkylthio, substituted cycloalkylthio, guanidino, substituted guanidino, halo, hydroxy, hydroxyamino, alkoxyamino, hydrazino, substituted hydrazino, heteroaryl, substituted heteroaryl, heteroaryloxy, substituted heteroaryloxy, heteroarylthio, substituted heteroarylthio, heterocyclic, substituted heterocyclic, heterocyclyloxy, substituted heterocyclyloxy, heterocyclylthio, substituted heterocyclylthio, nitro, $SO_3H$, substituted sulfonyl, sulfonyloxy, thioacyl, thiocyanate, thiol, alkylthio, and substituted alkylthio, wherein said substituents are as defined herein. The term "substituted cycloalkyl" includes substituted cycloalkenyl groups.

"Cycloalkyloxy" refers to -O-cycloalkyl wherein cycloalkyl is as defined herein.

"Substituted cycloalkyloxy" refers to -O-(substituted cycloalkyl) wherein substituted cycloalkyl is as defined herein.

"Cycloalkylthio" refers to -S-cycloalkyl wherein substituted cycloalkyl is as defined herein.

"Substituted cycloalkylthio" refers to -S-(substituted cycloalkyl) wherein substituted cycloalkyl is as defined herein.

"Guanidino" refers to the group $-NHC(=NH)NH_2$.

"Substituted guanidino" refers to $-NR^{29}C(=NR^{29})N(R^{29})_2$ where each $R^{29}$ is independently selected from the

group consisting of hydrogen, alkyl, substituted alkyl, aryl, substituted aryl, heteroaryl, substituted heteroaryl, heterocyclyl, and substituted heterocyclyl and two $R^{29}$ groups attached to a common guanidino nitrogen atom are optionally joined together with the nitrogen bound thereto to form a heterocyclic or substituted heterocyclic group, provided that at least one $R^{29}$ is not hydrogen, and wherein said substituents are as defined herein.

"Halo" or "halogen" refers to fluoro, chloro, bromo and iodo.

"Haloalkyl" refers to substitution of alkyl groups with 1 to 5 or, in some embodiments, 1 to 3 halo groups, *e.g.,* $-CH_2Cl$, $-CH_2F$, $-CH_2Br$, -CFClBr, $-CH_2CH_2Cl$, - $CH_2CH_2F$, $-CF_3$, $-CH_2CF_3$, $-CH_2CCl_3$, and the like, and further includes those alkyl groups such as perfluoroalkyl in which all hydrogen atoms are replaced by fluorine atoms.

"Haloalkoxy" refers to substitution of alkoxy groups with 1 to 5 or, in some embodiments, 1 to 3 halo groups, *e.g.,* $-OCH_2Cl$, $-OCH_2F$, $-OCH_2CH_2Br$, $-OCH_2CH_2Cl$, $-OCF_3$, and the like.

"Hydroxy" or "hydroxyl" refers to the group -OH.

"Heteroalkyl" means an alkyl radical as defined herein with 1, 2 or 3 substituents independently selected from cyano, $-OR^w$, $-NR^xR^y$, $-SR^z$, $-S(O)R^z$, and $-S(O)_2R^z$ (where n is 0, 1, or 2), with the understanding that the point of attachment of the heteroalkyl radical is through a carbon atom of the heteroalkyl radical. $R^w$ is hydrogen, alkyl, cycloalkyl, cycloalkyl-alkyl, aryl, arylalkyl, alkoxycarbonyl, aryloxycarbonyl, carboxamido, or mono-or di-alkylcarbamoyl. $R^x$ is hydrogen, alkyl, cycloalkyl, cycloalkyl-alkyl, aryl or arylalkyl. $R^y$ is hydrogen, alkyl, cycloalkyl, cycloalkyl-alkyl, aryl, arylalkyl, alkoxycarbonyl, aryloxycarbonyl, carboxamido, mono- or di-alkylcarbamoyl or alkylsulfonyl. $R^z$ is hydrogen, alkyl, cycloalkyl, cycloalkyl-alkyl, aryl, arylalkyl, amino, mono-alkylamino, di-alkylamino, or hydroxyalkyl. Representative examples include, for example, 2-hydroxyethyl, 2,3-dihydroxypropyl, 2-methoxyethyl, benzyloxymethyl, 2-cyanoethyl, and 2-methylsulfonyl-ethyl. For each of the above, $R^w$, $R^x$, $R^y$, and $R^z$ can be further substituted by amino, fluorine, alkylamino, di-alkylamino, OH or alkoxy. Additionally, the prefix indicating the number of carbon atoms (*e.g.,* $C_1$-$C_{1o}$) refers to the total number of carbon atoms in the portion of the heteroalkyl group exclusive of the cyano, $-OR^w$, $-NR^xR^y$, $-SR^z$, - S(O) $R^z$, or $-S(O)_2R^z$ portions.

"Heteroaryl" refers to an aromatic group of from 1 to 14 carbon atoms and 1 to 6 heteroatoms selected from the group consisting of oxygen, nitrogen, and sulfur and includes a 5- to 18- member ring or ring system that includes a single ring (*e.g.,* imidazolyl) or multiple rings (*e.g.,* benzimidazol-2-yl and benzimidazol-6-yl). For multiple ring systems, including fused, bridged, and spiro ring systems having aromatic and non-aromatic rings, the term "heteroaryl" applies if there is at least one ring heteroatom and the point of attachment is at an atom of an aromatic ring (*e.g.,* 1,2,3,4-tetrahydroquinolin-6-yl and 5,6,7,8-tetrahydroquinolin-3-yl). In one embodiment, the nitrogen and/or the sulfur ring atom (s) of the heteroaryl group are optionally oxidized to provide for the N-oxide (N→O), sulfinyl, or sulfonyl moieties. More specifically the term heteroaryl includes, but is not limited to, pyridyl, furanyl, thienyl, thiazolyl, isothiazolyl, tetrazolyl, triazolyl, imidazolyl, isoxazolyl, pyrrolyl, pyrazolyl, pyridazinyl, pyrimidinyl, benzofuranyl, tetrahydrobenzofuranyl, isobenzofuranyl, benzothiazolyl, benzoisothiazolyl, benzotriazolyl, indolyl, isoindolyl, benzoxazolyl, quinolyl, tetrahydroquinolinyl, isoquinolyl, quinazolinonyl, benzimidazolyl, benzisoxazolyl, or benzothienyl.

"N-linked" refers to nitrogen containing groups in which the point of attachment is to the nitrogen atom of the nitrogen containing group. For example, "N-linked tetrazolyl" is a group in which the point of attachment is to a nitrogen atom of the tetrazolyl group. Similarly, N-linked triazolyl, N-linked imidazolyl, N-linked pyrazolyl and N-linked pyrrolyl are groups in which the point of attachment is to a nitrogen atom of the triazole, imidazole, pyrazole, and pyrrol group, respectively. Similarly, "N-linked imidazolyl" refers to an imidazole in which the point of attachment is to the nitrogen atom.

"Substituted heteroaryl" refers to heteroaryl groups that are substituted with from 1 to 8, or, in some embodiments, 1 to 5, or 1 to 3, or 1 to 2 substituents selected from the group consisting of the substituents defined for substituted aryl.

"Heteroaryloxy" refers to -O-heteroaryl wherein heteroaryl is as defined herein.

"Substituted heteroaryloxy" refers to the group -O-(substituted heteroaryl) wherein heteroaryl is as defined herein.

"Heteroarylthio" refers to the group -S-heteroaryl wherein heteroaryl is as defined herein.

"Substituted heteroarylthio" refers to the group -S-(substituted heteroaryl) wherein heteroaryl is as defined herein.

"Heterocycle" or "heterocyclic" or "heterocyclo" or "heterocycloalkyl" or "heterocyclyl" refers to a saturated or partially saturated cyclic group having from 1 to 14 carbon atoms and from 1 to 6 heteroatoms selected from the group consisting of nitrogen, sulfur, or oxygen and includes single ring and multiple ring systems including fused, bridged, and spiro ring systems. For multiple ring systems having aromatic and/or non-aromatic rings, the term "heterocyclic", "heterocycle", "heterocyclo", "heterocycloalkyl" or "heterocyclyl" applies when there is at least one ring heteroatom and the point of attachment is at an atom of a non-aromatic ring (*e.g.,* 1,2,3,4-tetrahydroquinoline-3-yl, 5,6,7,8-tetrahydroquinoline-6-yl, and decahydroquinolin-6-yl). In one embodiment, the nitrogen and/or sulfur atom(s) of the heterocyclic group are optionally oxidized to provide for the N-oxide, sulfinyl, and sulfonyl moieties. More specifically the heterocyclyl includes, but is not limited to, tetrahydropyranyl, piperidinyl, N-methylpiperidin-3-yl, piperazinyl, N-methylpyrrolidin-3-yl, 3-pyrrolidinyl, 2-pyrrolidon-1-yl, morpholinyl, and pyrrolidinyl. A prefix indicating the number of carbon atoms (*e.g.,* $C_3$-$C_{10}$) refers to the total number of carbon atoms in the portion of the heterocyclyl group exclusive of the number of heteroatoms.

"Substituted heterocycle" or "substituted heterocyclic" or "substituted heterocyclo" or "substituted heterocycloalkyl" or "substituted heterocyclyl" refers to heterocyclic groups, as defined herein, that are substituted with from 1 to 5 or, in some embodiments, 1 to 3 of the substituents as defined for substituted cycloalkyl.

"Heterocyclyloxy" refers to the group -O-heterocyclyl wherein heterocyclyl is as defined herein.

"Substituted heterocyclyloxy" refers to the group -O-(substituted heterocyclyl) wherein heterocyclyl is as defined herein.

"Heterocyclylthio" refers to the group -S-heterocycyl wherein heterocyclyl is as defined herein.

"Substituted heterocyclylthio" refers to the group -S-(substituted heterocycyl) wherein heterocyclyl is as defined herein.

Examples of heterocycle and heteroaryl groups include, but are not limited to, azetidine, pyrrole, imidazole, pyrazole, pyridine, pyrazine, pyrimidine, pyridazine, indolizine, isoindole, indole, dihydroindole, indazole, purine, quinolizine, isoquinoline, quinoline, phthalazine, naphthylpyridine, quinoxaline, quinazoline, cinnoline, pteridine, carbazole, carboline, phenanthridine, acridine, phenanthroline, isothiazole, phenazine, isoxazole, phenoxazine, phenothiazine, imidazolidine, imidazoline, piperidine, piperazine, indoline, phthalimide, 1,2,3,4-tetrahydroisoquinoline, 4,5,6,7-tetrahydrobenzo[b]thiophene, thiazole, thiazolidine, thiophene, benzo[b]thiophene, morpholinyl, thiomorpholinyl (also referred to as thiamorpholinyl), 1,1-dioxothiomorpholinyl, piperidinyl, pyrrolidine, and tetrahydrofuranyl.

"Nitro" refers to the group $-NO_2$.

"Oxo" refers to the atom (=O).

"Oxide" refers to products resulting from the oxidation of one or more heteroatoms. Examples include N-oxides, sulfoxides, and sulfones.

"Spirocycloalkyl" refers to a 3- to 10- member cyclic substituent formed by replacement of two hydrogen atoms at a common carbon atom with an alkylene group having 2 to 9 carbon atoms, as exemplified by the following structure wherein the methylene group shown below attached to bonds marked with wavy lines is substituted with a spirocycloalkyl group:

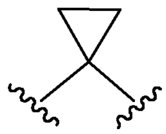
.

"Sulfonyl" refers to the divalent group $-S(O)_2-$.

"Substituted sulfonyl" refers to the group $-S(O)_2$-alkyl, $-S(O)_2$-substituted alkyl, $-S(O)_2$-alkenyl, $-S(O)_2$-substituted alkenyl, $-S(O)_2$-alkynyl, $-S(O)_2$-substituted alkynyl, $-S(O)_2$-cycloalkyl, $-S(O)_2$-substituted cylcoalkyl, $-S(O)_2$-aryl, $-S(O)_2$-substituted aryl, $-S(O)_2$-heteroaryl, $-S(O)_2$-substituted heteroaryl, $-S(O)_2$-heterocyclic, $-S(O)_2$-substituted heterocyclic, wherein alkyl, substituted alkyl, alkenyl, substituted alkenyl, alkynyl, substituted alkynyl, cycloalkyl, substituted cycloalkyl, aryl, substituted aryl, heteroaryl, substituted heteroaryl, heterocyclic and substituted heterocyclic are as defined herein. Substituted sulfonyl includes groups such as methyl-$S(O)_2$-, phenyl-$S(O)_2$-, and 4-methylphenyl-S(O)2-.

"Sulfonyloxy" refers to the group $-OS(O)_2$-alkyl, $-OS(O)_2$-substituted alkyl, $-OS(O)_2$-alkenyl, $-OS(O)_2$-substituted alkenyl, $-OS(O)_2$-cycloalkyl, $-OS(O)_2$-substituted cylcoalkyl, $-OS(O)_2$-aryl, $-OS(O)_2$-substituted aryl, $-OS(O)_2$-heteroaryl, $-OS(O)_2$-substituted heteroaryl, $-OS(O)_2$-heterocyclic, $-OS(O)_2$-substituted heterocyclic, wherein alkyl, substituted alkyl, alkenyl, substituted alkenyl, alkynyl, substituted alkynyl, cycloalkyl, substituted cycloalkyl, aryl, substituted aryl, heteroaryl, substituted heteroaryl, heterocyclic and substituted heterocyclic are as defined herein.

"Thioacyl" refers to the groups H-C(S)-, alkyl-C(S)-, substituted alkyl-C(S)-, alkenyl-C(S)-, substituted alkenyl-C(S)-, alkynyl-C(S)-, substituted alkynyl-C(S)-, cycloalkyl-C(S)-, substituted cycloalkyl-C(S)-, aryl-C(S)-, substituted aryl-C(S)-, heteroaryl-C(S)-, substituted heteroaryl-C(S)-, heterocyclic-C(S)-, and substituted heterocyclic-C(S)-, wherein alkyl, substituted alkyl, alkenyl, substituted alkenyl, alkynyl, substituted alkynyl, cycloalkyl, substituted cycloalkyl, aryl, substituted aryl, heteroaryl, substituted heteroaryl, heterocyclic and substituted heterocyclic are as defined herein.

"Thiol" refers to the group -SH.

"Alkylthio" refers to the group -S-alkyl wherein alkyl is as defined herein.

"Substituted alkylthio" refers to the group -S-(substituted alkyl) wherein substituted alkyl is as defined herein.

"Thiocarbonyl" refers to the divalent group -C(S)- which is equivalent to -C(=S)-.

"Thione" refers to the atom (=S).

"Thiocyanate" refers to the group -SCN.

"Compound" and "compounds" as used herein refers to a compound encompassed by the generic formulae disclosed herein, any subgenus of those generic formulae, and any forms of the compounds specificed by the generic and subgeneric formulae, such as a pharmaceutically acceptable salt. Unless specified otherwise, the term further includes the racemates, stereoisomers, and tautomers of the compound or compounds.

"Racemates" refers to a mixture of enantiomers.

"Solvate" or "solvates" of a compound refer to those compounds, where compounds are as defined herein, that are bound to a stoichiometric or non-stoichiometric amount of a solvent. Solvates of a compound includes solvates of all forms of the compound such as the oxide, ester, prodrug, or pharmaceutically acceptable salt of the disclosed generic and subgeneric formulae. Preferred solvents are volatile, non-toxic, and/or acceptable for administration to humans. The present invention provides solvates of the compounds disclosed herein and of the compounds of Formula (A)-(D) and (I)-(XIV).

"Stereoisomer" or "stereoisomers" refer to compounds that differ in the chirality of one or more stereocenters. Stereoisomers include enantiomers and diastereomers. The compounds of this invention may exist in stereoisomeric form if they possess one or more asymmetric centers or a double bond with asymmetric substitution and, therefore, can be produced as individual stereoisomers or as mixtures. Unless otherwise indicated, the description is intended to include individual stereoisomers as well as mixtures. The methods for the determination of stereochemistry and the separation of stereoisomers are well-known in the art (*see* discussion in Chapter 4 of Advanced Organic Chemistry, 4th ed., J. March, John Wiley and Sons, New York, 1992).

"Tautomer" refers to alternate forms of a compound that differ in the position of a proton, such as enol-keto and imine-enamine tautomers, or the tautomeric forms of heteroaryl groups containing a ring atom attached to both a ring -NH- moiety and a ring =N-moiety such as pyrazoles, imidazoles, benzimidazoles, triazoles, and tetrazoles.

"Prodrug" refers to any derivative of a compound of the embodiments that is capable of directly or indirectly providing a compound of the embodiments or an active metabolite or residue thereof when administered to a patient. Prodrugs of a compound of the present invention are prepared by modifying functional groups present in the compound in such a way that the modifications may be cleaved *in vivo* to release the parent compound, or an active metabolite. For example, prodrugs include compounds wherein a hydroxy, amino, or sulfhydryl group in a compound is bonded to any group that may be cleaved *in vivo* to regenerate the free hydroxyl, amino, or sulfhydryl group, respectively. Particularly favored derivatives and prodrugs are those that increase the bioavailability of the compounds of the embodiments when such compounds are administered to a patient (*e.g.,* by allowing an orally administered compound to be more readily absorbed into the blood) or which enhance delivery of the parent compound to a biological compartment (*e.g.,* the brain or lymphatic system) relative to the parent species. Prodrugs include ester, amide, and carbamate (*e.g.,* N, N-dimethylaminocarbonyl) forms of hydroxy functional groups of compounds of the invention. Examples of ester prodrugs include formate, acetate, propionate, butyrate, acrylate, and ethylsuccinate derivatives. An general overview of prodrugs is provided in T Higuchi and V Stella, Pro-drugs as Novel Delivery Systems, Vol. 14 of the A.C.S. Symposium Series, and in Edward B. Roche, ed., Bioreversible Carriers in Drug Design, American Pharmaceutical Association and Pergamon Press, 1987, both of which are incorporated herein by reference.

"Pharmaceutically acceptable salt" refers to pharmaceutically acceptable salts derived from a variety of organic and inorganic counter ions well known in the art and includes, by way of example only, sodium, potassium, calcium, magnesium, ammonium, and tetraalkylammonium. When the molecule contains a basic functionality, acid addition salts of organic or inorganic acids, such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl)benzoic acid, cinnamic acid, mandelic acid, methanesulfonic acid, ethanesulfonic acid, 1,2-ethane-disulfonic acid, 2-hydroxyethanesulfonic acid, benzenesulfonic acid, 4-chlorobenzenesulfonic acid, 2-naphthalenesulfonic acid, oxalic acid, 4-toluenesulfonic acid, camphorsulfonic acid, methanesulfonic acid, 4-methylbicyclo[2.2.2]-oct-2-ene-1-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like. Salts can also be formed when an acidic proton present in the parent compound is either replaced by a metal ion, *e.g.,* an alkali metal ion, an alkaline earth ion, or an aluminum ion; or coordinates with an organic base such as ethanolamine, diethanolamine, triethanolamine, trimethylamine, N-methylglucamine, and the like. Pharmaceutically acceptable salts are suitable for administration in a patient and possess desirable pharmacological properties. Suitable salts further include those described in P. Heinrich Stahl, Camille G. Wermuth (Eds.), Handbook of Pharmaceutical Salts Properties, Selection, and Use; 2002.

Unless indicated otherwise, the nomenclature of substituents that are not explicitly defined herein are arrived at by naming the terminal portion of the functionality followed by the adjacent functionality toward the point of attachment. For example, the substituent "arylalkyloxycabonyl" refers to the group (aryl)-(alkyl)-O-C(O)-.

It is understood that in all substituted groups defined above, polymers arrived at by defining substituents with further substituents to themselves (*e.g.,* substituted aryl having a substituted aryl group as a substituent which is itself substituted with a substituted aryl group, which is further substituted by a substituted aryl group, etc.) are not intended for inclusion herein. In such cases, the maximum number of such substitutions is three. For example, serial substitutions of substituted aryl groups with two other substituted aryl groups are limited to -substituted aryl-(substituted aryl)-substituted aryl.

Similarly, it is understood that the above definitions are not intended to include impermissible substitution patterns (*e.g.,* methyl substituted with 5 fluoro groups or heteroaryl groups having two adjacent oxygen ring atoms). Such impermissible substitution patterns are well known to the skilled artisan.

The terms "optional" or "optionally" as used throughout the specification means that the subsequently described event or circumstance may but need not occur, and that the description includes instances where the event or circumstance occurs and instances in which it does not. For example, "heterocyclo group optionally mono- or di-substituted with an alkyl group" means that the alkyl may but need not be present, and the description includes situations where the heterocyclo group is mono- or disubstituted with an alkyl group and situations where the heterocyclo group is not substituted with the alkyl group.

Turning next to the compositions of the invention, the term "pharmaceutically acceptable carrier or excipient" means a carrier or excipient that is useful in preparing a pharmaceutical composition that is generally safe, and possesses acceptable toxicities. Acceptable carriers or excipients include those that are acceptable for veterinary use as well as human pharmaceutical use. A "pharmaceutically acceptable carrier or excipient" as used in the specification and claims includes both one and more than one such carrier or excipient.

With reference to the methods of the present invention, the following terms are used with the noted meanings:

The terms "treating" or "treatment" of a disease includes:

(1) preventing or reducing the risk of developing the disease, *i.e.,* causing the clinical symptoms of the disease not to develop in a mammal that may be exposed to or predisposed to the disease but does not yet experience or display symptoms of the disease,
(2) inhibiting the disease, *i.e.,* arresting or reducing the development of the disease or its clinical symptoms, or
(3) relieving the disease, *i.e.,* causing regression of the disease or its clinical symptoms.

A preferred embodiment of the invention is treatment of a disease that consists of relieving the disease.

The term "diagnosing" refers to determining the presence or absence of a particular disease or condition. Additionally, the term refers to determining the level or severity of a particular disease or condition, as well as monitoring of the disease or condition to determine its response to a particular therapeutic regimen.

The term "therapeutically effective amount" means the amount of the subject compound that will elicit the biological or medical response of a tissue, system, animal or human that is being sought by the researcher, veterinarian, medical doctor or other clinician. "A therapeutically effective amount" includes the amount of a compound that, when administered to a mammal for treating a disease, is sufficient to effect such treatment for the disease. The "therapeutically effective amount" will vary depending on the compound, the disease and its severity and the age, weight, etc., of the mammal to be treated.

The term "mammal" includes, without limitation, humans, domestic animals (*e.g.,* dogs or cats), farm animals (cows, horses, or pigs), and laboratory animals (mice, rats, hamsters, guinea pigs, pigs, rabbits, dogs, or monkeys).

The term "insulin resistance" can be defined generally as a disorder of glucose metabolism. More specifically, insulin resistance can be defined as the diminished ability of insulin to exert its biological action across a broad range of concentrations producing less than the expected biologic effect (*see, e.g.,* Reaven GM, J. Basic & Clin. Phys. & Pharm. (1998) 9:387-406 and Flie J, Ann. Rev. Med. (1983) 34:145-60). Insulin resistant persons have a diminished ability to properly metabolize glucose and respond poorly, if at all, to insulin therapy. Manifestations of insulin resistance include insufficient insulin activation of glucose uptake, oxidation and storage in muscle and inadequate insulin repression of lipolysis in adipose tissue and of glucose production and secretion in liver. Insulin resistance can cause or contribute to polycystic ovarian syndrome, impaired glucose tolerance, gestational diabetes, metabolic syndrome, hypertension, obesity, atherosclerosis and a variety of other disorders. Eventually, the insulin resistant individuals can progress to a point where a diabetic state is reached.

The term "diabetes mellitus" or "diabetes" means a disease or condition that is generally characterized by metabolic defects in production and utilization of glucose that result in the failure to maintain appropriate blood sugar levels in the body. The result of these defects is elevated blood glucose, referred to as "hyperglycemia." Two major forms of diabetes are Type I diabetes and Type II diabetes. As described above, Type I diabetes is generally the result of an absolute deficiency of insulin, the hormone that regulates glucose utilization. Type II diabetes often occurs in the face of normal, or even elevated levels of insulin and can result from the inability of tissues to respond appropriately to insulin. Most Type II diabetic patients are insulin resistant and have a relative deficiency of insulin, in that insulin secretion can not compensate for the resistance of peripheral tissues to respond to insulin. In addition, many Type II diabetics are obese. Other types of disorders of glucose homeostasis include impaired glucose tolerance, which is a metabolic stage intermediate between normal glucose homeostasis and diabetes, and gestational diabetes mellitus, which is glucose intolerance in pregnancy in women with no previous history of Type I or Type II diabetes.

The term "metabolic syndrome" refers to a cluster of metabolic abnormalities including abdominal obesity, insulin resistance, glucose intolerance, diabetes, hypertension and dyslipidemia. These abnormalities are known to be

associated with an increased risk of vascular events.

The term "abdominal obesity" is defined by a cutoff point of waist circumference ≥ 102 cm in men and ≥ 80 cm in women, as recommended by the third report of the national cholesterol education program expert panel on detection, evaluation, and treatment of high blood cholesterol in adults (NCEP/ATP Panel III).

The guidelines for diagnosis of Type II diabetes, impaired glucose tolerance, and gestational diabetes have been outlined by the American Diabetes Association (*see, e.g.,* The Expert Committee on the Diagnosis and Classification of Diabetes Mellitus, Diabetes Care, (1999) Vol. 2 (Suppl 1):S5-19).

The term "secretagogue" means a substance or compound that stimulates secretion. For example, an insulin secretagogue is a substance or compound that stimulates secretion of insulin.

The term "symptom" of diabetes, includes, but is not limited to, polyuria, polydipsia, and polyphagia, as used herein, incorporating their common usage. For example, "polyuria" means the passage of a large volume of urine during a given period; "polydipsia" means chronic, excessive thirst; and "polyphagia" means excessive eating. Other symptoms of diabetes include, *e.g.,* increased susceptibility to certain infections (especially fungal and staphylococcal infections), nausea, and ketoacidosis (enhanced production of ketone bodies in the blood).

The term "complication" of diabetes includes, but is not limited to, microvascular complications and macrovascular complications. Microvascular complications are those complications that generally result in small blood vessel damage. These complications include, e.g., retinopathy (the impairment or loss of vision due to blood vessel damage in the eyes); neuropathy (nerve damage and foot problems due to blood vessel damage to the nervous system); and nephropathy (kidney disease due to blood vessel damage in the kidneys). Macrovascular complications are those complications that generally result from large blood vessel damage. These complications include, *e.g.,* cardiovascular disease and peripheral vascular disease. Cardiovascular disease refers to diseases of blood vessels of the heart. *See, e.g.,* Kaplan RM, et al., "Cardiovascular diseases" in Health and Human Behavior, pp. 206-242 (McGraw-Hill, New York 1993). Cardiovascular disease is generally one of several forms, including, *e.g.,* hypertension (also referred to as high blood pressure), coronary heart disease, stroke, and rheumatic heart disease. Peripheral vascular disease refers to diseases of any of the blood vessels outside of the heart. It is often a narrowing of the blood vessels that carry blood to leg and arm muscles.

The term "atherosclerosis" encompasses vascular diseases and conditions that are recognized and understood by physicians practicing in the relevant fields of medicine. Atherosclerotic cardiovascular disease, coronary heart disease (also known as coronary artery disease or ischemic heart disease), cerebrovascular disease and peripheral vessel disease are all clinical manifestations of atherosclerosis and are therefore encompassed by the terms "atherosclerosis" and "atherosclerotic disease".

The term "antihyperlipidemic" refers to the lowering of excessive lipid concentrations in blood to desired levels.

The term "modulate" or "modulating" refers to the treating, prevention, suppression, enhancement, or induction of a function or condition. For example, compounds can modulate Type II diabetes by increasing insulin in a human, thereby suppressing hyperglycemia. Compounds can also modulate GPR120 by acting as GPR120 agonists.

The term "triglyceride(s)" ("TGs"), as used herein, incorporates its common usage. TGs consist of three fatty acid molecules esterified to a glycerol molecule. TGs serve to store fatty acids that are used by muscle cells for energy production or are taken up and stored in adipose tissue.

Because cholesterol and TGs are water insoluble, they must be packaged in special molecular complexes known as "lipoproteins" in order to be transported in the plasma. Lipoproteins can accumulate in the plasma due to overproduction and/or deficient removal. There are at least five distinct lipoproteins differing in size, composition, density, and function. In the cells of the small intestine, dietary lipids are packaged into large lipoprotein complexes called "chylomicrons", which have a high TG and low-cholesterol content. In the liver, TG and cholesterol esters are packaged and released into plasma as TG-rich lipoprotein called very low density lipoprotein ("VLDL"), whose primary function is the endogenous transport of TGs made in the liver or released by adipose tissue. Through enzymatic action, VLDL can be either reduced and taken up by the liver, or transformed into intermediate density lipoprotein ("IDL"). IDL, is in turn, either taken up by the liver, or is further modified to form low density lipoprotein ("LDL"). LDL is either taken up and broken down by the liver, or is taken up by extrahepatic tissue. High density lipoprotein ("HDL") helps remove cholesterol from peripheral tissues in a process called reverse cholesterol transport.

The term "dyslipidemia" refers to abnormal levels of lipoproteins in blood plasma including both depressed and/or elevated levels of lipoproteins (e.g., elevated levels of LDL and/or VLDL, and depressed levels of HDL).

The term "hyperlipidemia" includes, but is not limited to, the following:

(1) *Familial Hyperchylomicronemia,* a rare genetic disorder that causes a deficiency in an enzyme, LP lipase, that breaks down fat molecules. The LP lipase deficiency can cause the accumulation of large quantities of fat or lipoproteins in the blood;

(2) *Familial Hypercholesterolemia,* a relatively common genetic disorder caused where the underlying defect is a series of mutations in the LDL receptor gene that result in malfunctioning LDL receptors and/or absence of the LDL

receptors. This brings about ineffective clearance of LDL by the LDL receptors resulting in elevated LDL and total cholesterol levels in the plasma;

(3) *Familial Combined Hyperlipidemia,* also known as multiple lipoprotein-type hyperlipidemia is an inherited disorder where patients and their affected first-degree relatives can at various times manifest high cholesterol and high triglycerides. Levels of HDL cholesterol are often moderately decreased;

(4) *Familial Defective Apolipoprotein B-100* is a relatively common autosomal dominant genetic abnormality. The defect is caused by a single nucleotide mutation that produces a substitution of glutamine for arginine, which can cause reduced affinity of LDL particles for the LDL receptor. Consequently, this can cause high plasma LDL and total cholesterol levels;

*(5) Familial Dysbetaliproteinemia,* also referred to as Type III Hyperlipoproteinemia, is an uncommon inherited disorder resulting in moderate to severe elevations of serum TG and cholesterol levels with abnormal apolipoprotein E function. HDL levels are usually normal; and

(6) *Familial Hypertriglyceridemia,* is a common inherited disorder in which the concentration of plasma VLDL is elevated. This can cause mild to moderately elevated TG levels (and usually not cholesterol levels) and can often be associated with low plasma HDL levels.

Risk factors for hyperlipidemia include, but are not limited to, the following: (1) disease risk factors, such as a history of Type I diabetes, Type II diabetes, Cushing's syndrome, hypothyroidism and certain types of renal failure; (2) drug risk factors, which include, birth control pills; hormones, such as estrogen, and corticosteroids; certain diuretics; and various β-blockers; (3) dietary risk factors include dietary fat intake per total calories greater than 40%; saturated fat intake per total calories greater than 10%; cholesterol intake greater than 300 mg per day; habitual and excessive alcohol use; and obesity.

The terms "obese" and "obesity" refers to, according to the World Health Organization, a Body Mass Index ("BMI") greater than 27.8 kg/$m^2$ for men and 27.3 kg/$m^2$ for women (BMI equals weight (kg)/height ($m^2$)). Obesity is linked to a variety of medical conditions including diabetes and hyperlipidemia. Obesity is also a known risk factor for the development of Type II diabetes (*see, e.g.,* Barrett-Conner E, Epidemol. Rev. (1989) 11:172-181; and Knowler, et al., Am. J. Clin. Nutr. (1991) 53:1543-1551).

The term "pancreas" refers to a gland organ in the digestive and endocrine system of vertebrates, including mammals. The pancreas secretes both digestive enzymes and hormones such as insulin, GLP-1 and GIP as well as other hormones.

The term "islet" or "islet of Langerhans" refers to endocrine cells of the pancreas that are grouped together in islets and secrete insulin and other hormones.

The term "beta cell" refers to cells found in the islet of Langerhans that secrete insulin, amylin, and other hormones.

The term "endocrine cell" refers to cells that secrete hormones into the blood stream. Endocrine cells are found various glands and organ systems of the body including the pancreas, intestines, and other organs.

The term "L cell" refers to gut endocrine cells that produce GLP-1.

The term "K cell" refers to gut endocrine cells that produce GIP.

The term "incretin" refers to a group of hormones that increases insulin secretion in response to food intake. Incretins include GLP-1 and GIP.

The term "insulin" refers to a polypeptide hormone that regulates glucose metabolism. Insulin binds to insulin receptors in insulin sensitive cells and mediates glucose uptake. Insulin is used to treat Type I diabetes and may be used to treat Type II diabetes.

The term "GLP-1" or "glucagon-like peptide" is a peptide hormone primarily produced by L cells. GLP-1 increases insulin secretion, decreases glucagon secretion, increases beta cell mass and insulin gene expression, inhibits acid secretion and gastric emptying in the stomach, and decreases food intake by increasing satiety.

The term "GIP" or "gastric inhibitory peptide" or "glucose dependent insulinotropic polypeptide" refers to a peptide hormone produced primarily by K cells. GIP stimulates insulin secretion. GIP also has significant effects on lipid metabolism.

The term "cAMP" or "cyclic AMP" or "cyclic adenosine monophosphate" refers to an intracellular signaling molecule involved in many biological processes, including glucose and lipid metabolism.

The term "agonist" refers to a compound that binds to a receptor and triggers a response in a cell. An agonist mimics the effect of an endogenous ligand, a hormone for example, and produces a physiological response similar to that produced by the endogenous ligand.

The term "partial agonist" refers to a compound that binds to a receptor and triggers a partial response in a cell. A partial agonist produces only a partial physiological response of the endogenous ligand.

Accordingly, in one embodiment provided is a compound of Formula (A)

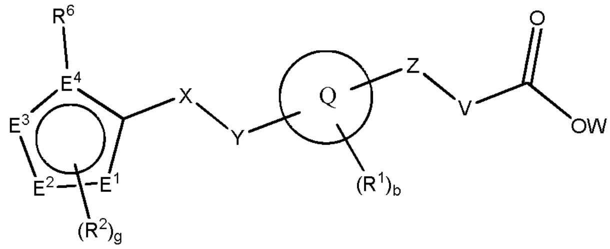

(A)

or a pha

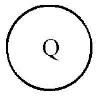

rmaceutically acceptable salt thereof, wherein: represents a 5-10 membered monocyclic or bicyclic aryl group, a 5-10 membered monocyclic or bicyclic heteroaryl group, a 5-10 membered monocyclic or bicyclic cycloalkyl group, a 5-10 membered monocyclic or bicyclic heterocycloalkyl group, or a 8-10 membered bicyclic group wherein an aryl or a 5-6 membered heteroaryl ring is fused to a 5-6 membered cycloalkyl or heterocycloalkyl ring;

| | |
|---|---|
| $E^1$, $E^2$ and $E^3$ | are independently selected from the group consisting of C, N and S; |
| $E^4$ | is selected from the group consisting of C and N; |
| X | is selected from the group consisting of $-CH_2-$, -C(O)- and $-C(O)CH_2-$; |
| Y | is selected from the group consisting of $-CH_2-$, -NH- and -O-; |
| Z | is selected from the group consisting of a $-(CR^4R^5)_n-$, -S-, -C(O)-and $-CR^4=CR^5-$; |
| V | is selected from the group consisting of a bond, $-(CR^4R^5)_n-$, $-CR^4=CR^5-$, and $-O-CR^4R^5-$; |
| W | is selected from the group consisting of H, $C_{1-6}$alkyl and substituted $C_{1-6}$alkyl; |
| $R^1$ | is independently selected from the group consisting of halo, $C_{1-6}$alkyl, substituted $C_{1-6}$alkyl, $C_{3-7}$cycloalkyl, substituted $C_{3-7}$cycloalkyl, $C_{2-6}$alkenyl, substituted $C_{2-6}$alkenyl, $C_{2-6}$alkynyl, substituted $C_{2-6}$alkynyl, CN, $-OR^a$, $-NR^aR^b$, $-C(O)R^a$, $-C(O)OR^a$, $-NR^aC(O)R^b$, $-SR^a$, $-S(O)R^a$ and $-S(O)_2R^a$; |
| $R^2$ | is independently selected from the group consisting of halo, $C_{1-6}$alkyl, substituted $C_{1-6}$alkyl, $C_{3-7}$cycloalkyl, substituted $C_{3-7}$cycloalkyl, $C_{2-6}$alkenyl, substituted $C_{2-6}$alkenyl, $C_{2-6}$alkynyl, substituted $C_{2-6}$alkynyl, aryloxy,-substituted aryloxy, CN, $-OR^a$, $-NR^aR^b$, $-C(O)R^a$, $-C(O)OR^a$, $-C(O)NR^aR^b$, $-NR^aC(O)R^b$, $-SR^a$, $-S(O)R^a$ and $-S(O)_2R^a$; each of $R^a$ and $R^b$ is independently selected from the group consisting of H, $C_{1-6}$alkyl, substituted $C_{1-6}$alkyl, $C_{3-7}$cycloalkyl, $C_{2-6}$alkenyl and $C_{2-6}$alkynyl; |
| $R^4$ | is independently selected from the group consisting of H, halo, $C_{1-6}$alkyl, substituted $C_{1-6}$alkyl, $C_{1-6}$alkoxy and substituted $C_{1-6}$alkoxy; |
| $R^5$ | is independently selected from the group consisting of H, halo, $C_{1-6}$alkyl, substituted $C_{1-6}$alkyl and $C_{1-6}$alkoxy; |
| $R^6$ | is selected from the group consisting of halo, $C_{1-6}$alkyl, substituted $C_{1-6}$alkyl, $C_{1-6}$alkoxy, substituted $C_{1-6}$alkoxy, aryl, substituted aryl, heteroaryl and substituted heteroaryl; |

optionally $R^4$ and $R^5$ cyclize to form a $C_{3-7}$saturated ring or a spiro $C_{3-7}$cycloalkyl group;
the subscript b is 0, 1, 2, 3 or 4;
the subscript g is 0, 1 or 2; and
the subscript n is independently 1, 2 or 3.

In some aspects, X is $-CH_2-$; Y is -O-; Z is $-(CR^4R^5)_n$; and V is a bond. In further aspects, Q is selected from the group consisting of

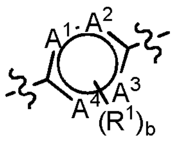, 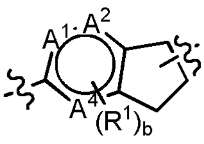, 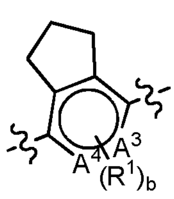 and 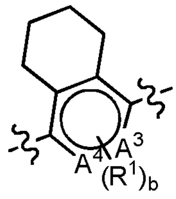

wherein $A^1$, $A^2$, $A^3$ and $A^4$ are independently selected from the group consisting of C and N, with the proviso that only 0, 1 or 2 of $A^1$, $A^2$, $A^3$ and $A^4$ is N and $R^1$ and subscript b are as previously defined.

In one embodiment, provided is a compound of Formula (B)

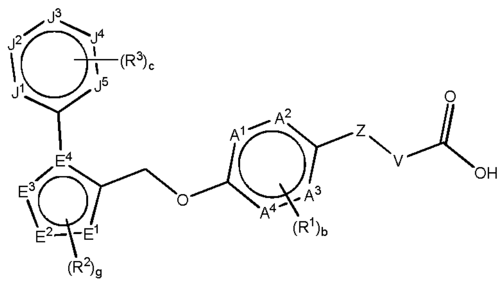

(B)

or a pharmaceutically acceptable salt thereof, wherein:

| | |
|---|---|
| $A^1$, $A^2$, $A^3$ and $A^4$ | are independently selected from the group consisting of C and N, with the proviso that only 0, 1 or 2 of $A^1$, $A^2$, $A^3$ and $A^4$ is N; |
| $J^1$, $J^2$, $J^3$, $J^4$ and $J^5$ | are independently selected from the group consisting ofN and C, with the proviso that only 0, 1 or 2 of $J^1$, $J^2$, $J^3$, $J^4$ and $J^5$ is N; |
| each $R^3$ | is independently selected from the group consisting of halo, $C_{1-6}$alkyl, substituted $C_{1-6}$alkyl, $C_{3-7}$cycloalkyl, substituted $C_{3-7}$cycloalkyl, $C_{2-6}$alkenyl, substituted $C_{2-6}$alkenyl, $C_{2-6}$alkynyl, substituted $C_{2-6}$alkynyl, cyano, $-OR^a$, $-NR^aR^b$, $-C(O)R^a$, $-C(O)OR^a$, $-C(O)NR^aR^b$, $-NR^aC(O)R^b$, $-SR^a$, $-S(O)R^a$ and $-S(O)_2R^a$; |

the subscript c is 0, 1, 2 or 3; and
$E^1$, $E^2$, $E^3$, $E^4$, $R^1$, $R^2$, Z, V, $R^a$, $R^b$, subscript b and subscript g are as previously defined.

In some aspects, Z and V taken together are selected from the group consisting of

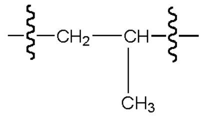, 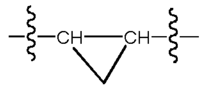 and 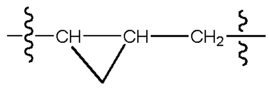.

In some aspects, $E^1$ and $E^2$ are both C; and $E^3$ and $E^4$ are both N. In some such aspects, $A^1$, $A^2$, $A^3$ and $A^4$ are all C. In other aspects $J^1$, $J^2$, $J^3$, $J^4$ and $J^5$ are all C. In further aspects, the subscript g is 0 or 1; the subscript c is 0 or 1; and the subscript b is 0, 1 or 2. In other aspects, $R^1$ is halo, $C_{1-3}$alkyl or $CF_3$; g is 0 or g is 1 and $R^2$ is $CH_3$; and $R^3$ is $C_{1-4}$alkyl or halo.

In one embodiment, provided is a compound of Formula (C)

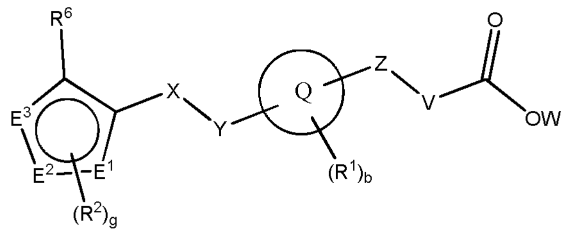

(C)

or a pharmaceutically acceptable salt thereof, wherein:

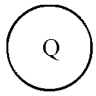

represents a 5-10 membered monocyclic or bicyclic aryl group, a 5-10 membered monocyclic or bicyclic heteroaryl group, a 5-10 membered monocyclic or bicyclic cycloalkyl group, a 5-10 membered monocyclic or bicyclic heterocycloalkyl group, or a 8-10 membered bicyclic group wherein an aryl or a 5-6 membered heteroaryl ring is fused to a 5-6 membered cycloalkyl or heterocycloalkyl ring;

| | |
|---|---|
| $E^1$, $E^2$ and $E^3$ | are independently selected from the group consisting of C, N and O; |
| X | is selected from the group consisting of $-CH_2-$ and $-C(O)CH_2-$; |
| Y | is selected from the group consisting of $-CH_2-$ and -O-; |
| Z | is selected from the group consisting of $-(CR^4R^5)_n-$, -S-, -C(O)-, and $-CR^4=CR^5-$; |
| V | is selected from the group consisting of a bond, $-(CR^4R^5)_n-$, $-CR^4=CR^5-$, and $-O-CR^4R^5-$; |
| W | is selected from the group consisting of H, $C_{1-6}$alkyl and substituted $C_{1-6}$alkyl; |
| $R^1$ | is independently selected from the group consisting of halo, $C_{1-6}$alkyl, substituted $C_{1-6}$alkyl, $C_{3-7}$cycloalkyl, substituted $C_{3-7}$cycloalkyl, $C_{2-6}$alkenyl, substituted $C_{2-6}$alkenyl, $C_{2-6}$alkynyl, substituted $C_{2-6}$alkynyl, CN, $-OR^a$, $-NR^aR^b$, $-C(O)R^a$, $-C(O)OR^a$, $-NR^aC(O)R^b$, $-SR^a$, $-S(O)R^a$ and $-S(O)_2R^a$; |
| $R^2$ | is independently selected from the group consisting of halo, $C_{1-6}$alkyl, substituted $C_{1-6}$alkyl, $C_{3-7}$cycloalkyl, substituted $C_{3-7}$cycloalkyl, $C_{2-6}$alkenyl, substituted $C_{2-6}$alkenyl, $C_{2-6}$alkynyl, substituted $C_{2-6}$alkynyl, aryloxy,-substituted aryloxy, CN, $-OR^a$, $-NR^aR^b$, $-C(O)R^a$, $-C(O)OR^a$, $-C(O)NR^aR^b$, $-NR^aC(O)R^b$, $-SR^a$, $-S(O)R^a$ and $-S(O)_2R^a$; |
| each of $R^a$ and $R^b$ | is independently selected from the group consisting of H, $C_{1-6}$alkyl, substituted $C_{1-6}$alkyl, $C_{3-7}$cycloalkyl, $C_{2-6}$alkenyl and $C_{2-6}$alkynyl; |
| $R^4$ | is independently selected from the group consisting of H, halo, $C_{1-6}$alkyl, substituted $C_{1-6}$alkyl, $C_{1-6}$alkoxy and substituted $C_{1-6}$alkoxy; |
| $R^5$ | is independently selected from the group consisting of H, halo, $C_{1-6}$alkyl, substituted $C_{1-6}$alkyl and $C_{1-6}$alkoxy; optionally $R^4$ and $R^5$ cyclize to form a $C_{3-7}$saturated ring or a spiro $C_{3-7}$cycloalkyl group; |
| $R^6$ | is selected from the group consisting of aryl, substituted aryl, heteroaryl and substituted heteroaryl; |

the subscript b is 0, 1, 2, 3 or 4;
the subscript g is 0, 1, 2 or 3 and;
the subscript n is independently 0, 1 or 2;
with the proviso that the compound is not methyl 3-(4-((3-(2,6-dichlorophenyl)-5-isopropylisoxazol-4-yl)methoxy)phenyl)propanoate, 3-(4-((3-(2-chloro-6-methylphenyl)-5-isopropylisoxazol-4-yl)methoxy)phenyl)propanoic acid, 3-(4-((3-(2,6-dichlorophenyl)-5-isopropylisoxazol-4-yl)methoxy)phenyl)propanoic acid, ethyl 3-(4-((4-(3-chlorophenyl)-2-(trifluoromethyl)furan-3-yl)methoxy)-2-methylphenyl)propanoate and 3-(3-fluoro-5-methyl-4-((3-methyl-5-phenylisoxazol-4-yl)methoxy)phenyl)propanoic acid.

In some aspects, X is $-CH_2-$; Y is -O-; Z is $-(CR^4R^5)_n$ ; and V is a bond. In some such aspects, Q is selected from the group consisting of

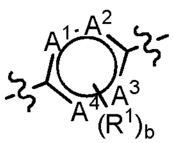 , 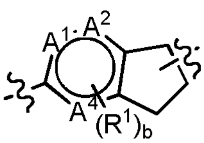 , 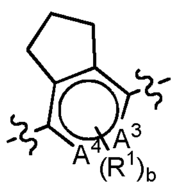 and 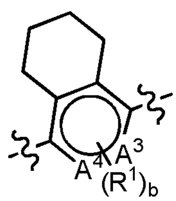

wherein $A^1$, $A^2$, $A^3$ and $A^4$ are independently selected from the group consisting of C and N, with the proviso that only 0, 1 or 2 of $A^1$, $A^2$, $A^3$ and $A^4$ is N and $R^1$ and subscript b are as previously defined.

In one embodiment, provided is a compound of Formula (D)

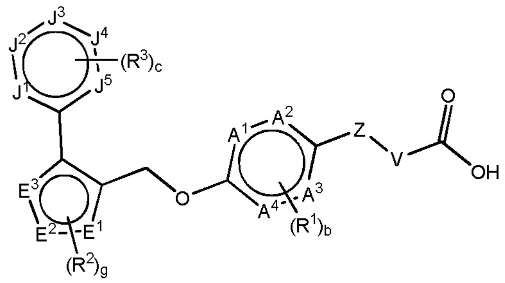

(D)

or a pharmaceutically acceptable salt thereof, wherein:

$A^1$, $A^2$, $A^3$ and $A^4$ are independently selected from the group consisting of C and N, with the proviso that only 0, 1 or 2 of $A^1$, $A^2$, $A^3$ and $A^4$ is N;
$J^1$, $J^2$, $J^3$, $J^4$ and $J^5$ are independently selected from the group consisting ofN and C, with the proviso that only 0, 1 or 2 of $J^1$, $J^2$, $J^3$, $J^4$ and $J^5$ is N;
each $R^3$ is independently selected from the group consisting of halo, $C_{1-6}$alkyl, substituted $C_{1-6}$alkyl, $C_{3-7}$cycloalkyl, substituted $C_{3-7}$cycloalkyl, $C_{2-6}$alkenyl, substituted $C_{2-6}$alkenyl, $C_{2-6}$alkynyl, substituted $C_{2-6}$alkynyl, cyano, $-OR^a$, $-NR^aR^b$, $-C(O)R^a$, $-C(O)OR^a$, $-C(O)NR^aR^b$, $-NR^aC(O)R^b$, $-SR^a$, $-S(O)R^a$ and $-S(O)_2R^a$;

the subscript c is 0, 1, 2 or 3; and
$E^1$, $E^2$, $E^3$, $R^1$, $R^2$, Z, V, $R^a$, $R^b$, subscript b and subscript g are as previously defined.

In some aspects, Z and V taken together are selected from the group consisting of

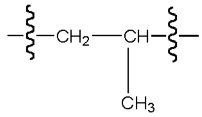 , 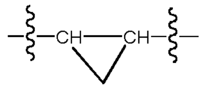 and 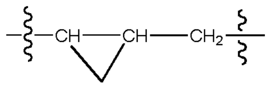 .

In some aspects, $E^1$ is C and one of $E^2$ and $E^3$ is N and the other of $E^2$ and $E^3$ is O or N.

In some aspects, $A^1$, $A^2$, $A^3$ and $A^4$ are all C. In further aspects, $J^1$, $J^2$, $J^3$, $J^4$ and $J^5$ are all C.

In some aspects, the subscript g is 0 or 1; the subscript c is 0 or 1 and the subscript b is 0, 1 or 2.

In some aspects, $R^1$ is halo, $C_{1-3}$alkyl or $CF_3$; g is 0 or g is 1 and $R^2$ is $CH_3$; and $R^3$ is $C_{1-4}$alkyl or halo.

In one embodiment, provided is a compound of Formula (I)

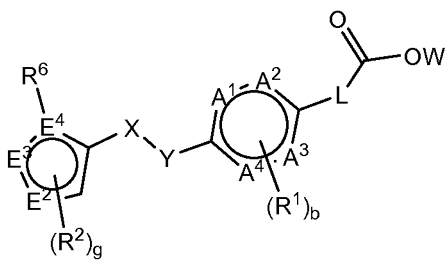

(I)

or a pharmaceutically acceptable salt thereof, wherein:

$A^1$, $A^2$, $A^3$, and $A^4$ are independently selected from the group consisting of N and C, with the proviso that only 0, 1, or 2 of $A^1$, $A^2$, $A^3$, and $A^4$ is N;
$E^4$ is C or N;
$E^2$ and $E^3$ are independently selected from the group consisting of C, N, and S, provided that $E^2$ and $E^3$ are not both C or both S;

one of X and Y is $CH_2$ and the other of X and Y is selected from the group consisting of $CH_2$, -NH-, -O-, -S-, -S(O)-, and $-S(O)_2-$;
L is $-(CR^4R^5)_n-$ wherein optionally one $-(CR^4R^5)-$ is replaced with -O- or -S-;

W is selected from the group consisting of H, $C_{1-10}$ alkyl, and substituted $C_{1-10}$ alkyl;
the subscript b is 0, 1, or 2;
the subscript g is 0, 1, or 2;
the subscript n is 0, 1, 2, 3, or 4;
$R^1$ is independently selected from the group consisting of halo, $C_{1-10}$ alkyl, substituted $C_{1-10}$ alkyl, $C_{3-7}$ cycloalkyl, substituted $C_{3-7}$ cycloalkyl, $C_{2-10}$ alkenyl, substituted $C_{2-10}$ alkenyl, $C_{2-10}$ alkynyl, substituted $C_{2-10}$ alkynyl, CN, $-OR^a$, $-NR^aR^b$, $-C(O)R^a$, $-C(O)OR^a$, $-C(O)NR^aR^b$, $-NR^aC(O)R^b$, $-SR^a$, $-S(O)R^a$, and $-S(O)_2R^a$;
$R^2$ is independently selected from the group consisting of halo, $C_{1-10}$ alkyl, substituted $C_{1-10}$ alkyl, $C_{3-7}$ cycloalkyl, substituted $C_{3-7}$ cycloalkyl, $C_{2-10}$ alkenyl, substituted $C_{2-10}$ alkenyl, $C_{2-10}$ alkynyl, substituted $C_{2-10}$ alkynyl, CN, $-OR^a$, $-NR^aR^b$, $-C(O)R^a$, $-C(O)OR^a$, $-C(O)NR^aR^b$, $-NR^aC(O)R^b$, $-SR^a$, $-S(O)R^a$, and $-S(O)_2R^a$;
each of $R^a$ and $R^b$ is independently selected from the group consisting of H, $C_{1-10}$ alkyl, substituted $C_{1-10}$ alkyl, $C_{3-10}$ cycloalkyl, $C_{2-10}$ alkenyl, and $C_{2-10}$ alkynyl;
$R^4$ is independently selected from the group consisting of H, fluoro, $C_{1-6}$ alkyl, substituted $C_{1-6}$ alkyl, and $C_{1-6}$ alkoxy;
$R^5$ is independently selected from the group consisting of H, fluoro, $C_{1-6}$ alkyl, substituted $C_{1-6}$ alkyl, and $C_{1-6}$ alkoxy; optionally one of $R^4$ and one of $R^5$ cyclize to form a $C_{3-6}$ saturated ring; and
$R^6$ is selected from the group consisting of $C_{1-6}$ alkyl, substituted $C_{1-6}$ alkyl, $C_{1-6}$ alkoxy, substituted $C_{1-6}$ alkoxy, aryl, substituted aryl, heteroaryl, and substituted heteroaryl;

provided that when n is 0, X is $CH_2$, Y is O, and $R^6$ is 2-chlorophenyl, then $R^2$ is not 3-trifluoromethyl; and provided that the compound is not 2-{[4-({[1-(2-chlorophenyl)-3-(trifluoromethyl)-1H-pyrazol-5-yl]methyl}oxy)phenyl]oxy}propanoic acid.

In some aspects, $E^2$ is C. In further aspects, $E^3$ and $E^4$ are N.

In one embodiment, provided is a compound of Formula (II)

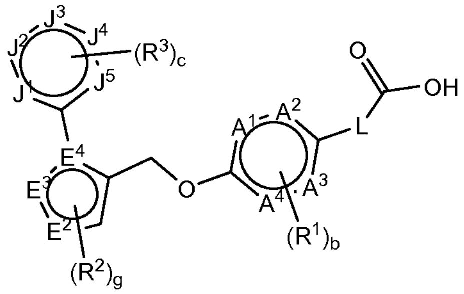

(II)

or a pharmaceutically acceptable salt thereof, wherein:

$J^1$, $J^2$, $J^3$, $J^4$, and $J^5$ are independently selected from the group consisting ofN and C, with the proviso that only 0, 1, or 2 of $J^1$, $J^2$, $J^3$, $J^4$, and $J^5$ is N; the subscript c is 0, 1, 2, or 3;

$R^3$ is independently selected from the group consisting of halo, $C_{1-10}$ alkyl, substituted $C_{1-10}$ alkyl, $C_{3-7}$ cycloalkyl, substituted $C_{3-7}$ cycloalkyl, $C_{2-10}$ alkenyl, substituted $C_{2-10}$ alkenyl, $C_{2-10}$ alkynyl, substituted $C_{2-10}$ alkynyl, CN, $-OR^a$, $-NR^aR^b$, $-C(O)R^a$, $-C(O)OR^a$, $-C(O)NR^aR^b$, $-NR^aC(O)R^b$, $-SR^a$, $-S(O)R^a$, and $-S(O)_2R^a$; and

$R^a$, $R^b$, $A^1$, $A^2$, $A^3$, $A^4$, $E^2$, $E^3$, $E^4$, L, $R^1$, $R^2$, subscript b, and subscript g are as previously defined for Formula (I).

In one embodiment, provided is a compound of Formula (III)

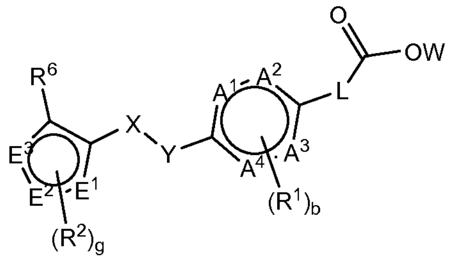

(III)

or a pharmaceutically acceptable salt thereof, wherein:

$A^1$, $A^2$, $A^3$, and $A^4$ are independently selected from the group consisting of N and C, with the proviso that only 0, 1, or 2 of $A^1$, $A^2$, $A^3$, and $A^4$ is N;
one of $E^1$, $E^2$, and $E^3$ is N or S and the other of $E^1$, $E^2$, and $E^3$ is independently C or N;

one of X and Y is $CH_2$ and the other of X and Y is selected from the group consisting of $CH_2$, -NH-, -O-, -S-, -S(O)-, and $-S(O)_2-$;
L is $-(CR^4R^5)_n-$ wherein optionally one $-(CR^4R^5)-$ is replaced with -O- or -S-;
W is selected from the group consisting of H, $C_{1-10}$ alkyl, and substituted $C_{1-10}$ alkyl;

the subscript b is 0, 1, or 2;
the subscript g is 0, 1, or 2;
the subscript n is 0, 1, 2, 3, or 4
$R^1$ is independently selected from the group consisting of halo, $C_{1-10}$ alkyl, substituted $C_{1-10}$ alkyl, $C_{3-7}$ cycloalkyl, substituted $C_{3-7}$ cycloalkyl, $C_{2-10}$ alkenyl, substituted $C_{2-10}$ alkenyl, $C_{2-10}$ alkynyl, substituted $C_{2-10}$ alkynyl, CN, $-OR^a$, $-NR^aR^b$, $-C(O)R^a$, $-C(O)OR^a$, $-C(O)NR^aR^b$, $-NR^aC(O)R^b$, $-SR^a$, $-S(O)R^a$, and $-S(O)_2R^a$;
$R^2$ is independently selected from the group consisting of halo, $C_{1-10}$ alkyl, substituted $C_{1-10}$ alkyl, $C_{3-7}$ cycloalkyl, substituted $C_{3-7}$ cycloalkyl, $C_{2-10}$ alkenyl, substituted $C_{2-10}$ alkenyl, $C_{2-10}$ alkynyl, substituted $C_{2-10}$ alkynyl, CN, $-OR^a$, $-NR^aR^b$, $-C(O)R^a$, $-C(O)OR^a$, $-C(O)NR^aR^b$, $-NR^aC(O)R^b$, $-SR^a$, $-S(O)R^a$, and $-S(O)_2R^a$;
each of $R^a$ and $R^b$ is independently selected from the group consisting of H, $C_{1-10}$ alkyl, substituted $C_{1-10}$ alkyl, $C_{3-10}$ cycloalkyl, $C_{2-10}$ alkenyl, and $C_{2-10}$ alkynyl;
$R^4$ is independently selected from the group consisting of H, fluoro, $C_{1-6}$ alkyl, substituted $C_{1-6}$ alkyl, and $C_{1-6}$ alkoxy;
$R^5$ is independently selected from the group consisting of H, fluoro, $C_{1-6}$ alkyl, substituted $C_{1-6}$ alkyl, and $C_{1-6}$ alkoxy; optionally one of $R^4$ and one of $R^5$ cyclize to form a $C_{3-6}$ saturated ring; and
$R^6$ is selected from the group consisting of $C_{1-6}$ alkyl, substituted $C_{1-6}$ alkyl, $C_{1-6}$ alkoxy, substituted $C_{1-6}$ alkoxy, aryl, substituted aryl, heteroaryl, and substituted heteroaryl.

In one embodiment, provided is a compound of Formula (IV)

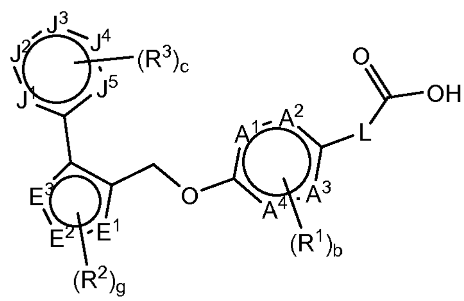

(IV)

or a pharmaceutically acceptable salt thereof, wherein:

$J^1$, $J^2$, $J^3$, $J^4$, and $J^5$ are independently selected from the group consisting ofN and C, with the proviso that only 0, 1, or 2 of $J^1$, $J^2$, $J^3$, $J^4$, and $J^5$ is N;

the subscript c is 0, 1, 2, or 3; and

$R^3$ is independently selected from the group consisting of halo, $C_{1-10}$ alkyl, substituted $C_{1-10}$ alkyl, $C_{3-7}$ cycloalkyl, substituted $C_{3-7}$ cycloalkyl, $C_{2-10}$ alkenyl, substituted $C_{2-10}$ alkenyl, $C_{2-10}$ alkynyl, substituted $C_{2-10}$ alkynyl, CN, $-OR^a$, $-NR^aR^b$, $-C(O)R^a$, $-C(O)OR^a$, $-C(O)NR^aR^b$, $-NR^aC(O)R^b$, $-SR^a$, $-S(O)R^a$, and $-S(O)_2R^a$; and

$R^a$, $R^b$, $A^1$, $A^2$, $A^3$, $A^4$, $E^1$, $E^2$, $E^3$, L, $R^1$, $R^2$, subscript b, and subscript g are as previously defined for Formula (III).

In some aspects, $E^1$ is S. In further aspects, $E^2$ and $E^3$ are N.

In other aspects, $E^1$ is S and $E^3$ is N.

In one embodiment, provided is a compound of Formula (V)

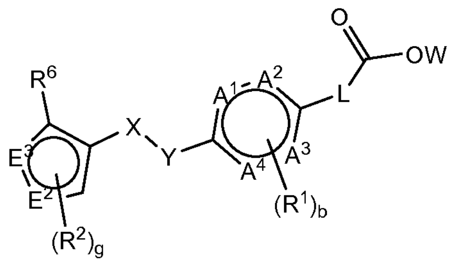

(V)

or a pharmaceutically acceptable salt thereof, wherein:

$A^1$, $A^2$, $A^3$, and $A^4$ are independently selected from the group consisting of N and C, with the proviso that only 0, 1, or 2 of $A^1$, $A^2$, $A^3$, and $A^4$ is N;

one of $E^2$ and $E^3$ is N and the other of $E^2$ and $E^3$ is O, N, or S;
one of X and Y is $CH_2$ and the other of X and Y is selected from the group consisting of $CH_2$, -NH-, -O-, -S-, -S(O)-, and $-S(O)_2-$;

L is $-(CR^4R^5)_n-$ wherein optionally one $-(CR^4R^5)-$ is replaced with -O- or -S-;
W is selected from the group consisting of H, $C_{1-10}$ alkyl, and substituted $C_{1-10}$ alkyl;
the subscript b is 0, 1, or 2;
the subscript g is 0, 1, or 2;
the subscript n is 0, 1, 2, 3, or 4
$R^1$ is independently selected from the group consisting of halo, $C_{1-10}$ alkyl, substituted $C_{1-10}$ alkyl, $C_{3-7}$ cycloalkyl, substituted $C_{3-7}$ cycloalkyl, $C_{2-10}$ alkenyl, substituted $C_{2-10}$ alkenyl, $C_{2-10}$ alkynyl, substituted $C_{2-10}$ alkynyl, CN, $-OR^a$, $-NR^aR^b$, $-C(O)R^a$, $-C(O)OR^a$, $-C(O)NR^aR^b$, $-NR^aC(O)R^b$, $-SR^a$, $-S(O)R^a$, and $-S(O)_2R^a$;
$R^2$ is independently selected from the group consisting of halo, $C_{1-10}$ alkyl, substituted $C_{1-10}$ alkyl, $C_{3-7}$ cycloalkyl, substituted $C_{3-7}$ cycloalkyl, $C_{2-10}$ alkenyl, substituted $C_{2-10}$ alkenyl, $C_{2-10}$ alkynyl, substituted $C_{2-10}$ alkynyl, CN, $-OR^a$, $-NR^aR^b$, $-C(O)R^a$, $-C(O)OR^a$, $-C(O)NR^aR^b$, $-NR^aC(O)R^b$, $-SR^a$, $-S(O)R^a$, and $-S(O)_2R^a$;
each of $R^a$ and $R^b$ is independently selected from the group consisting of H, $C_{1-10}$ alkyl, substituted $C_{1-10}$ alkyl, $C_{3-10}$ cycloalkyl, $C_{2-10}$ alkenyl, and $C_{2-10}$ alkynyl;
$R^4$ is independently selected from the group consisting of H, fluoro, $C_{1-6}$ alkyl, substituted $C_{1-6}$ alkyl, and $C_{1-6}$ alkoxy;
$R^5$ is independently selected from the group consisting of H, fluoro, $C_{1-6}$ alkyl, substituted $C_{1-6}$ alkyl, and $C_{1-6}$ alkoxy; optionally one of $R^4$ and one of $R^5$ cyclize to form a $C_{3-6}$ saturated ring; and
$R^6$ is selected from the group consisting of $C_{1-6}$ alkyl, substituted $C_{1-6}$ alkyl, $C_{1-6}$ alkoxy, substituted $C_{1-6}$ alkoxy, aryl, substituted aryl, heteroaryl, and substituted heteroaryl;

provided that the compound is not 3-(3-fluoro-5-methyl-4-((3-methyl-5-phenylisoxazol-4-yl)methoxy)phenyl)propanoic acid, 3-(4-((3-(2-chloro-6-methylphenyl)-5-isopropylisoxazol-4-yl)methoxy)phenyl)propanoic acid, or 3-(4-((3-(2,6-dichlorophenyl)-5-isopropylisoxazol-4-yl)methoxy)phenyl)propanoic acid.

In one embodiment, provided is a compound of Formula (VI)

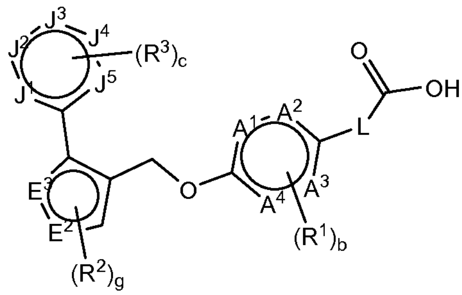

(VI)

or a pharmaceutically acceptable salt thereof, wherein:

| | |
|---|---|
| $J^1$, $J^2$, $J^3$, $J^4$, and $J^5$ | are independently selected from the group consisting ofN and C, with the proviso that only 0, 1, or 2 of $J^1$, $J^2$, $J^3$, $J^4$, and $J^5$ is N;<br>the subscript c is 0, 1, 2, or 3; |
| $R^3$ | is independently selected from the group consisting of halo, $C_{1-10}$ alkyl, substituted $C_{1-10}$ alkyl, $C_{3-7}$ cycloalkyl, substituted $C_{3-7}$ cycloalkyl, $C_{2-10}$ alkenyl, substituted $C_{2-10}$ alkenyl, $C_{2-10}$ alkynyl, substituted $C_{2-10}$ alkynyl, CN, $-OR^a$, $-NR^aR^b$, $-C(O)R^a$, $-C(O)OR^a$, $-C(O)NR^aR^b$, $-NR^aC(O)R^b$, $-SR^a$, $-S(O)R^a$, and $-S(O)_2R^a$; and |
| $R^a$, $R^b$, $A^1$, $A^2$, $A^3$, $A^4$, $E^2$, $E^3$, L, $R^1$, $R^2$, subscript b, and subscript g | are as previously defined for Formula (V). |

In some aspects, $E^2$ is O.

In some aspects, $E^3$ is N.

In some aspects, $E^2$ is O and and $E^3$ is N.

In one embodiment, provided is a compound of Formula (VII)

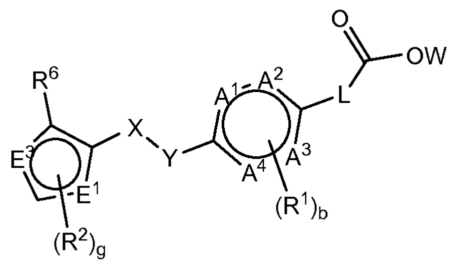

(VII)

or a pharmaceutically acceptable salt thereof, wherein:

| | |
|---|---|
| $A^1$, $A^2$, $A^3$, and $A^4$ | are independently selected from the group consisting ofN and C, with the proviso that only 0, 1, or 2 of $A^1$, $A^2$, $A^3$ and $A^4$ is N;<br>one of $E^1$ and $E^3$ is N and the other of $E^1$ and $E^3$ is O or S; |
| one of X and Y | is $CH_2$ and the other of X and Y is selected from the group consisting of $CH_2$, -NH-, -O-, -S-, -S(O)-, and $-S(O)_2-$; |
| L | is $-(CR^4R^5)_n-$ wherein optionally one $-(CR^4R^5)-$ is replaced with -O- or -S-; |
| W | is selected from the group consisting of H, $C_{1-10}$ alkyl, and substituted $C_{1-10}$ alkyl;<br>the subscript b is 0, 1, or 2;<br>the subscript g is 0, 1, or 2;<br>the subscript n is 0, 1, 2, 3, or 4;<br>$R^1$ is independently selected from the group consisting of halo, $C_{1-10}$ alkyl, substituted $C_{1-10}$ alkyl, $C_{3-7}$ cycloalkyl, substituted $C_{3-7}$ cycloalkyl, $C_{2-10}$ alkenyl, substituted $C_{2-10}$ alkenyl, $C_{2-10}$ alkynyl, substituted $C_{2-10}$ alkynyl, CN, $-OR^a$, $-NR^aR^b$, $-C(O)R^a$, $-C(O)OR^a$, $-C(O)NR^aR^b$, $-NR^aC(O)R^b$, $-SR^a$, $-S(O)R^a$, and $-S(O)_2R^a$; |

$R^2$ is independently selected from the group consisting of halo, $C_{1-10}$ alkyl, substituted $C_{1-10}$ alkyl, $C_{3-7}$ cycloalkyl, substituted $C_{3-7}$ cycloalkyl, $C_{2-10}$ alkenyl, substituted $C_{2-10}$ alkenyl, $C_{2-10}$ alkynyl, substituted $C_{2-10}$ alkynyl, CN, $-OR^a$, $-NR^aR^b$, $-C(O)R^a$, $-C(O)OR^a$, $-C(O)NR^aR^b$, $-NR^aC(O)R^b$, $-SR^a$, $-S(O)R^a$, and $-S(O)_2R^a$;

each of $R^a$ and $R^b$ is independently selected from the group consisting of H, $C_{1-10}$ alkyl, substituted $C_{1-10}$ alkyl, $C_{3-10}$ cycloalkyl, $C_{2-10}$ alkenyl, and $C_{2-10}$ alkynyl;

$R^4$ is independently selected from the group consisting of H, fluoro, $C_{1-6}$ alkyl, substituted $C_{1-6}$ alkyl, and $C_{1-6}$ alkoxy;

$R^5$ is independently selected from the group consisting of H, fluoro, $C_{1-6}$ alkyl, substituted $C_{1-6}$ alkyl, and $C_{1-6}$ alkoxy; optionally one of $R^4$ and one of $R^5$ cyclize to form a $C_{3-6}$ saturated ring; and

$R^6$ is selected from the group consisting of $C_{1-6}$ alkyl, substituted $C_{1-6}$ alkyl, $C_{1-6}$ alkoxy, substituted $C_{1-6}$ alkoxy, aryl, substituted aryl, heteroaryl, and substituted heteroaryl.

In one embodiment, provided is a compound of Formula (VIII)

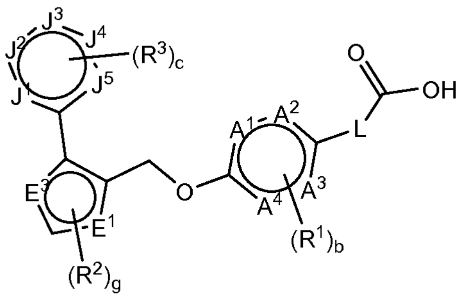

(VIII)

or a pharmaceutically acceptable salt thereof, wherein:
or a pharmaceutically acceptable salt thereof, wherein:

$J^1$, $J^2$, $J^3$, $J^4$, and $J^5$ are independently selected from the group consisting ofN and C, with the proviso that only 0, 1, or 2 of $J^1$, $J^2$, $J^3$, $J^4$, and $J^5$ is N;

the subscript c is 0, 1, 2, or 3; and

$R^3$ is independently selected from the group consisting of halo, $C_{1-10}$ alkyl, substituted $C_{1-10}$ alkyl, $C_{3-7}$ cycloalkyl, substituted $C_{3-7}$ cycloalkyl, $C_{2-10}$ alkenyl, substituted $C_{2-10}$ alkenyl, $C_{2-10}$ alkynyl, substituted $C_{2-10}$ alkynyl, CN, $-OR^a$, $-NR^aR^b$, $-C(O)R^a$, $-C(O)OR^a$, $-C(O)NR^aR^b$, $-NR^aC(O)R^b$, $-SR^a$, $-S(O)R^a$, and $-S(O)_2R^a$; and

$R^a$, $R^b$, $A^1$, $A^2$,

$A^3$, $A^4$, $E^1$,

$E^3$, L, $R^1$, $R^2$, subscript b, and subscript g are as previously defined for Formula (VII).

In some aspects, $E^1$ is S. In further aspects, $E^3$ is N.
In some aspects, $E^1$ is N. In further aspects, $E^3$ is S.
In one embodiment, provided is a compound of Formula (IX)

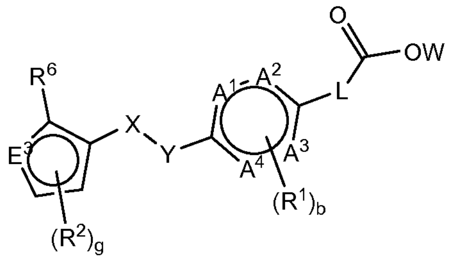

(IX)

or a pharmaceutically acceptable salt thereof, wherein:

| | |
|---|---|
| $A^1$, $A^2$, $A^3$, and $A^4$ | are independently selected from the group consisting of N and C, with the proviso that only 0, 1, or 2 of $A^1$, $A^2$, $A^3$, and $A^4$ is N; |
| $E^3$ | is selected from the group consisting of O, N, and S; |
| one of X and Y | is $CH_2$ and the other of X and Y is selected from the group consisting of $CH_2$, -NH-, -O-, -S-, -S(O)-, and $-S(O)_2-$; |
| L | is $-(CR^4R^5)_n-$ wherein optionally one $-(CR^4R^5)-$ is replaced with -O- or -S-; |
| W | is selected from the group consisting of H, $C_{1-10}$ alkyl, and substituted $C_{1-10}$ alkyl; |

the subscript b is 0, 1, or 2;
the subscript g is 0, 1, or 2;
the subscript n is 0, 1, 2, 3, or 4;
$R^1$ is independently selected from the group consisting of halo, $C_{1-10}$ alkyl, substituted $C_{1-10}$ alkyl, $C_{3-7}$ cycloalkyl, substituted $C_{3-7}$ cycloalkyl, $C_{2-10}$ alkenyl, substituted $C_{2-10}$ alkenyl, $C_{2-10}$ alkynyl, substituted $C_{2-10}$ alkynyl, CN, $-OR^a$, $-NR^aR^b$, $-C(O)R^a$, $-C(O)OR^a$, $-C(O)NR^aR^b$, $-NR^aC(O)R^b$, $-SR^a$, $-S(O)R^a$, and $-S(O)_2R^a$;
$R^2$ is independently selected from the group consisting of halo, $C_{1-10}$ alkyl, substituted $C_{1-10}$ alkyl, $C_{3-7}$ cycloalkyl, substituted $C_{3-7}$ cycloalkyl, $C_{2-10}$ alkenyl, substituted $C_{2-10}$ alkenyl, $C_{2-10}$ alkynyl, substituted $C_{2-10}$ alkynyl, CN, $-OR^a$, $-NR^aR^b$, $-C(O)R^a$, $-C(O)OR^a$, $-C(O)NR^aR^b$, $-NR^aC(O)R^b$, $-SR^a$, $-S(O)R^a$, and $-S(O)_2R^a$;
each of $R^a$ and $R^b$ is independently selected from the group consisting of H, $C_{1-10}$ alkyl, substituted $C_{1-10}$ alkyl, $C_{3-10}$ cycloalkyl, $C_{2-10}$ alkenyl, and $C_{2-10}$ alkynyl;
$R^4$ is independently selected from the group consisting of H, fluoro, $C_{1-6}$ alkyl, substituted $C_{1-6}$ alkyl, and $C_{1-6}$ alkoxy;
$R^5$ is independently selected from the group consisting of H, fluoro, $C_{1-6}$ alkyl, substituted $C_{1-6}$ alkyl, and $C_{1-6}$ alkoxy; optionally one of $R^4$ and one of $R^5$ cyclize to form a $C_{3-6}$ saturated ring; and
$R^6$ is selected from the group consisting of $C_{1-6}$alkyl, substituted $C_{1-6}$ alkyl, $C_{1-6}$ alkoxy, substituted $C_{1-6}$ alkoxy, aryl, substituted aryl, heteroaryl, and substituted heteroaryl.

In one embodiment, provided is a compound of Formula (X)

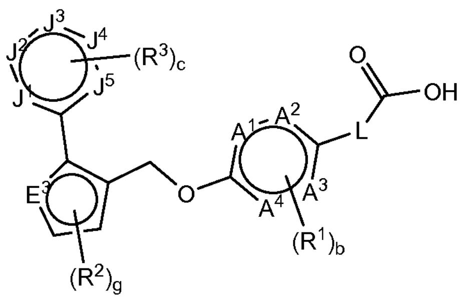

(X)

or a pharmaceutically acceptable salt thereof, wherein:

| | |
|---|---|
| $J^1$, $J^2$, $J^3$, $J^4$, and $J^5$ | are independently selected from the group consisting ofN and C, with the proviso that only 0, 1, or 2 of $J^1$, $J^2$, $J^3$, $J^4$, and $J^5$ is N; |

the subscript c is 0, 1, 2, or 3;

$R^3$ is independently selected from the group consisting of halo, $C_{1-10}$ alkyl, substituted $C_{1-10}$ alkyl, $C_{3-7}$ cycloalkyl, substituted $C_{3-7}$ cycloalkyl, $C_{2-10}$ alkenyl, substituted $C_{2-10}$ alkenyl, $C_{2-10}$ alkynyl, substituted $C_{2-10}$ alkynyl, CN, $-OR^a$, $-NR^aR^b$, $-C(O)R^a$, $-C(O)OR^a$, $-C(O)NR^aR^b$, $-NR^aC(O)R^b$, $-SR^a$, $-S(O)R^a$, and $-S(O)_2R^a$; and

$R^a$, $R^b$, $A^1$, $A^2$

, $A^3$, $A^4$, $E^3$, L,

$R^1$, $R^2$, subscript b, and subscript g are as previously defined for Formula (IX).

In some aspects, $E^3$ is O.

In one embodiment, provided is a compound of Formula (XI) or (XII)

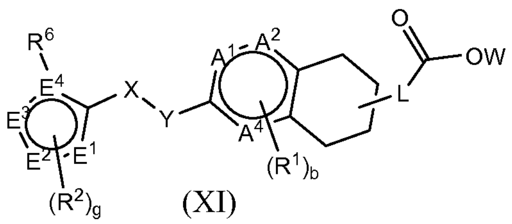

(XI)

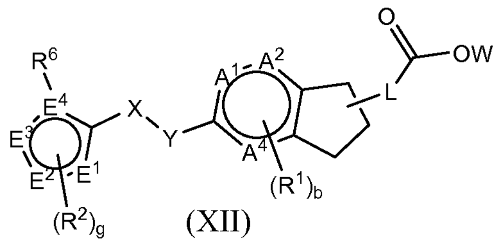

(XII)

or a pharmaceutically acceptable salt thereof, wherein:

$A^1$, $A^2$, and $A^4$ are independently selected from the group consisting of N and C, with the proviso that only 0, 1, or 2 of $A^1$, $A^2$, $A^3$, and $A^4$ is N;

$E^4$ is C or N;

$E^1$, $E^2$, and $E^3$ are independently selected from the group consisting of C, N, O and S; provided that when one of $E^1$, $E^2$, or $E^3$ is O or S, the other of $E^1$, $E^2$, or $E^3$ is independently C or N;

one of X and Y is $CH_2$ and the other of X and Y is selected from the group consisting of $CH_2$, -NH-, -O-, -S-, -S(O)-, and $-S(O)_2-$;

L is $-(CR^4R^5)_n-$ wherein optionally one $-(CR^4R^5)-$ is replaced with -O- or -S-;

W is selected from the group consisting of H, $C_{1-10}$ alkyl, and substituted $C_{1-10}$ alkyl;

the subscript b is 0, 1, or 2;

the subscript g is 0, 1, or 2;

the subscript n is 0, 1, 2, 3, or 4;

$R^1$ is independently selected from the group consisting of halo, $C_{1-10}$ alkyl, substituted $C_{1-10}$ alkyl, $C_{3-7}$ cycloalkyl, substituted $C_{3-7}$ cycloalkyl, $C_{2-10}$ alkenyl, substituted $C_{2-10}$ alkenyl, $C_{2-10}$ alkynyl, substituted $C_{2-10}$ alkynyl, CN, $-OR^a$, $-NR^aR^b$, $-C(O)R^a$, $-C(O)OR^a$, $-C(O)NR^aR^b$, $-NR^aC(O)R^b$, $-SR^a$, $-S(O)R^a$, and $-S(O)_2R^a$;

$R^2$ is independently selected from the group consisting of halo, $C_{1-10}$ alkyl, substituted $C_{1-10}$ alkyl, $C_{3-7}$ cycloalkyl, substituted $C_{3-7}$ cycloalkyl, $C_{2-10}$ alkenyl, substituted $C_{2-10}$ alkenyl, $C_{2-10}$ alkynyl, substituted $C_{2-10}$ alkynyl, CN, $-OR^a$, $-NR^aR^b$, $-C(O)R^a$, $-C(O)OR^a$, $-C(O)NR^aR^b$, $-NR^aC(O)R^b$, $-SR^a$, $-S(O)R^a$, and $-S(O)_2R^a$;

each of $R^a$ and $R^b$ is independently selected from the group consisting of H, $C_{1-10}$ alkyl, substituted $C_{1-10}$ alkyl, $C_{3-10}$ cycloalkyl, $C_{2-10}$ alkenyl, and $C_{2-10}$ alkynyl;

$R^4$ is independently selected from the group consisting of H, fluoro, $C_{1-6}$ alkyl, substituted $C_{1-6}$ alkyl, and $C_{1-6}$ alkoxy;

$R^5$ is independently selected from the group consisting of H, fluoro, $C_{1-6}$ alkyl, substituted $C_{1-6}$ alkyl, and $C_{1-6}$ alkoxy; optionally one of $R^4$ and one of $R^5$ cyclize to form a $C_{3-6}$ saturated ring; and

$R^6$ is selected from the group consisting of $C_{1-6}$alkyl, substituted $C_{1-6}$ alkyl, $C_{1-6}$ alkoxy, substituted $C_{1-6}$ alkoxy, aryl, substituted aryl, heteroaryl, and substituted heteroaryl.

In one embodiment, provided is a compound of Formula (XIII) or (XIV)

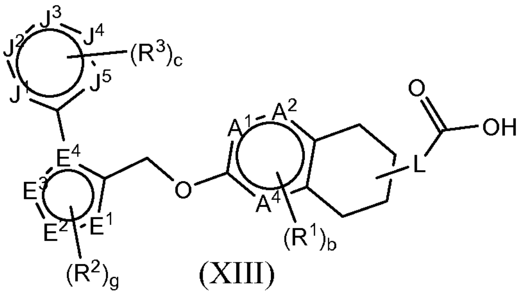
(XIII)

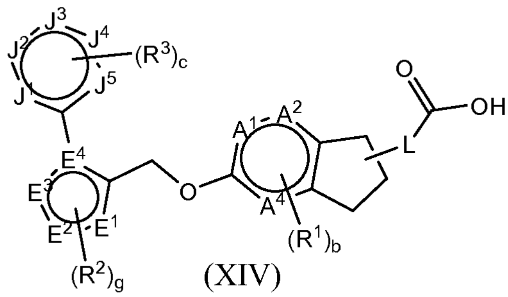
(XIV)

or a pharmaceutically acceptable salt thereof, wherein:

| | |
|---|---|
| $J^1$, $J^2$, $J^3$, $J^4$, and $J^5$ | are independently selected from the group consisting ofN and C, with the proviso that only 0, 1, or 2 of $J^1$, $J^2$, $J^3$, $J^4$, and $J^5$ is N; the subscript c is 0, 1, 2, or 3; |
| $R^3$ | is independently selected from the group consisting of halo, $C_{1-10}$ alkyl, substituted $C_{1-10}$ alkyl, $C_{3-7}$ cycloalkyl, substituted $C_{3-7}$ cycloalkyl, $C_{2-10}$ alkenyl, substituted $C_{2-10}$ alkenyl, $C_{2-10}$ alkynyl, substituted $C_{2-10}$ alkynyl, CN, $-OR^a$, $-NR^aR^b$, $-C(O)R^a$, $-C(O)OR^a$, $-C(O)NR^aR^b$, $-NR^aC(O)R^b$, $-SR^a$, $-S(O)R^a$, and $-S(O)_2R^a$; and |
| $R^a$, $R^b$, $A^1$, $A^2$, $A^4$, $E^1$, $E^2$, $E^3$, $E^4$, L, $R^1$, $R^2$, subscript b, and subscript g | are as previously defined for Formula (XI and XII). |

In some aspects, $E^1$ and $E^2$ are C. In further aspects, $E^3$ and $E^4$ are N.

In aspects provided are compounds of Formula (A)-(D) and (I)-(XIV) and pharmaceutically acceptable salts thereof having the following features in combination with any of the above embodiments and features.

In some aspects, L is $-(CR^4R^5)_2-$. In some further aspects, $J^1$, $J^2$, $J^3$, $J^4$ , and $J^5$ are C. In other further aspects $A^1$, $A^2$, $A^3$, and $A^4$ are C. In still other further aspects, $J^1$, $J^2$, $J^3$, $J^4$, and $J^5$ are C and $A^1$, $A^2$, $A^3$, and $A^4$ are C.

In some aspects, L is $-(CR^4R^5)_2-$ and $R^3$ is halo, $C_{1-10}$ alkyl, or substituted $C_{1-10}$ alkyl. In some further aspects $R^2$ is $C_{1-10}$ alkyl or substituted $C_{1-10}$ alkyl. In still further aspects $R^1$ is halo, $C_{1-10}$ alkyl, substituted $C_{1-10}$ alkyl, $C_{1-6}$ alkoxy, or substituted $C_{1-6}$ alkoxy.

In some aspects, L is $-(CR^4R^5)_2-$, $R^1$ is halo, $R^2$ is $C_{1-10}$ alkyl, and $R^3$ is halo or substituted $C_{1-10}$ alkyl.

In some aspects, $R^6$ is selected from the group consisting of $C_{1-6}$ alkyl, substituted $C_{1-6}$ alkyl, $C_{1-6}$ alkoxy, and substituted $C_{1-6}$ alkoxy. In some such aspects, $R^6$ is selected from the group consisting of methyl, ethyl, propyl, isopropyl, butyl, isobutyl, and t-butyl.

In one embodiment, provided are ester prodrugs of the compounds of Formula (A)-(D) and (I)-(XIV). In some embodiments, the ester prodrugs are compounds wherein the carboxylic acid group is derivatized to be an ester, such as when W in the formulae is alkyl or substituted alkyl.

In other embodiments provided are compound agonists or a pharmaceutically acceptable salt thereof or synthetic intermediates thereof as exemplified in Chemical Examples section below.

*Preparation of Compounds of the Invention*

The compounds of the present invention can be prepared in a number of ways familiar to one skilled in the art of organic chemistry synthesis. The synthetic route of compounds in the present invention is not limited to the methods outlined herein or as provided in the Examples. Individual compounds may require manipulation of the conditions in order to accommodate various functional groups and may require appropriate use of protecting groups. Purification, if necessary, can be accomplished on a silica gel column eluted with the appropriate organic solvent system. Also, reverse phase HPLC or recrystallization may be employed.

*Compositions and Methods of Treatment*

In accordance with the present invention methods of treating a disease or condition selected from the group consisting of Type I diabetes, Type II diabetes and metabolic syndrome are provided. The method comprises administering to a subject in need of such treatment an effective amount of a compound of the present invention.

In another aspect, methods of raising intracellular levels of $Ca^{2+}$ in a cell expressing GPR120 are provided. The method comprises exposing a cell that expresses GPR120 to a compound of the invention. $Ca^{2+}$ levels are determined by the methods disclosed in the Example sections herein.

In one embodiment, the cell that expresses GPR120 is a pancreatic cell, an islet cell, or a beta cell, an intestinal endocrine cell, an L cell or a K cell.

Another aspect of the invention provides a method of stimulating insulin production in a mammal, in particular a human. The method comprises administering an effective amount of a compound of the invention to the mammal. In response to administration of a compound to the subject, insulin is produced by the beta cells. Biological Example 3 provides detailed methods by which a skilled artisan can measure insulin secretion in laboratory animals in response to administration of a compound of the invention.

In another aspect, the invention provides a method of stimulating insulin secretion in a mammal, in particular a human. The method comprises administering an effective amount of a compound of the invention to the mammal. In response to administration of a compound to the subject, insulin is secreted into the blood stream by the beta cells.

A further aspect of the invention provides a method of stimulating glucose-depependent insulin secretion in a mammal, in particular a human. The method comprises administering an effective amount of a compound of the invention to the mammal. After administration to the subject, insulin is secreted into the blood stream by the beta cells in a glucose-dependent manner. Biological Example 4 provides methods that show the blood glucose lowering effects of the compounds of the invention.

In another embodiment, the invention provides methods of lowering blood glucose in a mammal, preferably a human. The method comprises administering an effective amount of a compound of the invention to the mammal. In response to administration of a compound to the subject, blood glucose levels are lowered. The method further comprises steps to measure blood glucose levels before and after administration of a compound of the invention. Blood glucose levels are easily measured by numerous commercially available glucose monitoring devices that measure blood glucose from samples of blood or urine. Blood glucose can also be measured by commercially available glucometers that do not require blood or urine samples. Biological Examples 3 and 4 provide methods that teach how to measure improvements in diabetes paramaters, including blood glucose monitoring.

Another aspect of the invention provides a method of stimulating incretin production in a mammal, in particular a human. The method comprises administering an effective amount of a compound of the invention to the mammal. In response to administration of a compound to the subject, glucagon-like peptide 1 and glucose-dependent insulinotropic polypeptide is produced by the intestinal endocrine cells. Biological Example 5 provides detailed methods by which a skilled artisan can measure incretin production in laboratory animals in response to administration of a compound of the invention.

*Combination Therapy*

As noted above, the compounds of the present invention will, in some instances, be used in combination with other therapeutic agents to bring about a desired effect. Selection of additional agents will, in large part, depend on the desired target therapy (*see, e.g.,* Turner N, et al., Prog. Drug Res. (1998) 51:33-94; Haffner S, Diabetes Care (1998) 21:160-178; and DeFronzo R, et al. (eds.), Diabetes Reviews (1997) Vol. 5 No. 4). A number of studies have investigated the benefits of combination therapies with oral agents (*see, e.g.,* Mahler R, J. Clin. Endocrinol. Metab. (1999) 84:1165-71; United Kingdom Prospective Diabetes Study Group: UKPDS 28, Diabetes Care (1998) 21:87-92; Bardin CW (ed.), Current Therapy in Endocrinology and Metabolism, 6th Ed. (Mosby - Year Book, Inc., St. Louis, MO 1997); Chiasson J, et al., Ann. Intern. Med. (1994) 121:928-935; Coniff R, et al., Clin. Ther. (1997) 19:16-26; ConiffR, et al., Am. J. Med. (1995) 98:443-451; and Iwamoto Y, et al., Diabet. Med. (1996) 13:365-370; Kwiterovich P, Am. J. Cardiol (1998) 82 (12A):3U-17U). These studies indicate that diabetes modulation can be further improved by the addition of a second agent to the therapeutic regimen. Combination therapy includes administration of a single pharmaceutical dosage formulation that contains a compound having the general structure of Formula (A)-(D) and (I)-(XIV) and one or more additional active agents, as well as administration of a compound of Formula (A)-(D) and (I)-(XIV) and each active agent in its own separate pharmaceutical dosage formulation. For example, a compound of Formula (A)-(D) and (I)-(XIV) and a DPP4 inhibitor can be administered to the human subject together in a single oral dosage composition, such as a tablet or capsule, or each agent can be administered in separate oral dosage formulations. Where separate dosage formulations are used, a compound of Formula (A)-(D) and (I)-(XIV) and one or more additional active agents can be administered at essentially the same time (i. e., concurrently), or at separately staggered times (*i.e.*, sequentially).

Combination therapy is understood to include all these regimens.

An example of combination therapy can be seen in modulating (preventing the onset of the symptoms or complications associated with) diabetes (or treating, preventing or reducing the risk of developing diabetes and its related symptoms, complications, and disorders), wherein the compounds of Formula (A)-(D) and (I)-(XIV) can be effectively used in combination with, for example, biguanides (such as metformin); thiazolidinediones (such as ciglitazone, pioglitazone, troglitazone, and rosiglitazone); dipeptidyl-peptidase-4 ("DPP4") inhibitors (such as vildagliptin and sitagliptin); glucagonlike peptide-1 ("GLP-1") receptor agonists (such as exanatide) (or GLP-1 mimetics); PPAR gamma agonists or partial agonists; dual PPAR alpha, PPAR gamma agonists or partial agonists; dual PPAR delta, PPAR gamma agonists or partial agonists; pan PPAR agonists or partial agonists; dehydroepiandrosterone (also referred to as DHEA or its conjugated sulphate ester, DHEA-$SO_4$); antiglucocorticoids; TNF$\alpha$ inhibitors; $\alpha$-glucosidase inhibitors (such as acarbose, miglitol, and voglibose); sulfonylureas (such as chlorpropamide, tolbutamide, acetohexamide, tolazamide, glyburide, gliclazide, glynase, glimepiride, and glipizide); pramlintide (a synthetic analog of the human hormone amylin); other insulin secretogogues (such as repaglinide, gliquidone, and nateglinide); insulin (or insulin mimetics); glucagon receptor antagonists; gastric inhibitory peptide ("GIP"); or GIP mimetics; as well as the active agents discussed below for treating obesity, hyperlipidemia, atherosclerosis and/or metabolic syndrome.

Another example of combination therapy can be seen in treating obesity or obesity-related disorders, wherein the compounds of Formula (A)-(D) and (I)-(XIV) can be effectively used in combination with, for example, phenylpropanolamine, phenteramine; diethylpropion; mazindol; fenfluramine; dexfenfluramine; phentiramine, $\beta$-3 adrenoceptor agonist agents; sibutramine; gastrointestinal lipase inhibitors (such as orlistat); and leptins. Other agents used in treating obesity or obesity-related disorders wherein the compounds of Formula (A)-(D) and (I)-(XIV) can be effectively used in combination with, for example, cannabinoid-1 ("CB-1") receptor antagonists (such as rimonabant); PPAR delta agonists or partial agonists; dual PPAR alpha, PPAR delta agonists or partial agonists; dual PPAR delta, PPAR gamma agonists or partial agonists; pan PPAR agonists or partial agonists; neuropeptide Y; enterostatin; cholecytokinin; bombesin; amylin; histamine $H_3$ receptors; dopamine $D_2$ receptors; melanocyte stimulating hormone; corticotrophin releasing factor; galanin; and gamma amino butyric acid (GABA).

Still another example of combination therapy can be seen in modulating hyperlipidemia (treating hyperlipidemia and its related complications), wherein the compounds of Formula (A)-(D) and (I)-(XIV) can be effectively used in combination with, for example, statins (such as atorvastatin, fluvastatin, lovastatin, pravastatin, and simvastatin), CETP inhibitors (such as torcetrapib); a cholesterol absorption inhibitor (such as ezetimibe); PPAR alpha agonists or partial agonists; PPAR delta agonists or partial agonists; dual PPAR alpha, PPAR delta agonists or partial agonists; dual PPAR alpha, PPAR gamma agonists or partial agonists; dual PPAR delta, PPAR gamma agonists or partial agonists; pan PPAR agonists or partial agonists; fenofibric acid derivatives (such as gemfibrozil, clofibrate, fenofibrate, and bezafibrate); bile acid-binding resins (such as colestipol or cholestyramine); nicotinic acid; probucol; betacarotene; vitamin E; or vitamin C.

A further example of combination therapy can be seen in modulating atherosclerosis, wherein a compound of Formula (A)-(D) and (I)-(XIV) is administered in combination with one or more of the following active agents: an antihyperlipidemic agent; a plasma HDL-raising agent; an antihypercholesterolemic agent, such as a cholesterol biosynthesis inhibitor, e.g., an hydroxymethylglutaryl (HMG) CoA reductase inhibitor (also referred to as statins, such as lovastatin, simvastatin, pravastatin, fluvastatin, and atorvastatin); an HMG-CoA synthase inhibitor; a squalene epoxidase inhibitor; or a squalene synthetase inhibitor (also known as squalene synthase inhibitor); an acyl-coenzyme A cholesterol acyltransferase (ACAT) inhibitor, such as melinamide; probucol; nicotinic acid and the salts thereof and niacinamide; a cholesterol absorption inhibitor, such as $\beta$-sitosterol; a bile acid sequestrant anion exchange resin, such as cholestyramine, colestipol or dialkylaminoalkyl derivatives of a cross-linked dextran; an LDL receptor inducer; fibrates, such as clofibrate, bezafibrate, fenofibrate, and gemfibrizol; vitamin $B_6$ (also known as pyridoxine) and the pharmaceutically acceptable salts thereof, such as the HCl salt; vitamin $B_{12}$ (also known as cyanocobalamin); vitamin $B_3$ (also known as nicotinic acid and niacinamide); anti-oxidant vitamins, such as vitamin C and E and beta carotene; a $\beta$-blocker; an angiotensin II antagonist; an angiotensin converting enzyme inhibitor; PPAR alpha agonists or partial agonists; PPAR delta agonists or partial agonists; PPAR gamma agonists or partial agonists; dual PPAR alpha, PPAR delta agonists or partial agonists; dual PPAR alpha, PPAR gamma agonists or partial agonists; dual PPAR delta, PPAR gamma agonists or partial agonists; pan PPAR agonists or partial agonists; and a platelet aggregation inhibitor, such as fibrinogen receptor antagonists (*i.e.*, glycoprotein IIb/IIIa fibrinogen receptor antagonists) and aspirin. As noted above, the compounds of Formula (A)-(D) and (I)-(XIV) can be administered in combination with more than one additional active agent, for example, a combination of a compound of Formula (A)-(D) and (I)-(XIV) with an HMG-CoA reductase inhibitor (e.g., atorvastatin, fluvastatin, lovastatin, pravastatin, and simvastatin) and aspirin, or a compound of Formula (A)-(D) and (I)-(XIV) with an HMG-CoA reductase inhibitor and a $\beta$-blocker.

Additionally, an effective amount of a compound of Formula (A)-(D) and (I)-(XIV) and a therapeutically effective amount of one or more active agents selected from the group consisting of: an antihyperlipidemic agent; a plasma HDL-raising agent; an antihypercholesterolemic agent, such as a cholesterol biosynthesis inhibitor, for example, an HMG-CoA reductase inhibitor; an HMG-CoA synthase inhibitor; a squalene epoxidase inhibitor, or a squalene synthetase inhibitor (also known as squalene synthase inhibitor); an acyl-coenzyme A cholesterol acyltransferase inhibitor; probucol; nicotinic acid and the salts thereof; CETP inhibitors such as torcetrapib; a cholesterol absorption inhibitor such as ezetimibe; PPAR alpha agonists or partial agonists; PPAR delta agonists or partial agonists; dual PPAR alpha, PPAR delta agonists or partial agonists; dual PPAR alpha, PPAR gamma agonists or partial agonists; dual PPAR delta, PPAR gamma agonists or partial agonists; pan PPAR agonists or partial agonists;niacinamide; a cholesterol absorption inhibitor; a bile acid sequestrant anion exchange resin; a LDL receptor inducer; clofibrate, fenofibrate, and gemfibrozil; vitamin $B_6$ and the pharmaceutically acceptable salts thereof; vitamin $B_{12}$; an anti-oxidant vitamin; a β-blocker; an angiotensin II antagonist; an angiotensin converting enzyme inhibitor; a platelet aggregation inhibitor; a fibrinogen receptor antagonist; aspirin; phentiramines,
β -3 adrenergic receptor agonists; sulfonylureas, biguanides, α-glucosidase inhibitors, other insulin secretogogues, and insulin can be used together for the preparation of a pharmaceutical composition useful for the above-described treatments.

An additional example of combination therapy can be seen in modulating metabolic syndrome (or treating metabolic syndrome and its related symptoms, complications and disorders), wherein the compounds of Formula (A)-(D) and (I)-(XIV) can be effectively used in combination with, for example, the active agents discussed above for modulating or treating diabetes, obesity, hyperlipidemia, atherosclerosis, and/or their respective related symptoms, complications and disorders.

In a further embodiment, a compound of the present invention can be administered in combination with halofenic acid, an ester of halofenic acid, or another prodrug of halofenic acid, preferably with (-)-(4-chlorophenyl)-(3-triftuoromethylphenoxy)-acetic acid 2-acetylaminoethyl ester.

In particular, this invention provides methods of treating a mammal, in particular a human by administering a compound of Formula (A)-(D) and (I)-(XIV) and a DPP4 inhibitor.

The DPP4 inhibitors useful in the present invention are sitagliptin (Merck), vildagliptin (Novartis), BMS-477118 (saxagliptin) (Bristol-Myers Squibb), R1438 (amino-methylpyridine) (Roche), NVP DPP728 (Novartis), PSN9301 (Prosidion), P32/98 (isoleucine thiozolidide) (Probiodrug), GSK823093C (Denagliptin) (Glaxo Smithkline), SYR-322 (Alogliptin) (Takeda), NN-7201 (NovoNordisk), ALS2-0426 (Alantos). (Green BD, Flatt PR, Bailey CJ, Dipeptidyl peptidase IB (DPP4) inhibitors: a newly emerging drug class for the treatment of Type II diabetes, Diabetes Vasc. Dis. Res. 2006, 3: 159-165). Preferred DPP4 inhibitors are sitagliptin, vildagliptin, Denagliptin, saxagliptin, and alogliptin). Even more preferred CPP4 inhibitors are sitagliptin and vildagliptin.

The compound of Formula (A)-(D) and (I)-(XIV) and DPP4 inhibitor are administered in a single dosage or in separate dosages. The single dosage is administered once a day or multiple times a day. When the compound of Formula (A)-(D) and (I)-(XIV) and DPP4 inhibitor are administered is separate dosages, the dosages can be administered once a day or multiple times a day.

The dosing of a compound of Formula (A)-(D) and (I)-(XIV) and DPP4 inhibitor can be dosed at the same time, within several minutes, or separated by hours. By way of example, a compound of Formula (A)-(D) and (I)-(XIV) and DPP4 inhibitor can be dosed together in the morning, with no further dosing for the remainder of the day. Alternatively, in the morning, a compound of Formula (A)-(D) and (I)-(XIV) and a DPP4 inhibitor is dosed followed with a second dose of a compound of Formula (A)-(D) and (I)-(XIV) and/or a DPP4 inhibitor in the evening or after a meal.

It can be necessary to administer dosages of the compound of Formula (A)-(D) and (I)-(XIV) and/or DPP4 inhibitor once a day or more than once a day, or before or after a meal, as will be apparent to those skilled in the art. Further, it is noted that the clinician or treating physician will know how and when to start, interrupt, adjust, or terminate therapy in conjunction with individual patient response.

In one embodiment, when the compound of the present invention and the DPP4 inhibitor are administered in a single dosage, the compound of Formula (A)-(D) and (I)-(XIV) and DPP4 inhibitor are formulated into a single pill, single table, or a single capsule. When the compound of Formula (I), (I)-(XIV) and DPP4 inhibitor are administered in separate dosages, the compound of Formula (A)-(D) and (I)-(XIV) is formulated into a pill, tablet or capsule and the DPP4 inhibitor is formulated into a separate pill or capsule.

When the compound of Formula (A)-(D) and (I)-(XIV) and DPP4 inhibitor are administered in separate dosages, the compound of this invention can be administered first and the DPP4 inhibitor can be administered next, following administration of the compound of Formula (A)-(D) and (I)-(XIV). Alternatively, the DPP4 inhibitor can be administered first and the compound of this invention can be administered next, following administration of the DPP4 inhibitor. The time between the first administration and the second administration can be varied by a skilled practitioner. In one embodiment, the first administration (a compound of Formula (A)-(D) and (I)-(XIV) or DPP4 inhibitor), is followed immediately by the second administration (a compound of Formula (A)-(D) and (I)-(XIV) or DPP4 inhibitor). In another embodiment, the second administration is within 2 minutes, 5 minutes, 10 minutes, 15 minutes, 30 minutes, or 60 minutes, 1 hour, 2 hours, 3 hours, 4 hours, 5 hours, 6 hours, 7 hours, 8 hours, 9 hours, 10 hours, 11 hours, or 12 hours following the first administration. Yet another embodiment provides for the administration of a compound for Formula (A)-(D) and

(I)-(XIV) and/or DPP4 inhibitor in the morning followed by the administration of a compound of Formula (A)-(D) and (I)-(XIV) and/or DPP4 inhibitor in the evening.

In addition, the present invention provides for kits with unit doses of the compounds of Formula (A)-(D) and (I)-(XIV) and/or DPP4 inhibitor, either in oral or injectable doses. In addition to the containers containing the unit doses will be an informational package insert describing the use and attendant benefits of the drugs in treating Type II diabetes, obesity, hyperlipidemia, atherosclerosis and metabolic syndrome, and/or their respective related symptoms, complications and disorders. Preferred compounds and unit doses are those described herein above.

Another aspect of this invention provides methods of lowering blood levels of glucose in a subject by administering a compound of Formula (A)-(D) and (I)-(XIV) and a DPP4 inhibitor. The method comprises administering an effective amount of a compound of the invention and DPP4 inhibitor to the mammal. The method further comprises steps to measure blood glucose levels before and after administration of a compound of Formula (A)-(D) and (I)-(XIV) and DPP4 inhibitor. Blood glucose levels are easily measured by numerous commercially available glucose monitoring devices that measure blood glucose from samples of blood or urine, or as taught herein. Blood glucose can also be measured by commercially available glucometers that do not require blood or urine samples.

Another aspect of this invention provides methods of lowering blood levels of insulin in a subject by administering a compound of Formula (A)-(D) and (I)-(XIV) and a DPP4 inhibitor. The method comprises administering an effective amount of a compound of Formula (A)-(D) and (I)-(XIV) and DPP4 inhibitor to the mammal. The method further comprises steps to measure blood insulin levels before and after administration of a compound of this invention and a DPP4 inhibitor. Blood insulin levels are easily measured by well-known insulin monitoring assays that measure insulin from samples of blood or urine, or as taught herein.

In another aspect, this invention provides methods of increasing blood levels of incretins in a subject by administering a compound of this invention and a DPP4 inhibitor. The incretins are GLP-1 and GIP. The method comprises administering an effective amount of a compound of Formula (A)-(D) and (I)-(XIV) and DPP4 inhibitor to the mammal. The method further comprises steps to measure blood incretin levels before and after administration of a compound of Formula (A)-(D) and (I)-(XIV) and a DPP4 inhibitor. Blood incretin levels are easily measured by well-known incretin monitoring assays, or as taught herein.

Yet another aspect of this invention provides methods of lowering blood triglyceride levels in a subject by administering a compound of Formula (A)-(D) and (I)-(XIV) and a DPP4 inhibitor. The method comprises administering an effective amount of a compound of the present invention and DPP4 inhibitor to the mammal. The method further comprises steps to measure blood triglycerides levels before and after administration of a compound of Formula (A)-(D) and (I)-(XIV) and DPP4 inhibitor. Blood triglyceride levels are easily measured by numerous commercially available devices that measure blood triglyceride levels from samples of blood.

A further aspect of this invention provides methods of lowering gastric emptying in a subject by administering a compound of the invention and a DPP4 inhibitor. The method comprises administering an effective amount of a compound of Formula (A)-(D) and (I)-(XIV) and DPP4 inhibitor to the mammal. The method further comprises steps to measure blood incretin levels before and after administration of a compound of Formula (A)-(D) and (I)-(XIV) and a DPP4 inhibitor. Blood incretin levels are easily measured by well-known incretin monitoring assays, or as taught herein.

Another aspect of this invention provides methods of increasing insulin production in the islet cells of a subject by administering a compound of Formula (A)-(D) and (I)-(XIV) and a DPP4 inhibitor. The method comprises administering an effective amount of a compound of Formula (A)-(D) and (I)-(XIV) and DPP4 inhibitor to the mammal. The method further comprises steps to measure insulin production in islet cells or the beta cells of the pancreas before and after administration of a compound of Formula (A)-(D) and (I)-(XIV) and a DPP4 inhibitor. The insulin production of islets and beta cells are easily measured by well-known assays, or as taught herein.

In yet another aspect, this invention provides methods of preserving islet function in a subject by administering a compound of the invention and a DPP4 inhibitor. The method comprises administering an effective amount of a compound of Formula (A)-(D) and (I)-(XIV) and DPP4 inhibitor to the mammal. The method further comprises steps to measure the function of islets or beta cell's ability to produce insulin before and after administration of a compound of Formula (A)-(D) and (I)-(XIV) and a DPP4 inhibitor. The insulin production of islets and beta cells are easily measured by well-known assays, or as taught herein.

The compounds of Formula (A)-(D) and (I)-(XIV) that are used in the methods of the present invention can be incorporated into a variety of formulations and medicaments for therapeutic administration. More particularly, the compounds of Formula (A)-(D) and (I)-(XIV) can be formulated into pharmaceutical compositions by combination with appropriate, pharmaceutically acceptable carriers or diluents, and can be formulated into preparations in solid, semi-solid, liquid or gaseous forms, such as tablets, capsules, pills, powders, granules, dragees, gels, slurries, ointments, solutions, suppositories, injections, inhalants and aerosols. As such, administration of the compounds can be achieved in various ways, including oral, buccal, rectal, parenteral, intraperitoneal, intradermal, transdermal, and/or intratracheal administration. Moreover, the compound can be administered in a local rather than systemic manner, in a depot or sustained release formulation. In addition, the compounds can be administered in a liposome.

The compounds of Formula (A)-(D) and (I)-(XIV) can be formulated with common excipients, diluents or carriers, and compressed into tablets, or formulated as elixirs or solutions for convenient oral administration, or administered by the intramuscular or intravenous routes. The compounds can be administered transdermally, and can be formulated as sustained release dosage forms and the like. Compounds of Formula (A)-(D) and (I)-(XIV) can be administered alone, in combination with each other, or they can be used in combination with other known compounds.

Suitable formulations for use in the present invention are found in Remington's Pharmaceutical Sciences (Mack Publishing Company (1985) Philadelphia, PA, 17th ed.), which is incorporated herein by reference. Moreover, for a brief review of methods for drug delivery, *see*, Langer, Science (1990) 249:1527-1533, which is incorporated herein by reference. The pharmaceutical compositions described herein can be manufactured in a manner that is known to those of skill in the art, *i.e*., by means of conventional mixing, dissolving, granulating, dragee-making, levigating, emulsifying, encapsulating, entrapping or lyophilizing processes. The following methods and excipients are merely exemplary and are in no way limiting.

For injection, the compound of Formula (A)-(D) and (I)-(XIV) and DPP4 inhibitor can be formulated into preparations by dissolving, suspending or emulsifying them in an aqueous or nonaqueous solvent, such as vegetable or other similar oils, synthetic aliphatic acid glycerides, esters of higher aliphatic acids or propylene glycol; and if desired, with conventional additives such as solubilizers, isotonic agents, suspending agents, emulsifying agents, stabilizers and preservatives. Preferably, the compounds of the present invention can be formulated in aqueous solutions, preferably in physiologically compatible buffers such as Hanks' solution, Ringer's solution, or physiological saline buffer. For transmucosal administration, penetrants appropriate to the barrier to be permeated are used in the formulation. Such penetrants are generally known in the art.

For oral administration, the compounds of Formula (A)-(D) and (I)-(XIV) and DPP4 inhibitors can be formulated readily by combining with pharmaceutically acceptable carriers that are well known in the art. Such carriers enable the compounds to be formulated as tablets, pills, dragees, capsules, emulsions, lipophilic and hydrophilic suspensions, liquids, gels, syrups, slurries, suspensions and the like, for oral ingestion by a patient to be treated. Pharmaceutical preparations for oral use can be obtained by mixing the compounds with a solid excipient, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone. If desired, disintegrating agents can be added, such as the cross-linked polyvinyl pyrrolidone, agar, or alginic acid or a salt thereof such as sodium alginate.

Dragee cores are provided with suitable coatings. For this purpose, concentrated sugar solutions can be used, which can optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments can be added to the tablets or dragee coatings for identification or to characterize different combinations of active compound doses.

Pharmaceutical preparations that can be used orally include push-fit capsules made of gelatin, as well as soft, sealed capsules made of gelatin and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds can be dissolved or suspended in suitable liquids, such as fatty oils, liquid paraffin, or liquid polyethylene glycols. In addition, stabilizers can be added. All formulations for oral administration should be in dosages suitable for such administration.

For buccal administration, the compositions can take the form of tablets or lozenges formulated in a conventional manner.

For administration by inhalation, the compounds for use according to the present invention are conveniently delivered in the form of an aerosol spray presentation from pressurized packs or a nebulizer, with the use of a suitable propellant, *e.g*., dichlorodifluoromethane, trichlorofluoromethane, dichlorotetrafluoroethane, carbon dioxide or other suitable gas, or from propellant-free, dry-powder inhalers. In the case of a pressurized aerosol the dosage unit can be determined by providing a valve to deliver a metered amount. Capsules and cartridges of, *e.g*., gelatin for use in an inhaler or insufflator can be formulated containing a powder mix of the compound and a suitable powder base such as lactose or starch.

The compounds can be formulated for parenteral administration by injection, *e.g*., by bolus injection or continuous infusion. Formulations for injection can be presented in unit dosage form, *e.g*., in ampoules or in multidose containers, with an added preservative. The compositions can take such forms as suspensions, solutions or emulsions in oily or aqueous vehicles, and can contain formulator agents such as suspending, stabilizing and/or dispersing agents.

Pharmaceutical formulations for parenteral administration include aqueous solutions of the active compounds in water-soluble form. Additionally, suspensions of the active compounds can be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions can contain substances that increase

the viscosity of the suspension, such as sodium carboxymethyl cellulose, sorbitol, or dextran. Optionally, the suspension can also contain suitable stabilizers or agents that increase the solubility of the compounds to allow for the preparation of highly concentrated solutions. Alternatively, the active ingredient can be in powder form for constitution with a suitable vehicle, *e.g.*, sterile pyrogen-free water, before use.

The compounds can also be formulated in rectal compositions such as suppositories or retention enemas, *e.g.*, containing conventional suppository bases such as cocoa butter, carbowaxes, polyethylene glycols or other glycerides, all of which melt at body temperature, yet are solidified at room temperature.

In addition to the formulations described previously, the compounds can also be formulated as a depot preparation. Such long acting formulations can be administered by implantation (for example, subcutaneously or intramuscularly) or by intramuscular injection. Thus, for example, the compounds can be formulated with suitable polymeric or hydrophobic materials (for example, as an emulsion in an acceptable oil) or ion exchange resins, or as sparingly soluble derivatives, for example, as a sparingly soluble salt.

Alternatively, other delivery systems for hydrophobic pharmaceutical compounds can be employed. Liposomes and emulsions are well known examples of delivery vehicles or carriers for hydrophobic drugs. In a presently preferred embodiment, long-circulating, *i.e.*, stealth liposomes can be employed. Such liposomes are generally described in Woodle, et al., U.S. Patent No. 5,013,556. The compounds of the present invention can also be administered by controlled release means and/or delivery devices such as those described in U.S. Patent Nos. 3,845,770; 3,916,899; 3,536,809; 3,598,123; and 4,008,719.

Certain organic solvents such as dimethylsulfoxide ("DMSO") also can be employed, although usually at the cost of greater toxicity. Additionally, the compounds can be delivered using a sustained-release system, such as semipermeable matrices of solid hydrophobic polymers containing the therapeutic agent. Various types of sustained-release materials have been established and are well known by those skilled in the art. Sustained-release capsules can, depending on their chemical nature, release the compounds for a few hours up to over 100 days.

The pharmaceutical compositions also can comprise suitable solid or gel phase carriers or excipients. Examples of such carriers or excipients include but are not limited to calcium carbonate, calcium phosphate, various sugars, starches, cellulose derivatives, gelatin, and polymers such as polyethylene glycols.

Pharmaceutical compositions suitable for use in the present invention include compositions wherein the active ingredients are contained in a therapeutically effective amount. The amount of composition administered will, of course, be dependent on the subject being treated, on the subject's weight, the severity of the affliction, the manner of administration and the judgment of the prescribing physician. Determination of an effective amount is well within the capability of those skilled in the art, especially in light of the detailed disclosure provided herein.

For any compound used in the method of the present invention, a therapeutically effective dose can be estimated initially from cell culture assays, animal models, or microdosing of human subjects.

Moreover, toxicity and therapeutic efficacy of the compounds described herein can be determined by standard pharmaceutical procedures in cell cultures or experimental animals, *e.g.*, by determining the $LD_{50}$, (the dose lethal to 50% of the population) and the $ED_{50}$ (the dose therapeutically effective in 50% of the population). The dose ratio between toxic and therapeutic effect is the therapeutic index and can be expressed as the ratio between $LD_{50}$ and $ED_{50}$. Compounds that exhibit high therapeutic indices are preferred. The data obtained from these cell culture assays and animal studies can be used in formulating a dosage range that is not toxic for use in humans. The dosage of such compounds lies preferably within a range of circulating concentrations that include the $ED_{50}$ with little or no toxicity. The dosage can vary within this range depending upon the dosage form employed and the route of administration utilized. The exact formulation, route of administration and dosage can be chosen by the individual physician in view of the patient's condition (*see, e.g.*, Fingl, et al., 1975 In: The Pharmacological Basis of Therapeutics, Ch. 1).

The amount of a compound of Formula (I) -(XIV) that can be combined with a carrier material to produce a single dosage form will vary depending upon the disease treated, the mammalian species, and the particular mode of administration. However, as a general guide, suitable unit doses for the compounds of the present invention can, for example, preferably contain between 0.1 mg to about 1000 mg of the active compound. A preferred unit dose is between 1 mg to about 500 mg. A more preferred unit dose is between 1 mg to about 300mg. Even more preferred unit dose is between 1 mg to about 100 mg. Such unit doses can be administered more than once a day, for example, 2, 3, 4, 5 or 6 times a day, but preferably 1 or 2 times per day, so that the total dosage for a 70 kg adult is in the range of 0.001 to about 15 mg per kg weight of subject per administration. A preferred dosage is 0.01 to about 1.5 mg per kg weight of subject per administration, and such therapy can extend for a number of weeks or months, and in some cases, years. It will be understood, however, that the specific dose level for any particular patient will depend on a variety of factors including the activity of the specific compound employed; the age, body weight, general health, sex and diet of the individual being treated; the time and route of administration; the rate of excretion; other drugs that have previously been administered; and the severity of the particular disease undergoing therapy, as is well understood by those of skill in the area.

A typical dosage can be one 1 mg to about 100 mg tablet or 1 mg to about 300 mg taken once a day, or, multiple

times per day, or one time-release capsule or tablet taken once a day and containing a proportionally higher content of active ingredient. The time-release effect can be obtained by capsule materials that dissolve at different pH values, by capsules that release slowly by osmotic pressure, or by any other known means of controlled release.

It can be necessary to use dosages outside these ranges in some cases as will be apparent to those skilled in the art. Further, it is noted that the clinician or treating physician will know how and when to start, interrupt, adjust, or terminate therapy in conjunction with individual patient response.

For the compositions, methods and kits provided above, one of skill in the art will understand that preferred compounds for use in each are those compounds that are noted as preferred above. Still further preferred compounds for the compositions, methods and kits are those compounds provided in the non-limiting Examples below.

## CHEMICAL EXAMPLES

General Methods. All operations involving moisture and/or oxygen sensitive materials were conducted under an atmosphere of dry nitrogen in pre-dried glassware. Unless noted otherwise, materials were obtained from commercially available sources and used without further purification.

Flash chromatography was performed on an Isco Combiflash Companion using RediSep *R*f silica gel cartridges by Teledyne Isco. Thin layer chromatography was performed using precoated plates purchased from E. Merck (silica gel 60 $PF_{254}$, 0.25 mm) and spots were visualized with long-wave ultraviolet light followed by an appropriate staining reagent.

Nuclear magnetic resonance ("NMR") spectra were recorded on a Varian Inova-400 resonance spectrometer. $^1$H NMR chemical shifts are given in parts per million (δ) downfield from tetramethylsilane ("TMS") using TMS or the residual solvent signal ($CHCl_3$ = δ 7.24, DMSO = δ 2.50) as internal standard. $^1$H NMR information is tabulated in the following format: multiplicity (s, singlet; d, doublet; t, triplet; q, quartet; m, multiplet), coupling constant(s) (*J*) in Hertz, number of protons. The prefix app is occasionally applied in cases where the true signal multiplicity was unresolved and br indicates the signal in question was broadened.

The compounds were named using ChemBioDraw Ultra Version 11.0.

LCMS analysis was performed using a PE SCIEX API 2000 spectrometer with a Phenomenex Luna 5 micron $C_{18}$ column.

Preparatory HPLC was performed on a Gilson HPLC 215 liquid handler with a Phenomenex column (Gemini 10 $\mu$, $C_{18}$, 110A) and a UV/VIS 156 detector.

When production of starting materials is not particularly described, the compounds are known or may be prepared analogously to methods known in the art or as disclosed in the examples. One of skill in the art will appreciate that synthetic methodologies described herein are only representative of methods for preparation of the compounds of the present invention, and that other well known methods may similarly be used. The present invention is further exemplified, but not limited, by the following examples that illustrate the preparation of the compounds of the invention.

Preparation of Intermediates

Intermediate 1 (3-Methyl-1-phenyl-1*H*-pyrazol-5-yl)methanol (**2**)

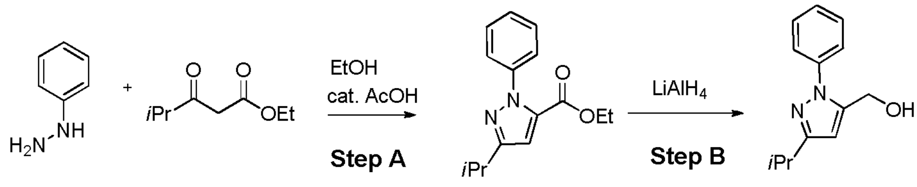


**1** **2**

**Step A:** To a solution of ethyl 4-methyl-3-oxopentanoate (2.5 g, 13.4 mmol) in ethanol (20 mL) was added phenylhydrazine and acetic acid (0.5 mL). The solution was stirred for 24 h and *concentrated in vacuo.* The residue contained two regioisomers of which the desired one was less polar. The mixture was purified by chromatography (0-20% EtOAc in hexanes) to give the ester **(1)** as a yellow oil.

**Step B:** The ester **(1)** (0.95 g, 3.68 mmol) was dissolved in anhydrous THF (4 mL) and cooled to 0 °C under nitrogen. Lithium aluminum hydride in THF (1.0 M, 4.05 mL, 4.05 mmol) was added over a ten minute period. After the addition was complete, the solution was allowed to warm to room temperature and stirred for an additional hour. The

solution was cooled to 0 °C and quenched by the addition of ethyl acetate (5 mL) followed by a saturated sodium sulfate aqueous solution (5 mL). The mixture was diluted with ethyl acetate and filtered through a pad of celite. The combined filtrates were dried over sodium sulfate and *concentrated in vacuo.* The residue was purified by silica gel chromatography (0-100% EtOAc in hexanes) to provide the desired alcohol **(2)** as a yellow oil.

Intermediate 2

5-(Bromomethyl)-4-phenyl-1,2,3-thiadiazole (5)

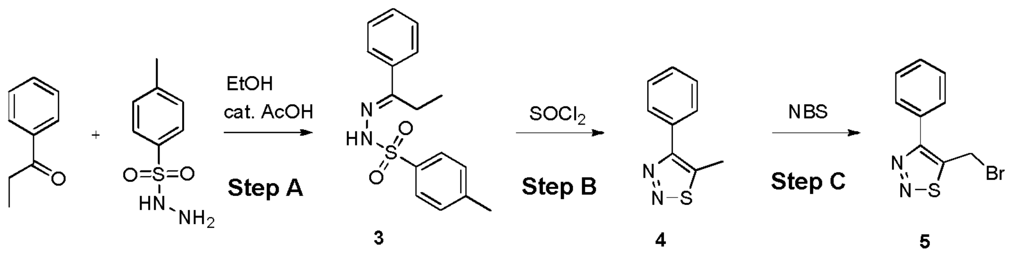


3 4 5

**Step A:** To a solution of propiophenone (2.5 g, 18.6 mmol) in ethanol (35 mL) was added 4-methylbenzenesulfonohydrazide (3.53 g, 20.5 mmol) followed by acetic acid (0.35 mL). The solution was refluxed for 1 h and cooled to room temperature. The solvent was *removed in vacuo* and the residue was rinsed with methanol. The white solid product (*E*)-4-methyl-*N'*-(1-phenylpropylidene)benzenesulfonohydrazide **(3)** was collected by filtration.

**Step B:** The hydrazone **(3)** (3.0 g, 9.9 mmol) was dissolved in thionyl chloride (10 mL) and stirred at rt for 1 h. The resulting solution was slowly poured into an aqueous solution of sodium hydroxide (1.0 N, 50 mL). The solution was extracted with DCM, the organic layer was dried over sodium sulfate, filtered, and *concentrated in vacuo.* The residue was purified by silica gel chromatography (0-30% EtOAc in hexanes) to provide 5-methyl-4-phenyl-1,2,3-thiadiazole **(4)** as a yellow oil.

**Step C:** To a solution ofthiadiazole **(4)** (1.6 g, 9.08 mmol) in tetrachloromethane (40 mL) was added *N*-bromosuccinimide (3.23 g, 18.2 mmol) and benzoyl peroxide (0.314 g, 0.91 mmol). The solution was refluxed for 24 hrs. After the solution was cooled to room temperature, the tetrachloromethane was *removed in vacuo.* The residue was dissolved in ethyl acetate and washed with water. The organic layer was separated, dried over sodium sulfate, filtered and *concentrated in vacuo.* The residue was chromatographed on silica gel (0-30% EtOAc in hexanes) to isolate 5-(bromomethyl)-4-phenyl-1,2,3-thiadiazole **(5).**

Intermediate 3

(1-Phenyl-1*H*-imidazol-5-yl)methanol **(8)**

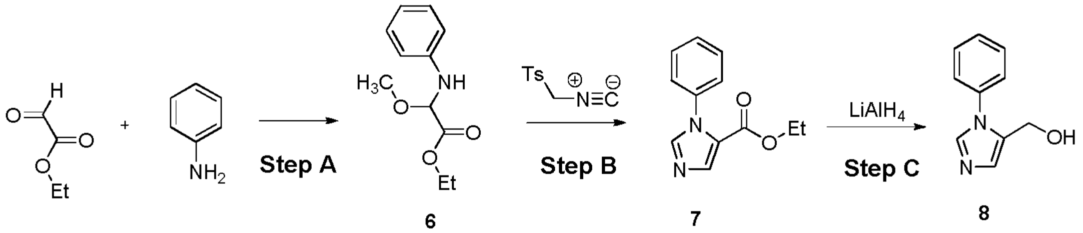


6 7 8

**Step A:** To a solution of aniline (2.3 mL, 25 mmol) in methanol (50 mL) was added ethyl glyoxalate. The solution was refluxed for 3.5 h, cooled to room temperature and the solvent was *removed in vacuo.* The resulting oil **(6)** was used in the next step without further purification.

**Step B:** To a solution of ethyl 2-methoxy-2-(phenylamino)acetate **(6)** (3.13 g, 15 mmol) in ethanol (30 mL) was added 1-(isocyanomethylsulfonyl)-4-methylbenzene (4.4 g, 22.5 mmol) and potassium carbonate (4.14 g, 30 mmol). The suspension was heated at 65 °C for four hours. After cooling to room temperature, the mixture was poured into

water and extracted with ethyl acetate. The organic layer was separated, dried over sodium sulfate, filtered and *concentrated in vacuo.* The residue was purified by preparatory HPLC to yield ethyl 1-phenyl-1H imidazole-5-carboxylate **(7).**

**Step C:** The ester (7) (0.80 g, 3.70 mmol) was dissolved in anhydrous THF (4 mL) and cooled to 0 °C under nitrogen. Lithium aluminum hydride in THF (1.0 M, 3.7 mL, 3.7 mmol) was added over a ten minute period. After the addition was complete, the solution was allowed to warm to room temperature and stirred for an additional sixty minutes. The solution was cooled to 0 °C and quenched by the addition of ethyl acetate (5 mL) followed by a saturated sodium sulfate aqueous solution (5 mL). The mixture was diluted with ethyl acetate and filtered through a pad of celite. The combined filtrates were dried over sodium sulfate and *concentrated in vacuo.* The residue oil was purified by silica gel chromatography (0-100% EtOAc in hexanes) to provide the desired alcohol **(8).**

Intermediate 4

5-(Bromomethyl)-1-isobutyl-3-methyl-1*H*-pyrazole **(11)**

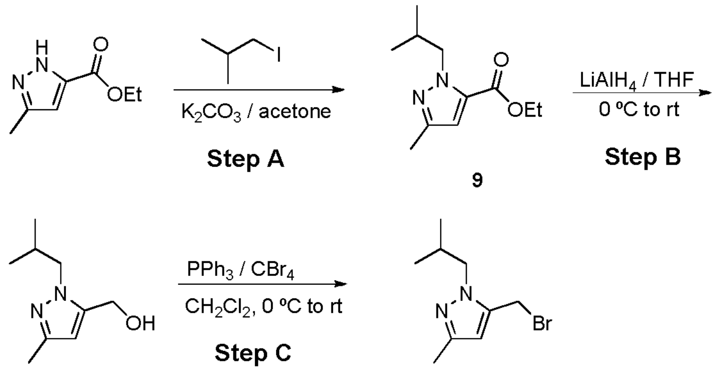


9

10

11

**Step A:** To a solution of ethyl 3-methylpyrazole-5-carboxylate (5.04 g, 0.032 mol) in acetone (20 mL) was added $K_2CO_3$ (18.14 g), followed by 1-iodo-2-methylpropane (22.21 g). The mixture was refluxed at 70 °C overnight. The mixture was filtered through celite, and the filtrate was *concentrated in vacuo.* The residue contained two regioisomers of which the desired one was less polar. Purification by flash chromatography on silica gel (0-30 % EtOAc in hexanes) gave 2.14 g of ethyl 1-isobutyl-3-methyl-1*H*-pyrazole-5-carboxylate **(9)** as a colorless liquid. $^1$H NMR (400 Hz, $CDCl_3$) δ: 6.61 (s, 1H), 4.38 - 4.24 (m, 4H), 2.27 (s, 3H), 2.24 - 2.13 (m, 1H), 1.36 (t, *J*= 7.1 Hz, 3H), 0.88 (d, *J*= 6.7 Hz, 6H).

**Step B:** To a solution of ethyl 1-isobutyl-3-methyl-1*H*-pyrazole-5-carboxylate **(9)** (1.99 g, 9.46 mmol) in THF (10 mL) at 0 °C was added a solution of lithium aluminum hydride in THF (1M, 15 mL, 15 mmol). After stirring at room temperature overnight, 10 mL of EtOAc was added and the mixture was stirred for 10 minutes. Water (15 mL) was added and the mixture was stirred for another 10 minutes, filtered through celite and rinsed with EtOAc. The filtrate was partitioned between EtOAc and $H_2O$/brine, washed with $H_2O$/brine, dried over $Na_2SO_4$ and *concentrated in vacuo* to afford 1.73 g of desired alcohol (10) as a colorless liquid. The product was sufficiently pure to be used directly in the subsequent bromination step. $^1$H NMR (400 MHz, $CDCl_3$) δ: 5.97 (s, 1H), 4.62 (d, *J* = 5.8 Hz, 2H), 3.87 (d, *J* = 7.5 Hz, 2H), 2.35 - 2.25 (m, 1H), 2.25 (s, 3H), 0.90 (d, *J* = 6.7 Hz, 6H).

**Step C:** To a solution of crude ethyl (1-isobutyl-3-methyl-1*H*-pyrazol-5-yl)methanol **(10)** (1.72 g) and $PPh_3$ (2.99 g) in dichloromethane (10 mL) at 0 °C was added $CBr_4$ (3.76 g). After stirring at room temperature for 1 h, the reaction solution was *concentrated in vacuo.* Purification by flash chromatography on silica gel (0-40% EtOAc in hexanes) gave 1.614 g of (bromomethyl)-1-isobutyl-3-methyl-1*H*-pyrazole **(11)** as a colorless liquid. $^1$H NMR (400 MHz, $CDCl_3$) δ: 6.05 (s, 1H), 4.43 (s, 2H), 3.85 (d, *J* = 7.5 Hz, 2H), 2.38 - 2.24 (m, 1H), 2.23 (s, 3H), 0.94 (d, *J* = 6.7 Hz, 6H).

Intermediate 5

Ethyl 2-(2-(3,5-difluoro-4-hydroxyphenyl)cyclopropyl)acetate **(20)**

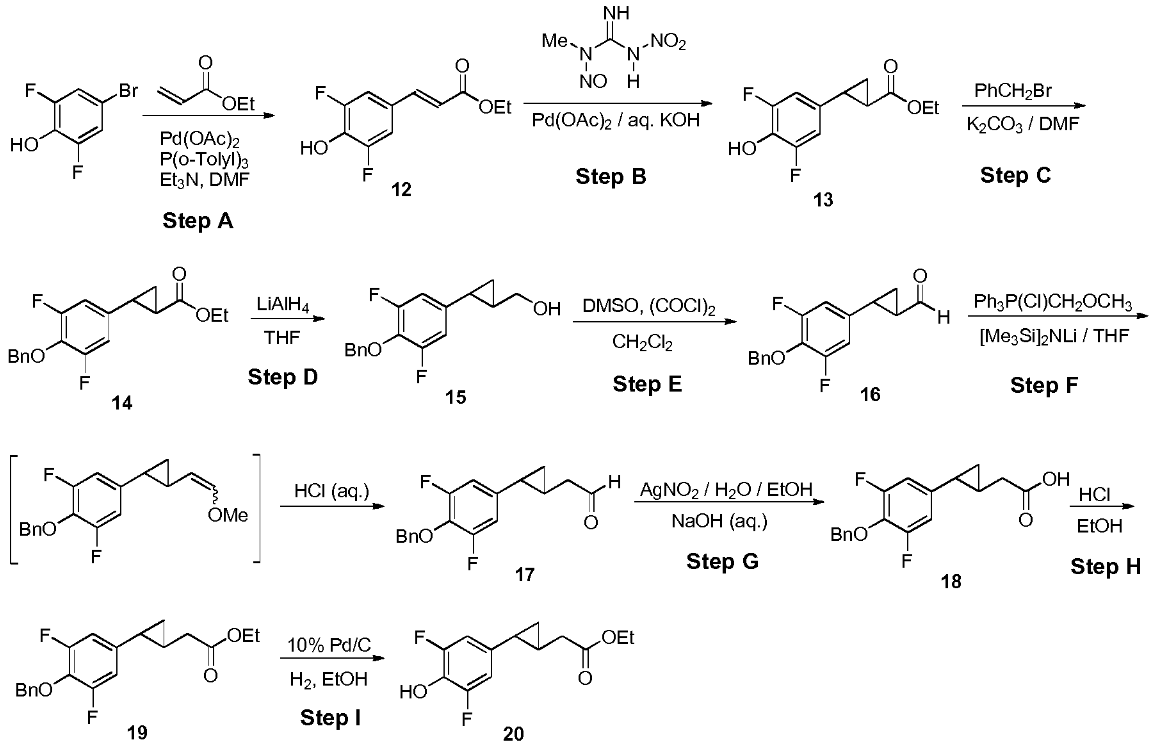


**Step A:** In a 350-mL pressure-tube was added 4-bromo-2,6-difluorophenol (23.82 g, 0.11 mol), triethylamine (55 mL, 0.39 mol), ethyl acrylate (34.27 g, 0.34 mol), DMF (50 mL), palladium (II) acetate (1.29 g, 5.75 mmol), and tri-*o*-tolyphosphine (2.34 g, 7.6 mmol) under $N_2$. The mixture was sealed in the glass tube and was stirred at 110 °C overnight (21 hours). The reaction was cooled to room temperature and EtOAc (150 mL) was added. The mixture was stirred for 30 minutes, filtered through celite and rinsed with EtOAc (100 mL × 3). The filtrate was acidified with 2N HCl to pH ~2. The organic layer was separated, and the aqueous layer was extracted with EtOAc (50 mL × 2). The organic layers were combined and washed with water ((100 mL × 2), brine (100 mL) and dried over $Na_2SO_4$. After filtration, heptane (200 mL) was added and the solution was *concentrated in vacuo.* The resulting precipitate was filtered, washed with heptane (50 mL × 2) and dried to afford the desired product **(12)** (17.09 g) as a light-yellow solid. The mother liquor was concentrated to obtain additional desired product (4.29 g) as a pale-yellow solid. $^1$H NMR (400 MHz, $CDCl_3$) δ: 7.50 (d, *J* = 15.9 Hz, 1H), 7.09 (d, *J* = 8.3 Hz, 2H), 6.29 (d, *J* = 15.9 Hz, 1H), 5.54 (*br,* 1H), 4.26 (q, *J* = 7.1 Hz, 2H), 1.33 (t, *J* = 7.1 Hz, 3H).

**Step B:** To a mixture of *N*-methyl-*N*'-nitro-*N*-nitrosoguanidine (TCI-America catalogue # M0527, 10 g on a dry weight basis, 0.068 mol) in ether (150 mL) at 0 °C was added a cold solution of KOH (12.60 g) in water (21 mL). After stirring for 2 minutes, a portion of the yellow ethereal solution of the resulting diazomethane was added to a solution of ethyl 3-(3,5-difluoro-4-hydroxyphenyl)acrylate **(12)** (2.28 g, 0.010 mol) in ether (100 mL) at 0 °C. A portion of palladium (II) acetate (0.372 g, 1.66 mmol) was added followed by an additional portion of diazomethane solution. This process was continued until all the diazomethane solution and palladium (II) acetate was added. The resulting dark mixture was stirred at 0-5 °C for 4 hours and acetic acid (6 drops) was added to quench any excess reagent. After removal of solvent, the residue was purified by chromatography on silica gel (0-30% EtOAc in hexanes) to afford 2.04 g of the desired product as a white solid (13). $^1$H NMR (400 MHz, $CDCl_3$) δ: 6.67 (d, *J* = 8.4 Hz, 2H), 5.05 (*br*, 1H), 4.20 (q, *J* = 7.1 Hz, 2H), 2.45 - 2.40 (m, 1H), 1.87 - 1.74 (m, 1H), 1.39 - 1.14 (m, 5H).

**Step C:** To a mixture of ethyl 2-(3,5-difluoro-4-hydroxyphenyl) cyclopropanecarboxylate **(13)** (2.04 g, 8.4 mmol) and $K_2CO_3$ (1.69 g, 12.2 mmol) in DMF (15 mL) was added benzyl bromide (1.88g, 11 mmol). The mixture was stirred at rt overnight and partitioned between ethyl acetate and water. The organic extract was washed with water and brine, dried over sodium sulfate and *concentrated in vacuo.* Purification by flash chromatography on silica gel (0-20% EtOAc in hexanes) gave 2.76 g of desired product **(14)** as a white solid. $^1$H NMR (400 MHz, $CDCl_3$) δ: 7.50 - 7.43 (m, 2H), 7.38-7.32 (m, 3H), 6.62 (d, *J* = 9.0 Hz, 2H), 5.12 (s, 2H), 4.19 - 4.11 (m, 2H), 2.43 - 2.38 (m, 1H), 1.89 - 1.76 (m, 1H), 1.65 - 1.58 (m, 1H), 1.29 - 1.15 (m, 4H).

**Step D:** To a solution of ethyl 2-(4-(benzyloxy)-3,5-diffuorophenyl)cyclo propanecarboxylate **(14)** (2.74 g, 8.24 mmol) in THF (10 mL) at 0 °C was added a solution of $LiAlH_4$ (IN in ether, 12.5 mL). After stirring at rt for 2 hours, 8 mL of EtOAc was added and the solution was stirred for 10 minutes. Water (10 mL) was added and the mixture was stirred for an additional 10 minutes, filtered through celite and rinsed with EtOAc. The filtration was partitioned between EtOAc

and $H_2O$/brine, washed with $H_2O$/brine, dried over $Na_2SO_4$ and concentrated *in vacuo* to afford 2.25 g of desired product **(15)** as a colorless liquid. The product was sufficiently pure to be used directly in subsequent Swern oxidation. $^1$H NMR (400 MHz, $CDCl_3$) δ: 7.49 - 7.39 (m, 2H), 7.39 - 7.33 (m, 3H), 6.59 (d, *J* = 9.2 Hz, 2H), 5.10 (s, 2H), 3.68 - 3.51 (m, 2H), 1.81 -1.68 (m, 1H), 1.47 - 1.20 (m, 1H), 1.02-0.83 (m, 2H).

**Step E:** DMSO (2.5 mL) was added to a solution of oxalyl choride (2.12 g, 16.7 mmol) in anhy. $CH_2Cl_2$ (15 mL) at -78° C, and then a solution of (2-(4-(benzyloxy)-3,5-difluorophenyl)-cyclopropyl)methanol **(15)** (2.25 g, 7.75 mmol) in $CH_2Cl_2$ (5 mL) was added, followed by $Et_3N$ (5.6 mL). Purification by flash chromatography on silica gel (0-30%) gave 2.07 g of desired product **(16)** as a colorless liquid. $^1$H NMR (400 MHz, $CDCl_3$) δ: 9.37 (s, 1H), 7.47 - 7.41 (m, 2H), 7.40 - 7.29 (m, 3H), 6.65 (d, *J* = 7.1 Hz, 2H), 5.13 (s, 2H), 2.59 - 2.45 (m, 1H), 2.19 - 2.10 (m, 1H), 1.78 - 1.65 (m, 1H), 1.51 - 1.36 (m, 1H).

**Steps F, G and H:** These reactions were conducted according to the protocol described in US patent (US 2004/0092538, pp 40-41).

**Step I:** To a solution of ethyl 2-(2-(4-(benzyloxy)-3,5-difluorophenyl)cyclopropyl) acetate **(19)** (0.782 g, 2.25 mmol) in EtOAc/EtOH (5 mL/10 mL) was added 159 mg of 10% Pd/C, and the mixture was stirred under an $H_2$ balloon overnight. After filtration through celite and washing with EtOH, the filtrate was *concentrated in vacuo* to afford 0.508 g of desired product **(20)** as a pale-yellow liquid. The product was sufficiently pure to be used directly in subsequent couplings. $^1$H NMR (400 MHz, $CDCl_3$) δ: 6.67 (d, *J* = 8.4 Hz, 2H), 4.96 (*br,* 1H), 4.23 - 4.05 (m, 2H), 2.50 - 2.26 (m, 2H), 1.70 - 1.66 (m, 1H), 1.33 - 1.19 (m, 4H), 0.97 - 0.79 (m, 2H).

Intermediate 6

(4-Isopropoxy-2-methylthiazol-5-yl)methanol (23)

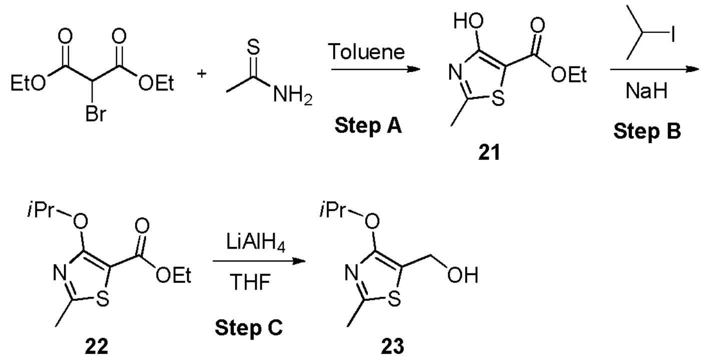


**Step A:** To a solution of bromomalonate (7.1 g, 29.7 mmol) in toluene (30 mL) was added thiacetamide (2.23 g, 29.7 mmol). After refluxing for 4 h, the solution was cooled and the solvent was *removed in vacuo.* The residue was dissolved in ethyl acetate, washed with water, saturated sodium bicarbonate and brine. The organic layer was separated, dried over sodium sulfate, filtered and *concentrated in vacuo.* The resulting solid **(21)** was washed with cold ether and collected by filtration.

**Step B:** To a solution **of (21)** and 2-iodopropane in DMF (2 mL) was added sodium hydride (60%, 0.026 g, 0.646 mmol). The mixture was heated at 50 °C for 18 h. After cooling, water was added (2 mL) and the solution was diluted with ethyl acetate (5 mL). The organic layer was separated and *concentrated in vacuo.* The residue was purified by silica gel chromatography (0-50 % EtOAc in hexanes) to provide the expected product **(22).**

**Step C:** The ester **(22)** (0.310 g, 1.35 mmol) was dissolved in anhydrous THF (4 mL) and cooled to 0 °C under nitrogen. Lithium aluminum hydride in THF (1.0 M, 1.6 mL, 1.6 mmol) was added over a ten minute period. After the addition was complete, the solution was allowed to warm to room temperature and stirred for an additional sixty minutes. The solution was cooled to 0 °C and quenched by the addition of ethyl acetate (5 mL) followed by a saturated sodium sulfate aqueous solution (5 mL). The mixture was diluted with ethyl acetate and filtered through a pad of celite. The combined filtrates were dried over sodium sulfate and concentrated *in vacuo.* The residue oil was purified by silica gel chromatography (0-100% EtOAc in hexanes) to provide the desired alcohol **(23).**

Intermediate 7

Methyl 2-(6-hydroxy-1,2,3,4-tetrahydronaphthalen-2-yl)acetate (26)

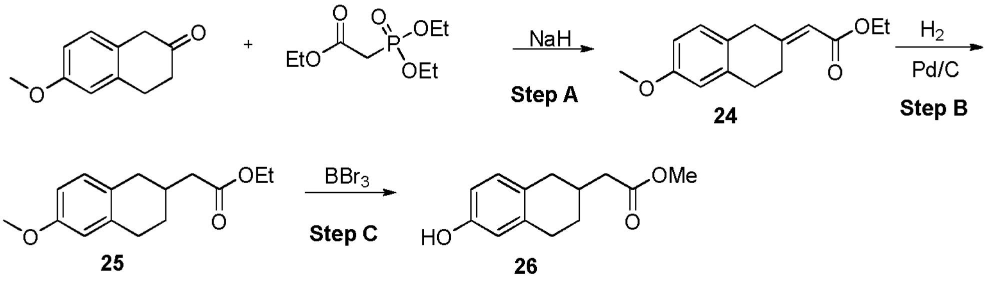


24 25 26

**Step A:** To a mixture of benzene (5 mL) and sodium hydride (680 mg, 17.01, mmol, 60% in mineral oil) at 0 °C was slowly added triethyl phosphonoacetate (1.82 mL, 9.08 mmol) and the reaction was stirred for 30 minutes. 6-methoxy-3,4-dihydronaphthalen-2(1H)-one (1g, 5.67 mmol) in benzene (1 mL) was added and stirred at room temperature overnight. The reaction was added to water (20 mL) and was extracted with ethyl acetate, dried over sodium sulfate, filtered and *concentrated in vacuo.* The residue was purified by flash column chromatography on silica gel with hexanes and EtOAc to afford (E)-ethyl 2-(6-methoxy-3,4-dihydronaphthalen-2(1H)-ylidene)acetate **(24).**

**Step B:** To a solution of (*E*)-ethyl 2-(6-methoxy-3,4-dihydronaphthalen-2(1*H*)-ylidene)acetate **(24)** (810 mg, 3.29 mmol) in ethanol (20 mL) was added Pd/C (81 mg, 10% Degussa type). A balloon of hydrogen gas was added and the reaction was evacuated and back-filled with hydrogen three times. The reaction was stirred overnight at room temperature, was filtered through a pad of celite and concentrated in *vacuo* to give ethyl 2-(6-methoxy-1,2,3,4-tetrahydronaphthalen-2-yl)acetate **(25).**

**Step C:** To a solution of ethyl 2-(6-methoxy-1,2,3,4-tetrahydronaphthalen-2-yl)acetate **(25)** (766 mg, 3.09 mmol) in (31 mL) of dichloromethane at 0 °C was added boron tribromide (2.92 mL, 30.9 mmol) and was stirred for three hours. The reaction was quenched with methanol extremely slowly, added to saturated sodium bicarbonate, and extracted with ethyl acetate. The organic layer was dried over sodium sulfate, filtered, and concentrated in *vacuo.* The residue was purified by flash column chromatography on silica gel with hexanes and EtOAc to afford methyl 2-(6-hydroxy-1,2,3,4-tetrahydronaphthalen-2-yl)acetate **(26).**

Intermediate 8

Ethyl 3-(3,5-difluoro-4-hydroxyphenyl)-2-methylpropanoate (30)

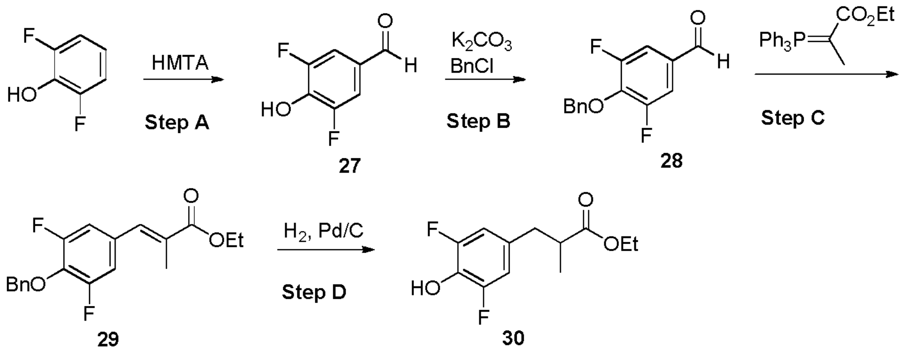


27 28 29 30

**Step A:** A solution of 2,6-difluorophenol (25g, 192 mmol), hexamethylenetetramine (26 g, 192 mmol) and trifluoroacetic acid (190 mmol) was refluxed overnight. The reaction was cooled and diluted with water (200 mL) and extracted with dichloromethane (3 x 100 mL). The organic layer was washed with 10% aqueous potassium carbonate (2 x 100 mL). The aqueous layer was acidified with concentrated hydrochloric acid and extracted with ethyl acetate. The organic layer was dried over sodium sulfate, filtered, and concentrated in *vacuo* to yield 3,5-difluoro-4-hydroxybenzaldehyde as a white solid **(27).** Upon sitting the desired product began to precipitate from the original aqueous layer that was extracted with dichloromethane. The layer was filtered to provide the product as long white crystals.

**Step B:** To a mixture of 3,5-difluoro-4-hydroxybenzaldehyde **(27)** (8.26 g, 52.2 mmol), and potassium carbonate (14.4 g, 104.4 mmol) in dimethylformamide (100 mL) was added benzyl chloride (7.2 mL, 62.7 mmol) and stirred overnight at 50 °C. The reaction was diluted with water and extracted with ethyl acetate (3 x 75 mL). The organic layer was dried over sodium sulfate, filtered, and concentrated in *vacuo.* The residue was purified by flash column chromatography on silica gel with hexanes and EtOAc to afford 4-(benzyloxy)-3,5-difluorobenzaldehyde **(28).**

**Step C:** A solution of 4-(benzyloxy)-3,5-difluorobenzaldehyde **(28)** (1.32 g, 5.34 mmol) and (1-ethoxycarbonylethylidene)triphenyl phosphorane (2.32 g, 6.41 mmol) in tetrahydrofuran (53 mL) was refluxed for 2 hours. The reaction was concentrated in *vacuo* and was purified by flash column chromatography on silica gel with hexanes and EtOAc to give (*E*)-ethyl 3-(4-(benzyloxy)-3,5-difluorophenyl)-2-methylacrylate **(29).**

**Step D:** To a solution (*E*)-ethyl 3-(4-(benzyloxy)-3,5-difluorophenyl)-2-methylacrylate **(29)** (1.4 g, 4.21 mmol) in ethanol (25 mL) was added Pd/C (140 mg, 10% Degussa type). A balloon of hydrogen gas was added and the reaction was evacuated and back-filled with hydrogen three times. The reaction was stirred overnight at room temperature, was filtered through a pad of celite and concentrated in *vacuo* to give ethyl 3-(3,5-difluoro-4-hydroxyphenyl)-2-methylpropanoate **(30).**

Intermediate 9

Ethyl 3-(3,5-difluoro-4-hydroxyphenyl)-2,2-dimethylpropanoate **(34)**

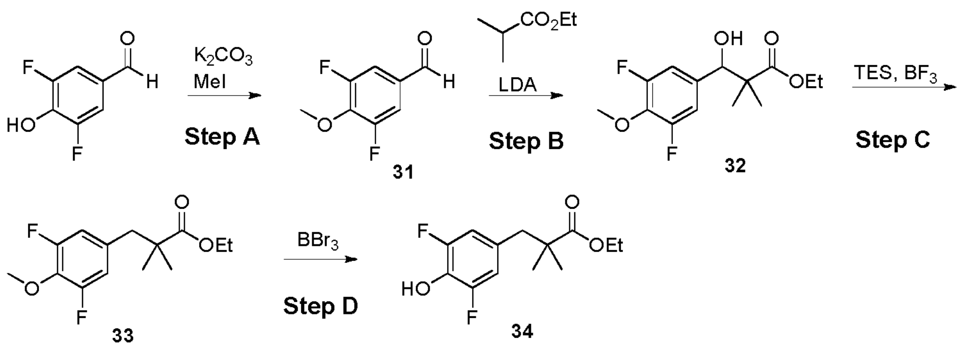


**Step A:** To a mixture of 3,5-difluoro-4-hydroxybenzaldehyde (2.55 g, 16.3 mmol), and potassium carbonate (4.5 g, 32.3 mmol) in dimethylformamide (30 mL) was added methyl iodide (1.2 mL, 19.4 mmol) and stirred overnight at room temperature. The reaction was diluted with water and extracted with ethyl acetate (3 x 75 mL). The organic layer was dried over sodium sulfate, filtered, and concentrated in *vacuo.* The residue was purified by flash column chromatography on silica gel with hexanes and EtOAc to afford 3,5-difluoro-4-methoxybenzaldehyde **(31).**

**Step B:** Lithium diisopropylamide (5 mL, 10.1 mmol, 2M tetrahydrofuran /heptane/ethylbenzene) was added to a solution of ethyl isobutyrate (1.36 mL, 10.1 mmol) in tetrahydrofuran (4 mL) at -78 °C and was stirred for 2 hours. 3,5-difluoro-4-methoxybenzaldehyde **(31)** (754 mg, 4.38 mmol) was added and the reaction was allowed to warm to room temperature overnight. Quenched with water and extracted with ethyl acetate (3 x 10 mL). The organic layer was dried over sodium sulfate, filtered, and concentrated in *vacuo.* The residue was purified by flash column chromatography on silica gel with hexanes and EtOAc to yield ethyl 3-(3,5-difluoro-4-methoxyphenyl)-3-hydroxy-2,2-dimethylpropanoate **(32).**

**Step C:** To a solution of ethyl 3-(3,5-difluoro-4-methoxyphenyl)-3-hydroxy-2,2-dimethylpropanoate **(32)** (200 mg, 0.69 mmol), triethylsilane (62 μL, 0.76 mmol) and dichloromethane (2 mL) at 0 °C was added boron trifluoride diethyl etherate (95 μL, 0.76 mmol) and stirred for two hours at room temperature. Starting material was still present by TLC so an additional 0.5 mL of triethylsilane and 0.5 mL of boron trifluoride diethyl etherate were added and stirred at room temperature overnight. The reaction was quenched with saturated sodium bicarbonate and extracted with ethyl acetate (3 x 10 mL). The organic layer was dried over sodium sulfate, filtered, and concentrated in *vacuo.* The residue was purified by flash column chromatography on silica gel with hexanes and EtOAc to give ethyl 3-(3,5-difluoro-4-methoxyphenyl)-2,2-dimethylpropanoate **(33).**

**Step D:** To a solution of ethyl 3-(3,5-difluoro-4-methoxyphenyl)-2,2-dimethylpropanoate **(33)** (155.7 mg, 0.57 mmol) in dichloromethane (6 mL) at 0 °C was added boron tribromide (0.54 mL, 5.71 mmol) and was stirred for three hours. The reaction was quenched with ethanol extremely slowly, added to saturated sodium bicarbonate, and extracted

with ethyl acetate. The organic layer was dried over sodium sulfate, filtered, and concentrated in *vacuo.* The residue was purified by flash column chromatography on silica gel with hexanes and EtOAc to afford ethyl 3-(3,5-difluoro-4-hydroxyphenyl)-2,2-dimethylpropanoate **(34).**

Intermediate 10

Ethyl 2-(3,5-difluoro-4-hydroxybenzyloxy)acetate (37)

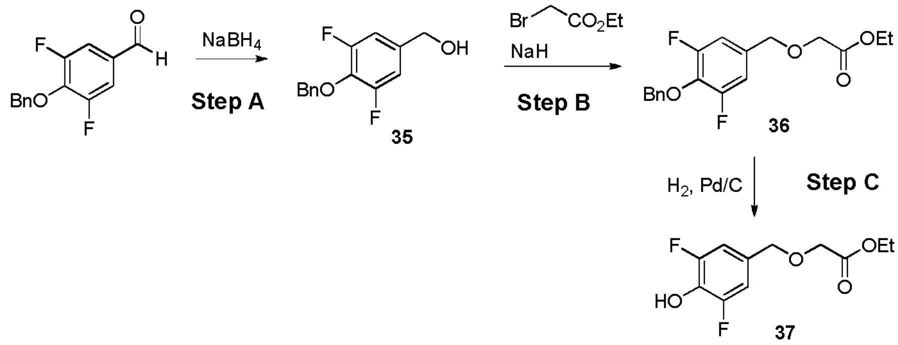


**Step A:** To a solution of 4-(benzyloxy)-3,5-difluorobenzaldehyde (1g, 4.03 mmol) in tetrahydrofuran:methanol (2:1) (22 mL) was added sodium borohydride (304 mg, 8.1 mmol) and was stirred for two hours. The reaction was quenched with water and extracted with ethyl acetate (3 x 25 mL). The organic layer was dried over sodium sulfate, filtered, and concentrated in *vacuo* to give (4-(benzyloxy)-3,5-difluorophenyl)methanol **(35).**

**Step B:** To a solution of (4-(benzyloxy)-3,5-difluorophenyl)methanol **(35)** (890 mg, 3.56 mmol) in tetrahydrofuran (35 mL) was added sodium hydride (156 mg, 3.91 mmol, 60% in mineral oil) and stirred at room temperature for 30 minutes. Ethyl bromoaceate (0.44 mL, 3.91 mmol) was added and the reaction was stirred overnight. The reaction was quenched with water and extracted with ethyl acetate (3 x 50 mL). The organic layer was dried over sodium sulfate, filtered, and concentrated in *vacuo.* The residue was purified by flash column chromatography on silica gel with hexanes and EtOAc to provide ethyl 2-(4-(benzyloxy)-3,5-difluorobenzyloxy)acetate **(36).**

**Step C:** To a solution ethyl 2-(4-(benzyloxy)-3,5-difluorobenzyloxy)acetate **(36)** (363 mg, 1.08 mmol) in ethanol (25 mL) was added Pd/C (36 mg, 10% Degussa type). A balloon of hydrogen gas was added and the reaction was evacuated and back-filled with hydrogen three times. The reaction was stirred overnight at room temperature, was filtered through a pad of celite and concentrated in *vacuo* to afford ethyl 2-(3,5-difluoro-4-hydroxybenzyloxy) acetate **(37).**

Intermediate 11

(*R*)-4-benzyl-3-((*S*)-3-(3,5-difluoro-4-hydroxyphenyl)-2-methylpropanoyl)oxazolidin-2-one **(40)** and (*R*)-4-benzyl-3-((*R*)-3-(3,5-difluoro-4-hydroxyphenyl)-2-methylpropanoyl)oxazolidin-2-one **(41)**

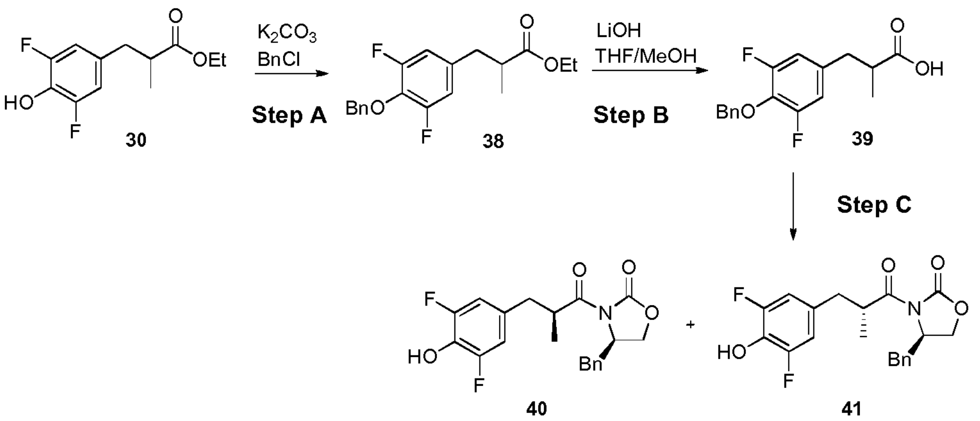


**Step A:** To a mixture of ethyl 3-(3,5-difluoro-4-hydroxyphenyl)-2-methylpropanoate **(30)** (930 mg, 3.81 mmol), and potassium carbonate (1.05 g, 7.62 mmol) in DMF (8 mL) was added benzyl chloride (0.53 mL, 4.57 mmol) and stirred overnight at 50 °C. The reaction was diluted with water and extracted with ethyl acetate (3 x 25 mL). The organic layer was dried over sodium sulfate, filtered, and concentrated in *vacuo.* The residue was purified by flash column chromatography on silica gel with hexanes and EtOAc to afford ethyl 3-(4-(benzyloxy)-3,5-difluorophenyl)-2-methylpropanoate **(38).**

**Step B:** To a mixture of 3-(4-(benzyloxy)-3,5-difluorophenyl)-2-methylpropanoate (38) (1.09 g, 3.26 mmol) in tetrahydrofuran (10 mL), water (10 mL), and methanol (20 mL) was added lithium hydroxide (547 mg, 13.04 mmol) and was stirred overnight at 80 °C. The reaction was concentrated, acidified with 1N hydrochloric acid, and extracted with ethyl acetate. The organic layer was dried over sodium sulfate, filtered, and concentrated in *vacuo* to yield 3-(4-(benzyloxy)-3,5-difluorophenyl)-2-methylpropanoic acid **(39).**

**Step C:** To a solution of 3-(4-(benzyloxy)-3,5-difluorophenyl)-2-methylpropanoic acid (0.99 g, 3.23 mmol) in tetrahydrofuran (2.5 mL) at 0 °C was added triethylamine (0.50 mL, 3.57 mmol) and pivaloyl chloride (0.44 mL, 3.57 mmol) and the reaction was stirred for 30 minutes. In a separate flask (R)-4-benzyloxazolidin-2-one (0.48 g, 2.69 mmol) was dissolved in tetrahydrofuran (4 mL) and cooled to -78 °C. nBuLi (1.77 mL, 2.69 mmol, 1.52 M in hexanes) was added and the reaction was stirred for 30 minutes. The solution of 3-(4-(benzyloxy)-3,5-difluorophenyl)-2-methylpropanoic acid was added to the (R)-4-benzyloxazolidin-2-one solution and stirred at -78 °C for 3 hours and at room temperature for 30 minutes. The reaction was quenched with saturated ammonium chloride and extracted with ethyl acetate. The organic layer was dried over sodium sulfate, filtered, and *concentrated in vacuo.* The residue was purified by flash column chromatography on silica gel with hexanes and EtOAc to afford the two diastereomers ((*R*)-4-benzyl-3-((*R*)-3-(3,5-difluoro-4-hydroxyphenyl)-2-methylpropanoyl)oxazolidin-2-one and ((*R*)-4-benzyl-3-((*S*)-3-(3,5-difluoro-4-hydroxyphenyl)-2-methylpropanoyl)oxazolidin-2-one.

Intermediate 12

Ethyl 2-(4-hydroxyphenylthio)acetate **(42)**

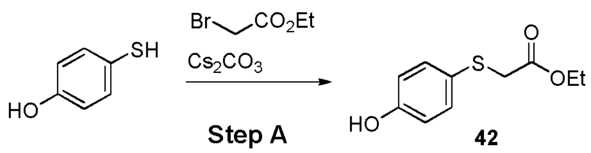


**Step A:** To a solution of 4-mercaptophenol (49.7 mg, 0.39 mmol) in tetrahydrofuran (2 mL) was added cesium carbonate (128 mg, 0.39 mmol) and ethyl bromoaceate (44 μL, 0.39 mmol) and the reaction was stirred at 50 °C overnight. The reaction was quenched with water and extracted with ethyl acetate (3 x 10 mL). The organic layer was dried over sodium sulfate, filtered, and concentrated in *vacuo.* The residue was purified by flash column chromatography on silica gel with hexanes and EtOAc to afford ethyl 2-(4-hydroxyphenylthio)acetate **(42).**

Intermediate 13

(5-Phenylthiazol-4-yl)methanol **(44)**

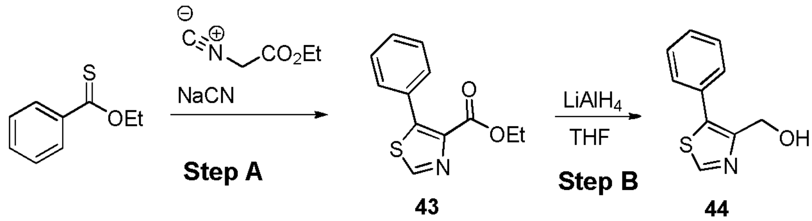


43 44

**Step A:** To a suspension of sodium cyanide (0.32 g, 6.5 mmol) in ethanol (65 mL), was added a solution of *O*-ethyl benzothioate (8.3g, 49.9 mmol) and ethyl isocyanoacetate (6.03 mL, 54.9 mmol) in ethanol (30 mL). The reaction was heated at 50 °C overnight and the concentrated in *vacuo.* The residue was purified by flash column chromatography on silica gel with hexanes and EtOAc to afford ethyl 5-phenylthiazole-4-carboxylate **(43).**

**Step B:** To a solution of ethyl 5-phenylthiazole-4-carboxylate **(43)** (1g, 4.28 mmol) in tetrahydrofuran (43 mL) at 0 °C was added lithium aluminum hydride (4.3 mL, 4.28 mmol, 1M in tetrahydrofuran) and the reaction was allowed to warm to room temperature overnight. The reaction was quenched with water, filtered through celite, and concentrated in *vacuo.* The residue was purified by flash column chromatography on silica gel with hexanes and EtOAc to provide (5-phenylthiazol-4-yl)methanol **(44).**

Intermediate 14

Ethyl 3-(3,5-difluoro-4-hydroxyphenyl)-2,2-difluoropropanoate **(46)**

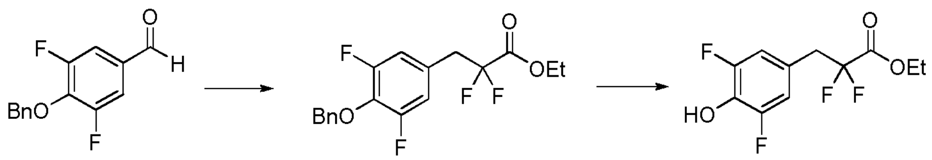

45 46

Compound **(46)** was prepared in a similar manner as that described in *Synthesis* **2000,** *13*, 1917-1924. $^1H$ NMR (400 MHz, $CDCl_3$) $\delta$: 6.83 (d, $J$ = 7.2 Hz, 2H), 4.28 (q, $J$ = 7.2 Hz, 2H), 3.27 (t, $J$ = 16 Hz, 2H), 1.3 (d, $J$ = 7.2 Hz, 2H).

Intermediate 15

Ethyl 3-(3,5-difluoro-4-hydroxyphenyl)-2-fluoropropanoate (48)

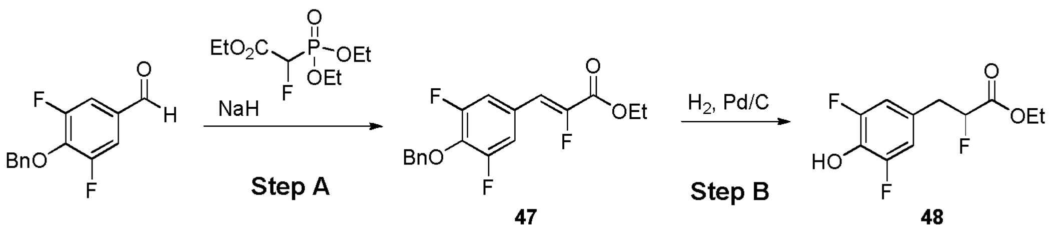


47 48

**Step A:** Sodium hydride (0.3 g, 7.5 mmol) was added to a mixture of 4-(benzyloxy)-3,5-difluorobenzaldehyde

(1.2 g, 5 mmol) and triethyl 2-fluoro-2-phosphonoacetate (1.2 g, 6mmol) in anhydrous THF (10 mL) at room temperature. The reaction was stirred for 2 hours and quenched with $H_2O$. The product was extracted with ethyl acetate. The organic phase was washed with $H_2O$, brine, dried with $Na_2SO_4$ and concentrated under reduced pressure. The residue was purified by flash chromatography on silica gel to yield a yellow oil **(47)** (1 g).

**Step B:** Compound **(47)** was dissolved in ethyl acetate (20 mL) and to the solution was added 100 mg of 10% Pd/C, and the mixture was stirred with $H_2$ balloons for 24 hours. After filtration through celite and washing with ethyl acetate, the filtrate was concentrated under reduced pressure. The residue was purified by flash chromatography on silica gel to yield a solid **(48)** (0.6 g). $^1H$ NMR (400 MHz, $CDCl_3$) δ: 6.81 (d, *J* = 8.4 Hz, 2H), 5.2 (*br,* 1H), 5.05 (ddd, *J* = 52, 7, 4 Hz, 1H), 4.24 (q, *J* = 7.2 Hz, 2H), 3.2-3 (m, 2H), 1.28 (d, *J* = 7.2 Hz, 2H).

Intermediate 16

Ethyl 2-(3,5-difluoro-4-hydroxybenzyl)cyclopropanecarboxylate (52)

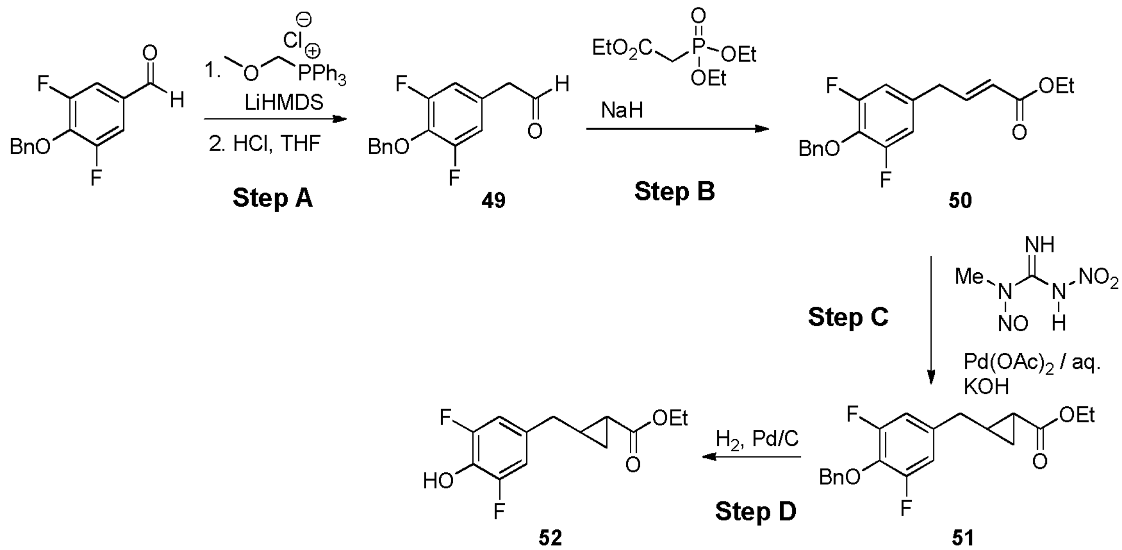


**Step A:** Lithium bis(trimethylsilyl) amide solution (30 mL, 30 mmol) was added to a mixture of methoxy methyltriphenyl phosphonium chloride (10.3 g, 30 mmol) in anhydrous THF (25 mL) at 0 °C under nitrogen. After 15 min, 4-(benzyloxy)-3,5-difluorobenzaldehyde (5 g, 20 mmol) in THF (25 mL) was added at 0 °C to the mixture. The reaction was stirred for 2 hours and quenched with $H_2O$. The product was extracted with ethyl acetate. The organic phase was washed with $H_2O$, brine, dried with $Na_2SO_4$ and concentrated under reduced pressure. The residue was purified by flash chromatography on silica gel to yield a yellow oil (5 g). The isolated oil was dissolved in THF (100 mL) and 2 N HCl (50 mL) solution. The resulting solution was stirred at 70 °C for 5 hours. The THF was evaporated and the residue was diluted with $H_2O$ and ethyl acetate. The organic phase was washed with $H_2O$, brine, dried with $Na_2SO_4$ and concentrated under reduced pressure. The residue was purified by flash chromatography on silica gel to yield a yellow oil **(49)** (3.3 g).

**Step B:** Sodium hydride (0.24 g, 6 mmol) was added to a mixture of 2-(4-(benzyloxy)-3,5-difluorophenyl) acetaldehyde **(49)** (1.3 g, 5 mmol) and triethyl phosphonoacetate (1.3 g, 6 mmol) in anhydrous THF (20 mL) at room temperature. The reaction was stirred for 5 hours and quenched with $H_2O$. The product was extracted with ethyl acetate. The organic phase was washed with $H_2O$, brine, dried with $Na_2SO_4$ and concentrated under reduced pressure. The residue was purified by flash chromatography on silica gel to yield a yellow oil **(50)** (1.1 g).

**Step C:** To a mixture of *N*-methyl-*N*'-nitro-*N*-nitrosoguanidine (10 g on a dry weight basis, 0.068 mol) in ether (150 mL) at 0 °C was added dropwise a cold solution of KOH (12.60 g) in water (21 mL). After stirring for 2 minutes, a yellow ethereal solution of resulting diazomethane on the top layer was added to a solution of ethyl (*E*)-ethyl 4-(4-(benzyloxy)-3,5-difluorophenyl)but-2-enoate **(50)** (1.1 g, 3.3 mol) in ether (50 mL) at 0 °C with palladium (II) acetate (0.4 g, 1.78 mmol) was added alternately. The resulting dark mixture was stirred at 0-5 °C for 4 hours. After removal of solvent, the residue was purified by chromatography on silica gel to afford the desired product **(51)** (1 g) as a yellow oil.

**Step D:** The ester **(51)** was dissolved in ethyl acetate (20 mL) and to the solution was added 100 mg of 10% Pd/C, and the mixture was stirred with $H_2$ balloons overnight. After filtration through celite and washing with ethyl acetate,

the filtrate was concentrated under reduced pressure. The residue was purified by flash chromatography on silica gel to yield a yellow oil **(52)** (0.7 g). $^1$H NMR (400 MHz, $CDCl_3$) δ: 6.67 (d, *J* = 8.4 Hz, 2H), 4.96 (*br,* 1H), 4.23 - 4.05 (m, 2H), 2.50 - 2.26 (m, 2H), 1.70 - 1.66 (m, 1H), 1.33 - 1.19 (m, 4H), 0.97 - 0.79 (m, 2H).

Intermediate 17

Ethyl 3-(3,5-difluoro-4-hydroxyphenyl)propanoate (53)

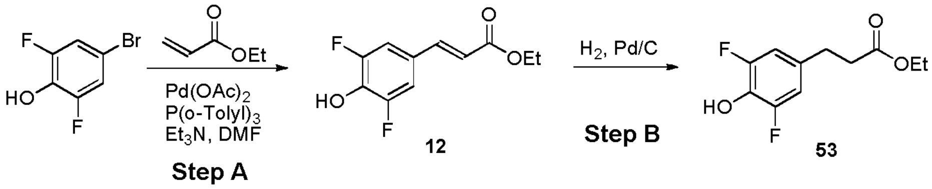


12

53

**Step A:** In a 350-mL pressure-glass was added 4-bromo-2,6-diffuorophenol (23.82 g, 0.11 mol), triethylamine (55 mL, 0.39 mol), ethyl acrylate (34.27 g, 0.34 mol), DMF (50 mL), palladium (II) acetate (1.29 g, 5.75 mmol), and followed by tri-*o*-tolyphosphine (2.34 g, 7.6 mmol) under $N_2$. The mixture in the sealed glass was stirred at 110 °C overnight (21 hours), cooled to room temperature and added EtOAc (150 mL) and stirred for 30 minutes, filtered through celite and rinsed with EtOAc (100 mL × 3). The filtrate was acidified with 2N HCl to pH -2. The organic layer was separated, and the aqueous layer was extracted with EtOAc (50 mL × 2). The organic layers were combined and washed with water ((100 mL × 2), brine (100 mL) and dried over $Na_2SO_4$. After filtration, heptane (200 mL) was added and the solution was *concentrated in vacuo.* The resulting precipitate was filtered, washed with heptane (50 mL × 2) and dried to afford the desired product (17.09 g) as a light-yellow solid. The mother liquor was concentrated to obtain additional desired product **(12)** (4.29 g) as a pale-yellow solid. $^1$H NMR (400 MHz, $CDCl_3$) δ: 7.50 (d, *J* = 15.9 Hz, 1H), 7.09 (d, *J* = 8.3 Hz, 2H), 6.29 (d, *J* = 15.9 Hz, 1H), 5.54 (*br,* 1H), 4.26 (q, *J* = 7.1 Hz, 2H), 1.33 (t, *J* = 7.1 Hz, 3H).

**Step B:** To a solution of (*E*)-ethyl 3-(3,5-difluoro-4-hydroxyphenyl)acrylate **(12)** (0.751 g, 3.29 mmol) in ethanol (20 mL) was added Pd/C (81 mg, 10% Degussa type). A balloon of hydrogen gas was added and the reaction was evacuated and back-filled with hydrogen three times. The reaction was stirred overnight at room temperature, was filtered through a pad of celite and concentrated in *vacuo* to give ethyl 3-(3,5-difluoro-4-hydroxyphenyl)propanoate **(53).**

Intermediate 18

(1-(4-chlorophenyl)-1H-pyrazol-5-yl)methanol **(251)**

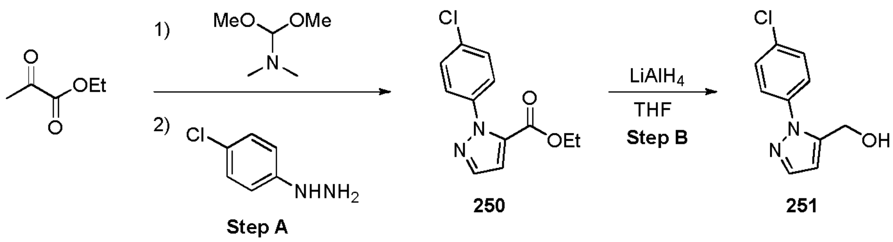


250

251

**Step A:** Ethyl pyruvate (1.91 mL, 17.2 mmol) and *N,N*-Dimethylformamide dimethyl acetal (2.29 mL, 17.2 mmol) were added to a scintillation vial and stirred overnight at room temperature. The dark red/brown solution was added to a solution of 4-chlorophenylhydrazine hydrochloride (3.08g, 17.2 mmol) in ethanol (50mL) and heated to 85 °C for 3 hours. The reaction was concentrated in *vacuo.* The residue was purified by flash column chromatography on silica gel with hexanes and EtOAc to afford ethyl 1-(4-chlorophenyl)-1H-pyrazole-5-carboxylate **(250).**

**Step B:** The ester **(250)** (0.600 g, 2.40 mmol) was dissolved in anhydrous THF (24 mL) and cooled to 0 °C under nitrogen. Lithium aluminum hydride in THF (1.0 M, 2.9 mL, 2.9 mmol) was added over a ten minute period. After the addition was complete, the solution was allowed to warm to room temperature and stirred for an additional sixty minutes. The solution was cooled to 0 °C and quenched by the addition of ethyl acetate (5 mL) followed by a saturated

sodium sulfate aqueous solution (5 mL). The mixture was diluted with ethyl acetate and filtered through a pad of celite. The combined filtrates were dried over sodium sulfate and *concentrated in vacuo.* The residue oil was purified by silica gel chromatography (0-100% EtOAc in hexanes) to provide the desired alcohol **(251).**

Intermediate 19

Ethyl 3-(3,5-difluoro-4-hydroxyphenyl)-2-ethoxypropanoate (254)

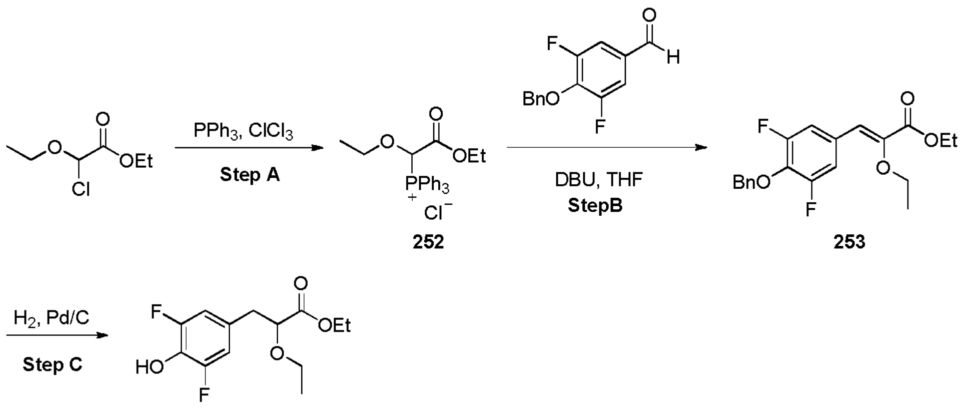


252

253

254

**Step A:** To a solution of 2-chloro-2-ethoxyacetic acid ethyl ester (10 g, 60 mmol) in chloroform (30 mL) was added triphenylphosphine (15.7 g, 60 mmol) and stirred overnight at room temperature. The solvent was removed in *vacuo,* and diethyl ether was added. The solvent was again removed and dried on high vacuum to give (1,2-diethoxy-2-oxoethyl)triphenylphosphonium chloride (21g, 82% yield) **(252)** as a foamy solid.

**Step B:** To a solution of (1,2-diethoxy-2-oxoethyl)triphenylphosphonium chloride (252) (1.61g, 3.76 mmol) in THF (56 mL) was added DBU (0.67 mL, 4.51 mmol) and the reaction was stirred for 10 minutes at room temperature. 4-(benzyloxy)-3,5-difluorobenzaldehyde (1.40 g, 5.64 mmol) was added in one portion and the reaction was stirred at room temperature for 18 hours. The solvent was removed in *vacuo,* diethyl ether was added and the solids filtered. The filtrate was concentrated in *vacuo* and the residue oil was purified by silica gel chromatography (0-30% EtOAc in hexanes) to provide (Z)-ethyl 3-(4-(benzyloxy)-3,5-difluorophenyl)-2-ethoxyacrylate **(253).**

**Step C:** To a solution (Z)-ethyl 3-(4-(benzyloxy)-3,5-difluorophenyl)-2-ethoxyacrylate **(253)** (1.3 g, 3.59 mmol) in ethanol (25 mL) was added Pd/C (140 mg, 10% Degussa type). A balloon of hydrogen gas was added and the reaction was evacuated and back-filled with hydrogen three times. The reaction was stirred overnight at room temperature, was filtered through a pad of celite and concentrated in *vacuo* to give ethyl 3-(3,5-difluoro-4-hydroxyphenyl)-2-ethoxypropanoate (0.81 g) **(254).**

Intermediate 20

ethyl 2-(6-fluoro-5-hydroxy-2,3-dihydro-1*H*-inden-1-yl)acetate (260)

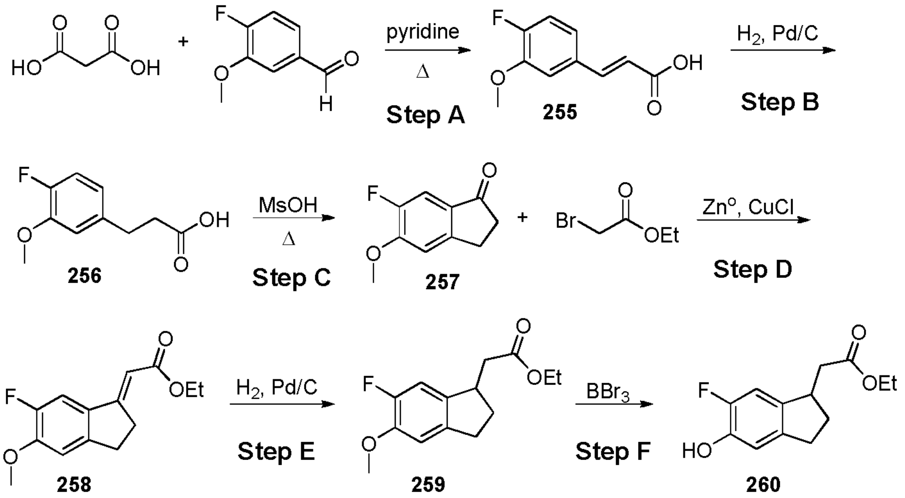


255 256 257 258 259 260

**Step A:** To a solution of malonic acid (21.5 g, 207 mmol) in pyridine (50 mL) was added 4-fluoro-3-methoxybenzaldehyde (16 g, 104 mmol) and piperidine (1.5 mL). The reaction was refluxed for 13 h. Water was added (25 mL) followed by concentrated HCl (40 mL). The precipitated product (255) was collected by filtration and washed with water.

**Step B:** To a solution of **(255)** (25 g, 127 mmol) in ethanol (40 mL) was added Pd/C (2 g, 10% Degussa type). A balloon of hydrogen gas was added and the reaction was evacuated and back-filled with hydrogen three times. The reaction was stirred overnight at room temperature, was filtered through a pad of celite and *concentrated in vacuo* to provide compound (256).

**Step C:** To a 20 mL $\mu$-wave tube was added compound **(256)** (2.00 g, 10.1 mmol) and methylsulfonic acid (15 mL). The tube was sealed and heated at 90 °C for 10 minutes. The resulting solution was poured into an ice bath, neutralized to pH 7 with aqueous NaOH. The resulting precipitate was collected by filtration and washed with water to provide compound **(257).**

**Step D:** To a solution of ketone **(257)** (3.56 g, 19.8 mmol) in toluene/THF (50:1, 40 mL) was added Zn° dust (2.6 g, 39.6 mmol) and copper (I) chloride (0.4 g, 3.96 mmol). The suspension was heated at 90 °C for 30 minutes. After cooling to room temperature, ethylbromoacetate (3.4 mL. 31.6 mmol) was added. The suspension was heated at 100 °C for 4 hours. After cooling to room temperature, an aqueous solution ofHCl (50 mL. 2N) was added and the solution was extrated with ethyl acetate (2 x 50 mL). The organic extracts were combined, dried over sodium sulfate, filtered and concentrated *in vacuo.* The residue was purified by silica gel chromatography (0-50% EtOAc in hexanes) to provide the desired ester **(258)** as a mixture of isomers.

**Step E:** To a solution of **(258)** (0.79 g, 3.2 mmol) in ethanol (10 mL) was added Pd/C (0.08 g, 10% Degussa type). A balloon of hydrogen gas was added and the reaction was evacuated and back-filled with hydrogen three times. The reaction was stirred overnight at room temperature, was filtered through a pad of celite and *concentrated in vacuo* to provide compound **(259).**

**Step F:** To a solution of ester **(259)** (1.06 g, 4.2 mmol) in dichloromethane (40 mL) at 0 °C was added borontribromide (3.96 mL, 41.9 mmol). The solution was stirred for 2 hours and quenched with ethanol (5 mL) followed by a saturated solution of sodium bicarbonate (5 mL). The organic layer was separated, dried over sodium sulfate, filtered and concentrated *in vacuo* to obtain the expected product **(260).**

Intermediate 21

methyl 2-(3,5-difluoro-4-hydroxyphenyl)cyclopent-1-enecarboxylate **(261)**

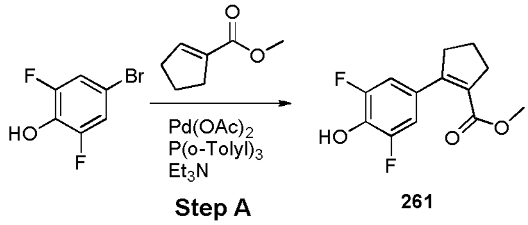


261

**Step A:** In a 350-mL pressure-glass was added 4-bromo-2,6-difluorophenol (5.0 g, 0.024 mol), triethylamine (100 mL), methyl 1-cyclopentene-1-carboxylate (4.5 g, 1.5eq.), palladium (II) acetate (0.27 g, 0.05eq.), followed by tri-o-tolyphosphine (0.73 g, 0.1eq.) under $N_2$. The mixture was sealed in the seal glass tube and stirred at 100 °C overnight. The reaction was cooled to room temperature and EtOAc was added (150 mL) and stirred for 30 minutes, filtered through celite and rinsed with EtOAc (100 mL × 3). The filtrate was acidified with 2N HCl to pH ~2. The organic layer was separated, and the aqueous layer was extracted with EtOAc (50 mL × 2). The organic layers were combined and washed with water (100 mL × 2), brine (100 mL) and dried over $Na_2SO_4$. The filtrate was concentrated in *vacuo* and the residue oil was purified by silica gel chromatography to afford the desired product **(261)** (5.43 g, 89%yield) as a light-yellow wet solid.

Intermediate 22

benzyl 3-(4-hydroxybenzylidene)cyclobutanecarboxylate **(262)**

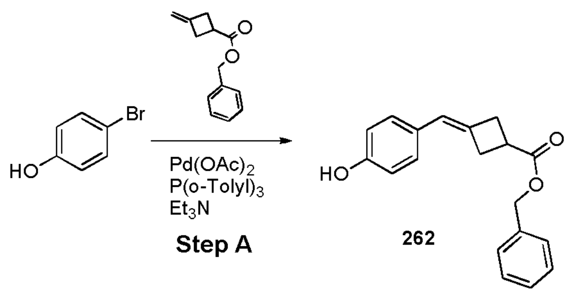


**Step A:** In a 100-mL pressure-glass was added 4-bromophenol (1.0 g, 0.0057 mol), triethylamine (30 mL), Benzyl 3-methylenecyclobutanecarboxylate(1.5 g, 1.3eq.), palladium (II) acetate (0.064 g, 0.05eq.), followed by tri-*o*-tolyphosphine (0.175 g, 0.1eq.) under $N_2$. The mixture was sealed in the seal glass tube and stirred at 100 °C overnight. The reaction was cooled to room temperature and EtOAc was added (100 mL) and stirred for 30 minutes, filtered through celite and rinsed with EtOAc (50 mL × 3). The filtrate was acidified with 2N HCl to pH -2. The organic layer was separated, and the aqueous layer was extracted with EtOAc (50 mL × 2). The organic layers were combined and washed with water (100 mL × 2), brine (100 mL) and dried over $Na_2SO_4$. After filtration, the solution was concentrated in *vacuo* and the residue oil was purified by silica gel chromatography to provide the desired product **(262)** (5.43 g, 58%yield).

Intermediate 23

ethyl 3-(2-ethyl-4-hydroxyphenyl)-2-methylpropanoate **(265)**

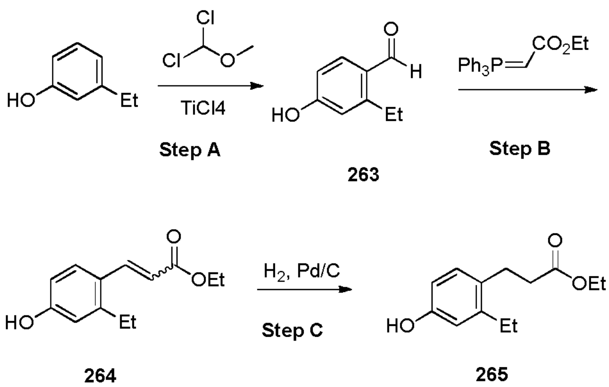


**Step A:** To a solution of 3-ethylphenol (6.11 g, 50 mmol) in $CH_2Cl_2$ was added $TiCl_4$ in $CH_2Cl_2$ (100 mL, 100 mmol) at 0 °C followed by dichloro(methoxy)methane (7.4 mL, 80 mmol). The reaction was stirred at 0 °C for 1 hour.

The mixture was poured into ice-water and extracted with ethyl acetate (3 x 25 mL). The organic layer was dried over sodium sulfate, filtered, and concentrated in *vacuo.* The residue was purified by flash column chromatography on silica gel with hexanes and EtOAc to afford 2-ethyl-4-hydroxybenzaldehyde **(263).** $^1$H NMR (400 MHz, $CDCl_3$) δ 10.07 (s, 1H), 7.76 (d, *J* = 8.3 Hz, 1H), 6.82 (d, *J* = 8.4 Hz, 1H), 6.77 (s, 1H), 3.02 (q, *J* = 7 Hz, 2H), 1.25 (t, *J* =7 Hz, 3H).

**Step B:** Compound **(264)** was prepared in a similar manner as that described for the synthesis of **29.**

**Step C:** Compound **(265)** was prepared in a similar manner as that described for the synthesis of **30.**

Intermediate 24

ethyl 2-(6-hydroxy-1,2,3,4-tetrahydronaphthalen-1-yl)acetate **(268)**

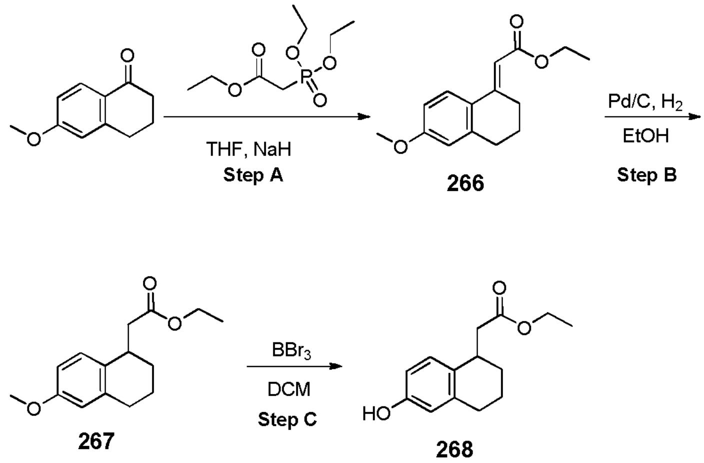


**Step A:** To the solution of 6-methoxy-3,4-dihydronaphthalen-1(2H)-one (5.0mmol), 0.881g) in THF (25.0mL) was added NaH (12.5mmol, 0.50g) and then ethyl 2-(diethoxyphosphoryl)acetate (12.5mmol, 2.5mL) under nitrogen at room temperature. The reaction mixture was heated at 60 °C for four hours. The reaction was cooled and quenched with water, extracted with EtOAc. The organic layer was washed with water, brine and dried over $Na_2SO_4$. The solvent was removed under reduced pressure and chromatographed (Silica gel, 30% EtOAc in Hexanes.) to give compound **(266)** (0.792g, 64.3%). LC-MS ESI *m/z*; found 247.0 $[M+H]^+$.

**Step B:** The mixture of compound **(266)** (0.792g, 3.22mmol) in EtOH (50.0mL) was reduced with Pd/C (0.100g) under hydrogen gas at room temperature for 12 hours to yield compound **(267)** (0.709g, 88.8%). $^1$H NMR (400 MHz, $CDCl_3$) δ 7.08 (d, *J* = 8.5Hz, 1H), 6.70 (dd, *J* = 8.5, 2.7 Hz, 1H), 6.61 (d, *J* = 2.5 Hz , 1H), 4.17 (q, *J* = 7.1 Hz , 2H), 3.77 (s, 3H), 3.38 - 3.22 (m, 1H), 2.82 - 2.60 (m, 3H), 2.57 - 2.41 (m, 1H), 1.96 - 1.62 (m, 4H), 1.27 (t, *J* = 7.1 Hz, 3H).

**Step C:** To the mixture of compound **(267)** (0.709g, 2.85mmol) in DCM (30.0mL) at 0 °C under nitrogen gas was added slowly boron tribromide (0.809mL, 8.56mmol). The reaction mixture was stirred at 0 °C for two hours then quenched slowly with EtOH at 0 °C. The reaction was quenched with $NaHCO_3$, extracted with DCM. The organic layer was washed with brine and dried over $Na_2SO_4$. The solvent was removed in *vacuo* to give compound **(268)** (0.600g, 89.9%). LC-MS ESI *m/z*; found: 235.3 $[M+H]^+$. $^1$H NMR (400 MHz, $CDCl_3$) δ 7.02 (d, *J* = 8.4 Hz, 1H), 6.62 (dd, *J* = 8.3, 2.6 Hz, 1H), 6.54 (d, *J* = 2.5 Hz, 1H), 4.17 (q, *J* = 7.1 Hz , 2H), 3.37 - 3.15 (m, 1H), 2.80 - 2.57 (m, 3H), 2.57 - 2.40 (m, 1H), 1.98 - 1.50 (m, 4H), 1.36 - 1.13 (m, 3H).

Intermediate 25

ethyl 3-(3,5-difluoro-4-hydroxyphenyl)butanoate **(270)**

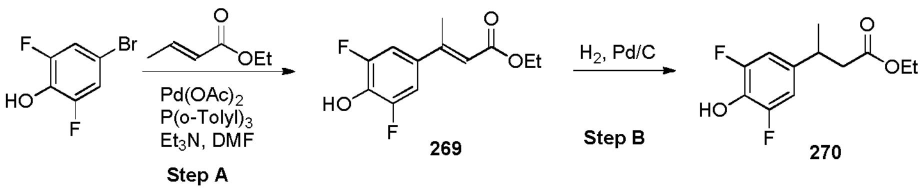


269

270

**Step A:** Compound **(269)** was prepared in a similar manner as that described for the synthesis of **12.**

**Step B:** Compound **(270)** was prepared in a similar manner as that described for the synthesis of **53.**

Intermediate 26

Ethyl 1-(4-chlorophenyl)-3-(trifluoromethyl)-1H-pyrazole-5-carboxylate **(271)**

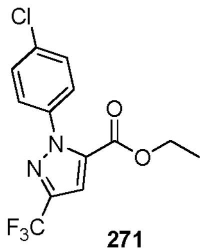

**271**

Compound **(271)** was prepared in a similar manner as that described in patent US639527B1. $^1H$ NMR (400 MHz, DMSO-$d_6$) δ 7.69 - 7.56 (m, 5H), 4.20 (q, *J* = 7.1 Hz, 2H), 1.16 (t, *J* = 7.1 Hz, 3H).

Intermediate 27

ethyl 3-(4-hydroxyphenyl)-3-methylbutanoate (275)

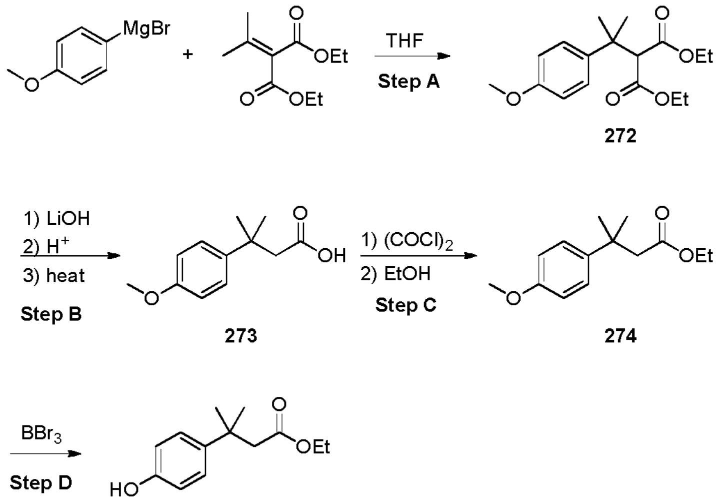


272

273

274

275

**Step A - Step C:** Compound **(274)** was prepared in a similar manner as that described in patent US5032577, example 175. $^1H$ NMR (400 MHz, $CDCl_3$) δ 7.33 - 7.23 (m, 3H), 6.84 (d, *J* = 8.8 Hz, 2H), 3.98 (q, *J* = 7.1 Hz, 2H), 3.79 (s, 3H), 2.57 (s, 2H), 1.43 (s, 6H), 1.10 (t, *J* = 7.1 Hz, 3H).

**Step D:** Compound **(275)** was prepared in a similar manner as that described for the synthesis of **34.**

Intermediate 28

methyl 4-(4-hydroxyphenyl)-4-oxobutanoate **(277)**

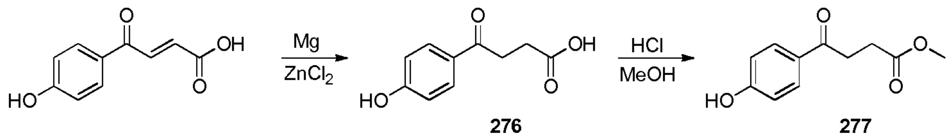


276 277

**Step A:** To a suspension of 4-hydroxybenzoylacrylic acid (0.43 g, 2.24 mmol) and magnesium (0.16 g, 6.58 mmol, 30 mesh) in water (6 mL) was added 20 drops of $ZnCl_2$. The mixture was stirred at room temperature for 4 days and the solvent was evaporated in *vacuo* to yield the desired unsaturated ketone **(276).**
**Step B:** This crude product **(276)** was dissolved in anhydrous MeOH (40 mL) and 4N HCl in dioxane (10 mL) was added. The mixture was stirred in a sealed flask at 55 °C overnight. After evaporation of solvent, the residue oil was purified by silica gel chromatography to provide the desired product **(277).** $^1$H NMR (400MHz, DMSO-$d_6$) δ: 7.83 (d, *J* = 9.2 Hz, 2H), 6.82 (d, *J* = 9.0 Hz, 2H), 3.57 (s, 3H), 3.18 (t, *J* = 6.0 Hz, 2H), 2.59 (t, *J* = 6.0 Hz, 2H).

Intermediate 29

ethyl 5-(4-hydroxyphenyl)-3-methylpentanoate **(279)**

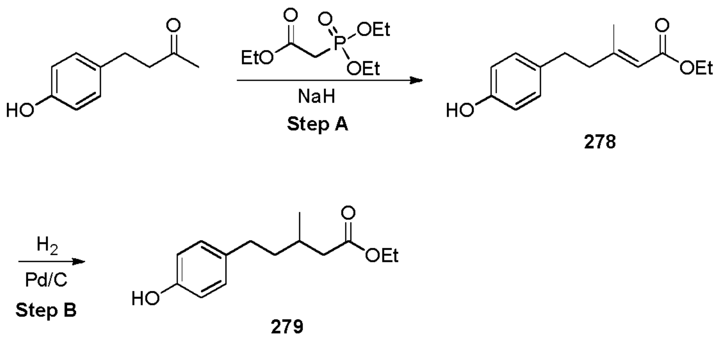


278

279

**Step A:** Compound **(278)** was prepared in a similar manner as that described for the synthesis of **266.**
**Step B:** Compound **(279)** was prepared in a similar manner as that described for the synthesis of **267.**

Intermediate 30

ethyl 4-(4-hydroxyphenyl)-3-methylbutanoate **(281)**

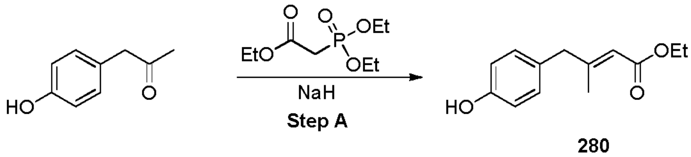


280

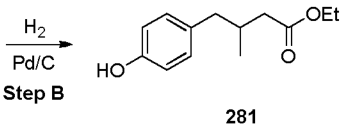


281

**Step A:** Compound **(280)** was prepared in a similar manner as that described for the synthesis of **266**.

**Step B:** Compound **(281)** was prepared in a similar manner as that described for the synthesis of **267.**

Intermediate 31 and 32

2-(5-Hydroxy-2,3-dihydro-1*H*-inden-1-yl)acetic acid **(282)** and 2-(5-hydroxy-2,3-dihydro-1*H*-inden-1-yl)propanoic acid **(283)**

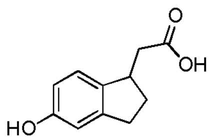

**282**

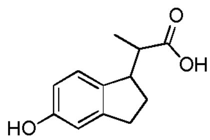

**283**

The above intermediates **(282)** and **(283)** were synthesized by adaptation of a method according to WO 2004/011445 A1.

The following intermediates were purchased from commercial sources and used to synthesize one or more of the representative compounds of the invention.

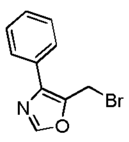

**284**

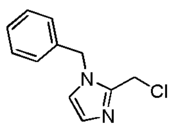

**285**

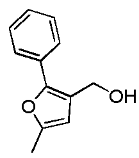

**286**

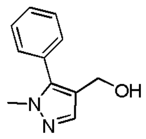

**287**

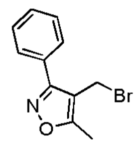

**288**

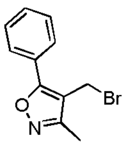

**289**

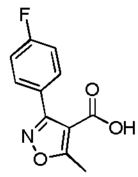

**290**

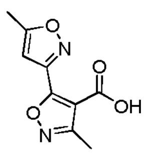

**291**

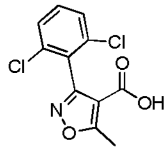

**292**

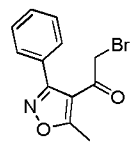

**293**

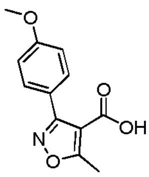

**294**

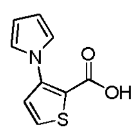

**295**

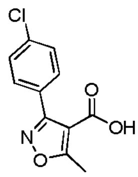

**296**

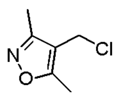

**297**

Preparation of Examples

Example 1

3-(3,5-difluoro-4-((3-isopropyl-1-phenyl-1*H*-pyrazol-5-yl)methoxy)phenyl)propanoic acid (**55**)

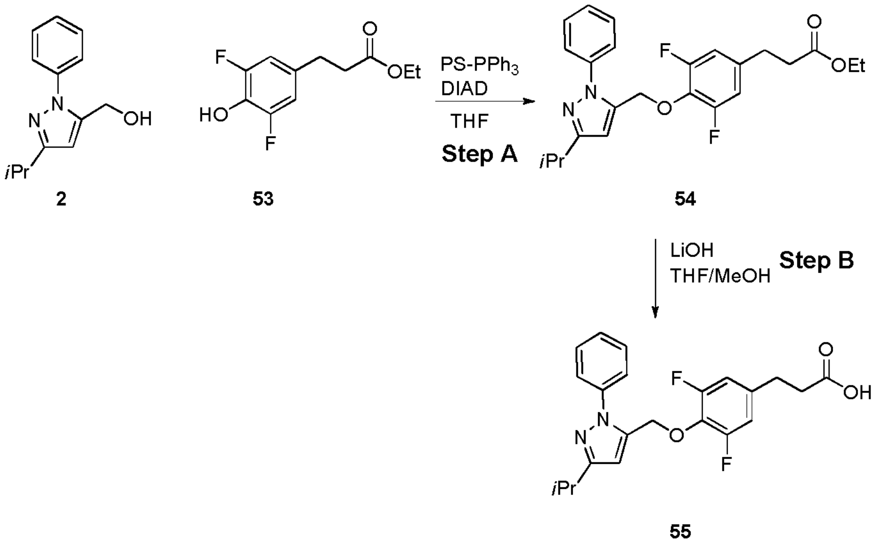


**Step A:** To a solution of intermediate **(2)** (0.150 g, 0.693 mmol) in THF (3 mL) was added intermediate **(53)** (0.160 g, 0.693 mmol), polymer supported triphenylphosphine (3 mmol/g, 0.347 g, 1.04 mmol) and diisopropylazodicarboxylate (0.205 mL, 1.04 mmol). The resulting suspension was stirred for 18 h. The reaction was diluted with ethyl acetate and filtered through a pad of celite. The filtrate was concentrated in vacuo and the residual was purified by silica gel chromatography (0-20 % EtOAc in hexanes) to yield the intermediate **(54).**

**Step B:** To a solution of intermediate **(54)** (0.270 g, 0.630 mmol) in THF (1 mL) and methanol (1 mL) was added a solution of lithium hydroxide (1.0 M, 1.0 mL). The reaction was stirred at room temperature for 4 h. The mixture was acidified with 1M HCl and diluted with EtOAc (5 mL). The organic layer was washed with brine (5 mL), dried over sodium sulfate and filtered. The filtrate was concentrated in vacuo and the residue was purified by silica gel chromatography (0-100 % EtOAc in hexanes) to isolate the title compound **(55).** $^1$H NMR (400 MHz, $CDCl_3$) $\delta$: 7.67 (d, 2H), 7.46 (t, 2H), 7.37 (t, 1H), 6.74 (d, 2H), 6.30 (s, 1H), 5.02 (s, 2H), 3.04 (m, 1H), 2.88 (t, 2H), 2.64 (t, 2H), 1.28 (d, 6H).

Example 2

ethyl 3-(3,5-difluoro-4-((4-phenyl-1,2,3-thiadiazol-5-yl)methoxy)phenyl)propanoate **(57)**

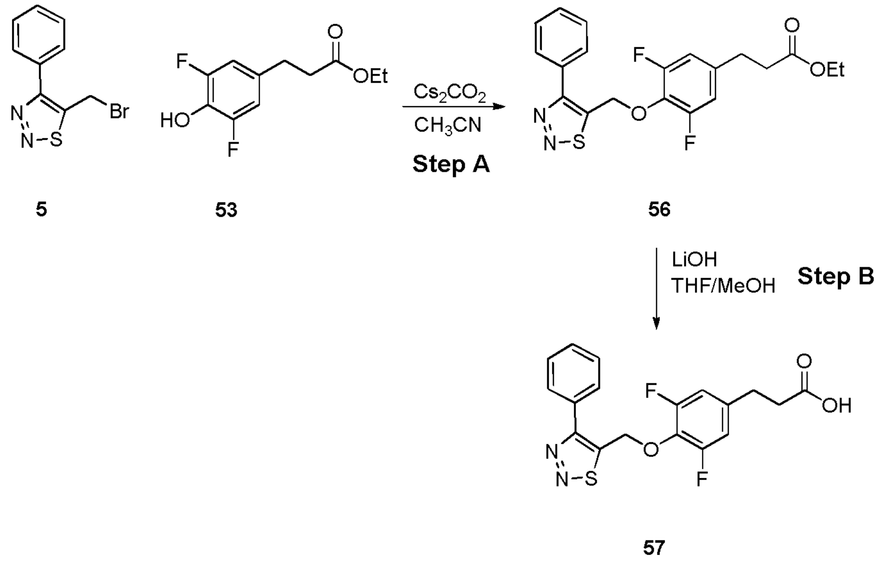


5 53 56 57

**Step A:** To a solution of intermediate (5) (0.200 g, 0.787 mmol) in acetonitrile (3 mL) was added intermediate (53) (0.181 g, 0.787 mmol) and cesium carbonate (0.308 g, 0.945). The resulting suspension was stirred at 80 °C for 4 h. The reaction was cooled to rt, diluted with ethyl acetate and filtered through a pad of celite. The filtrate was concentrated in vacuo and the residual was purified by silica gel chromatography (0-20 % EtOAc in hexanes) to yield the intermediate **(56).**

**Step B:** To a solution of intermediate **(56)** (0.318 g, 0.787 mmol) in THF (1 mL) and methanol (1 mL) was added a solution of lithium hydroxide (1.0 M, 1.0 mL). The reaction was stirred at room temperature for 4 h. The mixture was acidified with 1M HCl and diluted with EtOAc (5 mL). The organic layer was washed with brine (5 mL), dried over sodium sulfate and filtered. The filtrate was *concentrated in vacuo* and the residue was purified by silica gel chromatography (0-100 % EtOAc in hexanes) to isolate the title compound **(57).** $^1$H NMR (400 MHz, $CDCl_3$) δ: 7.77 (d, 2H), 7.55-7.45 (m, 3H), 6.79 (d, 2H), 5.52 (s, 2H), 2.89 (t, 2H), 2.67 (t, 2H).

Example 3

3-(3,5-difluoro-4-((1-phenyl-1*H*-imidazol-5-yl)methoxy)phenyl)propanoic acid **(59)**

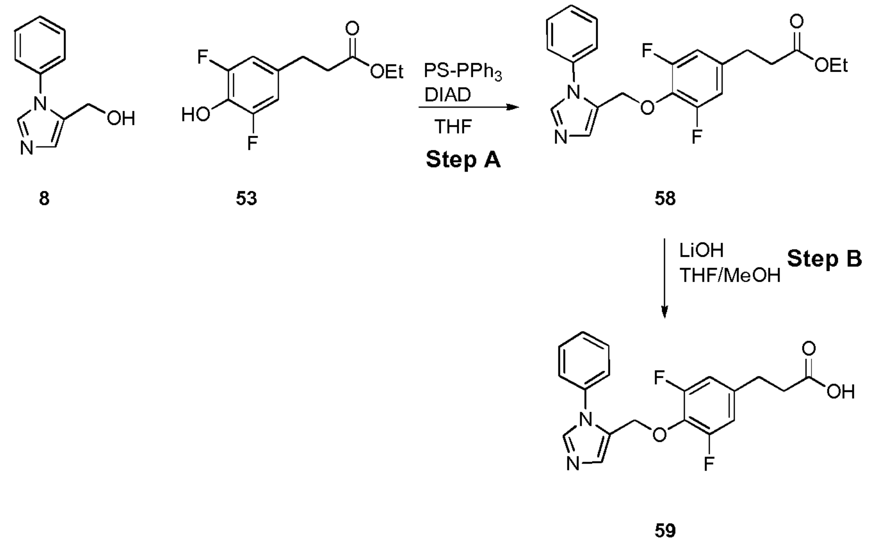


8 53 58 59

**Step A:** To a solution of intermediate **(8)** (0.200 g, 1.15 mmol) in THF (3 mL) was added intermediate (53) (0.264 g, 1.15 mmol), polymer supported triphenylphosphine (3 mmol/g, 0.573 g, 1.72 mmol) and diisopropylazodicarboxylate (0.340 mL, 1.72 mmol). The resulting suspension was stirred for 18 h. The reaction was diluted with ethyl acetate and filtered through a pad of celite. The filtrate was concentrated in vacuo and the residual was purified by silica gel chromatography (0-20 % EtOAc in hexanes) to yield the intermediate **(58).**

**Step B:** To a solution of intermediate **(58)** (0.040 g, 0.103 mmol) in THF (0.5 mL) and methanol (0.5 mL) was added a solution of lithium hydroxide (1.0 M, 0.5 mL). The reaction was stirred at room temperature for 4 h. The mixture was acidified with 1M HCl and diluted with EtOAc (3 mL). The organic layer was washed with brine (3 mL), dried over sodium sulfate and filtered. The filtrate was concentrated in vacuo and the residue was purified by silica gel chromatography (0-100 % EtOAc in hexanes) to isolate the title compound **(59).** $^1$H NMR (400 MHz, $CDCl_3$) δ: 7.85 (s, 1H), 7.56-7.54 (m, 5H), 7.06 (s, 1H), 6.74 (d, 2H), 4.98 (s, 2H), 2.88 (t, 2H), 2.63 (t, 2H).

Example 4

2-(2-(4-((1-(4-chlorophenyl)-3-methyl-1*H*-pyrazol-5-yl)methoxy)-3,5-difluorophenyl)cyclopropyl)acetic acid **(62)**

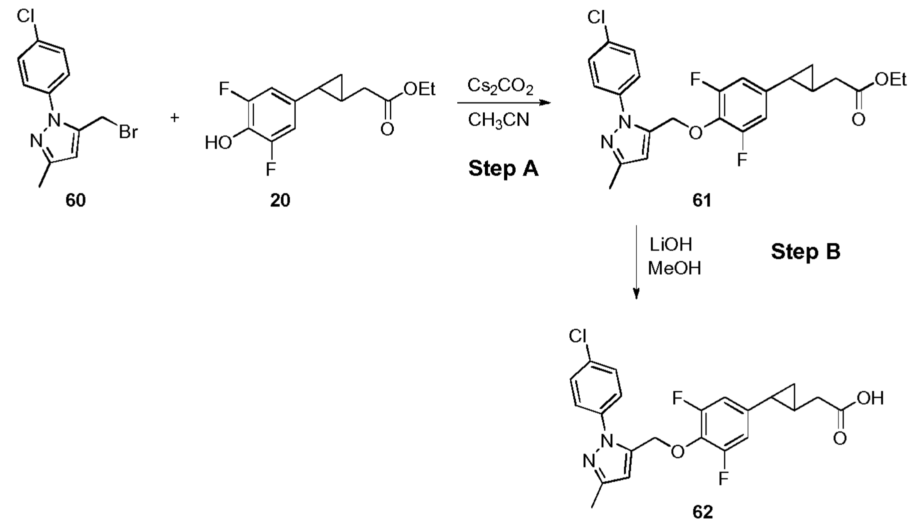


60 20 61 62

**Step A:** A mixture of ethyl 2-(2-(3,5-difluoro-4-hydroxyphenyl)cyclopropyl) acetate **(20)** (84.7 mg, 0.33 mmol), $Cs_2CO_3$ (170.8 mg, 0.52 mmol) and 5-(bromomethyl) - 1-(4-chlorophenyl)-3-methyl-1*H*-pyrazole **(60)** (103.7 mg, 0.36 mmol) in acetonitrile (3 mL) was stirred at 50 °C for 5 hours. The reaction mixture was filtrated through celite and rinsed with acetonitrile. The filtrate was concentrated in vacuo and purified by flash chromatography on silica gel (0-30%) gave108.6 mg of desired ester **(61)** as a colorless liquid. $^1$H NMR (400 MHz, $CDCl_3$) δ: 7.65 (d, *J*= 8.6 Hz, 2H), 7.44 (d, *J*= 8.6 Hz, 2H), 6.64 (d, *J* = 9.2 Hz, 2H), 6.26 (s, 1H), 4.97 (s, 2H), 4.17 (q, *J* = 6.9 Hz, 2H), 2.47 - 2.33 (m, 2H), 2.31 (s, 3H), 1.72 -1.68 (m, 1H), 1.33 - 1.20 (m, 4H), 1.00 - 0.86 (m, 2H).

**Step B:** To a solution of ethyl 2-(2-(4-((1-(4-chlorophenyl)-3-methyl-1*H*-pyrazol-5-yl)methoxy)-3,5-difluorophenyl)cyclopropyl)acetate **(61)** (108.6 mg, 0.24 mmol) obtained above in MeOH (2 mL) was added an aqueous solution of 10 % LiOH (2 mL). The reaction mixture was stirred at 50 °C for 2 hours. The reaction mixture was acidified with 6N HCl (1 mL) and partitioned between EtOAc and $H_2O$. The organic layer was washed with brine, dried over $Na_2SO_4$ and concentrated *in vacuo.* Purification by HPLC gave 73.5 mg of desired product **(62)** as a white solid. $^1$H NMR (400 MHz, DMSO-$d_6$) δ: 7.62 (d, *J*= 8.8 Hz, 2H), 7.55 (d, *J* = 8.8 Hz, 2H), 6.81 (d, *J* = 9.6 Hz, 2H), 6.33 (s, 1H), 5.07 (s, 2H), 2.38 - 2.23 (m, 2H), 2.19 (s, 3H), 1.79 - 1.67 (m, 1H), 1.28 - 1.15 (m, 1H), 1.03 - 0.77 (m, 2H).

Example 5

3-(3,5-difluoro-4-((1-phenyl-1*H*-1,2,3-triazol-5-yl)methoxy)phenyl)propanoic acid (65)

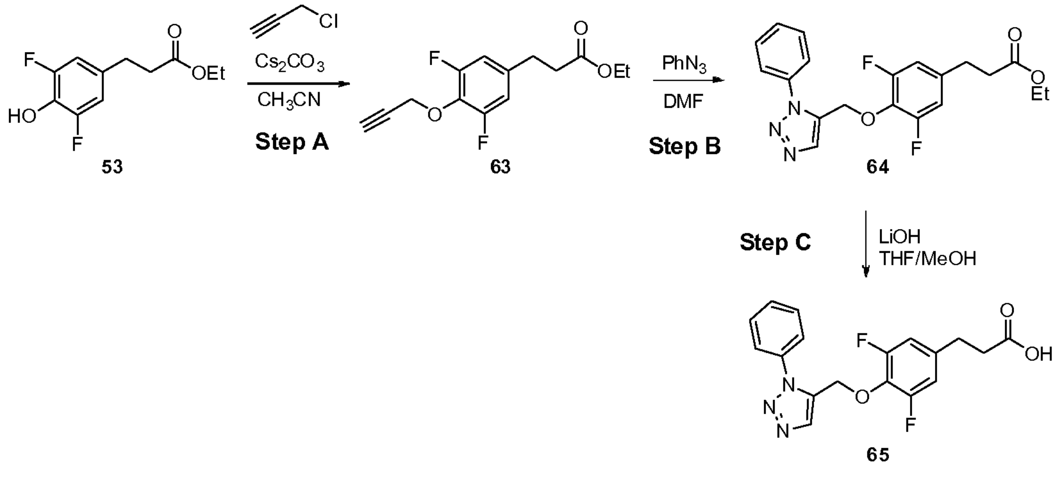


53 63 64 65

**Step A:** To a mixture of ethyl 3-(3,5-difluoro-4-hydroxyphenyl)propanoate (53) (1 g, 4.3 mmol) and $Cs_2CO_3$ (1.7 g, 5.16 mmol) in acetonitrile (25 mL) was added propargyl chloride solution in toluene (0.55g, 5.16 mmol). The mixture was stirred at 60 °C for 5 hours. After filtration through celite and washing with ethyl acetate, the filtrate was concentrated under reduced pressure. The residue was purified by flash chromatography on silica gel to yield white solid **(63).**

**Step B:** The solution of ethyl 3-(3,5-difluoro-4-(prop-2-ynyloxy)phenyl)propanoate **(63)** and phenylazide in anhydrous DMF (8 mL) was heated at 110 °C for 20 minutes in a microwave reactor. The reaction was diluted with $H_2O$. The product was extracted with ethyl acetate. The organic phase was washed with $H_2O$, brine, dried over $Na_2SO_4$ and concentrated *in vacuo.* The residue was purified by flash chromatography on silica gel to yield a yellow oil **(64).**

**Step C:** To a solution of intermediate **(64)** (0.100 g, 0.258 mmol) in THF (0.5 mL) and methanol (0.5 mL) was added a solution of lithium hydroxide (1.0 M, 0.5 mL). The reaction was stirred at room temperature for 4 h. The mixture was acidified with 1M HCl and diluted with EtOAc (3 mL). The organic layer was washed with brine (3 mL), dried over sodium sulfate and filtered. The filtrate was concentrated in vacuo and the residue was purified by silica gel chromatography (0-100 % EtOAc in hexanes) to isolate the title compound **(65).** $^1$H NMR (400 MHz, $CDCl_3$) δ: 7.75-7.71 (m, 3H), 7.6-7.5 (m, 3H), 6.76 (d, *J*= 9.2 Hz, 1H), 5.13 (s, 2H), 2.89 (t, *J*= 7.4 Hz, 2H), 2.66 (t, *J*= 7.4 Hz, 2H).

Representative compounds of the invention, prepared by following procedures described in the above examples using appropriate starting materials that will be apparent to those skilled in the art, are shown below.

Example 6

2-(2-(3,5-difluoro-4-((5-methyl-3-phenylisoxazol-4-yl)methoxy)phenyl)cyclopropyl)-acetic acid **(67)**

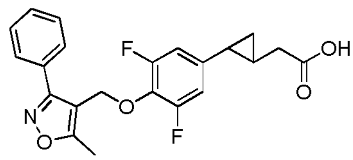

$^1$H NMR (400 MHz, DMSO-$d_6$) δ: 7.79 - 7.70 (m, 2H), 7.58 - 7.46 (m, 3H), 6.83 (d, *J*= 9.8 Hz, 2H), 4.95 (s, 2H), 2.41 - 2.17 (m, 2H), 2.29 (s, 3H), 1.83 - 1.63 (m, 1H), 1.29 -1.14 (m, 1H), 1.02 - 0.74 (m, 2H).

Example 7

3-(3,5-difluoro-4-((1-isobutyl-3-methyl-1*H*-pyrazol-5-yl)methoxy)phenyl)propanoic acid **(68)**

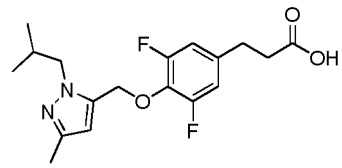

$^1$H NMR (400 MHz, $CDCl_3$) δ: 6.78 (d, *J*= 9.1 Hz, 2H), 6.08 (s, 1H), 5.04 (s, 2H), 4.02 (d, *J*= 7.6 Hz, 2H), 2.89 (t, *J*= 7.3 Hz, 2H), 2.72 (t, *J*= 7.3 Hz, 2H), 2.33 - 2.28 (m, 1H), 2.28 (s, 3H), 0.92 (d, *J* = 6.7 Hz, 6H)

Example 8

2-(2-(3,5-difluoro-4-((1-isobutyl-3-methyl-1*H*-pyrazol-5-yl)methoxy)phenyl)-cyclopropyl)acetic acid **(69)**

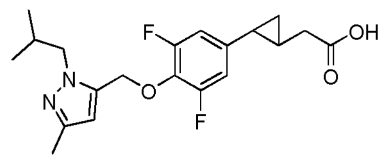

$^1$H NMR (400 MHz, CD $Cl_3$) δ: 6.65 (d, *J*= 9.4 Hz, 2H), 6.07 (s, 1H), 5.01 (s, 2H), 4.03 (d, *J*= 7.6 Hz, 2H), 2.57 - 2.31 (m, 2H), 2.42 - 2.29 (m, 1H), 2.27 (s, 3H), 1.79 - 1.66 (m, 1H), 1.36 - 1.27 (m, 1H), 1.00 - 0.93 (m, 2H), 0.92 (d, *J*= 6.7 Hz, 6H).

Example 9

2-(3,5-Difluoro-4-((1-isobutyl-3-methyl-1*H*-pyrazol-5-yl)methoxy)phenyl)-cyclopropanecarboxylic acid **(70)**

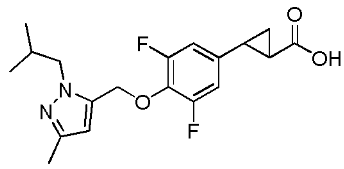

$^1$H NMR (400 MHz, CD $Cl_3$) δ: 6.68 (d, *J*= 9.1 Hz, 2H), 6.15 (s, 1H), 5.05 (s, 2H), 4.12 (d, *J*= 7.7 Hz, 2H), 2.58 - 2.45 (m, 1H), 2.33 (s, 3H), 2.31 - 2.22 (m, 1H), 1.93 - 1.81 (m, 1H), 1.74 - 1.61 (m, 1H), 1.38 - 1.29 (m, 1H), 0.99 (d, *J* = 6.7 Hz, 6H).

Example 10

3-(3,5-difluoro-4-((5-phenyloxazol-4-yl)methoxy)phenyl)propanoic acid **(71)**

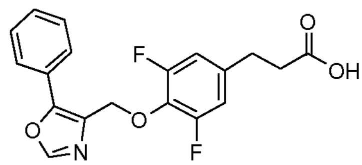

$^1$H NMR (400 MHz, $CDCl_3$) δ: 7.89 (s, 1H), 7.78 (d, 2H), 7.50 - 7.42 (m, 3H), 6.75 (d, 2H), 5.21 (s, 2H), 2.90 -

2.86 (t, 2H), 2.66 - 2.63 (t, 2H).

Example 11

3-(4-((1-benzyl-1*H*-imidazol-2-yl)methoxy)-3,5-difluorophenyl)propanoic acid (72)

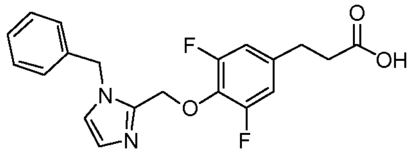

$^1$H NMR (400 MHz, $CDCl_3$) δ: 7.38 - 7.30 (m, 3H), 7.21 (d, 2H), 7.04 (s, 1H), 6.91 (s, 1H), 6.78 (d, 2H), 5.37 (s, 2H), 5.16 (s, 2H), 2.88 - 2.84 (t, 2H), 2.59 - 2.58 (t, 2H).

Example 12

3-(3,5-difluoro-4-((5-methyl-2-phenylfuran-3-yl)methoxy)phenyl)propanoic acid **(73)**

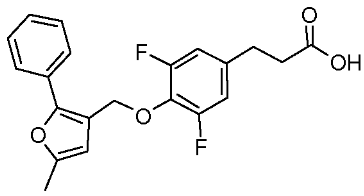

$^1$H NMR (400 MHz, $CDCl_3$) δ: 7.67 (d, 2H), 7.42 - 7.38 (m, 2H), 7.31 - 7.27 (m, 1H), 6.74 (d, 2H), 6.16 (s, 1H), 5.07 (s, 2H), 2.88 - 2.85 (t, 2H), 2.66 - 2.62 (t, 2H), 2.33 (s, 3H).

Example 13

3-(3,5-difluoro-4-((1-methyl-5-phenyl-1*H*-pyrazol-4-yl)methoxy)phenyl)propanoic acid **(74)**

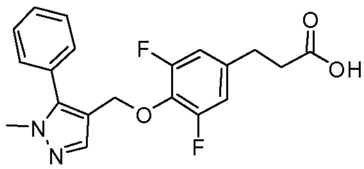

$^1$H NMR (400 MHz, $CDCl_3$) δ: 10.51 (*br,* 1H), 7.56 (s, 1H), 7.50 - 7.41 (m, 5H), 6.72 (d, 2H), 4.88 (s, 2H), 3.79 (s, 3H), 2.88 - 2.84 (t, 2H), 2.64 - 2.61 (t, 2H).

Example 14

3-(3,5-difluoro-4-((5-methyl-3-phenylisoxazol-4-yl)methoxy)phenyl)propanoic acid **(75)**

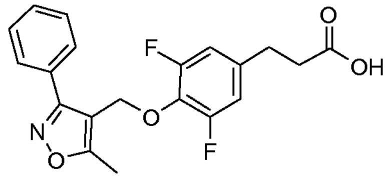

$^1$H NMR (400 MHz, $CDCl_3$) δ: 10.31 (br, 1H), 7.86 - 7.83 (m, 2H), 7.49 - 7.47 (m, 3H), 6.75 (d, 2H), 4.93 (s, 2H), 2.90 - 2.86 (t, 2H), 2.67 - 2.63 (t, 2H), 2.35 (s, 3H).

Example 15

3-(3,5-difluoro-4-((3-methyl-5-phenylisoxazol-4-yl)methoxy)phenyl)propanoic acid **(76)**

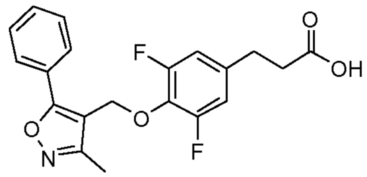

$^1$H NMR (400 MHz, $CDCl_3$) δ: 7.85 - 7.82 (m, 2H), 7.52 - 7.49 (m, 3H), 6.76 (d, 2H), 4.99 (s, 2H), 2.91 - 2.87 (t, 2H), 2.68 - 2.64 (t, 2H), 2.33 (s, 3H).

Example 16

2-(3,5-difluoro-4-((5-methyl-3-phenylisoxazol-4-yl)methoxy)phenyl) cyclopropanecarboxylic acid **(77)**

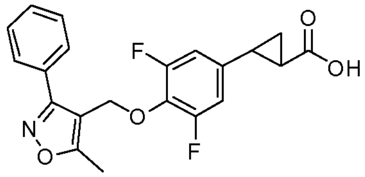

$^1$H NMR (400 MHz, $CDCl_3$) δ: 7.84 - 7.82 (m, 2H), 7.49 - 7.47 (m, 3H), 6.64 (d, 2H), 4.93 (s, 2H), 2.52 - 2.48 (m, 1H), 2.39 (s, 3H), 1.87 - 1.83 (m, 1H), 1.69 - 1.62 (m, 1H), 1.37 - 1.26 (m, 1H).

Example 17

2-(3,5-difluoro-4-((3-(4-fluorophenyl)-5-methylisoxazol-4-yl)methoxy)phenyl) cyclopropanecarboxylic acid **(78)**

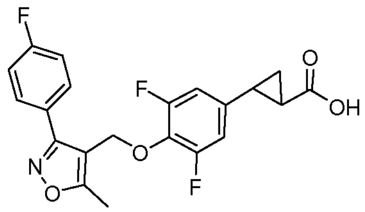

$^1$H NMR (400 MHz, $CDCl_3$) δ: 7.87 - 7.84 (m, 2H), 7.20 - 7.16 (m, 2H), 6.66 (d, 2H), 4.91 (s, 2H), 2.54 - 2.51 (m, 1H), 2.38 (s, 3H), 1.88 - 1.84 (m, 1H), 1.70 - 1.65 (m, 1H), 1.36 - 1.31 (m, 1H).

Example 18

3-(3,5-difluoro-4-((3-(4-fluorophenyl)-5-methylisoxazol-4-yl)methoxy)phenyl)propanoic acid **(79)**

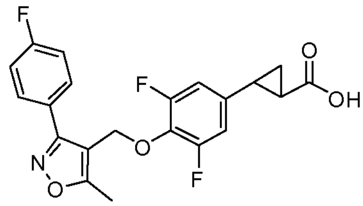

$^1$H NMR (400 MHz, $CDCl_3$) $\delta$: 7.89 - 7.85 (m, 2H), 7.20 - 7.16 (m, 2H), 6.77 (d, 2H), 4.91 (s, 2H), 2.91 - 2.87 (t, 2H), 2.68 - 2.65 (t, 2H), 2.35 (s, 3H).

Example 19

3-(4-((3',5-dimethyl-3,5'-biisoxazol-4'-yl)methoxy)-3,5-difluorophenyl)propanoic acid **(80)**

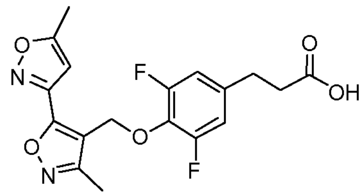

$^1$H NMR (400 MHz, $CDCl_3$) $\delta$: 6.75 (d, 2H), 6.44 (s, 1H), 5.29 (s, 2H), 2.87 - 2.84 (t, 2H), 2.59 - 2.55 (t, 2H), 2.52 (s, 3H), 2.44 (s, 3H).

Example 20

3-(4-((3-(1*H*-pyrrol-1-yl)thiophen-2-yl)methoxy)-3,5-difluorophenyl)propanoic acid **(81)**

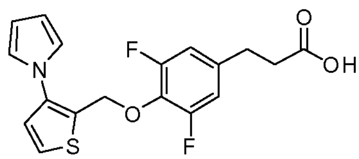

$^1$H NMR (400 MHz, $CDCl_3$) $\delta$: 7.34 (d, 1H), 7.05 (d, 1H), 7.02 (*app* s, 2H), 6.75 (d, 2H), 6.32 (*app* s, 2H), 5.15 (s, 2H), 2.90 - 2.86 (t, 2H), 2.67 - 2.64 (t, 2H).

Example 21

3-(3,5-difluoro-4-((3-(4-methoxyphenyl)-5-methylisoxazol-4-yl)methoxy)phenyl) propanoic acid **(82)**

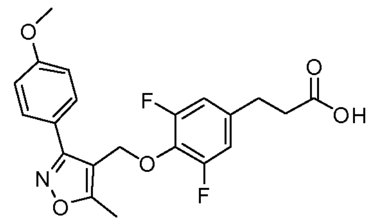

$^1H$ NMR (400 MHz, $CDCl_3$) δ: 7.82 (d, 2H), 7.01 (d, 2H), 6.76 (d, 2H), 4.93 (s, 2H), 3.87 (s, 3H), 2.91 - 2.87 (t, 2H), 2.68 - 2.65 (t, 2H), 2.34 (s, 3H).

Example 22

3-(4-((3-(2,6-dichlorophenyl)-5-methylisoxazol-4-yl)methoxy)-3,5-difluorophenyl) propanoic acid **(83)**

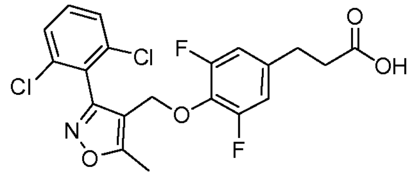

$^1H$ NMR (400 MHz, $CDCl_3$) δ: 7.41 - 7.33 (m, 3H), 6.65 (d, 2H), 4.79 (s, 2H), 2.86 - 2.82 (t, 2H), 2.64 - 2.60 (t, 2H), 2.58 (s, 3H).

Example 23

3-(4-((3,5-dimethylisoxazol-4-yl)methoxy)-3,5-difluorophenyl)propanoic acid **(84)**

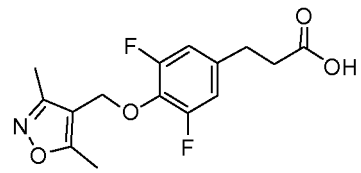

$^1H$ NMR (400 MHz, $CDCl_3$) δ: 6.75 (d, 2H), 4.89 (s, 2H), 2.89 - 2.86 (t, 2H), 2.67 - 2.63 (t, 2H), 2.30 (s, 6H).

Example 24

3-(4-((3-(4-chlorophenyl)-5-methylisoxazol-4-yl)methoxy)-3,5-difluorophenyl)propanoic acid **(85)**

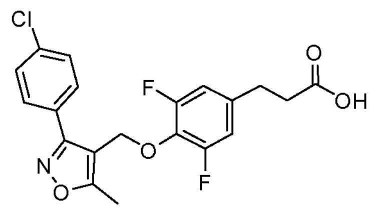

$^1H$ NMR (400 MHz, $CDCl_3$) δ: 7.82 (d, 2H), 7.48 (d, 2H), 6.78 (d, 2H), 4.91 (s, 2H), 2.90 - 2.86 (t, 2H), 2.62 -

2.58 (t, 2H), 2.37 (s, 3H).

Example 25

3-(3,5-difluoro-4-((1-isopropyl-3-methyl-1*H*-pyrazol-5-yl)methoxy)phenyl)propanoic acid **(86)**

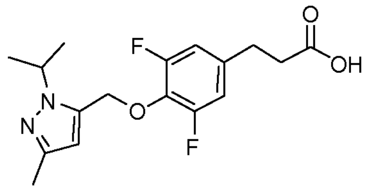

$^1$H NMR (400 MHz, $CDCl_3$) δ: 9.77 (br, 1H), 6.77 (d, 2H), 6.00 (s, 1H), 5.04 (s, 2H), 4.71 - 4.65 (m, 1H), 2.90 - 2.87 (t, 2H), 2.66 - 2.63 (t, 2H), 2.25 (s, 3H), 1.50 (d, 6H).

Example 26

3-(3,5-difluoro-4-(2-(5-methyl-3-phenylisoxazol-4-yl)-2-oxoethoxy)phenyl)propanoic acid **(87)**

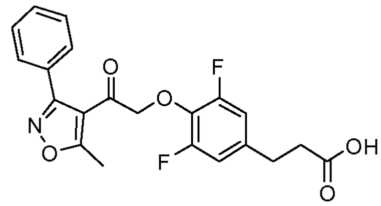

$^1$H NMR (400 MHz, $CDCl_3$) δ: 7.52 - 7.48 (m, 5H), 6.69 (d, 2H), 4.67 (s, 2H), 2.86 - 2.82 (t, 2H), 2.72 (s, 3H), 2.64 - 2.60 (t, 2H).

Example 27

3-(4-((1-(4-chlorophenyl)-3-methyl-1*H*-pyrazol-5-yl)methoxy)-3,5-difluorophenyl) butanoic acid **(88)**

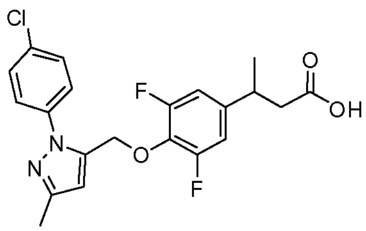

$^1$H NMR (400 MHz, $CDCl_3$) δ: 7.62 (d, 2H), 7.43 (d, 2H), 6.75 (d, 2H), 6.26 (s, 1H), 4.98 (s, 2H), 3.22 - 3.16 (m, 1H), 2.60 - 2.55 (m, 2H), 2.29 (s, 3H), 1.27 (d, 3H).

Example 28

3-(3,5-difluoro-4-((5-phenylthiazol-4-yl)methoxy)phenyl)propanoic acid **(89)**

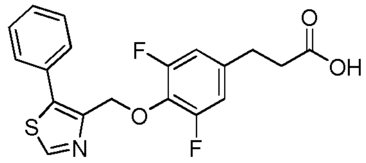

$^1H$ NMR (400 MHz, $CDCl_3$) δ 8.78 (s, 1H), 7.62 (d, *J*= 6.8 Hz, 2H), 7.45-7.44 (m, 3H), 6.75-6.73 (m, 2H), 5.19 (s, 2H), 2.88 (t, *J* = 7.6 Hz, 2H, 2.65 (t, *J* = 7.6 Hz, 2H).

Example 29

2-(4-((4-phenyl-1,2,3-thiadiazol-5-yl)methoxy)phenylthio)acetic acid **(90)**

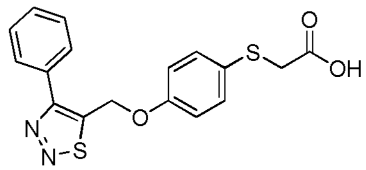

$^1H$ NMR (400 MHz, DMSO) δ 7.84 (d, *J*= 7.2 Hz, 2H), 7.58-7.52 (m, 3H), 7.36 (d, *J* = 8.8 Hz, 2H), 7.04 (d, *J* = 8.8, 2H), 5.66 (s, 2H), 3.66 (s, 2H).

Example 30

3-(3,5-difluoro-4-((2-methyl-4-phenylthiazol-5-yl)methoxy)phenyl)propanoic acid **(91)**

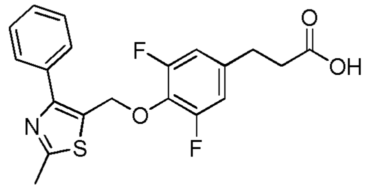

$^1H$ NMR (400 MHz, $CDCl_3$) δ 7.69 (d, J = 7.2 Hz, 2H), 7.45-7.38 (m, 3H), 6.75-6.73 (m, 2H), 5.23 (s, 2H), 2.90-2.86 (m, 2H), 2.74 (s, 3H), 2.67-2.63 (m, 2H).

Example 31

3-(3,5-difluoro-4-((4-(4-fluorophenyl)-1,2,3-thiadiazol-5-yl)methoxy)phenyl)propanoic acid **(92)**

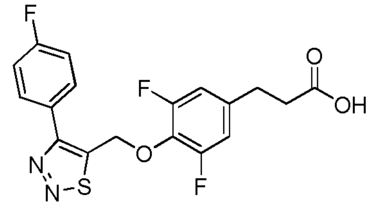

$^1$H NMR (400 MHz, $CDCl_3$) δ 7.81-7.78 (m, 2H), 7.26-7.21 (m, 3H), 6.81-6.79 (m, 2H), 5.47 (s, 2H), 2.91 (t, *J* = 7.4 Hz, 2H), 2.67 (t, *J* = 7.4 Hz, 2H).

Example 32

3-(4-((4-(3-chlorophenyl)-1,2,3-thiadiazol-5-yl)methoxy)-3,5-difluorophenyl)propanoic acid **(93)**

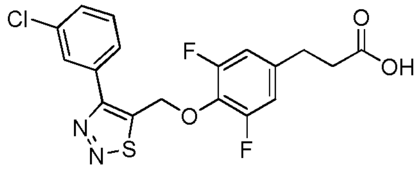

$^1$H NMR (400 MHz, $CDCl_3$) δ 7.79 (s, 1H), 7.70-7.66 (m, 1H), 7.47 (d, J = 4.8 Hz, 2H), 6.81-6.79 (m, 2H), 5.50 (s, 2H), 2.91 (t, *J* = 7.4 Hz, 2H), 2.67 (t, 7.4 Hz, 2H).

Example 33

2-(3,5-difluoro-4-((2-methyl-4-phenylthiazol-5-yl)methoxy)phenyl) cyclopropane carboxylic acid **(94)**

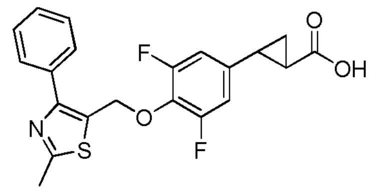

$^1$H NMR (400 MHz, $CDCl_3$) δ 7.67 (d, *J*= 7.6 Hz, 2H), 7.44-7.37 (m, 3H), 6.61-6.59 (m, 2H), 5.22 (s, 2H), 2.72 (s, 3H), 2.44-2.41 (m, 1H), 1.83-1.79 (m, 1H), 1.61-1.56 (m, 1H), 1.29-1.22 (m, 1H).

Example 34

2-(3,5-difluoro-4-((5-methyl-3-phenylisoxazol-4-yl)methoxy)benzyloxy)acetic acid **(95)**

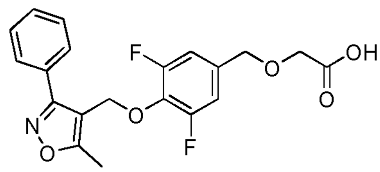

$^1$H NMR (400 MHz, $CDCl_3$) δ 7.87-7.84 (m, 2H), 7.50-7.49 (m, 3H), 6.94-6.92 (m, 2H), 4.98 (s, 2H), 4.57 (s,

2H), 4.18 (s, 2H), 2.42 (s, 3H).

Example 35

2-(4-((1-(4-chlorophenyl)-3-methyl-1*H*-pyrazol-5-yl)methoxy)-3,5-difluorobenzyloxy) acetic acid **(96)**

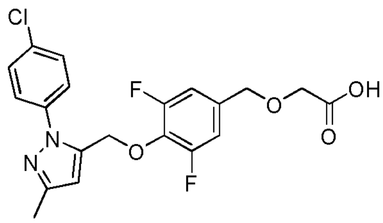

$^1$H NMR (400 MHz, DMSO) δ 7.63 (d, *J*= 9.0 Hz, 2H), 7.56 (d, *J*= 9.0 Hz, 2H), 7.08-7.06 (m, 2H), 6.32 (s, 2H), 5.13 (s, 2H), 4.47 (s, 2H), 4.07 (s, 2H), 2.19 (s, 3H).

Example 36

3-(4-((1-(4-chlorophenyl)-3-methyl-1*H*-pyrazol-5-yl)methoxy)-3,5-difluorophenyl)-2-methylpropanoic acid **(97)**

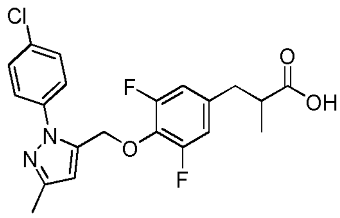

$^1$H NMR (400 MHz, $CDCl_3$) δ 7.65 (d, *J*= 9.2 Hz, 2H), 7.43 (d, *J*= 9.2 Hz, 2H), 6.75-6.73 (m, 2H), 6.24 (s, 1H), 5.00 (s, 2H), 3.00-2.94 (m, 1H), 2.74-2.70 (m, 1H), 2.65-2.60 (m, 1H), 2.30 (s, 3H), 1.20 (d, *J*= 7.2 Hz, 3H).

Example 37

3-(3,5-difluoro-4-((5-methyl-3-phenylisoxazol-4-yl)methoxy)phenyl)-2-methylpropanoic acid **(98)**

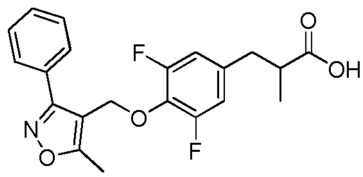

$^1$H NMR (400 MHz, $CDCl_3$) δ 7.88-7.85 (m, 2H), 7.50-7.48 (m, 3H), 6.75-6.73 (m, 2H), 4.94 (s, 2H), 2.99-2.94 (m, 1H), 2.75-2.71 (m, 1H), 2.67-2.62 (m, 1H), 2.35 (s, 3H), 1.21 (d, *J* = 7.2 Hz, 3H).

Example 38

3-(4-((1-(4-chlorophenyl)-3-methyl-1*H*-pyrazol-5-yl)methoxy)-3,5-difluorophenyl)-2,2-dimethylpropanoic acid **(99)**

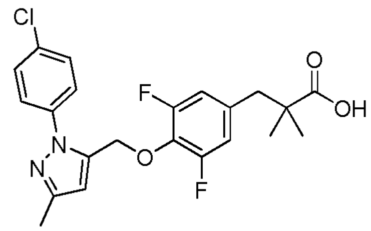

$^1H$ NMR (400 MHz, $CDCl_3$) δ 7.62 (d, *J* = 8.6 Hz, 2H), 7.45 (d, *J* = 8.6 Hz, 2H), 6.74-6.71 (m, 2H), 6.25 (s, 1H), 4.99 (s, 2H), 2.81 (s, 2H), 2.32 (s, 3H), 1.22 (s, 6H).

Example 39

2-(6-((1-(4-chlorophenyl)-3-methyl-1*H*-pyrazol-5-yl)methoxy)-1,2,3,4-tetrahydronaphthalen-2-yl)acetic acid **(100)**

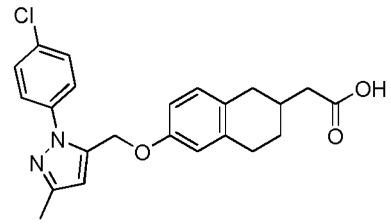

$^1H$ NMR (400 MHz, $CDCl_3$) δ 7.52 (d, *J*= 8.6 Hz, 2H), 7.38 (d, *J*= 8.6 Hz, 2H), 6.97 (d, *J* = 8.4 Hz, 1H), 6.70-6.68 (m, 1H), 6.63 (s, 1H), 6.34 (s, 1H), 4.88 (d, 2H), 2.92-2.80 (m, 3H), 2.49-2.45 (m, 1H), 2.41 (d, *J*= 6.4 Hz, 2H), 2.34 (s, 3H), 2.30-2.20 (m, 1H), 1.99-1.97 (m, 1H), 1.56-1.44 (m, 1H).

Example 40

2-(6-((5-methyl-3-phenylisoxazol-4-yl)methoxy)-1,2,3,4-tetrahydronaphthalen-2-yl)acetic acid **(101)**

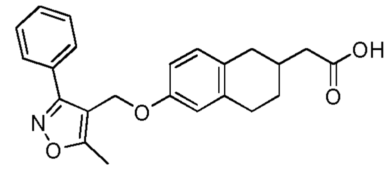

$^1H$ NMR (400 MHz, $CDCl_3$) δ 7.74 (m, 2H), 7.45-7.42 (m, 3H), 7.00 (d, *J*= 8.4 Hz, 1H), 6.75-6.73 (m, 1H), 6.68 (s, 1H), 4.81 (s, 2H), 2.93-2.82 (m, 3H), 2.51-2.50 (m, 1H), 2.49 (s, 3H), 2.44 (d, *J*= 7.2 Hz), 2.31-2.25 (m, 1H), 2.1-1.98 (m, 1H), 1.55-1.48 (m, 1H).

Example 41

3-(4-((1-tert-butyl-3-methyl-1*H*-pyrazol-5-yl)methoxy)-3,5-difluorophenyl)propanoic acid **(102)**

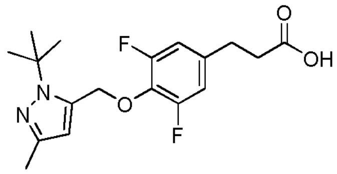

$^1H$ NMR (400 MHz, $CDCl_3$) δ 7.74 (m, 2H), 6.76 (m, 2H), 6.05 (s, 1H), 5.18 (s, 2H), 2.89 (t, *J* = 7.2 Hz, 2H), 2.66 (t, *J* = 7.2 Hz, 2H), 2.22 (s, 3H), 1.70 (s, 9H).

Example 42

3-(3,5-difluoro-4-((2-methyl-4-phenylthiazol-5-yl)methoxy)phenyl)-2-methylpropanoic acid **(103)**

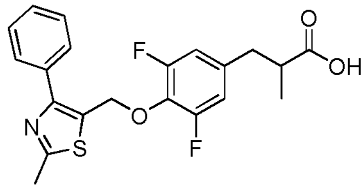

$^1H$ NMR (400 MHz, $CDCl_3$) δ 7.62-7.59 (m, 2H), 7.45-7.43 (m, 3H), 6.74-6.72 (m, 2H), 5.24 (s, 2H), 2.98-2.92 (m, 1H), 2.81 (s, 3H), 2.75-2.73 (m, 1H), 2.66-2.63 (m, 1H), 1.21 (d, *J*= 6.8 Hz, 3H).

Example 43

(*R*)-3-(4-((1-(4-chlorophenyl)-3-methyl-1*H*-pyrazol-5-yl)methoxy)-3,5-difluorophenyl)-2-methylpropanoic acid **(104)**

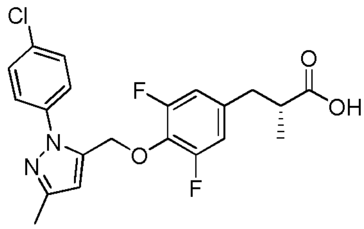

$^1H$ NMR (400 MHz, $CDCl_3$) δ 7.65 (d, *J*= 9.2 Hz, 2H), 7.43 (d, *J*= 9.2 Hz, 2H), 6.75-6.73 (m, 2H), 6.24 (s, 1H), 5.00 (s, 2H), 3.00-2.94 (m, 1H), 2.74-2.70 (m, 1H), 2.65-2.60 (m, 1H), 2.30 (s, 3H), 1.20 (d, *J*= 7.2 Hz, 3H).

Example 44

(*S*)-3-(4-((1-(4-chlorophenyl)-3-methyl-1*H*-pyrazol-5-yl)methoxy)-3,5-difluorophenyl)-2-methylpropanoic acid **(105)**

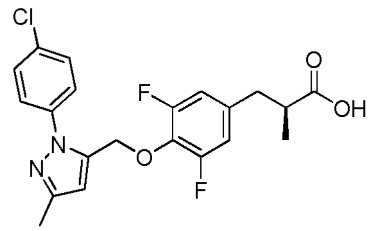

$^1H$ NMR (400 MHz, $CDCl_3$) δ 7.65 (d, *J*= 9.2 Hz, 2H), 7.43 (d, *J*= 9.2 Hz, 2H), 6.75-6.73 (m, 2H), 6.24 (s, 1H), 5.00 (s, 2H), 3.00-2.94 (m, 1H), 2.74-2.70 (m, 1H), 2.65-2.60 (m, 1H), 2.30 (s, 3H), 1.20 (d, *J*= 7.2 Hz, 3H).

Example 45

4-(4-((3-methyl-1-phenyl-1*H*-pyrazol-5-yl)methoxy)phenyl)butanoic acid **(106)**

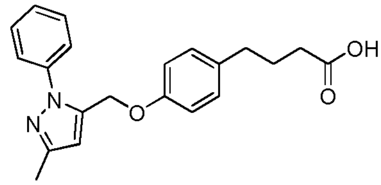

$^1H$ NMR (400 MHz, $CDCl_3$) δ: 7.53 (d, *J* = 7.6 Hz, 2H), 7.46-7.36 (m, 3H), 7.09 (d, *J* = 8.4 Hz, 2H), 6.82 (d, *J* = 8.4 Hz, 2H), 6.38 (s, 1H), 4.91 (s, 2H), 2.62 (t, *J* = 7.2 Hz, 2H), 2.38 (s, 3H), 2.36 (t, *J* = 7.2 Hz, 2H), 1.98-1.88 (m, 2H).

Example 46

3-(3,5-difluoro-4-((3-methyl-1-phenyl-1*H*-pyrazol-5-yl)methoxy)phenyl)propanoic acid **(107)**

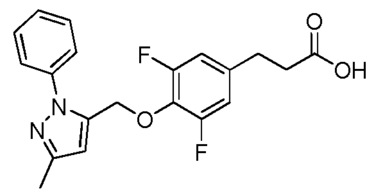

$^1H$ NMR (400 MHz, $CDCl_3$) δ: 7.64 (d, *J* = 7.8 Hz, 2H), 7.46 (t, *J* = 7.6 Hz, 2H), 7.37 (m, 1H), 6.74 (d, *J* = 8.4 Hz, 2H), 6.26 (s, 1H), 4.91 (s, 2H), 2.87 (t, *J* = 7.6 Hz, 2H), 2.62 (t, *J* = 7.6 Hz, 2H), 2.32 (s, 3H).

Example 47

3-(3,5-difluoro-4-((1-(4-fluorophenyl)-3-methyl-1*H*-pyrazol-5-yl)methoxy)phenyl) propanoic acid **(108)**

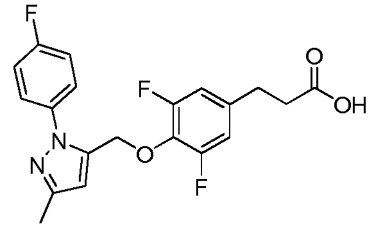

$^1H$ NMR (400 MHz, $CDCl_3$) δ: 7.66-7.62 (m, 2H), 7.15 (t, J= 8.4 Hz, 2H), 6.75 (d, *J* = 8.4 Hz, 2H), 6.25 (s, 1H), 4.98 (s, 2H), 2.89 (t, *J* = 7.6 Hz, 2H), 2.65 (t, *J* = 7.6 Hz, 2H), 2.31 (s, 3H).

Example 48

3-(3,5-difluoro-4-((1-phenyl-1*H*-pyrazol-5-yl)methoxy)phenyl)propanoic acid (109)

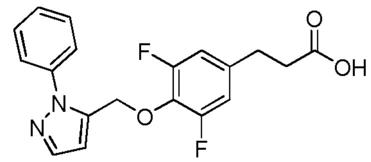

$^1H$ NMR (400 MHz, $CDCl_3$) δ: 7.69 (d, J= 7.6 Hz, 2H), 7.64 (d, *J* = 1.6 Hz, 1H), 7.5 (t, *J* = 7.6 Hz, 2H), 7.42 (m, 1H), 6.74 (d, *J* = 8.8 Hz, 2H), 6.44 (d, *J* = 1.6 Hz, 1H), 5.08 (s, 2H), 2.88 (t, *J* = 7.6 Hz, 2H), 2.65 (t, J= 7.6 Hz, 2H).

Example 49

3-(3,5-difluoro-4-((1-(4-methoxyphenyl)-3-methyl-1*H*-pyrazol-5-yl)methoxy)phenyl) propanoic acid (110)

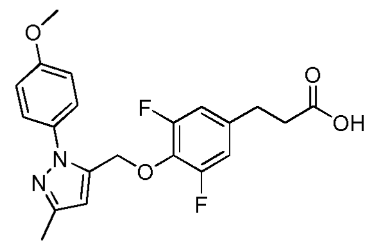

$^1H$ NMR (400 MHz, $CDCl_3$) δ: 7.54 (d, *J* = 8.4 Hz, 2H), 6.97 (d, *J* = 8.4 Hz, 2H), 6.74 (d, *J* = 8.8 Hz, 2H), 6.22 (s, 1H), 4.98 (s, 2H), 3.85 (s, 3H), 2.88 (t, *J* = 7.6 Hz, 2H), 2.64 (t, *J* = 7.6 Hz, 2H), 2.31 (s, 3H).

Example 50

3-(4-((1-(4-chlorophenyl)-3-methyl-1*H*-pyrazol-5-yl)methoxy)-3,5-difluorophenyl) propanoic acid **(111)**

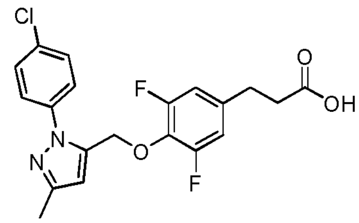

$^1H$ NMR (400 MHz, DMSO-$d^6$) δ: 7.61 (d, *J* = 8.4 Hz, 2H), 7.54 (d, *J* = 8.4 Hz, 2H), 6.97 (d, *J* = 9.2 Hz, 2H), 6.31 (s, 1H), 5.08 (s, 2H), 2.74 (t, *J* = 7.8 Hz, 2H), 2.51 (t, *J* = 7.8 Hz, 2H), 2.19 (s, 3H).

Example 51

3-(3,5-difluoro-4-((1-(4-fluorophenyl)-4-methyl-1*H*-pyrazol-5-yl)methoxy)phenyl) propanoic acid **(112)**

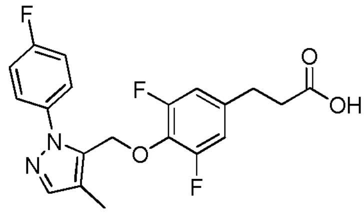

$^1H$ NMR (400 MHz, $CDCl_3$) δ: 7.71-7.68 (m, 2H), 7.5 (s, 1H), 7.17 (t, *J* = 8.6 Hz, 2H), 6.77 (d, *J* = 8.8 Hz, 2H), 4.95 (s, 2H), 2.9 (t, *J* = 7.6 Hz, 2H), 2.66 (t, *J* = 7.6 Hz, 2H), 2.01 (s, 3H).

Example 52

3-(4-((4-(4-chlorophenyl)-1,2,3-thiadiazol-5-yl)methoxy)-3,5-difluorophenyl)-2,2-difluoropropanoic acid **(113)**

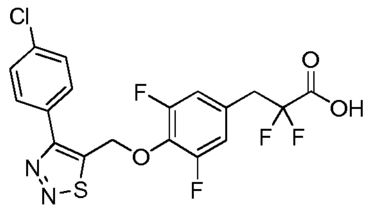

$^1H$ NMR (400 MHz, $CDCl_3$) δ: 7.73 (d, *J* = 8.4 Hz, 2H), 7.52 (d, *J* = 8.4 Hz, 2H), 6.89 (d, *J* = 8.4 Hz, 2H), 5.51 (s, 2H), 3.33 (t, *J* = 16 Hz, 2H).

Example 53

3-(4-((1-(3,4-difluorophenyl)-3-methyl-1*H*-pyrazol-5-yl)methoxy)-3,5-difluorophenyl) propanoic acid **(114)**

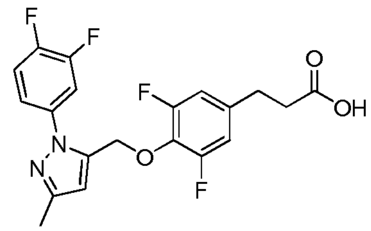

$^1H$ NMR (400 MHz, $CDCl_3$) δ: 7.62-7.57 (m, 1H), 7.52-7.46 (m, 1H), 7.29-7.22 (m, 1H), 6.77 (d, *J* = 8.8 Hz, 2H), 6.25 (s, 1H), 4.99 (s, 2H), 2.89 (t, *J* = 7.4 Hz, 2H), 2.66 (t, *J* = 7.4 Hz, 2H), 2.3 (s, 3H).

Example 54

3-(4-((1-(4-chlorophenyl)-3-methyl-1*H*-pyrazol-5-yl)methoxy)-3,5-difluorophenyl)-2-fluoropropanoic acid (115)

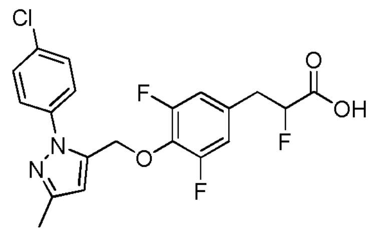

$^1H$ NMR (400 MHz, DMSO-$d^6$) δ: 7.6 (d, *J*= 9 Hz, 2H), 7.45 (d, *J*= 9 Hz, 2H), 6.81 (d, *J*= 8.8 Hz, 2H), 6.23 (s, 1H), 5.1 (ddd, *J*= 48.8, 6.8, 4 Hz, 1H), 5.01 (s, 2H), 328-3.05 (m, 2H), 2.26 (s, 3H).

Example 55

2-(4-((1-(4-chlorophenyl)-3-methyl-1*H*-pyrazol-5-yl)methoxy)-3,5-difluorobenzyl) cyclopropanecarboxylic acid (116)

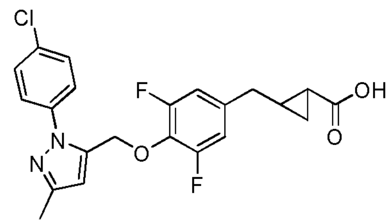

$^1H$ NMR (400 MHz, $CDCl_3$) δ: 7.64 *(d, J*= 8.8 Hz, 2H), 7.43 *(d, J*= 8.8 Hz, 2H), 6.63 (d, *J*= 9.2 Hz, 2H), 6.26 (s, 1H), 4.98 (s, 2H), 2.49 (dd, *J*= 16.4, 7 Hz, 1H), 2.38 (dd, *J* = 16, 7.2 Hz, 1H), 2.31 (s, 3H), 1.74-1.68 (m, 1H), 1.38-1.28 (m, 1H), 1.0-0.88 (m, 2H).

Example 56

3-(4-((1-(4-chlorophenyl)-3-(trifluoromethyl)-1*H*-pyrazol-5-yl)methoxy)-3,5-difluorophenyl)propanoic acid **(117)**

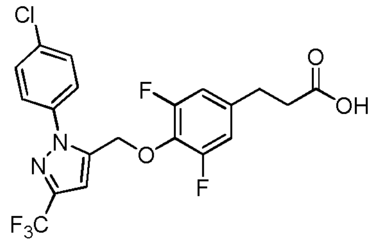

$^1H$ NMR (400 MHz, $CDCl_3$) δ: 7.68 *(d, J*= 9 Hz, 2H), 7.5 (d*, J*= 9 Hz, 2H), 6.78 (d, *J*= 8.8 Hz, 2H), 6.72 (s, 1H), 5.03 (s, 2H), 2.9 (t, *J* = 7.6 Hz, 2H), 2.66 (t, *J*= 7.6 Hz, 2H).

Example 57

3-(4-((1-(4-chlorophenyl)-3-(trifluoromethyl)-1*H*-pyrazol-5-yl)methoxy)-3,5-difluorophenyl)-2,2-difluoropropanoic acid **(118)**

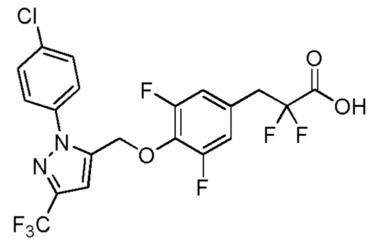

$^1H$ NMR (400 MHz, $CDCl_3$) δ: 7.64 (d, *J* = 8.8 Hz, 2H), 7.54 (d, *J* = 8.8 Hz, 2H), 6.88 (d, *J* = 8.8 Hz, 2H), 6.73 (s, 1H), 5.07 (s, 2H), 3.33 (t, *J* = 16 Hz, 2H).

Example 58

2-(5-((5-methyl-3-phenylisoxazol-4-yl)methoxy)-2,3-dihydro-1*H*-inden-1-yl)acetic acid **(119)**

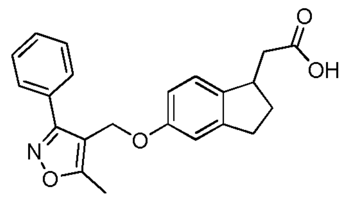

$^1H$ NMR (400 MHz, $CDCl_3$) δ: 7.76 - 7.72 (m, 2H), 7.48 - 7.42 (m, 3H), 7.15 (d, *J* = 8.4 Hz, 1H), 6.85 (d, *J* = 2.4 Hz, 1H), 6.80 (dd, *J* = 8.4, 2.4 Hz, 1H), 4.84 (s, 2H), 3.62 - 3.54 (m, 1H), 2.98 - 2.78 (m, 3H), 2.56 - 2.40 (m, 5H), 1.88 - 1.76 (m, 1H).

Example 59

2-(5-((1-(4-chlorophenyl)-3-methyl-1*H*-pyrazol-5-yl)methoxy)-2,3-dihydro-1*H*-inden-1-yl)acetic acid **(120)**

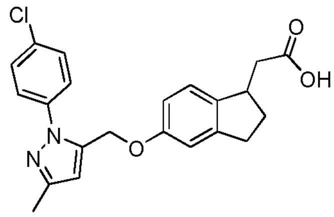

$^1H$ NMR (400 MHz, $CDCl_3$) δ: 7.55 - 7.50 (m, 2H), 7.41 - 7.37 (m, 2H), 7.11 (d, *J* = 8.4 Hz, 1H), 6.79 (d, *J* = 2.4 Hz, 1H), 6.74 (dd, *J* = 8.4, 2.4 Hz, 1H), 6.36 (s, 1H), 4.89 (s, 2H), 3.60 - 3.50 (m, 1H), 2.96-2.76 (m, 3H), 2.52 - 2.38 (m, 2H), 2.36 (s, 3H), 1.84 - 1.72 (m, 1H).

Example 60

2-(5-((5-methyl-3-phenylisoxazol-4-yl)methoxy)-2,3-dihydro-1*H*-inden-1-yl)propanoic acid **(121)**

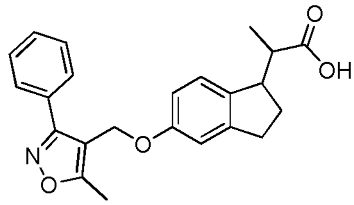

LC-MS ESI *m/z*; found 378 [M+H]$^+$.

Example 61

2-(5-((1-(4-chlorophenyl)-3-methyl-1*H*-pyrazol-5-yl)methoxy)-2,3-dihydro-1*H*-inden-1-yl)propanoic acid **(122)**

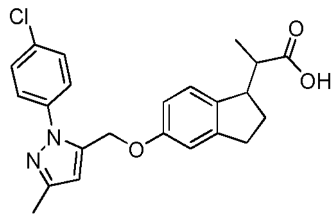

LC-MS ESI *m/z*; found: 411 [M+H]$^+$.

Example 62

3-(4-((4-(4-chlorophenyl)-1,2,3-thiadiazol-5-yl)methoxy)-3,5-difluorophenyl)propanoic acid **(123)**

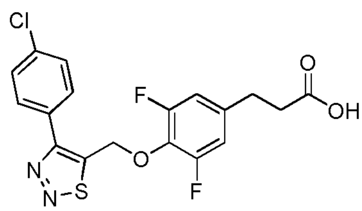

$^1$H NMR (400 MHz, $CDCl_3$) δ: 7.75 (d, 2H), 7.51 (d, 2H), 6.80 (d, 2H), 5.48 (s, 2H), 2.91 (t, 2H), 2.70 (t, 2H).

Example 63

2-(3,5-difluoro-4-((4-phenyl-1,2,3-thiadiazol-5-yl)methoxy)phenyl)cyclopropane carboxylic acid **(124)**

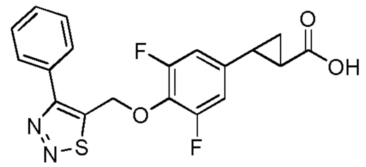

$^1$H NMR (400 MHz, $CDCl_3$) δ: 7.76 (d, 2H), 7.52-7.48 (m, 3H), 6.69 (d, 2H), 5.52 (s, 2H), 2.56-2.48 (m, 1H), 1.89-1.82 (m, 1H), 1.71-1.64 (m, 1H), 1.38-1.30 (m, 1H).

Example 64

2-(3,5-difluoro-4-((3-methyl-1-phenyl-1*H*-pyrazol-5-yl)methoxy)phenyl)cyclopropane carboxylic acid **(125)**

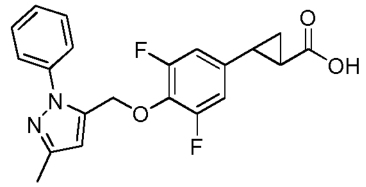

$^1$H NMR (400 MHz, $CDCl_3$) δ: 7.64 (d, 2H), 7.47 (t, 2H), 7.39 (t, 1H), 6.64 (d, 2H), 6.25 (s, 1H), 5.03 (s, 2H), 2.54-2.46 (m, 1H), 2.33 (s, 3H), 1.88-1.82 (m, 1H), 1.69-1.62 (m, 1H), 1.35-1.32 (m, 1H).

Example 65

3-(3,5-difluoro-4-((1-phenyl-3-(trifluoromethyl)-1*H*-pyrazol-5-yl)methoxy)phenyl) propanoic acid **(126)**

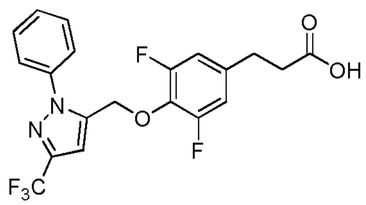

$^1$H NMR (400 MHz, $CDCl_3$) δ: 7.67 (d, 2H), 7.52-7.42 (m, 3H), 6.99 (s, 1H), 6.79-6.76 (m, 2H), 5.06 (s, 2H), 2.89 (t, 2H), 2.66 (t, 2H).

Example 66

(*R*)-2-(4-((4-(4-chlorophenyl)-1,2,3-thiadiazol-5-yl)methoxy)-3,5-difluorophenoxy) propanoic acid **(127)**

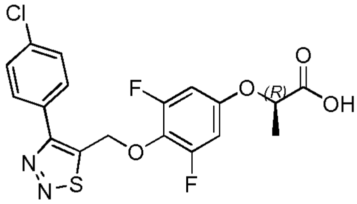

$^1H$ NMR (400 MHz, DMSO-$d_6$) δ: 7.87 (d, *J* = 8.4 Hz, 2H), 7.64 (d, *J* = 8.4 Hz, 2H), 6.98 (d, *J* = 9.2 Hz, 2H), 6.81 (d, *J* = 9.2 Hz, 2H), 5.60 (s, 2H), 4.70 (q, *J* = 6.6 Hz, 1H)), 1.44 (d, *J* = 6.8 Hz, 3H). LC-MS ESI *m/z*; found 389.2 [M - H]$^-$.

Example 67

4-(4-((4-(4-chlorophenyl)-1,2,3-thiadiazol-5-yl)methoxy)phenyl)-4-oxobutanoic acid (128)

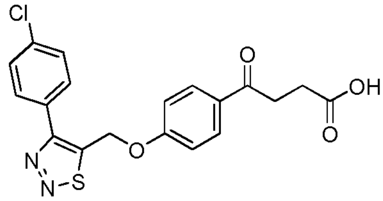

$^1H$ NMR (400 MHz, DMSO-$d_6$) δ: 7.97 (d, *J* = 8.4 Hz, 2H), 7.89 (d, *J* = 8.8 Hz, 2H), 7.65 (d, *J* = 7.6 Hz, 2H), 7.18 (d, *J* = 9.2 Hz, 2H), 5.80 (s, 2H), 3.18 (t, *J* = 7.6 Hz, 3H), 2.54 (t, *J* = 7.6 Hz, 3H). LC-MS: 401.0 [M - H]$^-$.

Example 68

(*Z*)-4-(4-((4-(4-chlorophenyl)-1,2,3-thiadiazol-5-yl)methoxy)phenyl)-4-(methoxyimino) butanoic acid (129)

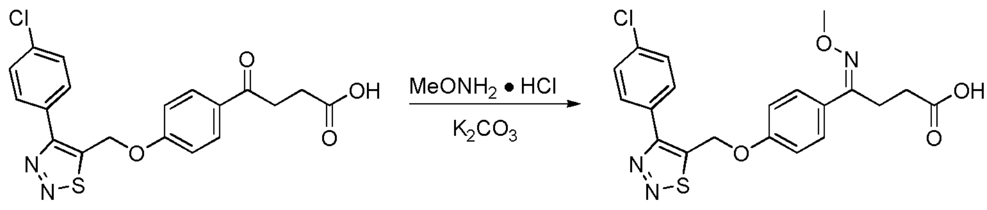


**128** **129**

A mixture of compound (128) (30 mg, 0.074 mmol), methoxylamine hydrochloride (12.4 mg, 0.148 mmol), and $K_2CO_3$ (0.2 g, 1.44 mmol) in absolute ethanol (2.5 mL) was stirred at 75 °C overnight. After evaporation of solvent, the residue was purified by reverse phase HPLC to give the desired product **(129).** $^1H$ NMR (400 MHz, DMSO-$d_6$) δ: 7.89 (d, *J* = 8.4 Hz, 2H), 7.65 (d, *J* = 8.4 Hz, 2H), 7.60 (d, *J* = 8.4 Hz, 2H), 7.07 (d, *J* = 8.4 Hz, 2H), 5.71 (s, 2H), 3.86 (s, 3H), 2.83 (t, *J* = 7.6 Hz, 3H), 2.18 (t, *J* = 7.6 Hz, 3H).

Example 69

(E)-4-(4-((4-(4-chlorophenyl)-1,2,3-thiadiazol-5-yl)methoxy)phenyl)-3-methylbut-2-enoic acid **(130)**

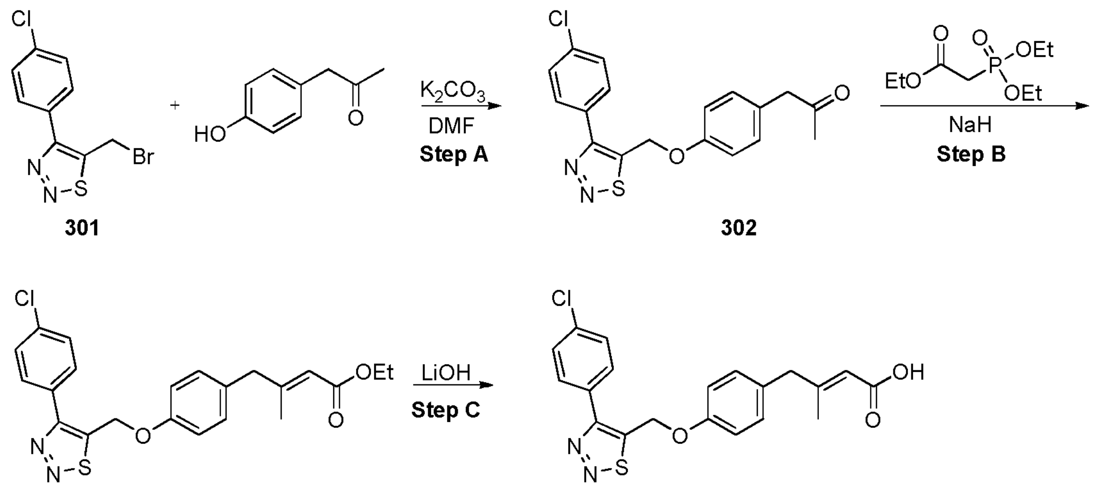


301 302 303 130

**Step A:** Compound **(302)** was prepared in a similar manner as that described for the synthesis of **56.**

**Step B:** To a mixture of compound **(302)** (0.1 g, 0.279 mmol) and NaH (60% dispersion in mineral oil, 18 mg) in anhydrous THF (4 mL) at 0 °C was added dropwise triethyl 2-phosphonoacetate (0.089 mL, 0.446 mmol). The reaction mixture was stirred at 0 °C for 1 hour and at room temperature for 2 hours. The reaction was quenched with ice water (30 mL) and the mixture was extracted with EtOAc (3 x 50 mL). The solvent was removed in *vacuo* and the product **(303)** was used in the next step with out further purification.

**Step C:** Compound **(130)** was prepared in a similar manner as that described for the synthesis of **57.** $^1$H NMR (400 MHz, DMSO-$d_6$) δ: 7.91-7.88 (m, 2H), 7.65 (d, *J* = 8.4 Hz, 2H), 7.23-7.13 (m, 2H), 7.07-7.01 (d, *J* = 8.4 Hz, 2H), 5.69-5.66 (m, 2H), 3.37 (s, 1H), 3.07 (s, 2H), 1.88 (s, 3H).

Example 70

(E)-5-(4-((4-(4-chlorophenyl)-1,2,3-thiadiazol-5-yl)methoxy)phenyl)-3-methylpent-2-enoic acid (131)

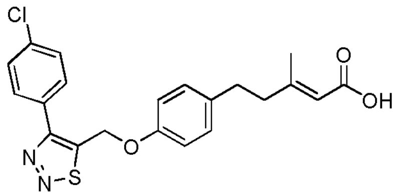

$^1$H NMR (400 MHz, DMSO-$d_6$) δ: 7.89 (d, *J* = 8.4 Hz, 2H), 7.65 (d, *J* = 8.4 Hz, 2H), 7.18-7.12 (m, 2H), 6.99-6.97 (m, 2H), 5.66 (s, 2H), 3.16 (s, 1H), 2.07-2.66 (m, 2H), 2.38-2.34(m, 2H), 2.09 (m, 3H).

Example 71

5-(4-((1-(4-chlorophenyl)-3-methyl-1*H*-pyrazol-5-yl)methoxy)phenyl)-3-methyl pentanoic acid (132)

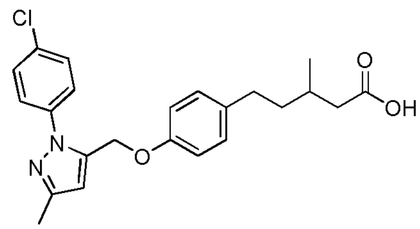

$^1H$ NMR (400 MHz, DMSO-$d_6$) δ: 11.99 (s, 1H), 7.58 (d, J = 8.4 Hz, 2H), 7.53 (d, *J* = 8.4 Hz, 2H), 7.08 (d, *J* = 8.8 Hz, 2H), 6.86 (d, *J* = 8.0 Hz, 2H), 6.44 (s, 1H), 5.05 (s, 2H), 2.54-2.51 (m, 2H), 2.28-2.23 (m, 4H), 2.05-2.00 (m, 1H), 1.83-1.78 (m, 1H), 1.55-1.51 (m, 1H), 1.41-1.38 (m, 1H), 0.91 (d, *J* = 6.8 Hz, 3H).

Example 72

4-(4-((1-(4-chlorophenyl)-3-methyl-1*H*-pyrazol-5-yl)methoxy)phenyl)-3-methylbutanoic acid **(133)**

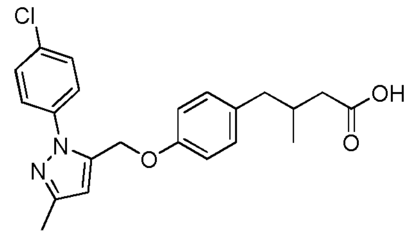

$^1H$ NMR (400 MHz, DMSO-$d_6$) δ: 12.03 (s, 1H), 7.58 (d, *J* = 8.8 Hz, 2H), 7.53 (d, *J* = 8.8 Hz, 2H), 7.06 (d, *J* = 8.4 Hz, 2H), 6.88 (d, *J* = 8.0 Hz, 2H), 6.45 (s, 1H), 5.05 (s, 2H), 2.53-2.51 (m, 1H), 2.39-2.34 (m, 1H), 2.22 (s, 3H), 2.20-2.15 (m, 1H), 2.06-1.96 (m, 2H), 0.83 (d, *J* = 6.4 Hz, 3H).

Example 73

3-(4-((4-(3,4-difluorophenyl)-1,2,3-thiadiazol-5-yl)methoxy)-3,5-difluorophenyl) propanoic acid **(134)**

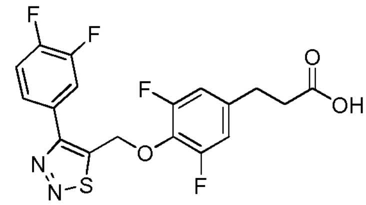

$^1H$ NMR (400 MHz, $CDCl_3$) δ: 7.73-7.68 (m, 1H), 7.59-7.53 (m, 1H), 7.34 (t, 1H), 6.80 (d, 2H), 5.47 (s, 2H), 2.91 (t, 2H), 2.67 (t, 2H).

Example 74

3-(4-((1-(3,4-dichlorophenyl)-3-methyl-1*H*-pyrazol-5-yl)methoxy)-3,5-difluorophenyl) propanoic acid **(135)**

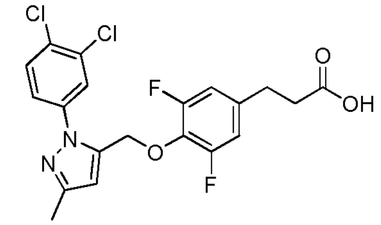

$^1$H NMR (400 MHz, $CDCl_3$) δ: 7.84 (d, 1H), 7.63 (d, 1H), 7.60 (d, 1H), 6.79-6.74 (m, 2H), 6.26 (s, 1H), 5.01 (s, 2H), 2.91-2.84 (m, 2H), 2.68-2.62 (m, 2H), 2.31 (s, 3H).

Example 75

3-(4-((4-isopropoxy-2-methylthiazol-5-yl)methoxy)phenyl)propanoic acid **(136)**

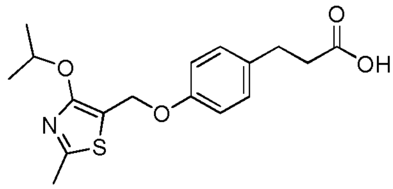

$^1$H NMR (400 MHz, $CDCl_3$) δ: 7.11 (d, 2H), 6.90 (d, 2H), 5.04 (s, 2H), 5.02-4.95 (m, 1H), 2.89 (t, 2H), 2.64 (t, 2H), 2.59 (s, 3H), 1.31 (d, 6H).

Example 76

3-(3,5-difluoro-4-((1-(4-isopropylphenyl)-3-methyl-1H-pyrazol-5-yl)methoxy)phenyl)-2-methylpropanoic acid **(137)**

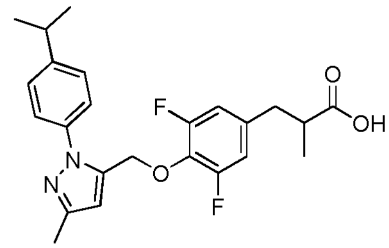

$^1$H NMR (400 MHz, $CDCl_3$) δ: 7.52 (d, *J* = 8.4 Hz, 2H), 7.29 (d, *J* = 8.4 Hz, 2H), 6.70 (d, *J* = 8.8 Hz, 2H), 6.20 (s, 1H), 5.00 (s, 2H), 2.96 - 2.90 (m, 2H), 2.66 - 2.63 (m, 1H), 2.58 - 2.53 (m, 1H), 2.28 (s, 3H), 1.26 (d, *J* = 8.5 Hz, 6H), 1.12 (d, *J* = 9.0 Hz, 3H). LC-MS ESI *m/z*; found 429.5 $[M+H]^+$.

Example 77

3-(3,5-difluoro-4-((3-methyl-1-(4-(trifluoromethyl)phenyl)-1H-pyrazol-5-yl)methoxy)phenyl)-2-methylpropanoic acid **(138)**

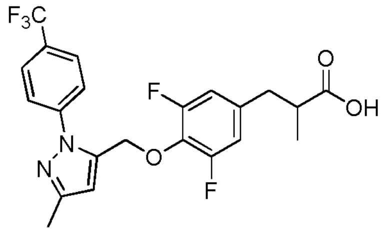

$^1H$ NMR (400 MHz, $CDCl_3$) δ: 7.87 (d, *J* = 8.4 Hz, 2H), 7.73 (d, *J* = 8.4 Hz, 2H), 6.73 (d, *J* = 8.8 Hz, 2H), 6.27 (s, 1H), 5.02 (s, 2H), 2.97 - 2.92 (m, 1H), 2.71 - 2.68 (m, 1H), 2.63 - 2.58 (m, 1H), 2.31 (s, 3H), 1.18 (d, *J* = 7.2 Hz, 3H). LC-MS ESI *m*/*z*; found 455.5 $[M+H]^+$.

Example 78

3-(4-((1-(4-chlorophenyl)-3-methyl-1H-pyrazol-5-yl)methoxy)-3-(trifluoromethyl)phenyl)-2-methylpropanoic acid **(139)**

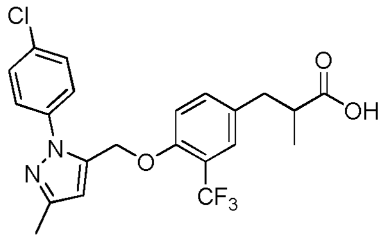

$^1H$ NMR (400 MHz, $CDCl_3$) δ: 7.52 (d, *J* = 8.4 Hz, 2H), 7.42 - 7.39 (m, 3H), 7.31 (m, 1H) 6.91 (d, *J* = 8.4 Hz, 1H), 6.36 (s, 1H), 4.98 (s, 2H), 3.04 - 3.00 (m, 1H), 2.76 - 2.69 (m, 2H), 2.35 (s, 3H), 1.20 (d, *J* = 7.2 Hz, 3H). LC-MS ESI *m*/*z*; found 454.15 $[M+H]^+$.

Example 79

3-(4-((1-(4-carbamoylphenyl)-3-methyl-1H-pyrazol-5-yl)methoxy)-3,5-difluorophenyl)-2-methylpropanoic acid **(140)**

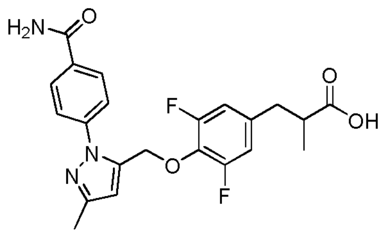

$^1H$ NMR (400 MHz, $CDCl_3$) δ: 7.87 (d, *J* = 8.8 Hz, 2H), 7.74 (d, *J* = 8.0 Hz, 2H), 6.73 (d, *J* = 9.2 Hz, 2H), 6.34 (s, 1H), 5.10 - 5.01 (m, 2H), 2.92 - 2.86 (m, 1H), 2.76 - 2.66 (m, 2H), 2.34 (s, 3H), 1.25 (d, *J* = 6.8 Hz, 3H). LC-MS ESI *m*/*z*; found 430.1 $[M+H]^+$.

Example 80

3-(4-((1-(4-cyanophenyl)-3-methyl-1H-pyrazol-5-yl)methoxy)-3,5-difluorophenyl)-2-methylpropanoic acid **(141)**

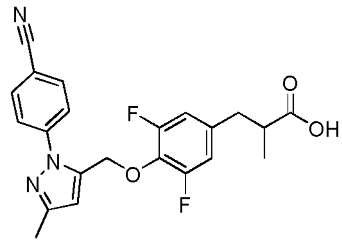

$^1H$ NMR (400 MHz, $CDCl_3$) δ: 7.93 (d, *J* = 8.8 Hz, 2H), 7.78 (d, *J* = 8.4 Hz, 2H), 6.76 (d, *J* = 9.2 Hz, 2H), 6.30 (s, 1H), 5.04 (s, 2H), 3.00 - 2.95 (m, 1H), 2.76 - 2.65 (m, 2H), 2.32 (s, 3H), 1.22 (d, *J* = 6.8 Hz, 3H). LC-MS ESI *m/z*; found 411.8 $[M+H]^+$.

Example 81

3-(4-((1-(2,3-dihydro-1H-inden-5-yl)-3-methyl-1H-pyrazol-5-yl)methoxy)-3,5-difluorophenyl)-2-methylpropanoic acid **(142)**

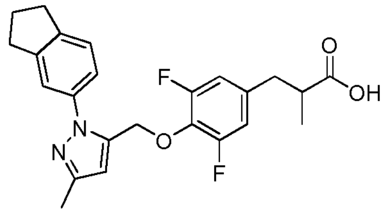

$^1H$ NMR (400 MHz, $CDCl_3$) δ: 7.45 (s, 1H), 7.37 (d, *J* = 8.0 Hz, 1H), 7.28 (m, 1H), 6.71 (d, *J* = 9.2 Hz, 2H), 6.23 (s, 1H), 5.00 (s, 2H), 2.97 - 2.92 (m, 5H), 2.71 - 2.57 (m, 2H), 2.30 (s, 3H), 2.15 - 2.10 (m, 2H), 1.16 (d, *J* = 6.8 Hz, 3H). LC-MS ESI *m/z*; found 426.8 $[M+H]^+$.

Example 82

3-(4-((1-(4-butylphenyl)-3-methyl-1H-pyrazol-5-yl)methoxy)-3,5-difluorophenyl)-2-methylpropanoic acid **(143)**

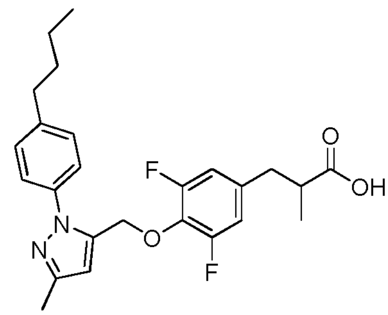

$^1H$ NMR (400 MHz, $CDCl_3$) δ: 7.52 (d, *J* = 8.4 Hz, 2H), 7.26 (d, *J* = 8.4 Hz, 2H), 6.72 (d, *J* = 9.2 Hz, 2H), 6.22 (s, 1H), 5.02 (s, 2H), 2.96 - 2.94 (m, 1H), 2.74 - 2.61 (m, 4H), 2.31 (s, 3H), 1.64 - 1.60 (m, 2H), 1.39 - 1.34 (m, 2H), 1.20 (d, *J* = 6.8 Hz, 3H), 0.94 (t, *J* = 7.2 Hz, 3H). LC-MS ESI *m/z*; found 442.9 $[M+H]^+$.

Example 83

3-(4-((1-(4-ethylphenyl)-3-methyl-1H-pyrazol-5-yl)methoxy)-3,5-difluorophenyl)-2-methylpropanoic acid **(144)**

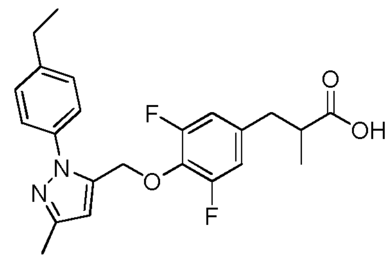

$^1H$ NMR (400 MHz, $CDCl_3$) $\delta$: 7.53 (d, *J* = 8.0 Hz, 2H), 7.27 (d, *J* = 8.8 Hz, 2H), 6.72 (d, *J* = 9.2 Hz, 2H), 6.22 (s, 1H), 5.02 (s, 2H), 2.98 - 2.93 (m, 1H), 2.74 - 2.58 (m, 4H), 2.30 (s, 3H), 1.26 (t, *J* = 8.0 Hz, 3H), 1.18 (d, *J* = 6.8 Hz, 3H). LC-MS ESI *m*/*z*; found 414.6 $[M+H]^+$.

Example 84

3-(3,5-difluoro-4-((3-methyl-1-p-tolyl-1H-pyrazol-5-yl)methoxy)phenyl)-2-methylpropanoic acid **(145)**

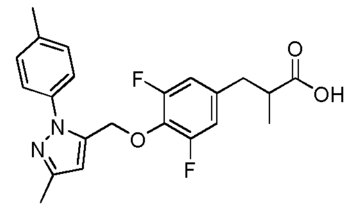

$^1H$ NMR (400 MHz, $CDCl_3$) $\delta$: 7.52 (d, *J* = 8.4 Hz, 2H), 7.25 (d, *J* = 8.8 Hz, 2H), 6.72 (d, *J* = 9.2 Hz, 2H), 6.23 (s, 1H), 5.00 (s, 2H), 2.99 - 2.94 (m, 1H), 2.73 - 2.70 (m, 1H), 2.64 - 2.59 (m, 1H), 2.40 (s, 3H), 2.30 (s, 3H), 1.19 (d, *J* = 6.8 Hz, 3H). LC-MS ESI *m*/*z*; found 400.5 $[M+H]^+$.

Example 85

3-(3,5-difluoro-4-((1-(3-isopropylphenyl)-3-methyl-1H-pyrazol-5-yl)methoxy)phenyl)-2-methylpropanoic acid **(146)**

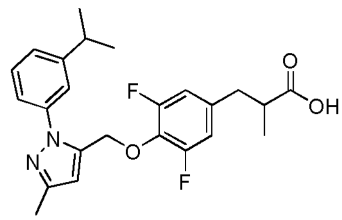

$^1H$ NMR (400 MHz, $CDCl_3$) $\delta$: 7.44 (s, 1H) 7.40 (m, 2H), 7.30 (m, 1H), 6.72 (d, *J* = 8.8 Hz, 2H), 6.29 (s, 1H), 5.03 (s, 2H), 2.98 - 2.93 (m, 2H), 2.74 - 2.72 (m, 1H), 2.64 - 2.59 (m, 1H) 2.36 (s, 3H), 1.26 (d, *J* = 6.8 Hz, 6H), 1.20 (d, *J* = 7.2 Hz, 3H). LC-MS ESI *m*/*z*; found 428.7 $[M+H]^+$.

Example 86

3-(4-((1-(4-chlorophenyl)-3-methyl-1H-pyrazol-5-yl)methoxy)phenyl)-2-methylpropanoic acid **(147)**

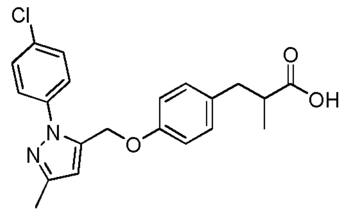

$^1$H NMR (400 MHz, $CDCl_3$) δ: 7.51 (d, *J* = 8.4 Hz, 2H), 7.38 (d, *J* = 8.8 Hz, 2H), 7.11 (d, *J* = 8.8 Hz, 2H), 6.84 (d, *J* = 8.8 Hz, 2H), 6.35 (s, 1H), 4.89 (s, 2H), 3.04 - 2.99 (m, 1H), 2.75 - 2.62 (m, 2H), 2.43 (s, 3H), 1.18 (d, *J* = 6.8 Hz, 3H). LC-MS ESI *m/z*; found 384.7 $[M+H]^+$.

Example 87

3-(4-((1-(3,4-dimethylphenyl)-3-methyl-1H-pyrazol-5-yl)methoxy)-3,5-difluorophenyl)-2-methylpropanoic acid **(148)**

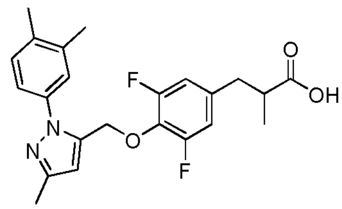

$^1$H NMR (400 MHz, $CDCl_3$) δ: 7.39 (s, 1H) 7.35 (d, *J* = 8.4 Hz, 1H), 7.20 (d, *J* = 8.4 Hz, 1H), 6.72 (d, *J* = 9.2 Hz, 2H), 6.23 (s, 1H), 5.01 (s, 2H), 2.97 - 2.94 (m, 1H), 2.73 - 2.71 (m, 1H), 2.64 - 2.61 (m, 1H) 2.31 (s, 3H), 2.30 (s, 6H), 1.20 (d, *J* = 7.2 Hz, 3H). LC-MS ESI *m/z*; found 413.2 $[M - H]^-$.

Example 88

3-(4-((1-(3-chloro-4-methylphenyl)-3-methyl-1H-pyrazol-5-yl)methoxy)-3,5-difluorophenyl)-2-methylpropanoic acid **(149)**

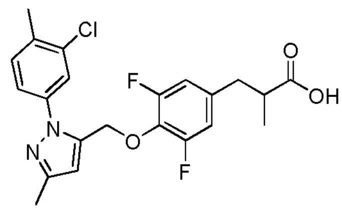

$^1$H NMR (400 MHz, $CDCl_3$) δ: 7.67 (d, *J* = 2.0 Hz, 1H) 7.50 (dd, *J* = 7.6, 2.0 Hz, 1H), 7.30 (d, *J* = 8.0 Hz, 1H), 6.75 (d, *J* = 8.4 Hz, 2H), 6.23 (s, 1H), 5.01 (s, 2H), 3.00 - 2.95 (m, 1H), 2.75 - 2.70 (m, 1H), 2.64 - 2.60 (m, 1H), 2.42 (s, 3H), 2.30 (s, 3H), 1.20 (d, *J* = 7.2 Hz, 3H). LC-MS ESI *m/z*; found 433.1 $[M - H]^-$.

Example 89

3-(4-((1-(3-chlorophenyl)-3-methyl-1H-pyrazol-5-yl)methoxy)-3,5-difluorophenyl)-2-methylpropanoic acid (150)

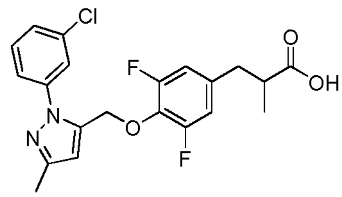

$^1$H NMR (400 MHz, $CDCl_3$) δ: 7.71 (s, 1H) 7.61 (d, *J* = 7.6 Hz, 1H), 7.42 - 7.34 (m, 2H), 6.73 (d, *J* = 8.8 Hz, 2H), 6.25 (s, 1H), 5.03 (s, 2H), 3.00 - 2.95 (m, 1H), 2.74 - 2.70 (m, 1H), 2.65 - 2.60 (m, 1H) 2.31 (s, 3H), 1.20 (d, *J* = 7.2 Hz, 3H). LC-MS ESI *m*/*z*; found 419.2 $[M - H]^-$.

Example 90

3-(4-((1-(4-ethylphenyl)-3-methyl-1H-pyrazol-5-yl)methoxy)-3-fluorophenyl)-2-methylpropanoic acid **(151)**

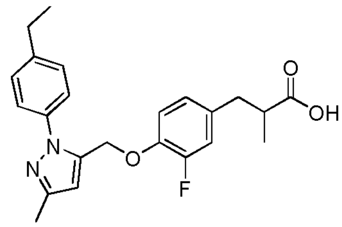

$^1$H NMR (400 MHz, $CDCl_3$) δ: 7.45 (d, *J* = 8.4 Hz, 2H), 7.25 (d, *J* = 8.8 Hz, 2H), 6.92 (m, 1H), 6.84 (m, 2H), 6.31 (s, 1H), 4.97 (s, 2H), 3.04 - 2.95 (m, 1H), 2.75 - 2.59 (m, 4H), 2.33 (s, 3H), 1.25 (t, *J* = 7.6 Hz, 3H), 1.18 (d, *J* = 6.8 Hz, 3H). LC-MS ESI *m*/*z*; found 396.7 $[M+H]^+$.

Example 91

3-(3,5-difluoro-4-((1-phenyl-1H-pyrazol-5-yl)methoxy)phenyl)-2-methylpropanoic acid **(152)**

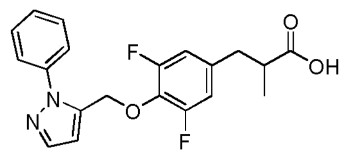

$^1$H NMR (400 MHz, $CDCl_3$) δ: 7.71 - 7.69 (m, 2H) 7.61 (d, *J* = 1.6 Hz, 1H), 7.52 - 7.48 (m, 2H), 7.44 - 7.40 (m, 1H), 6.72 (d, *J* = 9.2 Hz, 2H), 6.42 (d, *J* = 1.6 Hz, 1H), 5.08 (s, 2H), 2.99 - 2.94 (m, 1H), 2.76 - 2.70 (m, 1H), 2.65 - 2.60 (m, 1H), 1.20 (d, *J* = 7.2 Hz, 3H). LC-MS ESI *m*/*z*; found 372.8 $[M+H]^+$.

Example 92

3-(4-((1-(3,5-dimethylphenyl)-3-methyl-1H-pyrazol-5-yl)methoxy)-3,5-difluorophenyl)-2-methylpropanoic acid **(153)**

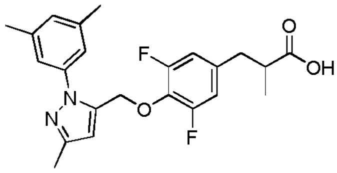

$^1$H NMR (400 MHz, $CDCl_3$) δ: 7.24 (s, 2H) 7.00 (s, 1H), 6.72 (d, *J* = 8.4 Hz, 2H), 6.25 (s, 1H), 5.00 (s, 2H), 2.97 - 2.92 (m, 1H), 2.70 - 2.65 (m, 1H), 2.61 - 2.55 (m, 1 H) 2.34 (s, 6H), 2.31 (s, 3H), 1.15 (d, *J* = 7.2 Hz, 3H). LC-MS ESI *m*/*z*; found 414.5 $[M+H]^+$.

Example 93

3-(4-((1-(4-chlorophenyl)-1H-pyrazol-5-yl)methoxy)-3,5-difluorophenyl)-2-methylpropanoic acid **(154)**

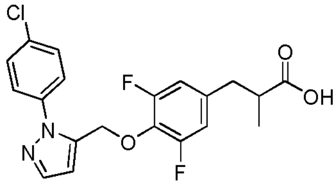

$^1$H NMR (400 MHz, $CDCl_3$) δ: 7.68 (d, *J* = 8.8 Hz, 2H) 7.64 (d, *J* = 1.6 Hz, 1H), 7.47 (d, *J* = 8.8 Hz, 2H), 6.74 (d, *J* = 9.2 Hz, 2H), 6.42 (d, *J* = 1.6 Hz, 1H), 5.08 (s, 2H), 2.99 - 2.94 (m, 1H), 2.75 - 2.70 (m, 1H), 2.65 - 2.60 (m, 1H), 1.20 (d, *J* = 7.2 Hz, 3H). LC-MS ESI *m*/*z*; found 406.8 $[M+H]^+$.

Example 94

3-(4-((1-(4-chloro-3-methylphenyl)-3-methyl-1H-pyrazol-5-yl)methoxy)-3,5-difluorophenyl)-2-methylpropanoic acid **(155)**

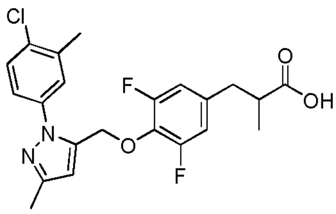

$^1$H NMR (400 MHz, $CDCl_3$) δ: 7.56 (bs, 1H) 7.48 - 7.41 (m, 2H), 6.75 (d, *J* = 8.4 Hz, 2H), 6.26 (s, 1H), 5.00 (s, 2H), 3.00 - 2.95 (m, 1H), 2.75 - 2.70 (m, 1H), 2.66 - 2.63 (m, 1H), 2.42 (s, 3H), 2.31 (s, 3H), 1.20 (d, *J* = 7.2 Hz, 3H). LC-MS ESI *m*/*z*; found 435.9 $[M+H]^+$.

Example 95

3-(4-((1-(4-ethylphenyl)-3-methyl-1H-pyrazol-5-yl)methoxy)-3-methylphenyl)-2-methylpropanoic acid **(156)**

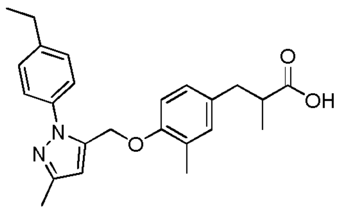

$^1H$ NMR (400 MHz, $CDCl_3$) δ: 7.42 (d, *J*= 8.0 Hz, 2H), 7.25 (d, *J*= 8.0 Hz, 2H), 6.97 (s, 1H), 6.93 (d, *J* = 8.0 Hz, 1H), 6.69 (d, *J* = 8.0 Hz, 1H), 6.33 (s, 1H), 4.92 (s, 2H), 3.00 - 2.95 (m, 1H), 2.75 - 2.56 (m, 4H), 2.37 (s, 3H), 2.15 (s, 3H), 1.24 (t, *J*= 7.6 Hz, 3H), 1.17 (d, *J*= 6.8 Hz, 3H). LC-MS ESI *m*/*z*; found 392.6 $[M+H]^+$.

Example 96

3-(4-((1-(4-ethylphenyl)-3-(trifluoromethyl)-1H-pyrazol-5-yl)methoxy)-3,5-difluorophenyl)-2-methylpropanoic acid (157)

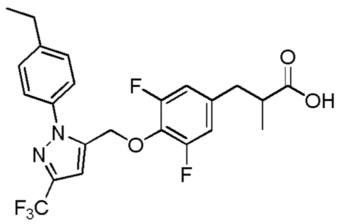

$^1H$ NMR (400 MHz, $CDCl_3$) δ: 7.57 (d, *J*= 8.4 Hz, 2H), 7.33 (d, *J*= 8.0 Hz, 2H), 6.74 (d, *J* = 9.2 Hz, 2H), 6.69 (s, 1H), 5.05 (s, 2H), 2.99 - 2.95 (m, 1H), 2.76 - 2.62 (m, 4H), 1.28 (t, *J* = 8.0 Hz, 3H), 1.20 (d, *J* = 6.8 Hz, 3H). LC-MS ESI *m*/*z*; found 469.1 $[M+H]^+$.

Example 97

3-(4-((1-(4-bromophenyl)-3-methyl-1H-pyrazol-5-yl)methoxy)-3,5-difluorophenyl)-2-methylpropanoic acid (158)

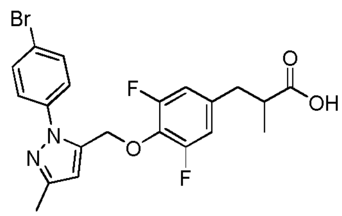

$^1H$ NMR (400 MHz, $CDCl_3$) δ: 7.60 (s, 4H), 6.74 (d, *J*= 9.2 Hz, 2H), 6.25 (s, 1H), 5.00 (s, 2H), 2.99 - 2.94 (m, 1H), 2.75 - 2.71 (m, 1H), 2.66 - 2.61 (m, 1H), 2.30 (s, 3H), 1.20 (d, *J*= 6.8 Hz, 3H). LC-MS ESI *m*/*z*; found 465.0 $[M+H]^+$.

Example 98

(R)-3-(4-((1-(4-ethylphenyl)-3-methyl-1H-pyrazol-5-yl)methoxy)-3,5-difluorophenyl)-2-methylpropanoic acid **(159)**

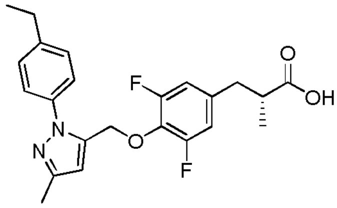

$^1$H NMR (400 MHz, $CDCl_3$) δ: 7.54 (d, J= 8.0 Hz, 2H), 7.28 (d, *J*= 8.8 Hz, 2H), 6.72 (d, *J* = 9.2 Hz, 2H), 6.22 (s, 1H), 5.01 (s, 2H), 2.99 - 2.93 (m, 1H), 2.74 - 2.58 (m, 4H), 2.30 (s, 3H), 1.26 (t, *J*= 8.0 Hz, 3H), 1.18 (d, *J*= 6.8 Hz, 3H). LC-MS ESI *m*/*z*; found 414.6 $[M+H]^+$.

Example 99

(S)-3-(4-((1-(4-ethylphenyl)-3-methyl-1H-pyrazol-5-yl)methoxy)-3,5-difluorophenyl)-2-methylpropanoic acid **(160)**

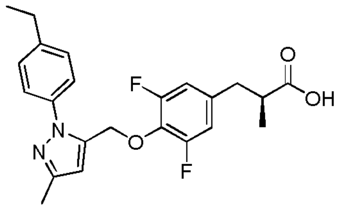

$^1$H NMR (400 MHz, $CDCl_3$) δ: 7.54 (d, J= 8.0 Hz, 2H), 7.28 (d, J= 8.8 Hz, 2H), 6.72 (d, *J* = 9.2 Hz, 2H), 6.22 (s, 1H), 5.01 (s, 2H), 2.99 - 2.93 (m, 1H), 2.74 - 2.58 (m, 4H), 2.30 (s, 3H), 1.26 (t, *J*= 8.0 Hz, 3H), 1.18 (d, *J*= 6.8 Hz, 3H). LC-MS ESI *m*/*z*; found 414.6 $[M+H]^+$.

Example 100

3-(4-((1-(4-ethylphenyl)-1H-pyrazol-5-yl)methoxy)-3,5-difluorophenyl)-2-methylpropanoic acid **(161)**

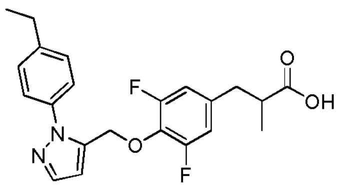

$^1$H NMR (400 MHz, $CDCl_3$) δ: 769 (d, *J*= 1.5 Hz, 1H) 7.54 (d, *J*= 8.3 Hz, 2H), 7.32 (d, *J* = 8.2 Hz, 2H), 6.72 (d, *J* = 8.8 Hz, 2H), 6.44 (d, *J* = 1.7 Hz, 1H), 5.06 (s, 2H), 2.96 (dd, *J*= 13.5, 7.3 Hz 1H), 2.75 - 2.69 (m, 3H), 2.62 (dd, *J*= 13.5, 7.2 Hz 1H), 1.28 (t, *J*= 7.6 Hz, 3H), 1.20 (d, *J*= 6.9 Hz, 3H). LC-MS ESI *m*/*z*; found 400.7 $[M+H]^+$.

Example 101

3-(4-((1-(4-chlorophenyl)-3-ethyl-1H-pyrazol-5-yl)methoxy)-3,5-difluorophenyl)-2-methylpropanoic acid **(162)**

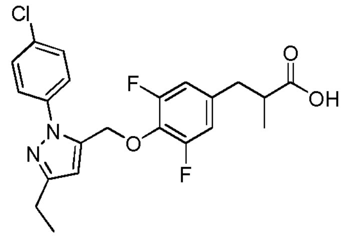

$^1$H NMR (400 MHz, $CDCl_3$) δ 7.65 (d, *J*= 8.6 Hz, 2H), 7.43 (d, *J* = 8.7 Hz, 2H), 6.80 - 6.65 (m, 2H), 6.27 (s, 1H), 5.00 (s, 2H), 3.04 - 2.83 (m, 1H), 2.83 - 2.55 (m, 4H), 1.31 - 1.10 (m, 6H). LC-MS ESI *m/z*; found 435.5 [M+H]$^+$.

Example 102

3-(4-((1-(4-chlorophenyl)-3-methyl-1H-pyrazol-5-yl)methoxy)-3,5-difluorophenyl)-2-ethoxypropanoic acid **(163)**

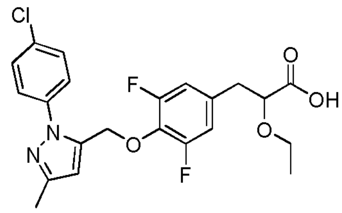

$^1$H NMR (400 MHz, $CDCl_3$) δ 7.65 (d, *J*= 8.4 Hz, 2H), 7.44 (d, *J*= 8.4 Hz, 2H), 6.80 (d, *J*= 8.6 Hz, 2H), 6.23 (s, 1H), 5.00 (s, 2H), 4.06 (m, 1H), 3.65 - 3.62 (m, 1H), 3.51 - 3.49 (m, 1H), 3.12 - 2.87 (m, 2H), 2.30 (s, 3H), 1.21 (t, *J*= 7.0 Hz, 3H). LC-MS ESI *m/z*; found 450.8 [M+H]$^+$.

Example 103

3-(4-((1-(4-Chlorophenyl)-3-methyl-1*H*-pyrazol-5-yl)methoxy)-2-methoxyphenyl)-2-methylpropanoic acid **(164)**

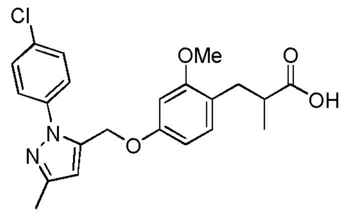

$^1$H NMR (400 MHz, $CDCl_3$) δ: 7.51 (d, *J*= 8.6 Hz, 2H), 7.39 (d, *J*= 8.6 Hz, 2H), 7.02 (d, *J* = 8.0 Hz, 1H), 6.43 (d, *J* = 2.4 Hz, 1H), 6.39 (dd, *J* = 2.4, 8.0 Hz, 1H), 6.35 (s, 1H), 4.90 (s, 2H), 3.76 (s, 3H), 2.99 - 2.94 (m, 1H), 2.84 - 2.79 (m, 1H), 2.67 - 2.62 (m, 1H), 2.34 (s, 3H), 1.15 (d, *J*= 7.2 Hz, 3H).

Example 104

3-(4-((1-(4-Cyclohexylphenyl)-3- methyl-1*H*-pyrazol-5- yl)methoxy)-3,5-difluorophenyl)-2-methylpropanoic acid **(165)**

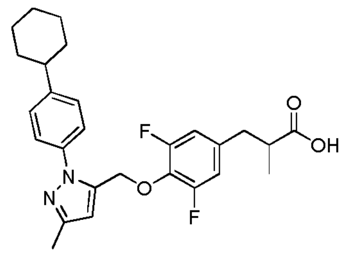

$^1H$ NMR (400 MHz, $CDCl_3$) δ $^1H$ NMR (400 MHz, $CDCl_3$) δ: 7.54 (d, *J*= 8.1 Hz, 2H), 7.26 (d, *J* = 8.1 Hz, 2H), 6.72 (d, *J* = 8.3 Hz, 2H), 6.22 (s, 1H), 5.02 (s, 2H), 2.99-2.94 (m, 1H), 2.74 - 2.71 (m, 1H), 2.61 - 2.54 (m, 2H), 2.30 (s, 3H), 1.90 - 1.74 (m, 5H), 1.45 - 1.27 (m, 5H), 1.20 (d, *J*= 6.9 Hz, 3H). LC-MS ESI *m/z*; found 469.1 $[M+H]^+$.

Example 105

3-(3,5-Difluoro-4-((3-methyl-1-(4-propylphenyl)-1*H*-pyrazol-5-yl)methoxy)phenyl)-2-methylpropanoic acid **(166)**

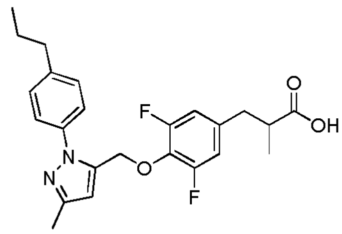

$^1H$ NMR (400 MHz, $CDCl_3$) δ: 7.53 (d, *J*= 8.0 Hz, 2H), 7.26 *(d, J*= 8.0 Hz, 2H), 6.72 (d, *J* = 9.2 Hz, 2H), 6.23 (s, 1H), 5.02 (s, 2H), 2.99 - 2.94 (m, 1H), 2.75 - 2.70 (m, 1H), 2.66 - 2.59 (m, 3H), 2.31 (s, 3H), 1.70 - 1.64 (m, 2H), 1.20 (d, *J* = 7.2 Hz, 3H), 0.95 (t, *J* = 7.2 Hz, 3H).

Example 106

2-(5-((1-(4-Chlorophenyl)-3-methyl-1*H*-pyrazol-5-yl)methoxy)-6-fluoro-2,3-dihydro-1*H*-inden-1-yl)acetic acid **(167)**

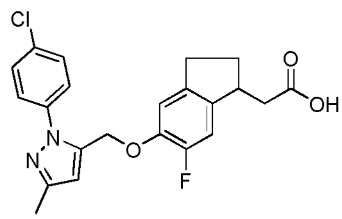

$^1H$ NMR (400 MHz, $CDCl_3$) δ: 7.56 (d, *J*= 8.8 Hz, 2H), 7.41 (d, *J*= 8.8 Hz, 2H), 6.95 (d, *J* = 10.8 Hz, 1H), 6.81 (d, *J* = 8.0 Hz, 1H), 6.34 (s, 1H), 4.94 (s, 2H), 3.57 - 3.53 (m, 1H), 2.87 - 2.80 (m, 1H), 2.79 - 2.72 (m, 2H), 2.53 - 2.42 (m, 2H), 2.34 (s, 3H), 1.82-1.77 (m, 1H).

Example 107

2-(4-((1-(4-Chlorophenyl)-3-methyl-1*H*-pyrazol-5-yl)methoxy)-3,5-difluorophenyl)cyclopent-1-enecarboxylic acid **(168)**

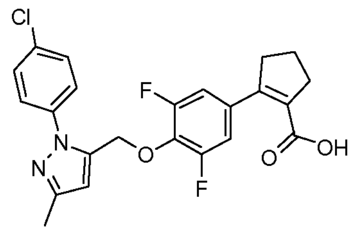

$^1H$ NMR (400 MHz, $CDCl_3$) δ: 7.63 (d, 2H), 7.43 (d, 2H), 6.71 (d, 2H), 6.29 (s, 1H), 4.98 (s, 2H), 4.05 - 3.03 (m, 1H), 2.67 - 2.49 (m, 4H), 2.31 (s, 3H), 1.88 - 1.83 (m, 1H), LC-MS ESI *m*/*z*; found 445.1 $[M+H]^+$.

Example 108

3-(4-((1-(4-Chlorophenyl)-3-methyl-1*H*-pyrazol-5-yl)methoxy)-3-fluorophenyl)-2-methylpropanoic acid **(169)**

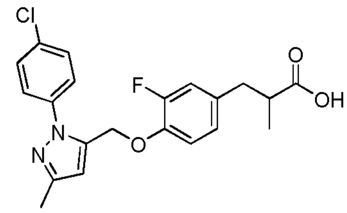

$^1H$ NMR (400 MHz, $CDCl_3$) δ: 10.32 (br, 1H), 7.54 (d, 2H), 7.40 (d, 2H), 6.92 (d, 1H), 6.84 (m, 2H), 6.34 (s, 1H), 4.94 (s, 2H), 3.00 - 2.95 (m, 1H), 2.73 - 2.59 (m, 2H), 2.34 (s, 3H), 1.17 (d, 3H), LC-MS ESI *m*/*z*; found 403 $[M+H]^+$.

Example 109

3-(4-((1-(4-Chlorophenyl)-3-methyl-1*H*-pyrazol-5-yl)methoxy)-3-methylphenyl)-2-methylpropanoic acid **(170)**

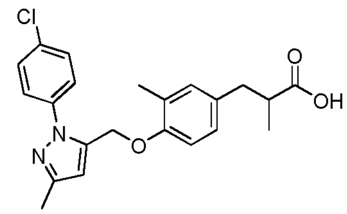

$^1H$ NMR (400 MHz, $CDCl_3$) δ: 9.42 (br, 1H), 7.50 (d, 2H), 7.38 (d, 2H), 6.95 (m, 2H), 6.71 (d, 1H), 6.34 (s, 1H), 4.90 (s, 2H), 3.00 - 2.95 (m, 1H), 2.73 - 2.67 (m, 1H), 2.60 - 2.55 (m, 1H), 2.35 (s, 3H), 2.13 (s, 3H), 1.55 (d, 3H), LC-MS ESI *m*/*z*; found 399 $[M+H]^+$.

Example 110

3-(4-((1-(5-Chloropyrimidin-2-yl)-3-methyl-1H-pyrazol-5-yl)methoxy)-3,5-difluorophenyl)-2-methylpropanoic acid **(171)**

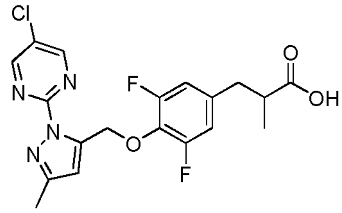

$^1$H NMR (400 MHz, $CDCl_3$) δ: 8.70 (s, 1H), 7.26 (s, 1H), 6.72 (d, 2H), 6.46 (s, 1H), 5.22 (s, 2H), 2.97 - 2.92 (m, 1H), 2.74 - 2.70 (m, 1H), 2.69 (s, 3H), 2.63 - 2.58 (m, 1H), 1.19 (d, 3H), LC-MS ESI *m/z*; found 423.1 $[M+H]^+$.

Example 111

3-(4-((1-(4-Chlorophenyl)-3-methyl-1*H*-pyrazol-5-yl)methoxy)benzylidene)cyclobutanecarboxylic acid **(172)**

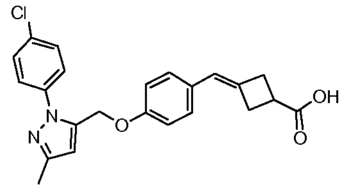

$^1$H NMR (400 MHz, $CDCl_3$) δ: 7.51 (d, 2H), 7.40 - 7.36 (m, 3H), 7.13 (d, 2H), 6.87 (d, 2H), 6.35 (s, 1H), 4.91 (s, 2H), 3.34 - 3.15 (m, 5H), 2.35 (s, 3H), LC-MS ESI *m/z*; found 409 $[M+H]^+$.

Example 112

3-(4-((1-(4-Chlorophenyl)-3-methyl-1*H*-pyrazol-5-yl)methoxy)-3-methoxyphenyl)-2-methylpropanoic acid **(173)**

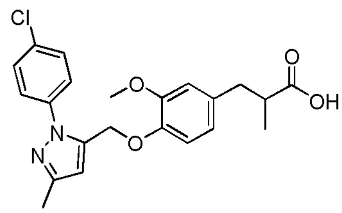

$^1$H NMR (400 MHz, $CDCl_3$) δ: 7.58 (d, 2H), 7.39 (d, 2H), 6.76 (d, 1H), 6.72 (s, 1H), 6.67 (d, 1H), 6.30 (s, 1H), 4.92 (s, 2H), 3.79 (s, 3H), 3.03 - 2.98 (m, 1H), 2.76 - 2.71 (m, 1H), 2.65 - 2.60 (m, 1H), 2.32 (s, 3H), 1.17 (d, 3H), LC-MS ESI *m/z*; found 415 $[M+H]^+$.

Example 113

3-(3,5-Dichloro-4-((1-(4-chlorophenyl)-3-methyl-1*H*-pyrazol-5-yl)methoxy)phenyl)-2-methylpropanoic acid **(174)**

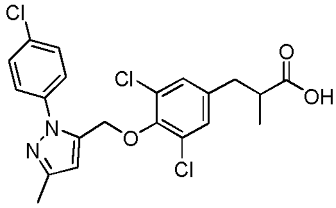

$^1H$ NMR (400 MHz, $CDCl_3$) δ: 7.70 (d, 2H), 7.45 (d, 2H), 7.14 (s, 2H), 6.31 (s, 1H), 4.94 (s, 2H), 3.00 - 2.95 (m, 1H), 2.76 - 2.71 (m, 1H), 2.64 - 2.59 (m, 1H), 2.34 (s, 3H), 1.21 (d, 3H), LC-MS ESI *m/z*; found 453.2 $[M+H]^+$.

Example 114

3-(3,5- Difluoro-4-((1-methyl-5-phenoxy-3-(trifluoromethyl)-1*H*-pyrazol-4-yl)methoxy)phenyl)-2-methylpropanoic acid **(175)**

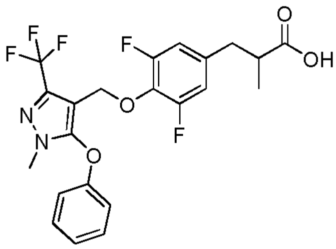

$^1H$ NMR (400 MHz, $CDCl_3$) δ: 7.34 - 7.30 (m, 2H), 7.15 (m, 1H), 6.95 (d, 2H), 6.64 (d, 2H), 4.84 (s, 2H), 3.70 (s, 3H), 2.96 - 2.90 (m, 1H), 2.71 - 2.66 (m, 1H), 2.58-2.54 (m, 1H), 1.16 (d, 3H). LC-MS ESI *m/z*; found 469.3 $[M - H]^-$.

Example 115

3-(4-((5-(4-Chlorophenoxy)-1-methyl-3-(trifluoromethyl)-1*H*-pyrazol-4-yl)methoxy)-3,5-difluorophenyl)-2-methylpropanoic Acid **(176)**

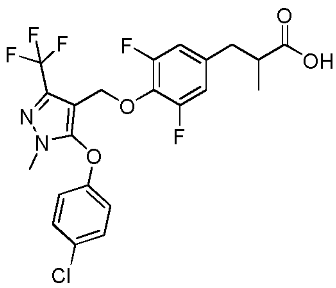

$^1H$ NMR (400 MHz, $CDCl_3$) δ: 7.27 (d, 2H), 6.92 (d, 2H), 6.64 (d, 2H), 4.84 (s, 2H), 3.70 (s, 3H), 2.96 - 2.91 (m, 1H), 2.71 - 2.66 (m, 1H), 2.59 - 2.54 (m, 1H), 1.16 (d, 3H). LC-MS ESI *m/z*; found 503.0 $[M - H]^-$.

Example 116

3-(3,5-Difluoro-4-((5-methyl-2-phenylfuran-3-yl)methoxy)phenyl)-2-methylpropanoic acid **(177)**

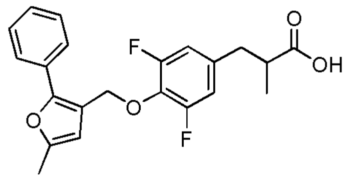

$^1H$ NMR (400 MHz, $CDCl_3$) δ: 7.68 (d, 2H), 7.44 (m, 2H), 7.30 (m, 1H), 6.73 (d, 2H), 6.16 (s, 1H), 5.08 (s, 2H), 3.00 - 2.95 (m, 1H), 2.74 - 2.70 (m, 1H), 2.64 - 2.58 (m, 1H), 2.33 (s, 3H), 1.19 (d, 3H). LC-MS ESI *m/z*; found 385.0 $[M - H]^-$.

Example 117

3-(4-((1-(4-chlorophenyl)-3-(trifluoromethyl)-1H-pyrazol-5-yl)methoxy)-3,5-difluorophenyl)-2-methylpropanoic acid **(178)**

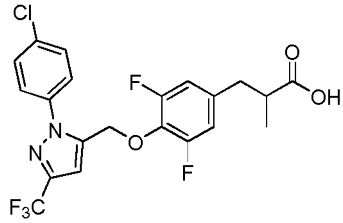

$^1H$ NMR (400 MHz, $CDCl_3$) δ 7.72 - 7.64 (m, 2H), 7.54 - 7.46 (m, 2H), 6.82-6.68 (m, 3H), 5.04 (s, 2H), 2.98 (dd, *J*= 13.6, 7.1 Hz, 1H), 2.79 - 2.59 (m, 2H), 1.22 (d, *J*= 6.9 Hz, 3H).

Example 118

3-(4-((1-(4-chlorophenyl)-3-methyl-1H-pyrazol-5-yl)methoxy)-2-ethylphenyl)propanoic acid **(179)**

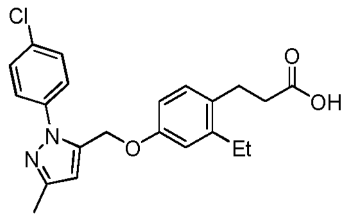

$^1H$ NMR (400 MHz, $CDCl_3$) δ 7.51 (d, *J*= 8 Hz, 2H), 7.38 (d, *J*= 8 Hz, 2H), 7.08 (d, *J*= 8.1 Hz, 1H), 6.72 (m, 2H), 6.34 (s, 1H), 4.89 (s, 2H), 2.92 (m, 2H), 2.62 (m, 4H), 2.34 (s, 3H), 1.21 (t, 3H).

Example 119

2-(5-((1-(4-chlorophenyl)-3-(trifluoromethyl)-1H-pyrazol-5-yl)methoxy)-2,3-dihydro-1H-inden-1-yl)acetic acid **(180)**

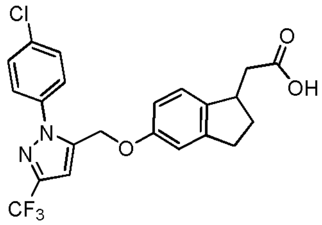

$^1H$ NMR (400 MHz, $CDCl_3$) δ 7.56 (d, *J*= 8.7 Hz, 2H), 7.45 (d, *J*= 8.8 Hz, 2H), 7.13 (d, *J* = 8.3 Hz , 1H), 6.81 (s, 1H), 6.77 (s, 1H), 6.72 (dd, *J* = 8.4 Hz , 2.2 Hz, 1H), 4.94 (s, 2H), 3.62 - 3.49 (m, 1H), 3.02 - 2.74 (m, 3H), 2.58 - 2.36 (m, 2H), 1.86 - 1.75 (m, 1H). LC-MS ESI *m/z*; found 451.1 $[M - H]^-$.

Example 120

2-(5-((1-(2,3-dihydro-1H-inden-5-yl)-3-methyl-1H-pyrazol-5-yl)methoxy)-2,3-dihydro-1H-inden-1-yl)acetic acid **(181)**

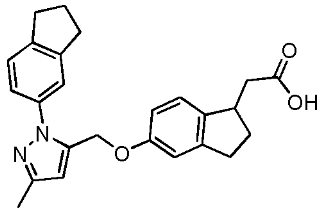

$^1H$ NMR (400 MHz, $CDCl_3$) δ 7.38 (s, 1H), 7.28-7.20 (m, 2H), 7.10 (d, *J*= 8.1 Hz, 1H), 6.78 (s, 1H), 6.74 (d, *J* = 8.4 Hz, 1H), 6.32 (s, 1H), 4.91 (s, 2H), 3.61 - 3.44 (m, 1H), 2.99 - 2.72 (m, 7H), 2.55 - 2.37 (m, 2H), 2.34 (s, 3H), 2.14 - 2.07 (m, 2H), 1.84 - 1.71 (m, 1H). LC-MS ESI *m/z*; found 402.7 $[M - H]^-$.

Example 121

2-(5-((1-(4-ethylphenyl)-3-methyl-1H-pyrazol-5-yl)methoxy)-2,3-dihydro-1H-inden-1-yl)acetic acid **(182)**

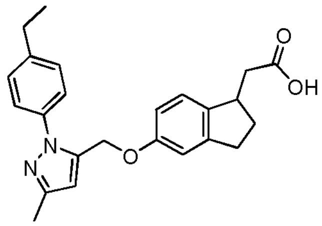

$^1H$ NMR (400 MHz, $CDCl_3$) δ 7.45 (d, *J* = 8.3 Hz, 2H), 7.24 (d, *J* = 8.4 Hz, 2H), 7.11 (d, *J* = 8.4 Hz, 1H), 6.79 (s, 2H), 6.33 (s, 1H), 4.91 (s, 2H), 3.60 - 3.49 (m, 1H), 2.77 (s, 3H), 2.73 - 2.62 (m, 2H), 2.53 - 2.39 (m, 2H), 2.35 (s, 3H), 1.84 - 1.73 (m, 1H), 1.24 (t, *J*= 7.6 Hz, 3H). LC-MS ESI *m/z*; found 390.7 $[M + H]^+$.

Example 122

3-(3,5-difluoro-4-((3-methyl-1-phenyl-1H-pyrazol-5-yl)methoxy)phenyl)-2-methylpropanoic acid **(183)**

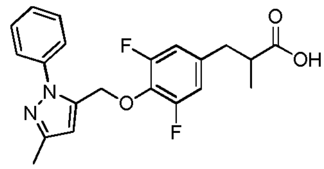

$^1H$ NMR (400 MHz, $CDCl_3$) δ 7.65 (d, *J* = 7.8 Hz, 2H), 7.47 (t, *J* = 7.7 Hz, 2H), 7.38 (t, *J* = 7.3 Hz, 1H), 6.78 - 6.67 (m, 2H), 6.25 (s, 1H), 5.03 (s, 2H), 2.96 (dd, *J* = 13.6 Hz , 7.0 Hz, 1H), 2.72 (dd, *J* = 14.1 Hz, 7.0 Hz , 1H), 2.61 (dd, *J* = 13.6 Hz, 7.3 Hz, 1H), 2.32 (s, 3H), 1.19 (d, *J*= 6.9 Hz , 3H). LC-MS ESI *m*/*z*; found 386.5 $[M + H]^+$.

Example 123

2-(6-((1-(4-ethylphenyl)-3-methyl-1H-pyrazol-5-yl)methoxy)-1,2,3,4-tetrahydronaphthalen-1-yl)acetic acid **(184)**

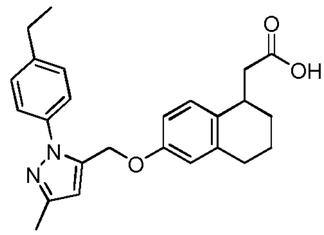

$^1H$ NMR (400 MHz, $CDCl_3$)) δ 7.44 (d, *J* = 8.3 Hz, 2H), 7.24 (d, *J* = 8.3 Hz, 2H), 7.10 (d, *J* = 8.4 Hz, 1H), 6.73 (d, *J* = 8.5 Hz, 1H), 6.63 (s, 1H), 6.33 (s, 1H), 4.90 (s, 2H), 3.40 - 3.23 (m, 1H), 2.79 - 2.49 (m, 6H), 2.35 (s, 3H), 1.99 - 1.87 (m, 1H), 1.88 - 1.66 (m, 4H), 1.24 (t, *J* = 7.6 Hz, 3H).

Example 124

3-(3,5-difluoro-4-((3-methyl-1-(pyridin-2-yl)-1H-pyrazol-5-yl)methoxy)phenyl)-2-methylpropanoic acid **(185)**

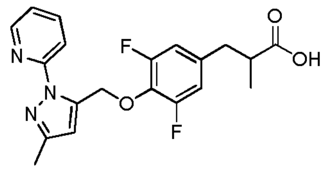

$^1H$ NMR (400 MHz, $CDCl_3$) δ 8.45 (d, *J*= 4.7 Hz, 1H), 7.78 (dd, *J*= 15.2 Hz, 7.5 Hz, 2H), 7.25 - 7.19 (m, 1H), 6.76 - 6.65 (m, 2H), 6.35 (s, 1H), 5.20 (s, 2H), 2.96 - 2.85 (m, 1H), 2.78 - 2.61 (m, 2H), 2.60 (s, 3H), 1.19 (d, *J* = 6.9 Hz, 3H). LC-MS ESI *m*/*z*; found 387.9 $[M + H]^+$.

Example 125

3-(4-((1-(4-chlorophenyl)-3-methyl-1H-pyrazol-5-yl)methoxy)phenyl)-3-methylbutanoic acid **(186)**

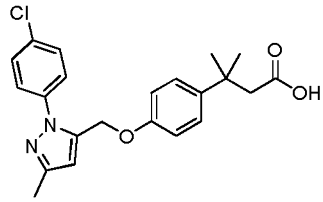

$^1H$ NMR (400 MHz, $CDCl_3$) δ 7.51 (d, $J$= 8.7 Hz, 2H), 7.38 (d, $J$= 8.6 Hz , 2H), 7.30 (d, $J$ = 8.8 Hz , 2H), 6.86 (d, $J$ = 8.7 Hz, 2H), 6.35 (s, 1H), 4.90 (s, 2H), 2.62 (s, 2H), 2.34 (s, 3H), 1.45 (s, 6H). LC-MS ESI $m/z$; found 398.7 $[M + H]^+$.

Example 126

3-(4-((1-(4-ethylphenyl)-3-methyl-1H-pyrazol-5-yl)methoxy)phenyl)-3-methylbutanoic acid **(187)**

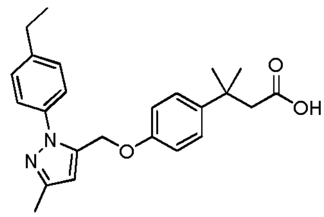

$^1H$ NMR (400 MHz, $CDCl_3$) δ 7.44 (d, $J$= 8.3 Hz , 2H), 7.33 - 7.17 (m, 4H), 6.87 (d, $J$ = 8.7 Hz, 2H), 6.33 (s, 1H), 4.92 (s, 2H), 2.71 - 2.59 (m, 4H), 2.34 (s, 3H), 1.45 (s, 6H), 1.23 (t, $J$=7.6 Hz, 3H). LC-MS ESI $m/z$; found 392.6 $[M + H]^+$.

Example 127

4-(4-((1-(4-chlorophenyl)-3-methyl-1H-pyrazol-5-yl)methoxy)phenyl)butanoic acid **(188)**

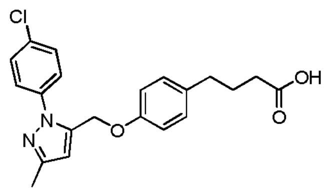

$^1H$ NMR (400 MHz, $CDCl_3$) δ 7.52 (d, $J$= 8.6 Hz, 2H), 7.38 (d, $J$= 8.6 Hz, 2H), 7.11 (d, $J$= 8.3 Hz, 2H), 6.84 (d, $J$= 8.4 Hz, 2H), 6.35 (s, 1H), 4.89 (s, 2H), 2.62 (t, $J$= 7.6 Hz, 2H), 2.42 - 2.31 (m, 5H), 2.01 - 1.86 (m, 2H). LC-MS ESI $m/z$; found 382.8 $[M - H]^-$.

Example 128

4-(4-((1-(4-chlorophenyl)-3-methyl-1H-pyrazol-5-yl)methoxy)-3,5-difluorophenyl)butanoic acid **(189)**

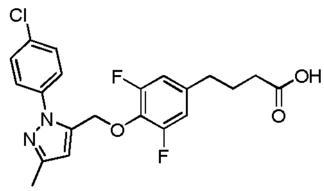

$^1$H NMR (400 MHz, $CDCl_3$) δ 7.65 (d, *J*= 8.6 Hz , 2H), 7.44 (d, *J*= 8.7 Hz , 2H), 6.72 (d, *J*= 8.8 Hz, 2H), 6.25 (s, 1H), 4.99 (s, 2H), 2.60 (t, *J*= 7.6 Hz , 2H), 2.37 (t, J= 7.3 Hz, 2H), 2.31 (s, 3H), 2.00 - 1.86 (m, 2H). LC-MS ESI *m*/*z*; found 419.0 [M - H]$^-$.

Example 129

4-(4-((1-(4-ethylphenyl)-3-methyl-1H-pyrazol-5-yl)methoxy)phenyl)butanoic acid **(190)**

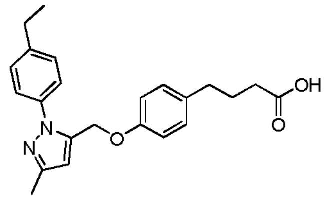

$^1$H NMR (400 MHz, $CDCl_3$) δ 7.44 (d, *J*= 8.1 Hz, 2H), 7.23 (d, *J*= 8.1 Hz , 2H), 7.09 (d, *J* = 8.1 Hz , 2H), 6.84 (d, *J* = 8.3 Hz , 2H), 6.33 (s, 1H), 4.91 (s, 2H), 2.75 - 2.52 (m, 4H), 2.43 - 2.26 (m, 5H), 2.00 - 1.83 (m, 2H), 1.34 - 1.12 (m, 3H). LC-MS ESI *m*/*z*; found 377.3 [M - H]$^-$.

Example 130

4-(4-((1-(4-ethylphenyl)-3-methyl-1H-pyrazol-5-yl)methoxy)-3,5-difluorophenyl)butanoic acid (191)

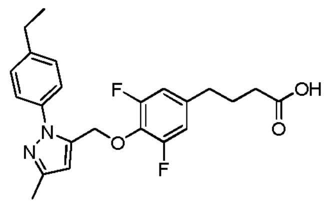

$^1$H NMR (400 MHz, $CDCl_3$) δ 7.55 (d, *J*= 7.8 Hz, 2H), 7.28 (d, *J*= 8.0 Hz, 2H), 6.71 (d, *J* = 8.7 Hz, 2H), 6.24 (s, 1H), 5.01 (s, 2H), 2.69 (q, *J* = 7.5 Hz, 2H), 2.59 (t, *J* = 7.7 Hz, 2H), 2.36 (t, *J*= 7.4 Hz, 2H), 2.31 (s, 3H), 1.8 - 1.84 (m, 2H), 1.26 (t, *J*= 7.5 Hz, 3H). LC-MS ESI *m*/*z*; found 413.3 [M - H]$^-$.

Example 131

4-(4-((1-(4-chlorophenyl)-3-methyl-1H-pyrazol-5-yl)methoxy)phenyl)-2-methylbutanoic acid **(192)**

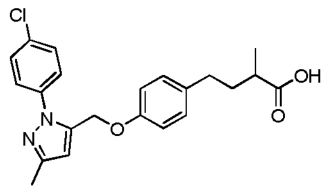

$^1$H NMR (400 MHz, $CDCl_3$) δ 7.51 (d, *J*= 8.0 Hz , 2H), 7.38 (d, *J*= 7.8 Hz, 2H), 7.11 (d, *J* = 8.1 Hz, 2H), 6.84 (d, *J*= 7.6 Hz , 2H), 6.35 (s, 1H), 4.89 (s, 2H), 2.61 (t, *J*= 7.6 Hz, 2H), 2.55 - 2.42 (d, *J* = 6.6 Hz , 1H), 2.33 (s, 3H), 2.09 - 1.93 (m, 1H), 1.78 - 1.60 (m, 1H), 1.22 (d, *J*= 6.8 Hz, 3H). LC-MS ESI *m*/*z*; found 397.4 $[M - H]^-$.

Example 132

4-(4-((1-(4-ethylphenyl)-3-methyl-1H-pyrazol-5-yl)methoxy)phenyl)-2-methylbutanoic acid **(193)**

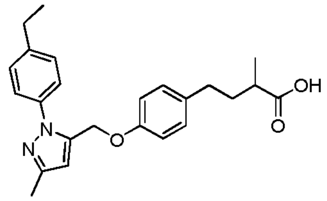

$^1$H NMR (400 MHz, $CDCl_3$) δ 7.43 (d, *J*= 8.2 Hz, 2H), 7.23 (d, *J*= 8.0 Hz , 2H), 7.10 (d, *J*= 8.2 Hz, 2H), 6.84 (d, *J* 8.3 Hz, 2H), 6.32 (s, 1H), 4.90 (s, 2H), 2.74 - 2.55 (m, 4H), 2.55 - 2.41(m, 1H), 2.34 (s, 3H), 2.07 - 1.91 (m, 1H), 1.77 - 1.62 (m, 1H), 1.32 - 1.16 (m, 6H). LC-MS ESI *m*/*z*; found 391.2 $[M - H]^-$.

Example 133

3-(4-((1-(4-chlorophenyl)-3-methyl-1H-pyrazol-5-yl)methoxy)-3-methoxyphenyl)propanoic acid **(194)**

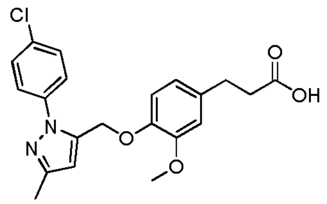

$^1$H NMR (400 MHz, $CDCl_3$) δ 7.58 (d, *J*= 8.7 Hz, 2H), 7.38 (d, *J*= 8.4 Hz, 2H), 6.83 - 6.64 (m, 3H), 6.31 (s, 1H), 4.93 (s, 2H), 3.81 (s, 3H), 2.91 (t, *J*= 7.8 Hz, 2H), 2.67 (t, *J*= 7.5 Hz, 2H), 2.31 (s, 3H). LC-MS ESI *m*/*z*; found 399.0 $[M - H]^-$.

Example 134

3-(4-((1-(4-chlorophenyl)-3-methyl-1H-pyrazol-5-yl)methoxy)-3-fluoro-2-methylphenyl)propanoic acid **(195)**

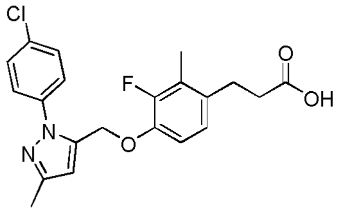

$^1H$ NMR (400 MHz, $CDCl_3$) $\delta$ 7.55 *(d, J*= 8.7 Hz, 2H), 7.40 (d, *J*= 8.7 Hz, 2H), 6.83 (d, *J*= 8.0 Hz, 1H), 6.73 (t, *J*= 8.0 Hz, 1H), 6.33 (s, 1H), 4.94 (s, 2H), 2.90 (t, *J*= 8.0 Hz, 2H), 2.62 (t, *J*= 8.0 Hz, 2H), 2.33 (s, 3H), 2.23 (s, 3H). LC-MS ESI *m/z*; found 402.7 $[M - H]^+$.

Example 135

3-(4-((1-(4-chlorophenyl)-3-methyl-1H-pyrazol-5-yl)methoxy)-2-ethyl-3-fluorophenyl)propanoic acid **(196)**

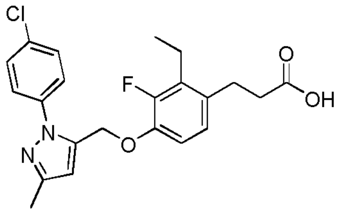

$^1H$ NMR (400 MHz, $CDCl_3$) $\delta$ 7.56 (d, *J*= 8.7 Hz, 2H), 7.40 (d, *J*= 8.7 Hz, 2H), 6.85 (d, *J*= 8.5 Hz, 1H), 6.74 (t, *J*= 8.2 Hz, 1H), 6.33 (s, 1H), 4.94 (s, 2H), 2.93 (t, *J* = 7.8 Hz, 2H), 2.73 - 2.54 (m, 4H), 2.33 (s, 3H), 1.18 (t, *J*= 7.5 Hz, 3H). LC-MS ESI *m/z*; found 416.9 $[M + H]^+$.

Example 136

3-(4-((1-(4-chlorophenyl)-3-methyl-1H-pyrazol-5-yl)methoxy)-2,3-dimethylphenyl)propanoic acid **(197)**

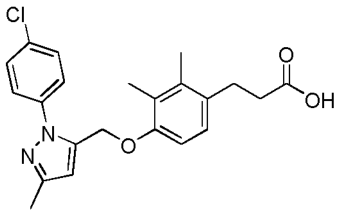

$^1H$ NMR (400 MHz, $CDCl_3$) $\delta$ 7.52 *(d, J*= 8.5 Hz, 2H), 7.38 *(d, J*= 8.7 Hz, 2H), 6.96 (d, *J* = 8.4 Hz, 1H), 6.65 (d, *J* = 8.4 Hz, 1H), 6.33 (s, 1H), 4.904 (s, 2H), 2.94 (t, *J* = 7.8 Hz, 2H), 2.60 (t, *J* = 7.8 Hz, 2H), 2.35 (s, 3H), 2.23 (s, 3H), 2.11 (s, 3H).

Example 137

3-(4-((1-(4-chlorophenyl)-1H-pyrazol-5-yl)methoxy)-3-fluoro-2-methylphenyl)propanoic acid **(198)**

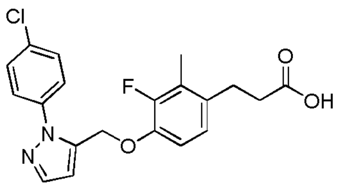

$^1H$ NMR (400 MHz, $CDCl_3$) δ 7.67 (s, 1H), 7.59 (d, *J*= 8.3, 2H), 7.43 (d, *J*= 8.5, 2H), 6.84 (d, *J* = 8.3, 1H), 6.73 (t, *J* = 8.0 Hz, 1H), 6.53 (s, 1H), 4.99 (s, 2H), 2.90 (t, J= 7.5, 2H), 2.62 (t, *J* = 8.3, 2H), 2.23 (s, 3H).

Example 138

3-(4-((1-(4-chlorophenyl)-1H-pyrazol-5-yl)methoxy)-2,3-dimethylphenyl)propanoic acid **(199)**

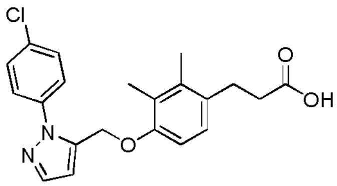

$^1H$ NMR (400 MHz, $CDCl_3$) δ 7.69 (s, 1H), 7.55 (d, *J*= 8.7, 2H), 7.41 (d, *J*= 8.7, 2H), 6.96 (d, *J* = 8.3, 1H), 6.66 (d, *J*= 8.3, 1H), 6.54 (s, 1H), 4.95 (s, 2H), 2.94 (t, *J* = 7.8, 2H), 2.60 (t, *J*= 7.8, 2H), 2.23 (s, 3H), 2.10 (s, 3H).

Example 139

3-(4-((3-(4-chlorophenyl)-5-methylisoxazol-4-yl)methoxy)-2,3-dimethylphenyl) propanoic acid **(200)**

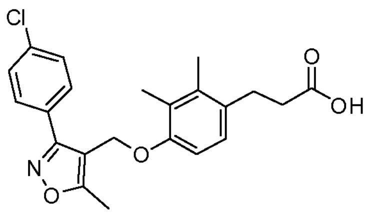

$^1H$ NMR (400 MHz, $CDCl_3$) δ: 7.69 (d, 2H), 7.40 (d, 2H), 6.99 (d, 1H), 6.71 (d, 1H), 4.79 (s, 2H), 2.97 - 2.94 (m, 2H), 2.64 - 2.60 (m, 2H), 2.45 (s, 3H), 2.23 (s, 3H), 2.09 (s, 3H). LC-MS ESI *m/z*; found 398.3.0 [M - H]$^-$.

Example 140

3-(4-((1-(4-ethylphenyl)-3-methyl-1H-pyrazol-5-yl)methoxy)-2,3-dimethylphenyl)propanoic acid **(201)**

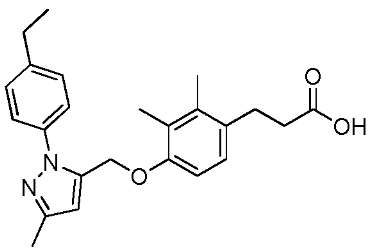

$^1$H NMR (400 MHz, $CDCl_3$) δ 7.44 (d, *J*= 8.1 Hz, 2H), 7.23 (d, *J*= 8.1 Hz, 2H), 6.94 (d, *J*= 8.5 Hz, 1H), 6.64 (d, *J*= 8.4 Hz, 1H), 6.31 (s, 1H), 4.91 (s, 2H), 2.93 (t, *J* = 7.9 Hz, 2H), 2.67 (q, *J*= 7.7 Hz, 2H), 2.59 (t, *J*= 7.8 Hz, 2H), 2.35 (s, 3H), 2.22 (s, 3H), 2.13 (s, 3H), 1.24 (t, *J*= 7.6 Hz, 3H). LC-MS ESI *m*/*z*; found 392.7 $[M + H]^+$.

Example 141

3-(4-((1-(4-ethylphenyl)-1H-pyrazol-5-yl)methoxy)-2,3-dimethylphenyl)propanoicacid **(202)**

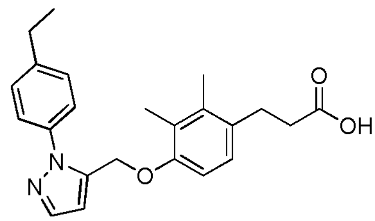

$^1$H NMR (400 MHz, $CDCl_3$) δ 7.67 (s, 1H), 7.47 (d, *J*= 8.0 Hz, 2H), 7.26 (d, *J*= 8.0 Hz, 2H), 6.95 (d, *J*= 8.2 Hz, 1H), 6.64 (d, *J*= 8.4 Hz, 1H), 6.52 (s, 1H), 4.96 (s, 2H), 2.93 (t, *J*= 7.9 Hz, 2H), 2.69 (q, *J*= 7.7 Hz, 2H), 2.59 (t, *J*= 7.8 Hz, 2H), 2.22 (s, 3H), 2.12 (s, 3H), 1.25 (t, *J*= 7.6 Hz, 3H). LC-MS ESI *m*/*z*; found 379.2 $[M + H]^+$.

Example 142

3-(4-((1-(4-chlorophenyl)-3-methyl-1H-pyrazol-5-yl)methoxy)-5,6,7,8-tetrahydro naphthalen-1-yl)propanoic acid **(203)**

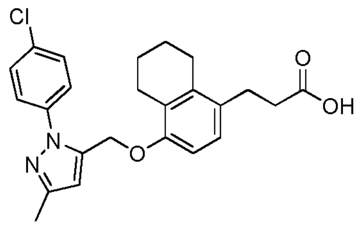

$^1$H NMR (400 MHz, $CDCl_3$) δ $^1$H NMR (400 MHz, $CDCl_3$) δ 7.45 - 7.33 (m, 4H), 6.94 (d, *J*= 8.5 Hz, 1H), 6.60 (d, *J*= 8.3 Hz, 1H), 6.35 (s, 1H), 4.89 (s, 2H), 2.88 (m, 2H), 2.73 - 2.51 (m, 6H), 2.37 (s, 3H), 1.75 (m, 4H). LC-MS ESI *m*/*z*; found 423.5 $[M - H]^-$.

Example 143

3-(7-((1-(4-chlorophenyl)-3-methyl-1H-pyrazol-5-yl)methoxy)-2,3-dihydro-1H-inden-4-yl)propanoic acid **(207)**

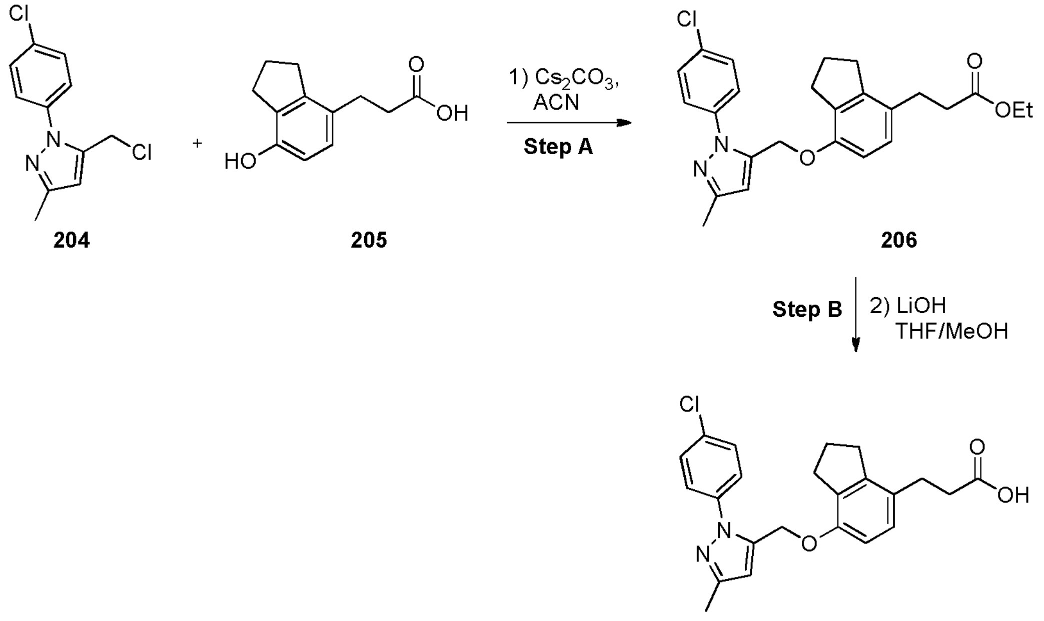


204 205 206 207

Step A: To a solution of intermediate **(204)** (0.100 g, 0.415 mmol) in acetonitrile (4 mL) is added intermediate **(205)** (0.085 g, 0.415 mmol) and cesium carbonate (0.162 g, 0.498). The resulting suspension is stirred at 50 °C for 4 h. The reaction is cooled to rt, diluted with ethyl acetate and filtered through a pad of celite. The filtrate is concentrated in vacuo and the residual is purified by silica gel chromatography (0-20 % EtOAc in hexanes) to yield the intermediate **(206).**

**Step B:** To a solution of intermediate **(206)** (0.182 g, 0.415 mmol) in THF (1 mL) and methanol (1 mL) is added a solution of lithium hydroxide (1.0 M, 1.0 mL). The reaction is stirred at room temperature for 4 h. The mixture is acidified with 1M HCl and the white solid is collected by filtration to afford the desired acid **(207).**

## BIOLOGICAL EXAMPLES

Biological Example 1

GPR120 stable cell line

Human GPR120 stable cell line was purchased from Multispan Inc (26219 Eden Landing Road, Hayward, CA94545). This GPR120 cell line was generated in HEK293 cells coexpressing Gqi5. In this cell line, the Flag epitope tag (DYKDDDDK) was fused to the amino terminus of human GPR120 protein.

Assay

The concentration of intracellular $Ca^{2+}$ was measured as described below. Human GPR120 cells were plated in 96 well plates (Poly-D-Lysine coated black/clear Plate, Greiner Bio-One) at 70,000 cells per well and cultured overnight in conditions of 37°C and 5% $CO_2$. A $Ca^{2+}$ assay dye stock solution was prepared by adding 10 mL of assay buffer (HBSS/20mM HEPES, pH 7.4) to FLIPR Calcium 4 Assay Bulk Kit (Molecular Devices). The 10 mL of $Ca^{2+}$ assay dye solution was prepared by further diluting 0.5 mL of $Ca^{2+}$ assay dye stock solution with 10 mL of assay buffer. The medium of the cells was removed and immediately 100 μl of the $Ca^{2+}$ assay dye solution was dispensed into each well followed by incubation at 37°C and 5% $CO_2$ for 50 minutes to incorporate the $Ca^{2+}$ dye into the cells. The cell plate was then placed in the Flexstation (Molecular Devices) for 20 minutes at 37°C. Compounds were dissolved in 100% DMSO and diluted to desired concentrations with assay buffer and placed in Flexstation simultaneously with the cell plate for 20 minute incubation at 37°C prior to reading. Fluorescence intensity was measured immediately following compound addition (25 μl/well) on the Flexstation at an excitation wavelength of 485 nm and an emission wavelength of 525 with 515 nm auto cutoff. The resulting increase in fluorescence intensities corresponds to increases in intracellular $Ca^{2+}$ levels.

Determination of activity of compounds

Compounds were dissolved in 100% DMSO to a concentration of 20 mM to provide stock solutions. To determine

activity against human GPR120, compounds were added with human GPR120 stably expressing cells (described above), at 8 desired concentrations ranging from 0.00001 to 20 μM, in 96 well plates and fluorescence intensities were measured for 90 seconds with 2-second intervals. An $EC_{50}$ value (concentration of the GPR120 agonist where 50% of the agonist's maximal activity is observed) was calculated using the changes (Max-Min) of fluorescent intensity.

To determine percent activity for a tested compound, the fluorescence intensity value obtained at a particular concentration were compared to the maximal fluorescence intensity value obtained for reference compound GW9508 (4-[[(3-phenoxyphenyl)methyl]amino]benzene propanoic acid; British Journal of Pharmacology 2006 148, 619-628) that acts as a full agonist of the GPR120 receptor. The maximal activity of GW9508 is designated as 100% activity. Typically, the GW9508 activity reached a maximum at aconcentration of approximately 6.7 μM. Activities of compounds that were tested according to this method are shown in Table 1 below and are expressed as % activity at 5 μM compound compared to the maximal activity of GW9508 at 6.7 μM.

In some embodiments, compounds of Formula (A)-(D) and (I)-(XIV) and pharmaceutically acceptable salts thereof have an $EC_{50}$ against human GPR120 of less than 10μM. In other aspects, the compounds have an an $EC_{50}$ of less than 1 μM.

**Table 1**

| Example No. | %Activity at 5μM | $EC_{50}$ ≤ 1μm | $EC_{50}$ between > 1 μM and ≤10 μM | $EC_{50}$> 10μM |
|---|---|---|---|---|
| 1 | 79 | + | | |
| 2 | 77 | + | | |
| 3 | 70 | | + | |
| 5 | 64 | | + | |
| 10 | 60 | + | | |
| 11 | 75 | | + | |
| 12 | 70 | | + | |
| 13 | 73 | | + | |
| 14 | 66 | + | | |
| 15 | 72 | + | | |
| 16 | 60 | + | | |
| 17 | 78 | | + | |
| 18 | 77 | + | | |
| 19 | 69 | + | | |
| 20 | 88 | + | | |
| 21 | 97 | + | | |
| 22 | 75 | | + | |
| 23 | 63 | | + | |
| 24 | 92 | + | | |
| 25 | 92 | | + | |
| 26 | 100 | + | | |
| 28 | 87 | + | | |
| 29 | 85 | | + | |
| 30 | 80 | | + | |
| 31 | 85 | + | | |
| 32 | 85 | + | | |
| 33 | 80 | + | | |
| 34 | 74 | | + | |

(continued)

| Example No. | %Activity at 5μM | $EC_{50} \leq 1\mu m$ | $EC_{50}$ between > 1 μM and ≤10 μM | $EC_{50}$> 10μM |
|---|---|---|---|---|
| 35 | 86 | + | | |
| 36 | 96 | + | | |
| 37 | 94 | + | | |
| 38 | 100 | | + | |
| 39 | 100 | + | | |
| 40 | 100 | + | | |
| 41 | 50 | + | | |
| 45 | 61 | + | | |
| 46 | 62 | + | | |
| 47 | 71 | + | | |
| 48 | 73 | + | | |
| 49 | 71 | + | | |
| 50 | 85 | + | | |
| 51 | 81 | + | | |
| 52 | 96 | + | | |
| 53 | 84 | + | | |
| 54 | 48 | + | | |
| 55 | 66 | + | | |
| 56 | 71 | + | | |
| 57 | 85 | + | | |
| 58 | 66 | + | | |
| 59 | 100 | + | | |
| 62 | 88 | + | | |
| 63 | 76 | + | | |
| 64 | 71 | + | | |
| 65 | 86 | + | | |
| 66 | 15 | | | + |
| 67 | 59 | | + | |
| 68 | 54 | | + | |
| 69 | 87 | | + | |
| 70 | 62 | | + | |
| 71 | 100 | + | | |
| 72 | 100 | + | | |
| 73 | 72 | + | | |
| 76 | 85 | + | | |
| 77 | 95 | + | | |
| 78 | 92 | + | | |
| 79 | 9 | | | + |

(continued)

| Example No. | %Activity at 5μM | $EC_{50}$ ≤ 1μm | $EC_{50}$ between > 1 μM and ≤10 μM | $EC_{50}$> 10μM |
|---|---|---|---|---|
| 80 | 92 | + | | |
| 81 | 106 | + | | |
| 82 | 68 | + | | |
| 83 | 88 | + | | |
| 84 | 106 | + | | |
| 85 | 95 | + | | |
| 86 | 98 | + | | |
| 87 | 106 | + | | |
| 88 | 107 | + | | |
| 89 | 107 | + | | |
| 90 | 99 | + | | |
| 91 | 64 | + | | |
| 92 | 58 | + | | |
| 93 | 108 | + | | |
| 94 | 108 | + | | |
| 95 | 100 | + | | |
| 96 | 97 | + | | |
| 97 | 109 | + | | |
| 98 | 104 | + | | |
| 99 | 73 | + | | |
| 100 | 109 | + | | |
| 101 | 113 | + | | |
| 102 | 61 | | + | |
| 103 | 78 | | + | |
| 104 | 27 | | | + |
| 105 | 99 | + | | |
| 106 | 88 | + | | |
| 107 | 32 | | + | |
| 108 | 106 | + | | |
| 109 | 114 | + | | |
| 110 | 19 | | | + |
| 111 | 80 | + | | |
| 112 | 83 | | + | |
| 113 | 92 | + | | |
| 114 | 111 | + | | |
| 115 | 118 | + | | |
| 116 | 115 | + | | |
| 117 | 105 | + | | |

(continued)

| Example No. | %Activity at 5μM | $EC_{50} \leq 1\mu m$ | $EC_{50}$ between > 1 μM and ≤10 μM | $EC_{50}$> 10μM |
|---|---|---|---|---|
| 118 | 103 | + | | |
| 119 | 92 | | | |
| 120 | 94 | + | | |
| 121 | 94 | + | | |
| 122 | 91 | + | | |
| 123 | 67 | | | + |
| 124 | 33 | | | + |
| 125 | 47 | | + | |
| 126 | 48 | | + | |
| 127 | 112 | + | | |
| 128 | 112 | + | | |
| 129 | 99 | + | | |
| 130 | 103 | + | | |
| 131 | 68 | | + | |
| 132 | 85 | | + | |
| 133 | 107 | | + | |

Biological Example 2

Glucose uptake in 3T3-L1 adipocytes

3T3-L1 fibroblasts are plated into growth medium (DMEM supplemented with 10% FBS, 1% Penicillin-Streptomycin) and grown to confluence for 7 days, with media changes every 2 to 3 days. Differentiation into adipocytes is induced by incubating the cells in DMEM supplemented with 10% FBS, 1% Penicillin-Streptomycin, 698 nM Bovine Insulin, 518 μM IBMX and 248 nM Dexamethasone. Glucose uptake activity is determined by measuring the uptake of 2-deoxy-D-[$^3$H] glucose. Briefly, 3T3-L1 adipocytes are washed two times with PBS, once with Fat Cell Buffer (FCB: 125mM NaCl, 5mM KCl, 1.8mM $CaCl_2$, 2.6mM $MgSO_4$, 25mM Hepes, 2mM pyruvate and 2% BSA, 0.2 μm sterile filtered) and are incubated with GPR120 agonists in FCB at 37°C for 30 minutes. Insulin is prepared at the indicated concentrations in FCB, added to the cells and incubated for 20 minutes at 37°C. Glucose uptake is initiated by the addition of 2-deoxy-D-[$^3$H] glucose (0.083μCi/mL and 1.1 mM 2-deoxy-D-glucose in FCB) and incubated for 10 minutes at 37°C. Glucose uptake is terminated by removing the contents of the wells and washing the cells three times with cold PBS. The cells are lysed with scintillation solution and 2-deoxy-D-[$^3$H] glucose retained by the cells is counted (MicroBeta TriLux 1450 - Perkin Elmer). Cell viability is assessed independently with the CellTitre-Glo Luminescent Cell Viability Assay Kit (Promega) as per manufacturer's instructions. Glucose uptake is quantified by normalizing the glucose uptake measurement for each compound treatment to the corresponding cell viability value. The fold induction of glucose uptake is calculated by normalizing all values against the average value of the basal value (taken as 1-fold).

Biological Example 3

Insulin Secretion (Islet Perifusion)

To determine the effect of GPR120 agonists on insulin secretion from islets, islets from Sprague Dawley rats are isolated and incuabated in vitro with GPR120 agonists in the presence of low and high glucose. 200-250g Sprague Dawley rats are obtained from Charles River laboratories and maintained on regular chow (Purina 5001). Before the procedure rats are anesthetized with intra peritoneal injection of pentobarbital at 200 mg/kg. The bile duct is clamped where it enters the duodenum, then a catheter is placed in the bile duct between the liver and the pancreas. The pancreas is infused through the catheter with a solution of 0.75mg/mL collagenase P (Roche) in HBSS buffer (Biowhitaker) supplemented with 0.1% glucose and 0.02% BSA. The pancreas is then excised from the rat and placed in 5mL of the

collagenase P solution in a 37°C waterbath for 8 minutes. After 8 minutes the digested pancreas is shaken vigorously by hand for 30 seconds. The resulting digest is washed four times in the HBSS buffer, then applied to a discontinuous ficoll gradient. To make the gradient, the digest is resuspended in 7.5mL of ficoll DL400 solution (Sigma) density 1.108, in a 15mL tube. Three 2mL layers of ficoll solution of decreasing density (1.096, 1.069, 1.037) are then added to the tube to create a density gradient. The gradient is centrifuged at 1500 rpm for 15 minutes after which islets are picked from the top two layers. Islets are washed four times in HBSS buffer, then cultured in RPMI 1640 media (Gibco) supplemented with 1% fetal bovine serum. The following day, 25 size-matched islets are placed in a perifusion chamber and exposed to Krebs Ringer Buffer (KRB;119mM NaCl, 4.7mM KCl, 25mM $NaHCO_3$, 2.5mM $CaCl_2$, 1.2 mM $MgSO_4$, 1.2mM $KH2PO_4$) at a rate of 1mL/minute, using a Cellex Acu-Sys S perifusion culture system. The islets are exposed to KRB containing glucose at 2mM for 30 minutes, followed with buffer containing 16mM glucose for 30 minutes, then returned to 2mM glucose for a further 30 minutes, in the presence of 0.1-100uM of the GPR120 agonist or vehicle (DMSO). Perifusate is collected at 1 minute intervals using a fraction collector, and assayed for insulin using an ELISA kit (Mercodia Ultrasensitive Rat Insulin ELISA Kit, ALPCO). Insulin secretion rate in response to glucose is plotted against time, and the AUC of the curve determined in order to quantify the insulin secretory response to 16mM glucose during the 30 minute perifusion. Statistical significance of differences in AUC between treated and untreated islets are determined by paired Students t test.

Biological Example 4

Oral and Intra-peritoneal Glucose Tolerance

8-10 week old male C57BL/6J mice (Harlan) were maintained on regular chow diet from Harlan (2018 Teklad Global). For the oral glucose tolerance test (OGTT), on the day of the experiment mice were fasted for 6 hours, then randomized into groups (n=10-15) to receive the tested GPR120 agonist at doses ranging from 30 mg/kg to 100mg/kg or the vehicle (1% CMC, 2% TWEEN 80). For the intra-peritoneal glucose tolerance test (IPGTT), the mice were fasted for overnight, then randomized into groups (n=10-15) to receive the tested GPR120 agonist at doses ranging from 30 mg/kg to 100mg/kg or the vehicle (1% CMC, 2% TWEEN 80). Compounds were delivered orally via gavage at 10mL/kg. Blood glucose levels were measured by glucometer (Ascensia Elite XL, Bayer) at time -30 minutes before administration of compound. Blood glucose was measured again after 30 minutes (at time 0), and then the mice were dosed orally with 3g/kg glucose at 10mL/kg or were dosed intrperitoneally with 2g/kg glucose at 10 mL/kg. Blood glucose measurements were taken 20, 40, 60, 90 and 120 minutes after glucose administration, by glucometer (Ascensia Elite XL, Bayer).

Glucose levels were plotted against time, and the incremental area under the curve (AUC) of the glucose excursion was determined from time 0 using Graphpad Prism 5.01. Outliers were excluded using Tukey's box plot outlier test, and statistical significance of differences in AUC of compound treatment compared to vehicle was determined by non-parametric Kruskal-Wallis test with Dunn's post test.

Table 2 below shows the mean percentage inhibition of the glucose excursion for the fifteen animals tested in each group. The compounds were tested at 100 mg/kg and the levels of blood glucose were determined in the presence and absence of the tested compounds. The percentage of glucose or intra-peritoneal reduction is reported as a percent reduction in AUC (area under the curve). The tested compound was selected as examples from the exemplified compounds. These results demonstrate that the GPR120 agonists can lower blood glucose in response to an oral glucose challenge.

**Table 2**

| Compound | dose (mg/kg) | %reduction in IPGTT AUC | % reduction in OGTT AUC |
|---|---|---|---|
| Example 36 | 100 | 24 | |
| Example 36 | 100 | | 45.2 |
| Example 36 | 30 | | 15 |
| Example 43 | 30 | | 20 |
| Example 43 | 100 | | 40 |
| Example 44 | 30 | | 33 |
| Example 44 | 100 | | 39 |

Biological Example 5

Incretin and enteroendocrine hormone measurement

The effect of GPR120 agonists on the secretion of insulin, Glucagon-like peptide-1 (GLP-1), glucose dependent insulinotropic peptide (GIP, Cholecystokinin (CCK) and Peptide YY (PYY) in C57BL/6J mice are determined as follows.
8-10 week old male C57BL/6J mice (Harlan) are maintained on a regular chow diet from Harlan (2018 Teklad Global). On the day of the experiment mice are fasted for 6 hours then randomized into treatment groups (n=15). All groups are treated with the DPPIV inhibitor sitagliptin at 1 mg/kg to prevent degredation of active GLP-1. GPR120 agonist compounds are dosed at concentrations ranging from 3-100mg/kg in 1% CMC, 2% TWEEN 80 either by oral gavage or intraperitoneal injection (i.p.) at -30 minutes. Sitagliptin is administered in the same dosing solution. Oral glucose at 3g/kg is administered at 0 minutes. At 3 minutes after glucose administration, animals are anesthetized with pentobarbital (40mg/mL in 10% ethanol) and at 4 minutes blood collected by heart puncture in microtainer tubes (BD) with potassium EDTA. For Glucose-independent incretin studies the same procedure is used but in the absence of oral glucose administration. Dosing of GPR120 agonist compounds and blood collection are as described above. For the GLP-1 assay, the collection tubes also contain a DPP-IV inhibitor provided in the GLP-1 assay kit.
Insulin is measured using the Mercodia mouse Insulin ELISA Kit (ALPCO) according to the manufacturer's instructions. Bioactive GLP-1 is measured using Glucagon-like peptide-1 (active) ELISA assay kit (Linco) according to the manufacturer's instructions. Total GIP (bioactive plus inactive) is measured using rat/mouse total GIP ELISA assay kit (Linco), according to the manufacturer's instructions. CCK (Nonsulfated Cholecystokinin Octapeptide, 26-33) is measured using human, rat, mouse CCK ELISA assay kit (Phoenix Pharmaceuticals), according to the manufacturer's instructions. PYY is measured using canine, mouse, porcine, rat PYY ELISA assay kit (Peninsula Laboratories), according to the manufacturer's instructions.

Biological Example 6

Gastric emptying

To evaluate the effects of GPR120 agonists on gastric emptying, 8-10 week old male C57BL/6J mice (Harlan) are fasted for 16-18 hours, thentreated orally or byintraperitoneal injection with either GPR120 agonists (1-100 mg/kg) or vehicle (1% CMC, 2% TWEEN 80) 30 minutes prior to initiation of the gastric emptying study. Phenol red (0.05% PR in deionized water) is administered either in an aqueous or glucose solution (0.05% in 20% glucose). Immediately after PR administration (0 min), control group animals are sacrificed by cervical dislocation and the average amount of phenol red recovered is measured as 100% phenol red retention. The remainder of the animals from each group are sacrificed at various time-points following phenol red administration. The stomachs are isolated after clamping at both the pyloric and the cardiac ends. Clamped stomachs are transferred to a 50 mL conical tube containing 5 mL deionized water. Clamps are removed and each stomach is cut into fine pieces with scissors and stomach content is extracted by centrifugation at 3000 rpm for 10 minutes and supernatant is filtered to remove particulates. 1mL of 1N NAOH is added to each 2 mL of filtered supernatant for color development. The concentration of phenol read is determine by measuring the absorbance of the extracted material at a wavelength of 558 nm and then converted to concentration by using the extinction coefficient of phenol red in aqueous solution.
The gastric emptying is calculated by the formula:

% Gastric emptying = ( (A-B) /A ) x 100, where A is the average amount (absorbance) of phenol red recovered immediately after ingestion (the 100% retained group) and B is the amount (absorbance) of phenol red remaining in the stomach at a given time after ingestion.

Biological Example 7

Improvement of diabetes parameters in animal models of diabetes

Female ZDF rats (Charles River laboratories) are obtained at 6 weeks of age and acclimatized for 1 week before being placed on a high fat diet (RD 13004, Research Diets). GPR120 compounds are administered to the rats by daily gavage at concentrations ranging from 0.3-300 mg/kg in 1% CMC, 2% TWEEN 80. Body weight and food intake is monitored daily. After 14 days of dosing, blood samples are taken from overnight fasted animals to measure glucose and insulin. Glucose is measured using a glucometer (Ascensia Elite XL, Bayer) and insulin is measured using rat insulin

ELISA kit (ALPCO). Insulin and glucose levels are compared to those of vehicle treated animals to determine efficacy.

Male high-fat diet-fed mice (Jackson),which has been placed on a high fat Diet D 12492 (Research diets, 60 kcal% fat) at the age of 4-weeks are obtained at 10 weeks of age and acclimatized for 1 week. GPR120 compounds are administered by daily gavage at concentrations ranging from 0.3-300 mg/kg in 1% CMC, 2% TWEEN 80. Body weight and food intake is monitored daily. After 14 days of dosing, blood samples are taken from overnight fasted animals to measure glucose and insulin. Glucose is measured using a glucometer (Ascensia Elite XL, Bayer), insulin is measured using mouse insulin ELISA kit (ALPCO). Insulin and glucose levels are compared to those of vehicle treated animals to determine efficacy.

The ob/ob mice (Jackson) are obtained at 6 weeks of age and acclimatized for 1 - 2 week. GPR120 compounds are administered by daily gavage at concentrations ranging from 0.3-300 mg/kg in 1% CMC, 2% TWEEN 80. Body weight and food intake is monitored daily. After 14 days of dosing, blood samples are taken from overnight fasted animals to measure glucose and insulin. Glucose is measured using a glucometer (Ascensia Elite XL, Bayer), insulin is measured using mouse insulin ELISA kit (ALPCO). Insulin and glucose levels are compared to those of vehicle treated animals to determine efficacy.

All patents, patent applications, publications and presentations referred to herein are incorporated by reference in their entirety. Any conflict between any reference cited herein and the teaching of this specification is to be resolved in favor of the latter. Similarly, any conflict between an art-recognized definition of a word or phrase and a definition of the word or phrase as provided in this specification is to be resolved in favor of the latter.

List of Embodiments

Embodiment 1: A compound of Formula (A)

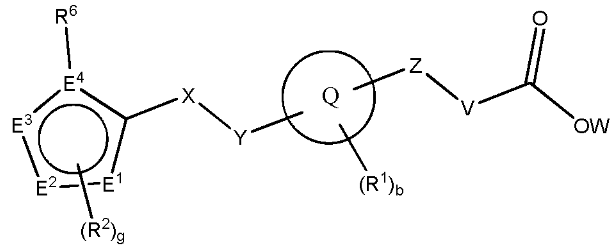

(A)

or a pharmaceutically acceptable salt thereof, wherein:

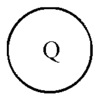

represents a 5-10 membered monocyclic or bicyclic aryl group, a 5-10 membered monocyclic or bicyclic heteroaryl group, a 5-10 membered monocyclic or bicyclic cycloalkyl group, a 5-10 membered monocyclic or bicyclic heterocycloalkyl group, or a 8-10 membered bicyclic group wherein an aryl or a 5-6 membered heteroaryl ring is fused to a 5-6 membered cycloalkyl or heterocycloalkyl ring;

| | |
|---|---|
| $E^1$, $E^2$ and $E^3$ | are independently selected from the group consisting of C, N and S; |
| $E^4$ | is selected from the group consisting of C and N; |
| X | is selected from the group consisting of $-CH_2-$, -C(O)- and $-C(O)CH_2-$; |
| Y | is selected from the group consisting of $-CH_2-$, -NH- and -O-; |
| Z | is selected from the group consisting of a $-(CR^4R^5)_n-$, -S-, -C(O)-and $-CR^4=CR^5-$; |
| V | is selected from the group consisting of a bond, $-(CR^4R^5)_n-$, $-CR^4=CR^5-$,and $-O-CR^4R^5-$; |
| W | is selected from the group consisting of H, $C_{1-6}$alkyl and substituted $C_{1-6}$alkyl; |
| $R^1$ | is independently selected from the group consisting of halo, $C_{1-6}$alkyl, substituted $C_{1-6}$alkyl, $C_{3-7}$cycloalkyl, substituted $C_{3-7}$cycloalkyl, $C_{2-6}$alkenyl, substituted $C_{26}$alkenyl, $C_{2-6}$alkynyl, substituted $C_{2-6}$alkynyl, CN, $-OR^a$, $-NR^aR^b$, $-C(O)R^a$, $-C(O)OR^a$, $-NR^aC(O)R^b$, $-SR^a$, $-S(O)R^a$ and $-S(O)_2R^a$; |

| | |
|---|---|
| $R^2$ | is independently selected from the group consisting of halo, $C_{1-6}$alkyl, substituted $C_{1-6}$alkyl, $C_{3-7}$cycloalkyl, substituted $C_{3-7}$cycloalkyl, $C_{2-6}$alkenyl, substituted $C_{26}$alkenyl, $C_{2-6}$alkynyl, substituted $C_{2-6}$alkynyl, aryloxy,-substituted aryloxy, CN, $-OR^a$, $-NR^aR^b$, $-C(O)R^a$, $-C(O)OR^a$, $-C(O)NR^aR^b$, $-NR^aC(O)R^b$, $-SR^a$, $-S(O)R^a$ and $-S(O)_2R^a$; |
| each of $R^a$ and $R^b$ | is independently selected from the group consisting of H, $C_{1-6}$alkyl, substituted $C_{1-6}$alkyl, $C_{3-7}$cycloalkyl, $C_{2-6}$alkenyl and $C_{2-6}$alkynyl; |
| $R^4$ | is independently selected from the group consisting of H, halo, $C_{1-6}$alkyl, substituted $C_{1-6}$alkyl, $C_{1-6}$alkoxy and substituted $C_{1-6}$alkoxy; |
| $R^5$ | is independently selected from the group consisting of H, halo, $C_{1-6}$alkyl, substituted $C_{1-6}$alkyl and $C_{1-6}$alkoxy; |

$R^6$ is selected from the group consisting of halo, $C_{1-6}$alkyl, substituted $C_{1-6}$alkyl, $C_{1-6}$alkoxy, substituted $C_{1-6}$alkoxy, aryl, substituted aryl, heteroaryl and substituted heteroaryl;

optionally $R^4$ and $R^5$ cyclize to form a $C_{3-7}$saturated ring or a spiro $C_{3-7}$cycloalkyl group;
the subscript b is 0, 1, 2, 3 or 4;
the subscript g is 0, 1 or 2; and
the subscript n is independently 1, 2 or 3.

Embodiment 2: The compound of embodiment 1, wherein: X is $-CH_2-$; Y is -O-; Z is $-(CR^4R^5)_n$; and V is a bond.

Embodiment 3: The compound of embodiment 2, wherein Q is selected from the group consisting of

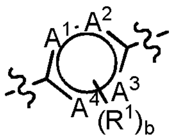 , 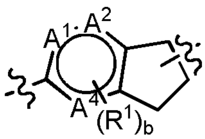 , 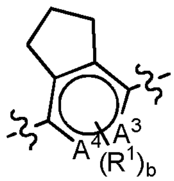 and 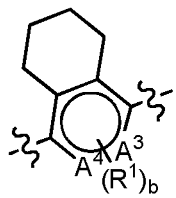

wherein $A^1$, $A^2$, $A^3$ and $A^4$ are independently selected from the group consisting of C and N, with the proviso that only 0, 1 or 2 of $A^1$, $A^2$, $A^3$ and $A^4$ is N and $R^1$ and subscript b are as previously defined.

Embodiment 4: The compound of embodiment 1 of Formula (B)

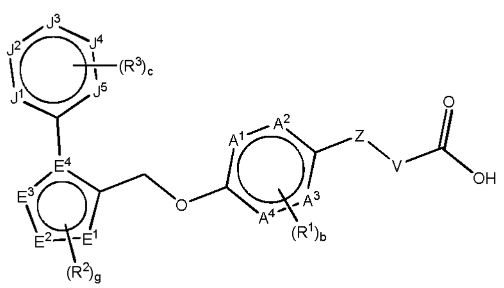

(B)

or a pharmaceutically acceptable salt thereof, wherein:

| | |
|---|---|
| $A^1$, $A^2$, $A^3$ and $A^4$ | are independently selected from the group consisting of C and N, with the proviso that only 0, 1 or 2 of $A^1$, $A^2$, $A^3$ and $A^4$ is N; |
| $J^1$, $J^2$, $J^3$, $J^4$ and $J^5$ | are independently selected from the group consisting ofN and C, with the proviso that only 0, 1 or 2 of $J^1$, $J^2$, $J^3$, $J^4$ and $J^5$ is N; |

| | |
|---|---|
| each $R^3$ | is independently selected from the group consisting of halo, $C_{1-6}$alkyl, substituted $C_{1-6}$alkyl, $C_{3-7}$cycloalkyl, substituted $C_{3-7}$cycloalkyl, $C_{2-6}$alkenyl, substituted $C_{2-6}$alkenyl, $C_{2-6}$alkynyl, substituted $C_{2-6}$alkynyl, cyano, $-OR^a$, $-NR^aR^b$, $-C(O)R^a$, $-C(O)OR^a$, $-C(O)NR^aR^b$, $-NR^aC(O)R^b$, $-SR^a$, $-S(O)R^a$ |

and $-S(O)_2R^a$;
the subscript c is 0, 1, 2 or 3; and

$E^1$, $E^2$, $E^3$, $E^4$, $R^1$, $R^2$, Z, V, $R^a$, $R^b$, subscript b and subscript g are as previously defined.

Embodiment 5: The compound of embodiment 4, wherein Z and V taken together are selected from the group consisting of

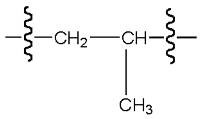 , 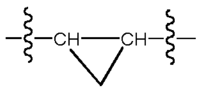 and 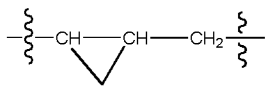 .

Embodiment 6: The compound of embodiment 5, wherein $E^1$ and $E^2$ are both C; and $E^3$ and $E^4$ are both N.

Embodiment 7: The compound of embodiment 6, wherein $A^1$, $A^2$, $A^3$ and $A^4$ are all C, and $J^1$, $J^2$, $J^3$, $J^4$ and $J^5$ are all C.

Embodiment 8: The compound of embodiment 7, wherein the subscript g is 0 or 1; the subscript c is 0 or 1; and the subscript b is 0, 1 or 2.

Embodiment 9: The compound of embodiment 8, wherein $R^1$ is halo, $C_{1-3}$alkyl or $CF_3$; g is 0 or g is 1 and $R^2$ is $CH_3$; and $R^3$ is $C_{1-4}$alkyl or halo.

Embodiment 10: A compound of Formula (C)

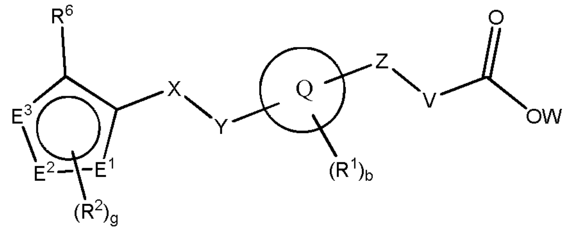

(C)

or a pharmaceutically acceptable salt thereof, wherein:

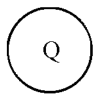

represents a 5-10 membered monocyclic or bicyclic aryl group, a 5-10 membered monocyclic or bicyclic heteroaryl group, a 5-10 membered monocyclic or bicyclic cycloalkyl group, a 5-10 membered monocyclic or bicyclic heterocycloalkyl group, or a 8-10 membered bicyclic group wherein an aryl or a 5-6 membered heteroaryl ring is fused to a 5-6 membered cycloalkyl or heterocycloalkyl ring;

| | |
|---|---|
| $E^1$, $E^2$ and $E^3$ | are independently selected from the group consisting of C, N and O; |
| X | is selected from the group consisting of $-CH_2-$ and $-C(O)CH_2-$; |
| Y | is selected from the group consisting of $-CH_2-$ and -O-; |
| Z | is selected from the group consisting of $-(CR^4R^5)_n-$, -S-, -C(O)-, and $-CR^4=CR^5-$; |
| V | is selected from the group consisting of a bond, $-(CR^4R^5)_n-$, $-CR^4=CR^5-$, and $-O-CR^4R^5-$; |
| W | is selected from the group consisting of H, $C_{1-6}$alkyl and substituted $C_{1-6}$alkyl; |
| $R^1$ | is independently selected from the group consisting of halo, $C_{1-6}$alkyl, substituted $C_{1-6}$alkyl, $C_{3-7}$cycloalkyl, substituted $C_{3-7}$cycloalkyl, $C_{2-6}$alkenyl, substituted $C_{26}$alkenyl, $C_{2-6}$alkynyl, substituted $C_{2-6}$alkynyl, CN, $-OR^a$, $-NR^aR^b$, $-C(O)R^a$, $-C(O)OR^a$, $-NR^aC(O)R^b$, $-SR^a$, $-S(O)R^a$ and $-S(O)_2R^a$; |
| $R^2$ | is independently selected from the group consisting of halo, $C_{1-6}$alkyl, substituted $C_{1-6}$alkyl, |

| | |
|---|---|
| | $C_{3-7}$cycloalkyl, substituted $C_{3-7}$cycloalkyl, $C_{2-6}$alkenyl, substituted $C_{26}$alkenyl, $C_{2-6}$alkynyl, substituted $C_{2-6}$alkynyl, aryloxy,-substituted aryloxy, CN, $-OR^a$, $-NR^aR^b$, $-C(O)R^a$, $-C(O)OR^a$, $-C(O)NR^aR^b$, $-NR^aC(O)R^b$, $-SR^a$, $-S(O)R^a$ and $-S(O)_2R^a$; |
| each of $R^a$ and $R^b$ | is independently selected from the group consisting of H, $C_{1-6}$alkyl, substituted $C_{1-6}$alkyl, $C_{3-7}$cycloalkyl, $C_{2-6}$alkenyl and $C_{2-6}$alkynyl; |
| $R^4$ | is independently selected from the group consisting of H, halo, $C_{1-6}$alkyl, substituted $C_{1-6}$alkyl, $C_{1-6}$alkoxy and substituted $C_{1-6}$alkoxy; |
| $R^5$ | is independently selected from the group consisting of H, halo, $C_{1-6}$alkyl, substituted $C_{1-6}$alkyl and $C_{1-6}$alkoxy; |

optionally $R^4$ and $R^5$ cyclize to form a $C_{3-7}$saturated ring or a spiro $C_{3-7}$cycloalkyl group;

$R^6$ is selected from the group consisting of aryl, substituted aryl, heteroaryl and substituted heteroaryl;

the subscript b is 0, 1, 2, 3 or 4;
the subscript g is 0, 1, 2 or 3 and;
the subscript n is independently 0, 1 or 2;
with the proviso that the compound is not methyl 3-(4-((3-(2,6-dichlorophenyl)-5-isopropylisoxazol-4-yl)methoxy)phenyl)propanoate, 3-(4-((3-(2-chloro-6-methylphenyl)-5-isopropylisoxazol-4-yl)methoxy)phenyl)propanoic acid, 3-(4-((3-(2,6-dichlorophenyl)-5-isopropylisoxazol-4-yl)methoxy)phenyl)propanoic acid, ethyl 3-(4-((4-(3-chlorophenyl)-2-(trifluoromethyl)furan-3-yl)methoxy)-2-methylphenyl)propanoate and 3-(3-fluoro-5-methyl-4-((3-methyl-5-phenylisoxazol-4-yl)methoxy)phenyl)propanoic acid.

Embodiment 11: The compound of embodiment 10, wherein: X is $-CH_2-$; Y is -O-; Z is $-(CR^4R^5)_n$ ; and V is a bond.

Embodiment 12: The compound of embodiment 11, wherein Q is selected from the group consisting of

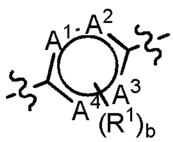 , 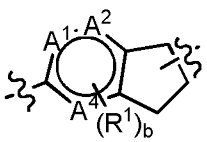 , 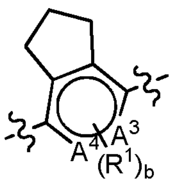 and 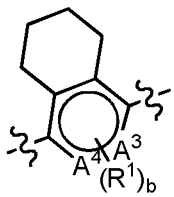

wherein $A^1$, $A^2$, $A^3$ and $A^4$ are independently selected from the group consisting of C and N, with the proviso that only 0, 1 or 2 of $A^1$, $A^2$, $A^3$ and $A^4$ is N and $R^1$ and subscript b are as previously defined.

Embodiment 13: The compound of embodiment 10 of Formula (D)

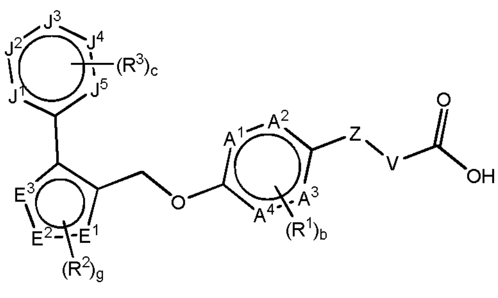

(D)

or a pharmaceutically acceptable salt thereof, wherein:

| | |
|---|---|
| $A^1$, $A^2$, $A^3$ and $A^4$ | are independently selected from the group consisting of C and N, with the proviso that only 0, 1 or 2 of $A^1$, $A^2$, $A^3$ and $A^4$ is N; |
| $J^1$, $J^2$, $J^3$, $J^4$ and $J^5$ | are independently selected from the group consisting ofN and C, with the proviso that only 0, 1 or 2 of $J^1$, $J^2$, $J^3$, $J^4$ and $J^5$ is N; |
| each $R^3$ | is independently selected from the group consisting of halo, $C_{1-6}$alkyl, substituted $C_{1-6}$alkyl, |

$C_{3-7}$cycloalkyl, substituted $C_{3-7}$cycloalkyl, $C_{2-6}$alkenyl, substituted $C_{2-6}$alkenyl, $C_{2-6}$alkynyl, substituted $C_{2-6}$alkynyl, cyano, $-OR^a$, $-NR^aR^b$, $-C(O)R^a$, $-C(O)OR^a$, $-C(O)NR^aR^b$, $-NR^aC(O)R^b$, $-SR^a$, $-S(O)R^a$ and - $S(O)_2R^a$;

the subscript c is 0, 1, 2 or 3; and
$E^1$, $E^2$, $E^3$, $R^1$, $R^2$, Z, V, $R^a$, $R^b$, subscript b and subscript g are as previously defined.

Embodiment 14: The compound of embodiment 13, wherein Z and V taken together are selected from the group consisting of

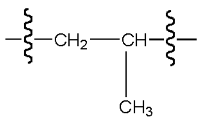 , 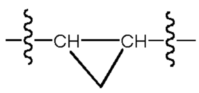 and 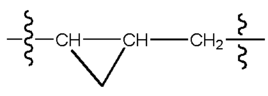 .

Embodiment 15: The compound of embodiment 14, wherein $E^1$ is C and one of $E^2$ and $E^3$ is N and the other of $E^2$ and $E^3$ is O or N.

Embodiment 16: The compound of embodiment 15, wherein $A^1$, $A^2$, $A^3$ and $A^4$ are all C, and $J^1$, $J^2$, $J^3$, $J^4$ and $J^5$ are all C.

Embodiment 17: The compound of embodiment 16, wherein the subscript g is 0 or 1; the subscript c is 0 or 1 and the subscript b is 0, 1 or 2.

Embodiment 18: The compound of embodiment 17, wherein $R^1$ is halo, $C_{1-3}$alkyl or $CF_3$; g is 0 or g is 1 and $R^2$ is $CH_3$; and $R^3$ is $C_{1-4}$alkyl or halo.

Embodiment 19: A compound or a pharmaceutically acceptable salt thereof of embodiment 1 or 10 selected from 3-(3,5-difluoro-4-((3-isopropyl-1-phenyl-1*H*-pyrazol-5-yl)methoxy)phenyl)propanoic acid **(55);**
ethyl 3-(3,5-difluoro-4-((4-phenyl-1,2,3-thiadiazol-5-yl)methoxy)phenyl)propanoate **(57);**
3-(3,5-difluoro-4-((1-phenyl-1*H*-imidazol-5-yl)methoxy)phenyl)propanoic acid **(59);** 2-(2-(4-((1-(4-chlorophenyl)-3-methyl-1*H*-pyrazol-5-yl)methoxy)-3,5-difluorophenyl)cyclopropyl)acetic acid **(62);**
3-(3,5-difluoro-4-((1-phenyl-1*H*-1,2,3-triazol-5-yl)methoxy)phenyl)propanoic acid **(65);**
2-(2-(3,5-difluoro-4-((5-methyl-3-phenylisoxazol-4-yl)methoxy)phenyl)cyclopropyl)-acetic acid **(67);**
3-(3,5-difluoro-4-((1-isobutyl-3-methyl-1*H*-pyrazol-5-yl)methoxy)phenyl)propanoic acid **(68);**
2-(2-(3,5-difluoro-4-((1-isobutyl-3-methyl-1*H*-pyrazol-5-yl)methoxy)phenyl)-cyclopropyl)acetic acid **(69);**
2-(3,5-Difluoro-4-((1-isobutyl-3-methyl-1*H*-pyrazol-5-yl)methoxy)phenyl)-cyclopropanecarboxylic acid **(70);**
3-(3,5-difluoro-4-((5-phenyloxazol-4-yl)methoxy)phenyl)propanoic acid **(71);**
3-(4-((1-benzyl-1*H*-imidazol-2-yl)methoxy)-3,5-difluorophenyl)propanoic acid **(72);**
3-(3,5-difluoro-4-((5-methyl-2-phenylfuran-3-yl)methoxy)phenyl)propanoic acid **(73);**
3-(3,5-difluoro-4-((1-methyl-5-phenyl-1*H*-pyrazol-4-yl)methoxy)phenyl)propanoic acid **(74);**
3-(3,5-difluoro-4-((5-methyl-3-phenylisoxazol-4-yl)methoxy)phenyl)propanoic acid **(75);**
3-(3,5-difluoro-4-((3-methyl-5-phenylisoxazol-4-yl)methoxy)phenyl)propanoic acid **(76);**
2-(3,5-difluoro-4-((5-methyl-3-phenylisoxazol-4-yl)methoxy)phenyl) cyclopropanecarboxylic acid **(77);**
2-(3,5-difluoro-4-((3-(4-fluorophenyl)-5-methylisoxazol-4-yl)methoxy)phenyl) cyclopropanecarboxylic acid **(78);**
3-(3,5-difluoro-4-((3-(4-fluorophenyl)-5-methylisoxazol-4-yl)methoxy)phenyl)propanoic acid **(79);**
3-(4-((3',5-dimethyl-3,5'-biisoxazol-4'-yl)methoxy)-3,5-difluorophenyl)propanoic acid **(80);**
3-(4-((3-(1*H*-pyrrol-1-yl)thiophen-2-yl)methoxy)-3,5-difluorophenyl)propanoic acid **(81);**
3-(3,5-difluoro-4-((3-(4-methoxyphenyl)-5-methylisoxazol-4-yl)methoxy)phenyl) propanoic acid **(82);**
3-(4-((3-(2,6-dichlorophenyl)-5-methylisoxazol-4-yl)methoxy)-3,5-difluorophenyl) propanoic acid **(83);**
3-(4-((3,5-dimethylisoxazol-4-yl)methoxy)-3,5-difluorophenyl)propanoic acid **(84);**
3-(4-((3-(4-chlorophenyl)-5-methylisoxazol-4-yl)methoxy)-3,5-difluorophenyl)propanoic acid **(85);**
3-(3,5-difluoro-4-((1-isopropyl-3-methyl-1*H*-pyrazol-5-yl)methoxy)phenyl)propanoic acid **(86);**
3-(3,5-difluoro-4-(2-(5-methyl-3-phenylisoxazol-4-yl)-2-oxoethoxy)phenyl)propanoic acid **(87);**
3-(4-((1-(4-chlorophenyl)-3-methyl-1*H*-pyrazol-5-yl)methoxy)-3,5-difluorophenyl) butanoic acid **(88);**
3-(3,5-difluoro-4-((5-phenylthiazol-4-yl)methoxy)phenyl)propanoic acid **(89);**
2-(4-((4-phenyl-1,2,3-thiadiazol-5-yl)methoxy)phenylthio)acetic acid **(90);**
3-(3,5-difluoro-4-((2-methyl-4-phenylthiazol-5-yl)methoxy)phenyl)propanoic acid **(91);**

3-(3,5-difluoro-4-((4-(4-fluorophenyl)-1,2,3-thiadiazol-5-yl)methoxy)phenyl)propanoic acid **(92);**
3-(4-((4-(3-chlorophenyl)-1,2,3-thiadiazol-5-yl)methoxy)-3,5-difluorophenyl)propanoic acid **(93);**
2-(3,5-difluoro-4-((2-methyl-4-phenylthiazol-5-yl)methoxy)phenyl) cyclopropane carboxylic acid **(94);**
2-(3,5-difluoro-4-((5-methyl-3-phenylisoxazol-4-yl)methoxy)benzyloxy)acetic acid **(95);**
2-(4-((1-(4-chlorophenyl)-3-methyl-1*H*-pyrazol-5-yl)methoxy)-3,5-difluorobenzyloxy) acetic acid **(96);**
3-(4-((1-(4-chlorophenyl)-3-methyl-1*H*-pyrazol-5-yl)methoxy)-3,5-difluorophenyl)-2-methylpropanoic acid **(97);**
3-(3,5-difluoro-4-((5-methyl-3-phenylisoxazol-4-yl)methoxy)phenyl)-2-methylpropanoic acid **(98);**
3-(4-((1-(4-chlorophenyl)-3-methyl-1*H*-pyrazol-5-yl)methoxy)-3,5-difluorophenyl)-2,2-dimethylpropanoic acid **(99);**
2-(6-((1-(4-chlorophenyl)-3-methyl-1*H*-pyrazol-5-yl)methoxy)-1,2,3,4-tetrahydronaphthalen-2-yl)acetic acid **(100);**
2-(6-((5-methyl-3-phenylisoxazol-4-yl)methoxy)-1,2,3,4-tetrahydronaphthalen-2-yl)acetic acid **(101);**
3-(4-((1-tert-butyl-3-methyl-1*H*-pyrazol-5-yl)methoxy)-3,5-difluorophenyl)propanoic acid **(102);**
3-(3,5-difluoro-4-((2-methyl-4-phenylthiazol-5-yl)methoxy)phenyl)-2-methylpropanoic acid **(103);**
(*R*)-3-(4-((1-(4-chlorophenyl)-3-methyl-1*H*-pyrazol-5-yl)methoxy)-3,5-difluorophenyl)-2-methylpropanoic acid **(104);**
(*S*)-3-(4-((1-(4-chlorophenyl)-3-methyl-1*H*-pyrazol-5-yl)methoxy)-3,5-difluorophenyl)-2-methylpropanoic acid **(105);**
4-(4-((3-methyl-1-phenyl-1*H*-pyrazol-5-yl)methoxy)phenyl)butanoic acid **(106);**
3-(3,5-difluoro-4-((3-methyl-1-phenyl-1*H*-pyrazol-5-yl)methoxy)phenyl)propanoic acid **(107);**
3-(3,5-difluoro-4-((1-(4-fluorophenyl)-3-methyl-1*H*-pyrazol-5-yl)methoxy)phenyl) propanoic acid **(108);**
3-(3,5-difluoro-4-((1-phenyl-1*H*-pyrazol-5-yl)methoxy)phenyl)propanoic acid **(109);**
3-(3,5-difluoro-4-((1-(4-methoxyphenyl)-3-methyl-1*H*-pyrazol-5-yl)methoxy)phenyl) propanoic acid **(110);**
3-(4-((1-(4-chlorophenyl)-3-methyl-1*H*-pyrazol-5-yl)methoxy)-3,5-difluorophenyl) propanoic acid **(111);**
3-(3,5-difluoro-4-((1-(4-fluorophenyl)-4-methyl-1*H*-pyrazol-5-yl)methoxy)phenyl) propanoic acid **(112);**
3-(4-((4-(4-chlorophenyl)-1,2,3-thiadiazol-5-yl)methoxy)-3,5-difluorophenyl)-2,2-difluoropropanoic acid **(113);**
3-(4-((1-(3,4-difluorophenyl)-3-methyl-1*H*-pyrazol-5-yl)methoxy)-3,5-difluorophenyl) propanoic acid **(114);**
3-(4-((1-(4-chlorophenyl)-3-methyl-1*H*-pyrazol-5-yl)methoxy)-3,5-difluorophenyl)-2-fluoropropanoic acid **(115);**
2-(4-((1-(4-chlorophenyl)-3-methyl-1*H*-pyrazol-5-yl)methoxy)-3,5-difluorobenzyl) cyclopropanecarboxylic acid **(116);**
3-(4-((1-(4-chlorophenyl)-3-(trifluoromethyl)-1*H*-pyrazol-5-yl)methoxy)-3,5-difluorophenyl)propanoic acid **(117);**
3-(4-((1-(4-chlorophenyl)-3-(trifluoromethyl)-1*H*-pyrazol-5-yl)methoxy)-3,5-difluorophenyl)-2,2-difluoropropanoic acid **(118);**
2-(5-((5-methyl-3-phenylisoxazol-4-yl)methoxy)-2,3-dihydro-1*H*-inden-1-yl)acetic acid **(119);**
2-(5-((1-(4-chlorophenyl)-3-methyl-1*H*-pyrazol-5-yl)methoxy)-2,3-dihydro-1*H*-inden-1-yl)acetic acid **(120);**
2-(5-((5-methyl-3-phenylisoxazol-4-yl)methoxy)-2,3-dihydro-1*H*-inden-1-yl)propanoic acid **(121);**
2-(5-((1-(4-chlorophenyl)-3-methyl-1*H*-pyrazol-5-yl)methoxy)-2,3-dihydro-1*H*-inden-1-yl)propanoic acid **(122);**
3-(4-((4-(4-chlorophenyl)-1,2,3-thiadiazol-5-yl)methoxy)-3,5-difluorophenyl)propanoic acid **(123);**
2-(3,5-difluoro-4-((4-phenyl-1,2,3-thiadiazol-5-yl)methoxy)phenyl)cyclopropane carboxylic acid **(124);**
2-(3,5-difluoro-4-((3-methyl-1-phenyl-1*H*-pyrazol-5-yl)methoxy)phenyl)cyclopropane carboxylic acid **(125);**
3-(3,5-difluoro-4-((1-phenyl-3-(trifluoromethyl)-1*H*-pyrazol-5-yl)methoxy)phenyl) propanoic acid **(126);**
4-(4-((4-(4-chlorophenyl)-1,2,3-thiadiazol-5-yl)methoxy)phenyl)-4-oxobutanoic acid **(128);**
(*E*)-4-(4-((4-(4-chlorophenyl)-1,2,3-thiadiazol-5-yl)methoxy)phenyl)-3-methylbut-2-enoic acid **(130);**
(E)-5-(4-((4-(4-chlorophenyl)-1,2,3-thiadiazol-5-yl)methoxy)phenyl)-3-methylpent-2-enoic acid **(131);**
5-(4-((1-(4-chlorophenyl)-3-methyl-1*H*-pyrazol-5-yl)methoxy)phenyl)-3-methyl pentanoic acid **(132);**
4-(4-((1-(4-chlorophenyl)-3-methyl-1*H*-pyrazol-5-yl)methoxy)phenyl)-3-methylbutanoic acid **(133);**
3-(4-((4-(3,4-difluorophenyl)-1,2,3-thiadiazol-5-yl)methoxy)-3,5-difluorophenyl) propanoic acid **(134);**
3-(4-((1-(3,4-dichlorophenyl)-3-methyl-1*H*-pyrazol-5-yl)methoxy)-3,5-difluorophenyl) propanoic acid **(135);**
3-(4-((4-isopropoxy-2-methylthiazol-5-yl)methoxy)phenyl)propanoic acid **(136);**
3-(3,5-difluoro-4-((1-(4-isopropylphenyl)-3-methyl-1H-pyrazol-5-yl)methoxy)phenyl)-2-methylpropanoic acid **(137);**
3-(3,5-difluoro-4-((3-methyl-1-(4-(trifluoromethyl)phenyl)-1*H*-pyrazol-5-yl)methoxy)phenyl)-2-methylpropanoic acid **(138);**
3-(4-((1-(4-chlorophenyl)-3-methyl-1H-pyrazol-5-yl)methoxy)-3-(trifluoromethyl)phenyl)-2-methylpropanoic acid **(139);**
3-(4-((1-(4-carbamoylphenyl)-3-methyl-1H-pyrazol-5-yl)methoxy)-3,5-difluorophenyl)-2-methylpropanoic acid **(140);**
3-(4-((1-(4-cyanophenyl)-3-methyl-1H-pyrazol-5-yl)methoxy)-3,5-difluorophenyl)-2-methylpropanoic acid **(141);**
3-(4-((1-(2,3-dihydro-1H-inden-5-yl)-3-methyl-1H-pyrazol-5-yl)methoxy)-3,5-difluorophenyl)-2-methylpropanoic acid **(142);**
3-(4-((1-(4-butylphenyl)-3-methyl-1H-pyrazol-5-yl)methoxy)-3,5-difluorophenyl)-2-methylpropanoic acid **(143);**

3-(4-((1-(4-ethylphenyl)-3-methyl-1H-pyrazol-5-yl)methoxy)-3,5-difluorophenyl)-2-methylpropanoic acid **(144);**
3-(3,5-difluoro-4-((3-methyl-1-p-tolyl-1H-pyrazol-5-yl)methoxy)phenyl)-2-methylpropanoic acid **(145);**
3-(3,5-difluoro-4-((1-(3-isopropylphenyl)-3-methyl-1H-pyrazol-5-yl)methoxy)phenyl)-2-methylpropanoic acid **(146);**
3-(4-((1-(4-chlorophenyl)-3-methyl-1H-pyrazol-5-yl)methoxy)phenyl)-2-methylpropanoic acid **(147);**
3-(4-((1-(3,4-dimethylphenyl)-3-methyl-1H-pyrazol-5-yl)methoxy)-3,5-difluorophenyl)-2-methylpropanoic acid **(148);**
3-(4-((1-(3-chloro-4-methylphenyl)-3-methyl-1 H-pyrazol-5-yl)methoxy)-3,5-difluorophenyl)-2-methylpropanoic acid **(149);**
3-(4-((1-(3-chlorophenyl)-3-methyl-1H-pyrazol-5-yl)methoxy)-3,5-difluorophenyl)-2-methylpropanoic acid **(150);**
3-(4-((1-(4-ethylphenyl)-3-methyl-1H-pyrazol-5-yl)methoxy)-3-fluorophenyl)-2-methylpropanoic acid **(151);**
3-(3,5-difluoro-4-((1-phenyl-1H-pyrazol-5-yl)methoxy)phenyl)-2-methylpropanoic acid **(152);**
3-(4-((1-(3,5-dimethylphenyl)-3-methyl-1H-pyrazol-5-yl)methoxy)-3,5-difluorophenyl)-2-methylpropanoic acid **(153);**
3-(4-((1-(4-chlorophenyl)-1H-pyrazol-5-yl)methoxy)-3,5-difluorophenyl)-2-methylpropanoic acid **(154);**
3-(4-((1-(4-chloro-3-methylphenyl)-3-methyl-1H-pyrazol-5-yl)methoxy)-3,5-difluorophenyl)-2-methylpropanoic acid **(155);**
3-(4-((1-(4-ethylphenyl)-3-methyl-1H-pyrazol-5-yl)methoxy)-3-methylphenyl)-2-methylpropanoic acid **(156);**
3-(4-((1-(4-ethylphenyl)-3-(trifluoromethyl)-1H-pyrazol-5-yl)methoxy)-3,5-difluorophenyl)-2-methylpropanoic acid **(157);**
3-(4-((1-(4-bromophenyl)-3-methyl-1H-pyrazol-5-yl)methoxy)-3,5-difluorophenyl)-2-methylpropanoic acid **(158);**
(R)-3-(4-((1-(4-ethylphenyl)-3-methyl-1H-pyrazol-5-yl)methoxy)-3,5-difluorophenyl)-2-methylpropanoic acid **(159);**
(S)-3-(4-((1-(4-ethylphenyl)-3-methyl-1H-pyrazol-5-yl)methoxy)-3,5-difluorophenyl)-2-methylpropanoic acid **(160);**
3-(4-((1-(4-ethylphenyl)-1H-pyrazol-5-yl)methoxy)-3,5-difluorophenyl)-2-methylpropanoic acid **(161);**
3-(4-((1-(4-chlorophenyl)-3-ethyl-1H-pyrazol-5-yl)methoxy)-3,5-difluorophenyl)-2-methylpropanoic acid **(162);**
3-(4-((1-(4-chlorophenyl)-3-methyl-1H-pyrazol-5-yl)methoxy)-3,5-difluorophenyl)-2-ethoxypropanoic acid **(163);**
3-(4-((1-(4-Chlorophenyl)-3-methyl-1*H*-pyrazol-5-yl)methoxy)-2-methoxyphenyl)-2-methylpropanoic acid **(164);**
3-(4-((1-(4-Cyclohexylphenyl)-3-methyl-1*H*-pyrazol-5-yl)methoxy)-3,5-difluorophenyl)-2-methylpropanoic acid **(165);**
3-(3,5-Difluoro-4-((3-methyl-1-(4-propylphenyl)-1*H*-pyrazol-5-yl)methoxy)phenyl)-2-methylpropanoic acid **(166);**
2-(5-((1-(4-Chlorophenyl)-3-methyl-1*H*-pyrazol-5-yl)methoxy)-6-fluoro-2,3-dihydro-1*H*-inden-1-yl)acetic acid **(167);**
2-(4-((1-(4-Chlorophenyl)-3-methyl-1*H*-pyrazol-5-yl)methoxy)-3,5-difluorophenyl)cyclopent-1-enecarboxylic acid **(168);**
3-(4-((1-(4-Chlorophenyl)-3-methyl-1*H*-pyrazol-5-yl)methoxy)-3-fluorophenyl)-2-methylpropanoic acid **(169);**
3-(4-((1-(4-Chlorophenyl)-3-methyl-1*H*-pyrazol-5-yl)methoxy)-3-methylphenyl)-2-methylpropanoic acid **(170);**
3-(4-((1-(5-Chloropyrimidin-2-yl)-3-methyl-1*H*-pyrazol-5-yl)methoxy)-3,5-difluorophenyl)-2-methylpropanoic acid **(171);**
3-(4-((1-(4-Chlorophenyl)-3-methyl-1*H*-pyrazol-5-yl)methoxy)benzylidene)cyclobutanecarboxylic acid **(172);**
3-(4-((1-(4-Chlorophenyl)-3-methyl-1*H*-pyrazol-5-yl)methoxy)-3-methoxyphenyl)-2-methylpropanoic acid **(173);**
3-(3,5-Dichloro-4-((1-(4-chlorophenyl)-3-methyl-1*H*-pyrazol-5-yl)methoxy)phenyl)-2-methylpropanoic acid **(174);**
3-(3,5-Difluoro-4-((1-methyl-5-phenoxy-3-(trifluoromethyl)-1*H*-pyrazol-4-yl)methoxy)phenyl)-2-methylpropanoic acid **(175);**
3-(4-((5-(4-Chlorophenoxy)-1-methyl-3-(trifluoromethyl)-1*H*-pyrazol-4-yl)methoxy)-3,5-difluorophenyl)-2-methylpropanoic Acid **(176);**
3-(3,5-Difluoro-4-((5-methyl-2-phenylfuran-3-yl)methoxy)phenyl)-2-methylpropanoic acid **(177);**
3-(4-((1-(4-chlorophenyl)-3-(trifluoromethyl)-1H-pyrazol-5-yl)methoxy)-3,5-difluorophenyl)-2-methylpropanoic acid **(178);**
3-(4-((1-(4-chlorophenyl)-3-methyl-1H-pyrazol-5-yl)methoxy)-2-ethylphenyl)propanoic acid **(179);**
2-(5-((1-(4-chlorophenyl)-3-(trifluoromethyl)-1H-pyrazol-5-yl)methoxy)-2,3-dihydro-1H-inden-1-yl)acetic acid **(180);**
2-(5-((1-(2,3-dihydro-1H-inden-5-yl)-3-methyl-1H-pyrazol-5-yl)methoxy)-2,3-dihydro-1H-inden-1-yl)acetic acid **(181);**
2-(5-((1-(4-ethylphenyl)-3-methyl-1H-pyrazol-5-yl)methoxy)-2,3-dihydro-1H-inden-1-yl)acetic acid **(182);**
3-(3,5-difluoro-4-((3-methyl-1-phenyl-1H-pyrazol-5-yl)methoxy)phenyl)-2-methylpropanoic acid **(183);**
2-(6-((1-(4-ethylphenyl)-3-methyl-1H-pyrazol-5-yl)methoxy)-1,2,3,4-tetrahydronaphthalen-1-yl)acetic acid **(184);**
3-(3,5-difluoro-4-((3-methyl-1-(pyridin-2-yl)-1H-pyrazol-5-yl)methoxy)phenyl)-2-methylpropanoic acid **(185);**
3-(4-((1-(4-chlorophenyl)-3-methyl-1H-pyrazol-5-yl)methoxy)phenyl)-3-methylbutanoic acid **(186);**
3-(4-((1-(4-ethylphenyl)-3-methyl-1H-pyrazol-5-yl)methoxy)phenyl)-3-methylbutanoic acid **(187);**
4-(4-((1-(4-chlorophenyl)-3-methyl-1H-pyrazol-5-yl)methoxy)phenyl)butanoic acid **(188);**
4-(4-((1-(4-chlorophenyl)-3-methyl-1H-pyrazol-5-yl)methoxy)-3,5-difluorophenyl)butanoic acid **(189);**

4-(4-((1-(4-ethylphenyl)-3-methyl-1H-pyrazol-5-yl)methoxy)phenyl)butanoic acid **(190);**
4-(4-((1-(4-ethylphenyl)-3-methyl-1H-pyrazol-5-yl)methoxy)-3,5-difluorophenyl)butanoic acid **(191);**
4-(4-((1-(4-chlorophenyl)-3-methyl-1H-pyrazol-5-yl)methoxy)phenyl)-2-methylbutanoic acid **(192);**
4-(4-((1-(4-ethylphenyl)-3-methyl-1H-pyrazol-5-yl)methoxy)phenyl)-2-methylbutanoic acid **(193);**
3-(4-((1-(4-chlorophenyl)-3-methyl-1H-pyrazol-5-yl)methoxy)-3-methoxyphenyl)propanoic acid **(194);**
3-(4-((1-(4-chlorophenyl)-3-methyl-1H-pyrazol-5-yl)methoxy)-3-fluoro-2-methylphenyl)propanoic acid **(195);**
3-(4-((1-(4-chlorophenyl)-3-methyl-1H-pyrazol-5-yl)methoxy)-2-ethyl-3-fluorophenyl)propanoic acid **(196);**
3-(4-((1-(4-chlorophenyl)-3-methyl-1H-pyrazol-5-yl)methoxy)-2,3-dimethylphenyl)propanoic acid **(197);**
3-(4-((1-(4-chlorophenyl)-1H-pyrazol-5-yl)methoxy)-3-fluoro-2-methylphenyl)propanoic acid **(198);**
3-(4-((1-(4-chlorophenyl)-1H-pyrazol-5-yl)methoxy)-2,3-dimethylphenyl)propanoic acid **(199);**
3-(4-((3-(4-chlorophenyl)-5-methylisoxazol-4-yl)methoxy)-2,3-dimethylphenyl) propanoic acid **(200);**
3-(4-((1-(4-ethylphenyl)-3-methyl-1H-pyrazol-5-yl)methoxy)-2,3-dimethylphenyl)propanoic acid **(201);**
3-(4-((1-(4-ethylphenyl)-1H-pyrazol-5-yl)methoxy)-2,3-dimethylphenyl)propanoicacid **(202);**
3-(4-((1-(4-chlorophenyl)-3-methyl-1H-pyrazol-5-yl)methoxy)-5,6,7,8-tetrahydro naphthalen-1-yl)propanoic acid **(203);** and
3-(7-((1-(4-chlorophenyl)-3-methyl-1H-pyrazol-5-yl)methoxy)-2,3-dihydro-1H-inden-4-yl)propanoic acid **(207).**

Embodiment 20: A composition comprising a compound of any one of embodiments 1 to 19 and a pharmaceutically acceptable carrier.

Embodiment 21: Use of a compound of any one of embodiments 1 to 19 in the preparation of a medicament for treating in a mammal a disease or condition selected from the group consisting of Type I diabetes, Type II diabetes and metabolic syndrome.

Embodiment 22: The use of embodiment 21, wherein said disease is Type II diabetes.

Embodiment 23: Use of a compound of any one of embodiments 1 to 19 in the preparation of a medicament for stimulating insulin production in a mammal.

Embodiment 24: The use of embodiment 23, wherein said mammal is a human.

Embodiment 25: Use of a compound of any one of embodiments 1 to 19 in the preparation of a medicament for lowering blood glucose in a mammal.

Embodiment 26: The use of embodiment 25, wherein said mammal is a human.

Embodiment 27: Use of a compound of any one of embodiments 1 to 19 in the preparation of a medicament for modulating GPR120 activity in a cell.

Embodiment 28: A compound of any one of embodiments 1 to 19 for use in one or more of treating Type I diabetes, treating Type II diabetes, treating metabolic syndrome, stimulating insulin production, or lowering blood glucose.

## Claims

**1.** A compound of Formula (A) or (C)

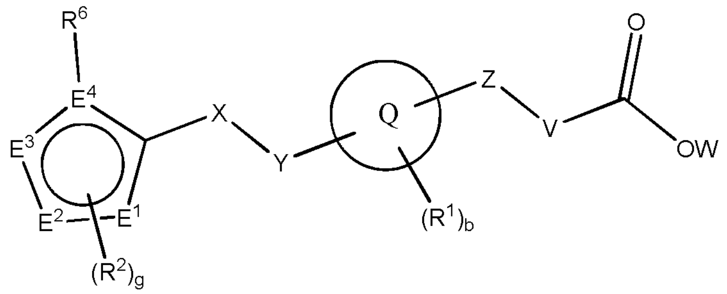

(A)

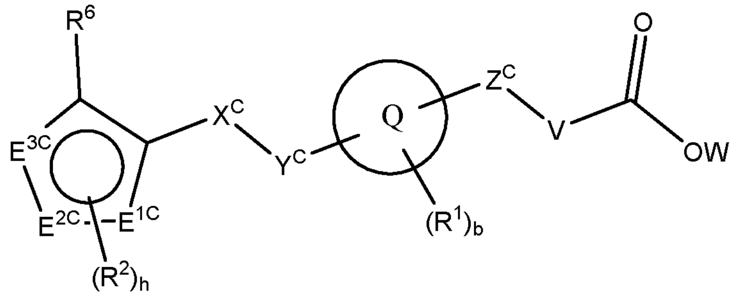

(C)

or a pharmaceutically acceptable salt thereof, wherein:

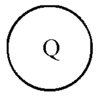

represents a 5-10 membered monocyclic or bicyclic aryl group, a 5-10 membered monocyclic or bicyclic heteroaryl group, a 5-10 membered monocyclic or bicyclic cycloalkyl group, a 5-10 membered monocyclic or bicyclic heterocycloalkyl group, or a 8-10 membered bicyclic group wherein an aryl or a 5-6 membered heteroaryl ring is fused to a 5-6 membered cycloalkyl or heterocycloalkyl ring;

$E^1$, $E^2$ and $E^3$ are independently selected from the group consisting of C, N and S;
$E^4$ is selected from the group consisting of C and N;
X is selected from the group consisting of $-CH_2-$, -C(O)- and $-C(O)CH_2-$;
Y is selected from the group consisting of $-CH_2-$, -NH- and -O-;
Z is selected from the group consisting of $-(CR^4R^5)_m-$, -S-, -C(O)-and $-CR^4=CR^5-$;
$E^{1C}$, $E^{2C}$ and $E^{3C}$ are independently selected from the group consisting of C, N and O;
$X^C$ is selected from the group consisting of $-CH_2-$ and $-C(O)CH_2-$;
$Y^C$ is selected from the group consisting of $-CH_2-$ and -O-;
$Z^C$ is selected from the group consisting of $-(CR^4R^5)_n-$, -S-, -C(O)-and $-CR^4=CR^5-$;
V is selected from the group consisting of a bond, $-(CR^4R^5)_n-$, $-CR^4=CR^5-$, and $-O-CR^4R^{5-}$;
W is selected from the group consisting of H, $C_{1-6}$alkyl and substituted $C_{1-6}$alkyl;
$R^1$ is independently selected from the group consisting of halo, $C_{1-6}$alkyl, substituted $C_{1-6}$alkyl, $C_{3-7}$cycloalkyl, substituted $C_{3-7}$cycloalkyl, $C_{2-6}$alkenyl, substituted $C_{26}$alkenyl, $C_{2-6}$alkynyl, substituted $C_{2-6}$alkynyl, CN, $-OR^a$, $-NR^aR^b$, $-C(O)R^a$, $-C(O)OR^a$, $-NR^aC(O)R^b$, $-SR^a$, $-S(O)R^a$ and $-S(O)_2R^a$;
$R^2$ is independently selected from the group consisting of halo, $C_{1-6}$alkyl, substituted $C_{1-6}$alkyl, $C_{3-7}$cycloalkyl, substituted $C_{3-7}$cycloalkyl, $C_{2-6}$alkenyl, substituted $C_{26}$alkenyl, $C_{2-6}$alkynyl, substituted $C_{2-6}$alkynyl, aryloxy, substituted aryloxy, CN, $-OR^a$, $-NR^aR^b$, $-C(O)R^a$, $-C(O)OR^a$, $-C(O)NR^aR^b$, $-NR^aC(O)R^b$, $-SR^a$, $-S(O)R^a$ and $-S(O)_2R^a$;
each of $R^a$ and $R^b$ is independently selected from the group consisting of H, $C_{1-6}$alkyl, substituted $C_{1-6}$alkyl, $C_{3-7}$cycloalkyl, $C_{2-6}$alkenyl and $C_{2-6}$alkynyl;
$R^4$ is independently selected from the group consisting of H, halo, $C_{1-6}$alkyl, substituted $C_{1-6}$alkyl, $C_{1-6}$alkoxy and substituted $C_{1-6}$alkoxy;
$R^5$ is independently selected from the group consisting of H, halo, $C_{1-6}$alkyl, substituted $C_{1-6}$alkyl and $C_{1-6}$alkoxy;
$R^6$ is selected from the group consisting of aryl, substituted aryl, heteroaryl and substituted heteroaryl;

optionally $R^4$ and $R^5$ cyclize to form a $C_{3-7}$saturated ring or a spiro $C_{3-7}$cycloalkyl group;
the subscript b is 0, 1, 2, 3 or 4;
the subscript g is 0, 1 or 2;
the subscript h is 0, 1, 2 or 3;
the subscript m is 1, 2 or 3; and
the subscript n is independently 0, 1 or 2;
with the proviso that the compound is not methyl 3-(4-((3-(2,6-dichlorophenyl)-5-isopropylisoxazol-4-yl)methoxy)phenyl)propanoate, 3-(4-((3-(2-chloro-6-methylphenyl)-5-isopropylisoxazol-4-yl)methoxy)phenyl)propanoic acid,

3-(4-((3-(2,6-dichlorophenyl)-5-isopropylisoxazol-4-yl)methoxy)phenyl)propanoic acid, ethyl 3-(4-((4-(3-chlorophenyl)-2-(trifluoromethyl)furan-3-yl)methoxy)-2-methylphenyl)propanoate and 3-(3-fluoro-5-methyl-4-((3-methyl-5-phenylisoxazol-4-yl)methoxy)phenyl)propanoic acid.

**2.** The compound of claim 1, wherein: X is $-CH_2-$; Y is -O-;Z is $-(CR^4R^5)_m$; $X^C$ is $-CH_2-$; $Y^C$ is -O-;$Z^C$ is $-(CR^4R^5)_n$ ; and V is a bond.

**3.** The compound of claim 2, wherein Q is selected from the group consisting of

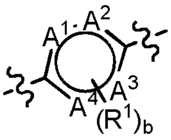 , 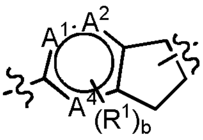 , 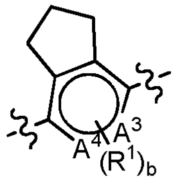 and 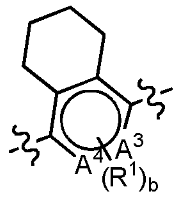

wherein $A^1$, $A^2$, $A^3$ and $A^4$ are independently selected from the group consisting of C and N, with the proviso that only 0, 1 or 2 of $A^1$, $A^2$, $A^3$ and $A^4$ is N and $R^1$ and subscript b are as previously defined.

**4.** The compound of claim 1 of Formula (B)

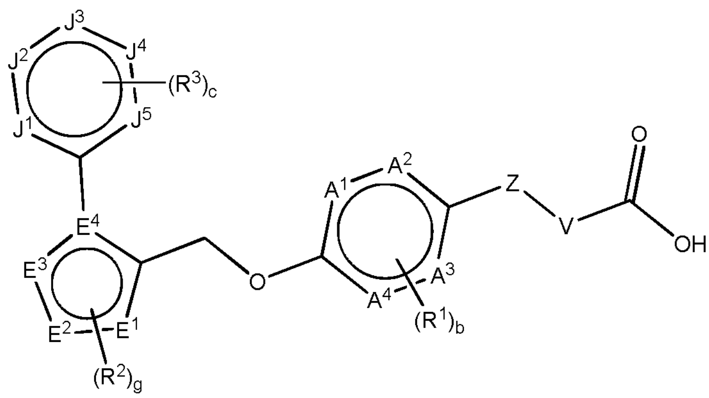

(B)

or a pharmaceutically acceptable salt thereof, wherein:

$A^1$, $A^2$, $A^3$ and $A^4$ are independently selected from the group consisting of C and N, with the proviso that only 0, 1 or 2 of $A^1$, $A^2$, $A^3$ and $A^4$ is N;
$J^1$, $J^2$, $J^3$, $J^4$ and $J^5$ are independently selected from the group consisting ofN and C, with the proviso that only 0, 1 or 2 of $J^1$, $J^2$, $J^3$, $J^4$ and $J^5$ is N;
each $R^3$ is independently selected from the group consisting of halo, $C_{1-6}$alkyl, substituted $C_{1-6}$alkyl, $C_{3-7}$cycloalkyl, substituted $C_{3-7}$cycloalkyl, $C_{2-6}$alkenyl, substituted $C_{2-6}$alkenyl, $C_{2-6}$alkynyl, substituted $C_{2-6}$alkynyl, cyano, $-OR^a$, $-NR^aR^b$, $-C(O)R^a$, $-C(O)OR^a$, $-C(O)NR^aR^b$, $-NR^aC(O)R^b$, $-SR^a$, $-S(O)R^a$ and $-S(O)_2R^a$;

the subscript c is 0, 1, 2 or 3; and

$E^1$, $E^2$, $E^3$, $E^4$,
$R^1$, $R^2$, Z, V, $R^a$, $R^b$, subscript b and subscript g are as previously defined.

**5.** The compound of claim 1 of Formula (D)

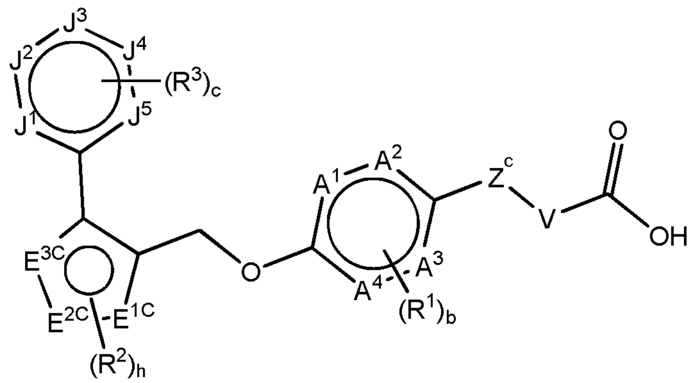

(D)

or a pharmaceutically acceptable salt thereof, wherein:

$A^1$, $A^2$, $A^3$ and $A^4$ are independently selected from the group consisting of C and N, with the proviso that only 0, 1 or 2 of $A^1$, $A^2$, $A^3$ and $A^4$ is N;
$J^1$, $J^2$, $J^3$, $J^4$ and $J^5$ are independently selected from the group consisting ofN and C, with the proviso that only 0, 1 or 2 of $J^1$, $J^2$, $J^3$, $J^4$ and $J^5$ is N;
each $R^3$ is independently selected from the group consisting of halo, $C_{1-6}$alkyl, substituted $C_{1-6}$alkyl, $C_{3-7}$cycloalkyl, substituted $C_{3-7}$cycloalkyl, $C_{2-6}$alkenyl, substituted $C_{2-6}$alkenyl, $C_{2-6}$alkynyl, substituted $C_{2-6}$alkynyl, cyano, $-OR^a$, $-NR^aR^b$, $-C(O)R^a$, $-C(O)OR^a$, $-C(O)NR^aR^b$, $-NR^aC(O)R^b$, $-SR^a$, $-S(O)R^a$ and $-S(O)_2R^a$;

the subscript c is 0, 1, 2 or 3; and

$E^{1C}$, $E^{2C}$, $E^{3C}$, $R^1$,
$R^2$, $Z^C$, V, $R^a$, $R^b$, subscript b and subscript h are as previously defined.

**6.** The compound of claim 1 or 5, wherein Z and V or $Z^C$ and V taken together are selected from the group consisting of

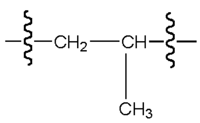 , 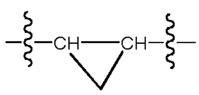 and 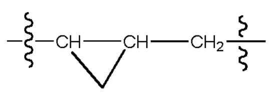 .

**7.** The compound of claim 6, wherein when the compound is of Formula (B), $E^1$ and $E^2$ are both C; and $E^3$ and $E^4$ are both N, when the compound is of Formula (D), $E^{1C}$ is C and one of $E^{2C}$ and $E^{3C}$ is N and the other of $E^{2C}$ and $E^{3C}$ is O or N.

**8.** The compound of claim 7, wherein $A^1$, $A^2$, $A^3$ and $A^4$ are all C, and $J^1$, $J^2$, $J^3$, $J^4$ and $J^5$ are all C.

**9.** The compound of claim 8, wherein the subscript g is 0 or 1; the subscript h is 0 or 1; the subscript c is 0 or 1; and the subscript b is 0, 1 or 2.

**10.** The compound of claim 9, wherein $R^1$ is halo, $C_{1-3}$alkyl or $CF_3$; g is 0 or g is 1; h is 0 or h is 1; and $R^2$ is $CH_3$; and $R^3$ is $C_{1-4}$alkyl or halo.

**11.** A compound of claim 1 selected from
3-(3,5-difluoro-4-((3-isopropyl-1-phenyl-1*H*-pyrazol-5-yl)methoxy)phenyl)propanoic acid **(55);**
ethyl 3-(3,5-difluoro-4-((4-phenyl-1,2,3-thiadiazol-5-yl)methoxy)phenyl)propanoate **(57);**
3-(3,5-difluoro-4-((1-phenyl-1*H*-imidazol-5-yl)methoxy)phenyl)propanoic acid **(59);**
2-(2-(4-((1-(4-chlorophenyl)-3-methyl-1*H*-pyrazol-5-yl)methoxy)-3,5-difluorophenyl)cyclopropyl)acetic acid **(62);**
3-(3,5-difluoro-4-((1-phenyl-1*H*-1,2,3-triazol-5-yl)methoxy)phenyl)propanoic acid **(65);**
2-(2-(3,5-difluoro-4-((5-methyl-3-phenylisoxazol-4-yl)methoxy)phenyl)cyclopropyl)-acetic acid **(67);**
3-(3,5-difluoro-4-((5-phenyloxazol-4-yl)methoxy)phenyl)propanoic acid **(71);**
3-(3,5-difluoro-4-((5-methyl-2-phenylfuran-3-yl)methoxy)phenyl)propanoic acid **(73);**

3-(3,5-difluoro-4-((1-methyl-5-phenyl-1*H*-pyrazol-4-yl)methoxy)phenyl)propanoic acid **(74);**
3-(3,5-difluoro-4-((5-methyl-3-phenylisoxazol-4-yl)methoxy)phenyl)propanoic acid **(75);**
3-(3,5-difluoro-4-((3-methyl-5-phenylisoxazol-4-yl)methoxy)phenyl)propanoic acid **(76);**
2-(3,5-difluoro-4-((5-methyl-3-phenylisoxazol-4-yl)methoxy)phenyl) cyclopropanecarboxylic acid **(77);**
2-(3,5-difluoro-4-((3-(4-fluorophenyl)-5-methylisoxazol-4-yl)methoxy)phenyl) cyclopropanecarboxylic acid **(78);**
3-(3,5-difluoro-4-((3-(4-fluorophenyl)-5-methylisoxazol-4-yl)methoxy)phenyl)propanoic acid **(79);**
3-(4-((3',5-dimethyl-3,5'-biisoxazol-4'-yl)methoxy)-3,5-difluorophenyl)propanoic acid **(80);**
3-(4-((3-(1*H*-pyrrol-1-yl)thiophen-2-yl)methoxy)-3,5-difluorophenyl)propanoic acid **(81);**
3-(3,5-difluoro-4-((3-(4-methoxyphenyl)-5-methylisoxazol-4-yl)methoxy)phenyl) propanoic acid **(82);**
3-(4-((3-(2,6-dichlorophenyl)-5-methylisoxazol-4-yl)methoxy)-3,5-difluorophenyl) propanoic acid **(83);**
3-(4-((3-(4-chlorophenyl)-5-methylisoxazol-4-yl)methoxy)-3,5-difluorophenyl)propanoic acid **(85);**
3-(3,5-difluoro-4-(2-(5-methyl-3-phenylisoxazol-4-yl)-2-oxoethoxy)phenyl)propanoic acid **(87);**
3-(4-((1-(4-chlorophenyl)-3-methyl-1*H*-pyrazol-5-yl)methoxy)-3,5-difluorophenyl) butanoic acid **(88);**
3-(3,5-difluoro-4-((5-phenylthiazol-4-yl)methoxy)phenyl)propanoic acid **(89);**
2-(4-((4-phenyl-1,2,3-thiadiazol-5-yl)methoxy)phenylthio)acetic acid **(90);**
3-(3,5-difluoro-4-((2-methyl-4-phenylthiazol-5-yl)methoxy)phenyl)propanoic acid **(91);**
3-(3,5-difluoro-4-((4-(4-fluorophenyl)-1,2,3-thiadiazol-5-yl)methoxy)phenyl)propanoic acid **(92);**
3-(4-((4-(3-chlorophenyl)-1,2,3-thiadiazol-5-yl)methoxy)-3,5-difluorophenyl)propanoic acid **(93);**
2-(3,5-difluoro-4-((2-methyl-4-phenylthiazol-5-yl)methoxy)phenyl) cyclopropane carboxylic acid **(94);**
2-(3,5-difluoro-4-((5-methyl-3-phenylisoxazol-4-yl)methoxy)benzyloxy)acetic acid **(95)**;
2-(4-((1-(4-chlorophenyl)-3-methyl-1*H*-pyrazol-5-yl)methoxy)-3,5-difluorobenzyloxy) acetic acid **(96);**
3-(4-((1-(4-chlorophenyl)-3-methyl-1*H*-pyrazol-5-yl)methoxy)-3,5-difluorophenyl)-2-methylpropanoic acid **(97);**
3-(3,5-difluoro-4-((5-methyl-3-phenylisoxazol-4-yl)methoxy)phenyl)-2-methylpropanoic acid **(98);**
3-(4-((1-(4-chlorophenyl)-3-methyl-1*H*-pyrazol-5-yl)methoxy)-3,5-difluorophenyl)-2,2-dimethylpropanoic acid **(99);**
2-(6-((1-(4-chlorophenyl)-3-methyl-1*H*-pyrazol-5-yl)methoxy)-1,2,3,4-tetrahydronaphthalen-2-yl)acetic acid **(100);**
2-(6-((5-methyl-3-phenylisoxazol-4-yl)methoxy)-1,2,3,4-tetrahydronaphthalen-2-yl)acetic acid **(101);**
3-(3,5-difluoro-4-((2-methyl-4-phenylthiazol-5-yl)methoxy)phenyl)-2-methylpropanoic acid **(103);**
(*R*)-3-(4-((1-(4-chlorophenyl)-3-methyl-1*H*-pyrazol-5-yl)methoxy)-3,5-difluorophenyl)-2-methylpropanoic acid **(104);**
(*S*)-3-(4-((1-(4-chlorophenyl)-3-methyl-1*H*-pyrazol-5-yl)methoxy)-3,5-difluorophenyl)-2-methylpropanoic acid **(105);**
4-(4-((3-methyl-1-phenyl-1*H*-pyrazol-5-yl)methoxy)phenyl)butanoic acid **(106);**
3-(3,5-difluoro-4-((3-methyl-1-phenyl-1*H*-pyrazol-5-yl)methoxy)phenyl)propanoic acid **(107);**
3-(3,5-difluoro-4-((1-(4-fluorophenyl)-3-methyl-1*H*-pyrazol-5-yl)methoxy)phenyl) propanoic acid **(108);**
3-(3,5-difluoro-4-((1-phenyl-1*H*-pyrazol-5-yl)methoxy)phenyl)propanoic acid **(109);**
3-(3,5-difluoro-4-((1-(4-methoxyphenyl)-3-methyl-1*H*-pyrazol-5-yl)methoxy)phenyl) propanoic acid **(110);**
3-(4-((1-(4-chlorophenyl)-3-methyl-1*H*-pyrazol-5-yl)methoxy)-3,5-difluorophenyl) propanoic acid **(111);**
3-(3,5-difluoro-4-((1-(4-fluorophenyl)-4-methyl-1*H*-pyrazol-5-yl)methoxy)phenyl) propanoic acid **(112);**
3-(4-((4-(4-chlorophenyl)-1,2,3-thiadiazol-5-yl)methoxy)-3,5-difluorophenyl)-2,2-difluoropropanoic acid **(113);**
3-(4-((1-(3,4-difluorophenyl)-3-methyl-1*H*-pyrazol-5-yl)methoxy)-3,5-difluorophenyl) propanoic acid **(114);**
3-(4-((1-(4-chlorophenyl)-3-methyl-1*H*-pyrazol-5-yl)methoxy)-3,5-difluorophenyl)-2-fluoropropanoic acid **(115);**
2-(4-((1-(4-chlorophenyl)-3-methyl-1*H*-pyrazol-5-yl)methoxy)-3,5-difluorobenzyl) cyclopropanecarboxylic acid **(116);**
3-(4-((1-(4-chlorophenyl)-3-(trifluoromethyl)-1*H*-pyrazol-5-yl)methoxy)-3,5-difluorophenyl)propanoic acid **(117);**
3-(4-((1-(4-chlorophenyl)-3-(trifluoromethyl)-1*H*-pyrazol-5-yl)methoxy)-3,5-difluorophenyl)-2,2-difluoropropanoic acid **(118);**
2-(5-((5-methyl-3-phenylisoxazol-4-yl)methoxy)-2,3-dihydro-1*H*-inden-1-yl)acetic acid **(119);**
2-(5-((1-(4-chlorophenyl)-3-methyl-1*H*-pyrazol-5-yl)methoxy)-2,3-dihydro-1*H*-inden-1-yl)acetic acid **(120);**
2-(5-((5-methyl-3-phenylisoxazol-4-yl)methoxy)-2,3-dihydro-1*H*-inden-1-yl)propanoic acid **(121);**
2-(5-((1-(4-chlorophenyl)-3-methyl-1*H*-pyrazol-5-yl)methoxy)-2,3-dihydro-1*H*-inden-1-yl)propanoic acid **(122);**
3-(4-((4-(4-chlorophenyl)-1,2,3-thiadiazol-5-yl)methoxy)-3,5-difluorophenyl)propanoic acid **(123);**
2-(3,5-difluoro-4-((4-phenyl-1,2,3-thiadiazol-5-yl)methoxy)phenyl)cyclopropane carboxylic acid **(124);**
2-(3,5-difluoro-4-((3-methyl-1-phenyl-1*H*-pyrazol-5-yl)methoxy)phenyl)cyclopropane carboxylic acid **(125);**
3-(3,5-difluoro-4-((1-phenyl-3-(trifluoromethyl)-1*H*-pyrazol-5-yl)methoxy)phenyl) propanoic acid **(126);**
4-(4-((4-(4-chlorophenyl)-1,2,3-thiadiazol-5-yl)methoxy)phenyl)-4-oxobutanoic acid **(128);**
(*E*)-4-(4-((4-(4-chlorophenyl)-1,2,3-thiadiazol-5-yl)methoxy)phenyl)-3-methylbut-2-enoic acid **(130);**
(*E*)-5-(4-((4-(4-chlorophenyl)-1,2,3-thiadiazol-5-yl)methoxy)phenyl)-3-methylpent-2-enoic acid **(131);**
5-(4-((1-(4-chlorophenyl)-3-methyl-1*H*-pyrazol-5-yl)methoxy)phenyl)-3-methyl pentanoic acid **(132);**

4-(4-((1-(4-chlorophenyl)-3-methyl-1*H*-pyrazol-5-yl)methoxy)phenyl)-3-methylbutanoic acid **(133);**
3-(4-((4-(3,4-difluorophenyl)-1,2,3-thiadiazol-5-yl)methoxy)-3,5-difluorophenyl) propanoic acid **(134);**
3-(4-((1-(3,4-dichlorophenyl)-3-methyl-1*H*-pyrazol-5-yl)methoxy)-3,5-difluorophenyl) propanoic acid **(135);**
3-(3,5-difluoro-4-((1-(4-isopropylphenyl)-3-methyl-1H-pyrazol-5-yl)methoxy)phenyl)-2-methylpropanoic acid **(137);**
3-(3,5-difluoro-4-((3-methyl-1-(4-(trifluoromethyl)phenyl)-1H-pyrazol-5-yl)methoxy)phenyl)-2-methylpropanoic acid **(138);**
3-(4-((1-(4-chlorophenyl)-3-methyl-1H-pyrazol-5-yl)methoxy)-3-(trifluoromethyl)phenyl)-2-methylpropanoic acid **(139);**
3-(4-((1-(4-carbamoylphenyl)-3-methyl-1H-pyrazol-5-yl)methoxy)-3,5-difluorophenyl)-2-methylpropanoic acid **(140);**
3-(4-((1-(4-cyanophenyl)-3-methyl-1H-pyrazol-5-yl)methoxy)-3,5-difluorophenyl)-2-methylpropanoic acid **(141);**
3-(4-((1-(2,3-dihydro-1H-inden-5-yl)-3-methyl-1H-pyrazol-5-yl)methoxy)-3,5-difluorophenyl)-2-methylpropanoic acid **(142);**
3-(4-((1-(4-butylphenyl)-3-methyl-1H-pyrazol-5-yl)methoxy)-3,5-difluorophenyl)-2-methylpropanoic acid **(143);**
3-(4-((1-(4-ethylphenyl)-3-methyl-1H-pyrazol-5-yl)methoxy)-3,5-difluorophenyl)-2-methylpropanoic acid **(144);**
3-(3,5-difluoro-4-((3-methyl-1-p-tolyl-1H-pyrazol-5-yl)methoxy)phenyl)-2-methylpropanoic acid **(145);**
3-(3,5-difluoro-4-((1-(3-isopropylphenyl)-3-methyl-1H-pyrazol-5-yl)methoxy)phenyl)-2-methylpropanoic acid **(146);**
3-(4-((1-(4-chlorophenyl)-3-methyl-1H-pyrazol-5-yl)methoxy)phenyl)-2-methylpropanoic acid **(147);**
3-(4-((1-(3,4-dimethylphenyl)-3-methyl-1H-pyrazol-5-yl)methoxy)-3,5-difluorophenyl)-2-methylpropanoic acid **(148);**
3-(4-((1-(3-chloro-4-methylphenyl)-3-methyl-1H-pyrazol-5-yl)methoxy)-3,5-difluorophenyl)-2-methylpropanoic acid **(149);**
3-(4-((1-(3-chlorophenyl)-3-methyl-1H-pyrazol-5-yl)methoxy)-3,5-difluorophenyl)-2-methylpropanoic acid **(150);**
3-(4-((1-(4-ethylphenyl)-3-methyl-1H-pyrazol-5-yl)methoxy)-3-fluorophenyl)-2-methylpropanoic acid **(151);**
3-(3,5-difluoro-4-((1-phenyl-1H-pyrazol-5-yl)methoxy)phenyl)-2-methylpropanoic acid **(152);**
3-(4-((1-(3,5-dimethylphenyl)-3-methyl-1H-pyrazol-5-yl)methoxy)-3,5-difluorophenyl)-2-methylpropanoic acid **(153);**
3-(4-((1-(4-chlorophenyl)-1H-pyrazol-5-yl)methoxy)-3,5-difluorophenyl)-2-methylpropanoic acid **(154);**
3-(4-((1-(4-chloro-3-methylphenyl)-3-methyl-1H-pyrazol-5-yl)methoxy)-3,5-difluorophenyl)-2-methylpropanoic acid **(155);**
3-(4-((1-(4-ethylphenyl)-3-methyl-1H-pyrazol-5-yl)methoxy)-3-methylphenyl)-2-methylpropanoic acid **(156);**
3-(4-((1-(4-ethylphenyl)-3-(trifluoromethyl)-1H-pyrazol-5-yl)methoxy)-3,5-difluorophenyl)-2-methylpropanoic acid **(157);**
3-(4-((1-(4-bromophenyl)-3-methyl-1H-pyrazol-5-yl)methoxy)-3,5-difluorophenyl)-2-methylpropanoic acid **(158);**
(R)-3-(4-((1-(4-ethylphenyl)-3-methyl-1H-pyrazol-5-yl)methoxy)-3,5-difluorophenyl)-2-methylpropanoic acid **(159);**
(S)-3-(4-((1-(4-ethylphenyl)-3-methyl-1H-pyrazol-5-yl)methoxy)-3,5-difluorophenyl)-2-methylpropanoic acid **(160);**
3-(4-((1-(4-ethylphenyl)-1H-pyrazol-5-yl)methoxy)-3,5-difluorophenyl)-2-methylpropanoic acid **(161);**
3-(4-((1-(4-chlorophenyl)-3-ethyl-1H-pyrazol-5-yl)methoxy)-3,5-difluorophenyl)-2-methylpropanoic acid **(162);**
3-(4-((1-(4-chlorophenyl)-3-methyl-1H-pyrazol-5-yl)methoxy)-3,5-difluorophenyl)-2-ethoxypropanoic acid **(163);**
3-(4-((1-(4-Chlorophenyl)-3-methyl-1*H*-pyrazol-5-yl)methoxy)-2-methoxyphenyl)-2-methylpropanoic acid **(164);**
3-(4-((1-(4-Cyclohexylphenyl)-3-methyl-1*H*-pyrazol-5-yl)methoxy)-3,5-difluorophenyl)-2-methylpropanoic acid **(165);**
3-(3,5-Difluoro-4-((3-methyl-1-(4-propylphenyl)-1*H*-pyrazol-5-yl)methoxy)phenyl)-2-methylpropanoic acid **(166);**
2-(5-((1-(4-Chlorophenyl)-3-methyl-1*H*-pyrazol-5-yl)methoxy)-6-fluoro-2,3-dihydro-1*H*-inden-1-yl)acetic acid **(167);**
2-(4-((1-(4-Chlorophenyl)-3-methyl-1*H*-pyrazol-5-yl)methoxy)-3,5-difluorophenyl)cyclopent-1-enecarboxylic acid **(168);**
3-(4-((1-(4-Chlorophenyl)-3-methyl-1*H*-pyrazol-5-yl)methoxy)-3-fluorophenyl)-2-methylpropanoic acid **(169);**
3-(4-((1-(4-Chlorophenyl)-3-methyl-1*H*-pyrazol-5-yl)methoxy)-3-methylphenyl)-2-methylpropanoic acid **(170);**
3-(4-((1-(5-Chloropyrimidin-2-yl)-3-methyl-1*H*-pyrazol-5-yl)methoxy)-3,5-difluorophenyl)-2-methylpropanoic acid **(171);**
3-(4-((1-(4-Chlorophenyl)-3-methyl-1*H*-pyrazol-5-yl)methoxy)benzylidene)cyclobutanecarboxylic acid **(172);**
3-(4-((1-(4-Chlorophenyl)-3-methyl-1*H*-pyrazol-5-yl)methoxy)-3-methoxyphenyl)-2-methylpropanoic acid **(173);**
3-(3,5-Dichloro-4-((1-(4-chlorophenyl)-3-methyl-1*H*-pyrazol-5-yl)methoxy)phenyl)-2-methylpropanoic acid **(174);**
3-(3,5-Difluoro-4-((5-methyl-2-phenylfuran-3-yl)methoxy)phenyl)-2-methylpropanoic acid **(177);**
3-(4-((1-(4-chlorophenyl)-3-(trifluoromethyl)-1H-pyrazol-5-yl)methoxy)-3,5-difluorophenyl)-2-methylpropanoic acid **(178);**
3-(4-((1-(4-chlorophenyl)-3-methyl-1H-pyrazol-5-yl)methoxy)-2-ethylphenyl)propanoic acid **(179);**
2-(5-((1-(4-chlorophenyl)-3-(trifluoromethyl)-1H-pyrazol-5-yl)methoxy)-2,3-dihydro-1*H*-inden-1-yl)acetic acid **(180);**

2-(5-((1-(2,3-dihydro-1H-inden-5-yl)-3-methyl-1H-pyrazol-5-yl)methoxy)-2,3-dihydro-1H-inden-1-yl)acetic acid **(181);**
2-(5-((1-(4-ethylphenyl)-3-methyl-1H-pyrazol-5-yl)methoxy)-2,3-dihydro-1H-inden-1-yl)acetic acid **(182);**
3-(3,5-difluoro-4-((3-methyl-1-phenyl-1H-pyrazol-5-yl)methoxy)phenyl)-2-methylpropanoic acid **(183);**
2-(6-((1-(4-ethylphenyl)-3-methyl-1H-pyrazol-5-yl)methoxy)-1,2,3,4-tetrahydronaphthalen-1-yl)acetic acid **(184);**
3-(3,5-difluoro-4-((3-methyl-1-(pyridin-2-yl)-1H-pyrazol-5-yl)methoxy)phenyl)-2-methylpropanoic acid **(185);**
3-(4-((1-(4-chlorophenyl)-3-methyl-1H-pyrazol-5-yl)methoxy)phenyl)-3-methylbutanoic acid **(186);**
3-(4-((1-(4-ethylphenyl)-3-methyl-1H-pyrazol-5-yl)methoxy)phenyl)-3-methylbutanoic acid **(187);**
4-(4-((1-(4-chlorophenyl)-3-methyl-1H-pyrazol-5-yl)methoxy)phenyl)butanoic acid (188);
4-(4-((1-(4-chlorophenyl)-3-methyl-1H-pyrazol-5-yl)methoxy)-3,5-difluorophenyl)butanoic acid **(189);**
4-(4-((1-(4-ethylphenyl)-3-methyl-1H-pyrazol-5-yl)methoxy)phenyl)butanoic acid (190);
4-(4-((1-(4-ethylphenyl)-3-methyl-1H-pyrazol-5-yl)methoxy)-3,5-difluorophenyl)butanoic acid **(191);**
4-(4-((1-(4-chlorophenyl)-3-methyl-1H-pyrazol-5-yl)methoxy)phenyl)-2-methylbutanoic acid **(192);**
4-(4-((1-(4-ethylphenyl)-3-methyl-1H-pyrazol-5-yl)methoxy)phenyl)-2-methylbutanoic acid **(193);**
3-(4-((1-(4-chlorophenyl)-3-methyl-1H-pyrazol-5-yl)methoxy)-3-methoxyphenyl)propanoic acid **(194);**
3-(4-((1-(4-chlorophenyl)-3-methyl-1H-pyrazol-5-yl)methoxy)-3-fluoro-2-methylphenyl)propanoic acid **(195);**
3-(4-((1-(4-chlorophenyl)-3-methyl-1H-pyrazol-5-yl)methoxy)-2-ethyl-3-fluorophenyl)propanoic acid **(196);**
3-(4-((1-(4-chlorophenyl)-3-methyl-1H-pyrazol-5-yl)methoxy)-2,3-dimethylphenyl)propanoic acid **(197);**
3-(4-((1-(4-chlorophenyl)-1H-pyrazol-5-yl)methoxy)-3-fluoro-2-methylphenyl)propanoic acid **(198);**
3-(4-((1-(4-chlorophenyl)-1H-pyrazol-5-yl)methoxy)-2,3-dimethylphenyl)propanoic acid **(199);**
3-(4-((3-(4-chlorophenyl)-5-methylisoxazol-4-yl)methoxy)-2,3-dimethylphenyl) propanoic acid **(200);**
3-(4-((1-(4-ethylphenyl)-3-methyl-1H-pyrazol-5-yl)methoxy)-2,3-dimethylphenyl)propanoic acid **(201);**
3-(4-((1-(4-ethylphenyl)-1H-pyrazol-5-yl)methoxy)-2,3-dimethylphenyl)propanoicacid **(202);**
3-(4-((1-(4-chlorophenyl)-3-methyl-1H-pyrazol-5-yl)methoxy)-5,6,7,8-tetrahydro naphthalen-1-yl)propanoic acid **(203);** and
3-(7-((1-(4-chlorophenyl)-3-methyl-1H-pyrazol-5-yl)methoxy)-2,3-dihydro-1H-inden-4-yl)propanoic acid **(207);**
or a pharmaceutically acceptable salt thereof.

**12.** A composition comprising a compound of any one of claims 1 to 11 and a pharmaceutically acceptable carrier.

**13.** A compound of any one of claims 1 to 11 for use in treating in a mammal a disease or condition selected from the group consisting of Type I diabetes, Type II diabetes and metabolic syndrome, stimulating insulin production in a mammal, lowering blood glucose in a mammal or modulating GPR120 activity in a cell.

**14.** The compound of claim 13, wherein said disease is Type II diabetes.

**15.** The compound of claim 13 or claim 14 wherein the mammal is a human.

Europäisches Patentamt
European Patent Office
Office européen des brevets

# EUROPEAN SEARCH REPORT

Application Number
EP 13 19 0133

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | EP 1 630 152 A1 (TAKEDA PHARMACEUTICAL [JP]) 1 March 2006 (2006-03-01)<br>* Examples 87, 138-140 * | 1-15 | INV.<br>C07D231/12<br>C07D333/16<br>C07D233/64<br>C07D409/04<br>C07D249/06<br>C07D261/08<br>C07D263/32<br>C07D277/24<br>C07D285/06<br>C07D307/42<br>A61K31/4155<br>A61K31/42<br>A61K31/4164 |
| Y | WO 2008/103501 A1 (IRM LLC; EPPLE ROBERT [US]; AZIMIOARA MIHAI [US]; COW CHRISTOPHER [US])<br>28 August 2008 (2008-08-28)<br>* Compounds on pages 30-36 * | 1-15 | |
| A | WO 03/072102 A1 (LILLY CO ELI [US]; CONNER SCOTT EUGENE [US]; MANTLO NATHAN BRYAN [US];) 4 September 2003 (2003-09-04)<br>* the whole document * | 1-15 | |
| A | WO 2004/031162 A1 (HOFFMANN LA ROCHE [CH]) 15 April 2004 (2004-04-15)<br>* the whole document * | 1-15 | |
| A | WO 2004/074284 A1 (PFIZER [US]; SU WEI-GUO [US]; ZEHNDER LUKE RAYMOND [US]; SKALITZKY DON) 2 September 2004 (2004-09-02)<br>* the whole document * | 1-15 | TECHNICAL FIELDS SEARCHED (IPC)<br>C07D<br>A61K |
| A | WO 03/015771 A1 (LION BIOSCIENCE AG [DE]; BAUER ULRIKE [DE]; CHERUVALLATH ZACH [US]; DE) 27 February 2003 (2003-02-27)<br>* claims 4, 6, 7, 9 * | 1-15 | |

The present search report has been drawn up for all claims

1

| Place of search | Date of completion of the search | Examiner |
|---|---|---|
| The Hague | 6 December 2013 | Hacking, Michiel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 13 19 0133

This annex lists the patent family members relating to the patent documents cited in the above-mentioned European search report.
The members are as contained in the European Patent Office EDP file on
The European Patent Office is in no way liable for these particulars which are merely given for the purpose of information.

06-12-2013

| Patent document cited in search report | Publication date | Patent family member(s) | Publication date |
|---|---|---|---|
| EP 1630152 A1 | 01-03-2006 | CA 2527691 A1 | 09-12-2004 |
| | | EP 1630152 A1 | 01-03-2006 |
| | | US 2006258722 A1 | 16-11-2006 |
| | | WO 2004106276 A1 | 09-12-2004 |
| WO 2008103501 A1 | 28-08-2008 | AU 2008218951 A1 | 28-08-2008 |
| | | CA 2677706 A1 | 28-08-2008 |
| | | CN 101646660 A | 10-02-2010 |
| | | EA 200901099 A1 | 30-04-2010 |
| | | EP 2125758 A1 | 02-12-2009 |
| | | JP 5175866 B2 | 03-04-2013 |
| | | JP 2010519306 A | 03-06-2010 |
| | | KR 20090113901 A | 02-11-2009 |
| | | US 2010035944 A1 | 11-02-2010 |
| | | WO 2008103501 A1 | 28-08-2008 |
| WO 03072102 A1 | 04-09-2003 | AT 415160 T | 15-12-2008 |
| | | AU 2003214932 A1 | 09-09-2003 |
| | | EP 1480642 A1 | 01-12-2004 |
| | | ES 2316736 T3 | 16-04-2009 |
| | | JP 2005528346 A | 22-09-2005 |
| | | US 2006084663 A1 | 20-04-2006 |
| | | WO 03072102 A1 | 04-09-2003 |
| WO 2004031162 A1 | 15-04-2004 | AR 041481 A1 | 18-05-2005 |
| | | AT 402153 T | 15-08-2008 |
| | | AU 2003273949 A1 | 23-04-2004 |
| | | BR 0315114 A | 16-08-2005 |
| | | CA 2499721 A1 | 15-04-2004 |
| | | CN 1703406 A | 30-11-2005 |
| | | CO 5540385 A2 | 29-07-2005 |
| | | DK 1551814 T3 | 27-10-2008 |
| | | EP 1551814 A1 | 13-07-2005 |
| | | ES 2311110 T3 | 01-02-2009 |
| | | GT 200300218 A | 24-05-2004 |
| | | HK 1083339 A1 | 14-11-2008 |
| | | HR P20050308 A2 | 31-07-2006 |
| | | IL 167250 A | 30-12-2010 |
| | | JP 4414886 B2 | 10-02-2010 |
| | | JP 2006504709 A | 09-02-2006 |
| | | KR 20050055750 A | 13-06-2005 |
| | | MX PA05003392 A | 22-06-2005 |
| | | MY 143576 A | 31-05-2011 |
| | | NO 330171 B1 | 28-02-2011 |
| | | NZ 538465 A | 31-08-2007 |
| | | PA 8584501 A1 | 21-05-2004 |



For more details about this annex : see Official Journal of the European Patent Office, No. 12/82

## ANNEX TO THE EUROPEAN SEARCH REPORT ON EUROPEAN PATENT APPLICATION NO. EP 13 19 0133

This annex lists the patent family members relating to the patent documents cited in the above-mentioned European search report.
The members are as contained in the European Patent Office EDP file on
The European Patent Office is in no way liable for these particulars which are merely given for the purpose of information.

06-12-2013

| Patent document cited in search report | Publication date | Patent family member(s) | Publication date |
|---|---|---|---|
| | | PE 05912004 A1 | 30-08-2004 |
| | | PL 208743 B1 | 30-06-2011 |
| | | PT 1551814 E | 22-09-2008 |
| | | RU 2303593 C2 | 27-07-2007 |
| | | SI 1551814 T1 | 31-10-2008 |
| | | TW I343915 B | 21-06-2011 |
| | | US 2004116487 A1 | 17-06-2004 |
| | | US 2005267180 A1 | 01-12-2005 |
| | | UY 28006 A1 | 30-04-2004 |
| | | WO 2004031162 A1 | 15-04-2004 |
| | | ZA 200502664 A | 22-02-2006 |
| WO 2004074284 A1 | 02-09-2004 | AR 044498 A1 | 14-09-2005 |
| | | BR PI0407735 A | 14-02-2006 |
| | | CA 2516475 A1 | 02-09-2004 |
| | | EP 1597257 A1 | 23-11-2005 |
| | | GT 200400019 A | 21-09-2004 |
| | | JP 2006518366 A | 10-08-2006 |
| | | MX PA05008922 A | 05-10-2005 |
| | | NL 1025542 A1 | 24-08-2004 |
| | | NL 1025542 C2 | 11-10-2005 |
| | | PA 8594401 A1 | 16-09-2004 |
| | | PE 02232005 A1 | 22-03-2005 |
| | | US 2004209929 A1 | 21-10-2004 |
| | | UY 28200 A1 | 30-09-2004 |
| | | WO 2004074284 A1 | 02-09-2004 |
| WO 03015771 A1 | 27-02-2003 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5013556 A
- US 3845770 A
- US 3916899 A
- US 3536809 A
- US 3598123 A
- US 4008719 A
- US 20040092538 A
- US 639527 B1
- US 5032577 A
- WO 2004011445 A1


### Non-patent literature cited in the description


- **KAHN SE.** *Am J Med,* 2000, vol. 108 (6a), 2S-8S
- **KAHN BB.** *Cell,* 1998, vol. 92, 593-596
- **CAVAGHAN MK et al.** *J Clin Invest,* 2000, vol. 106, 329-333
- **SALTIEL AR.** *Cell,* 2001, vol. 104, 517-529
- **PRENTKI M ; NOLAN CJ.** *J Clin Invest,* 2006, vol. 116, 1802-1812
- **KAHN SE.** *J Clin Endicrinol Metab,* 2001, vol. 86, 4047-4058
- *JAMA,* 1999, vol. 281, 2005-2012
- **LEVY J et al.** *Diabetes Med,* 1998, vol. 15, 290-296
- **ZHOU YP et al.** *J Biol Chem,* 2003, vol. 278, 51316-23
- **BELL GI ; POLONSKY KS.** *Nature,* 2001, vol. 414, 788-791
- **SAXENA R et al.** *Science,* 26 April 2007
- **VALGERDUR STEINTHORSDOTTIR et al.** *Nature Genetics,* 26 April 2007
- **THORENS B.** *Mol Membr Biol,* October 2001, vol. 18 (4), 265-73
- **MATSCHINSKY FM.** *Curr Diab Rep,* June 2005, vol. 5 (3), 171-6
- **GLOYN AL et al.** *J Biol Chem,* 25 January 2005, vol. 280 (14), 14105-13
- **GUERTIN KR ; GRIMSBY J.** *Curr Med Chem,* 2006, vol. 13 (15), 1839-43
- **MATSCHINSKY FM et al.** *Diabetes,* January 2006, vol. 55 (1), 1-12
- **GILON P ; HENQUIN JC.** *Endocr Rev,* October 2001, vol. 22 (5), 565-604
- **FILIPSSON K et al.** *Diabetes,* September 2001, vol. 50 (9), 1959-69
- **YAMADA H et al.** *Regul Pept,* 15 December 2004, vol. 123 (1-3), 147-53
- **QADER SS et al.** *Am J Physiol Endocrinol Metab,* May 2007, vol. 292 (5), E1447-55
- **DRUCKER DJ.** *J Clin Invest,* January 2007, vol. 117 (1), 24-32
- **HANSOTIA T et al.** *J Clin Invest,* 21 December 2006, vol. 117 (1), 143-52
- **GONZALEZ C et al.** *Expert Opin Investig Drugs,* August 2006, vol. 15 (8), 887-95
- **NAUCK MA et al.** *J Clin Invest,* 1993, vol. 91, 301-307
- **ELAHI D et al.** *Regul Pept,* 1994, vol. 51, 63-74
- **MENEILLY GS et al.** *Diabetes Care,* January 1993, vol. 16 (1), 110-4
- **VILSBOLL T et al.** *Diabetes,* vol. 50, 609-613
- **BRUBAKER P.** *Ann N YAcad Sci,* July 2006, vol. 1070, 10-26
- **REIMANN F et al.** *Diabetes,* December 2006, vol. 55 (2), S78-S85
- **BRUBAKER P.** *Ann N Y Acad Sci,* July 2006, vol. 1070, 10-26
- **FARILLA L et al.** *ndocrinology,* November 2002, vol. 143 (11), 4397-408
- **FARILLA L et al.** *Endocrinology,* December 2003, vol. 144 (12), 5149-58
- **LI Y et al.** *J Biol Chem,* 03 January 2003, vol. 278 (1), 471-8
- **FRIEDRICHSEN BN et al.** *J Endocrinol,* March 2006, vol. 188 (3), 481-92
- **KIM MJ et al.** *J Endocrinol,* March 2006, vol. 188 (3), 623-33
- **HUSSAIN MA et al.** *Mol Cell Biol,* October 2006, vol. 26 (20), 7747-59
- **HIRASAWA et al.** *Nature Medicine,* January 2005, vol. 11, 90-94
- **IAKOUBOV et al.** *Endocrinology,* March 2007, vol. 148 (3), 1089-1098
- **KATSUMA et al.** *J. Biol. Chem.,* May 2005, vol. 280, 19507-19515
- **MATSUMURA et al.** *Biomed. Res.,* February 2007, vol. 28 (1), 49-55
- **RAYASAM et al.** *Expert Opin. Ther. Targets,* May 2007, vol. 11 (5), 661-671

- **TANAKA et al.** *Naunyn Schmiedeberg Arch Pharmacol,* June 2008, vol. 377 (4-6), 515-22
- **GOTOH et al.** *Biochem. Biophys. Res. Commun.,* March 2007, vol. 354 (2), 591-597
- **RAYASAM et al.** *Expert Opin Ther Targets,* May 2007, vol. 11 (5), 661-671
- **J. MARCH.** Advanced Organic Chemistry. John Wiley and Sons, 1992
- **T HIGUCHI ; V STELLA.** Pro-drugs as Novel Delivery Systems. A.C.S. Symposium Series, vol. 14
- Bioreversible Carriers in Drug Design. American Pharmaceutical Association and Pergamon Press, 1987
- Handbook of Pharmaceutical Salts Properties, Selection, and Use. 2002
- **REAVEN GM.** *J. Basic & Clin. Phys. & Pharm.,* 1998, vol. 9, 387-406
- **FLIE J.** *Ann. Rev. Med.,* 1983, vol. 34, 145-60
- The Expert Committee on the Diagnosis and Classification of Diabetes Mellitus. *Diabetes Care,* 1999, vol. 2 (1), 5-19
- Cardiovascular diseases. **KAPLAN RM et al.** Health and Human Behavior. McGraw-Hill, 1993, 206-242
- **BARRETT-CONNER E.** *Epidemol. Rev.,* 1989, vol. 11, 172-181
- **KNOWLER et al.** *Am. J. Clin. Nutr.,* 1991, vol. 53, 1543-1551
- **TURNER N et al.** *Prog. Drug Res.,* 1998, vol. 51, 33-94
- **HAFFNER S.** *Diabetes Care,* 1998, vol. 21, 160-178
- Diabetes Reviews. 1997, vol. 5
- **MAHLER R.** *J. Clin. Endocrinol. Metab.,* 1999, vol. 84, 1165-71
- *Diabetes Care,* 1998, vol. 21, 87-92
- Current Therapy in Endocrinology and Metabolism. Mosby - Year Book, Inc, 1997
- **CHIASSON J et al.** *Ann. Intern. Med.,* 1994, vol. 121, 928-935
- **CONIFF R et al.** *Clin. Ther.,* 1997, vol. 19, 16-26
- **CONIFFR et al.** *Am. J. Med.,* 1995, vol. 98, 443-451
- **IWAMOTO Y et al.** *Diabet. Med.,* 1996, vol. 13, 365-370
- **KWITEROVICH P.** *Am. J. Cardiol,* 1998, vol. 82 (12A), 3U-17U
- **GREEN BD ; FLATT PR ; BAILEY CJ.** Dipeptidyl peptidase IB (DPP4) inhibitors: a newly emerging drug class for the treatment of Type II diabetes. *Diabetes Vasc. Dis. Res.,* 2006, vol. 3, 159-165
- Remington's Pharmaceutical Sciences. Mack Publishing Company, 1985
- **LANGER.** *Science,* 1990, vol. 249, 1527-1533
- **FINGL et al.** The Pharmacological Basis of Therapeutics. 1975
- *British Journal of Pharmacology,* 2006, vol. 148, 619-628